# EUROPEAN PATENT SPECIFICATION

(11) **EP 3 629 720 B1**
(45) Date of publication and mention of the grant of the patent: **19.01.2022**
(21) Application number: 19737613.0
(22) Date of filing: 14.06.2019
(51) Int. Cl.: A01K 67/027, C12N 15/85, C12N 5/10, C12N 5/12, C07K 14/705

(54) **NON-HUMAN ANIMALS CAPABLE OF DH-DH REARRANGEMENT IN THE IMMUNOGLOBULIN HEAVY CHAIN CODING SEQUENCES**
NICHT-MENSCHLICHE TIERE, DIE ZU DH-DH-REKOMBINATION IN DEN IMMUNOGLOBULIN-SCHWERKETTEN-KODIERUNGSSEQUENZEN FÄHIG SIND
ANIMAUX NON HUMAINS CAPABLES DE RÉARRANGER DH-DH DANS LES SÉQUENCES DE CODAGE DE LA CHAÎNE LOURDE DE L'IMMUNOGLOBULINE

(30) Priority: 14.06.2018 US 201862685203 P; 23.07.2018 US 201862702206 P; 01.03.2019 US 201962812580 P
(43) Date of publication of application: 08.04.2020
(60) Divisional application: 21213824.2
(73) Proprietor: Regeneron Pharmaceuticals, Inc., Tarrytown, NY 10591 (US)
(72) Inventor: MURPHY, Andrew J., Tarrytown, New York 10591 (US); MACDONALD, Lynn, Tarrytown, New York 10591 (US); GUO, Chunguang, Tarrytown, New York 10591 (US); MCWHIRTER, John, Tarrytown, New York 10591 (US); VORONINA, Vera, Tarrytown, New York 10591 (US)
(74) Representative: J A Kemp LLP
(86) International application number: PCT/US2019/037285
(87) International publication number: WO 2019/241692

(56) References cited:
- JUNG D. ET AL.: "Mechanism and control of V(D)J recombination at the immunoglobulin heavy chain locus", ANNU. REV. IMMUNOL., vol. 24, 16 January 2006 (2006-01-16), pages 541-570, XP002794627,
- KOKUBU F ET AL: "Diverse organization of immunoglobulin VH gene loci in a primitive vertebrate", THE EMBO JOURNAL, vol. 7, no. 11, November 1988 (1988-11), pages 3413-3422, XP002794628,
- TUAILLON N & CAPRA J.D.: "VHD rearrangements in human immunoglobulin heavy chain minilocus transgenic mice", EUR. J. IMMUNOL., vol. 30, 2000, pages 2998-3005, XP002794629,
- MEEK K.D., HASEMAN C.A. & CAPRA J.D.: "Novel rearrangements at the immunoglobulin D locus.", J. EXP. MED., vol. 170, July 1989 (1989-07), pages 39-57, XP002794630,
- TAYLOR L.D. ET AL: "A transgenic mouse that expresses a diversity of human sequence heavy and light chain immunoglobulins", NUCLEIC ACIDS RESEARCH, vol. 20, no. 23, 11 December 1992 (1992-12-11), pages 6287-6295, XP002041128,

### SEQUENCE LISTING

<110> Regeneron Pharmaceuticals, Inc. MURPHY, Andrew J. MACDONALD, Lynn GUO**,** Chunguang MCWHIRTER, John VORONINA, Vera
<120> NON-HUMAN ANIMALS CAPABLE OF DH-DH REARRANGEMENT AND USES THEREOF
<130> 009108.347/10347
<150> 62685203
   <151> 2018-06-14
<150> 62702206
   <151> 2018-07-23
<150> 62812580
   <151> 2019-03-01
<160> 151
<170> PatentIn version 3.5
<210> 1
   <211> 22
   <212> DNA
   <213> Artificial Sequence
<220>
   <223> hIgHJ2
<400> 1
   gaggctgtgc tactggtact tc 22
<210> 2
   <211> 21
   <212> DNA
   <213> Artificial Sequence
<220>
   <223> hIgHJ2
<400> 2
   ccgtggcacc ctggtcactg t 21
<210> 3
   <211> 19
   <212> DNA
   <213> Artificial Sequence
<220>
   <223> hIgHJ2
<400> 3
   tggtgcctgg acagagaag 19
<210> 4
   <211> 21
   <212> DNA
   <213> Artificial Sequence
<220>
   <223> VI741-42h1
<400> 4
   tttttgtgca ccccttaatg g 21
<210> 5
   <211> 22
   <212> DNA
   <213> Artificial Sequence
<220>
   <223> VI741-42h1
<400> 5
   atgtggtatt acgatttttg ga 22
<210> 6
   <211> 27
   <212> DNA
   <213> Artificial Sequence
<220>
   <223> VI741-42h1
<400> 6
   ctctgtgaca ctgtgggtat aataacc 27
<210> 7
   <211> 24
   <212> DNA
   <213> Artificial Sequence
<220>
   <223> VI742h2
<400> 7
   tgtcacagag tccatcaaaa acct 24
<210> 8
   <211> 14
   <212> DNA
   <213> Artificial Sequence
<220>
   <223> VI742h2
<400> 8
   caccaccccc tctc 14
<210> 9
   <211> 19
   <212> DNA
   <213> Artificial Sequence
<220>
   <223> VI742h2
<400> 9
   cctgagaccc tggcaagct 19
<210> 10
   <211> 19
   <212> DNA
   <213> Artificial Sequence
<220>
   <223> Neo
<400> 10
   ggtggagagg ctattcggc 19
<210> 11
   <211> 23
   <212> DNA
   <213> Artificial Sequence
<220>
   <223> Neo
<400> 11
   tgggcacaac agacaatcgg ctg 23
<210> 12
   <211> 17
   <212> DNA
   <213> Artificial Sequence
<220>
   <223> Neo
<400> 12
   gaacacggcg gcatcag 17
<210> 13
   <211> 19
   <212> DNA
   <213> Artificial Sequence
<220>
   <223> Jxn DH2-2
<400> 13
   atggcgcttg cagcgattc 19
<210> 14
   <211> 28
   <212> DNA
   <213> Artificial Sequence
<220>
   <223> Jxn DH2-2
<400> 14
   ccaatactaa cgaggaaatg caaaccca 28
<210> 15
   <211> 21
   <212> DNA
   <213> Artificial Sequence
<220>
   <223> Jxn DH2-2
<400> 15
   accctcactg ttgtccttct g 21
<210> 16
   <211> 23
   <212> DNA
   <213> Artificial Sequence
<220>
   <223> Jxn DH2-8
<400> 16
   gcaaacgtcc atctgaagga gaa 23
<210> 17
   <211> 27
   <212> DNA
   <213> Artificial Sequence
<220>
   <223> Jxn DH2-8
<400> 17
   caaataaacg atggcaggct acacccg 27
<210> 18
   <211> 22
   <212> DNA
   <213> Artificial Sequence
<220>
   <223> Jxn DH2-8
<400> 18
   gagttgccga accgacttat tg 22
<210> 19
   <211> 23
   <212> DNA
   <213> Artificial Sequence
<220>
   <223> Jxn DH2-15
<400> 19
   gcaatggtag gttcatgtcc atc 23
<210> 20
   <211> 20
   <212> DNA
   <213> Artificial Sequence
<220>
   <223> Jxn DH2-15
<400> 20
   accgccgctg gtactccaat 20
<210> 21
   <211> 19
   <212> DNA
   <213> Artificial Sequence
<220>
   <223> Jxn DH2-15
<400> 21
   ccctggctgt ctgggtttg 19
<210> 22
   <211> 21
   <212> DNA
   <213> Artificial Sequence
<220>
   <223> hIgHJ6
<400> 22
   cagcagaggg ttccatgaga a 21
<210> 23
   <211> 22
   <212> DNA
   <213> Artificial Sequence
<220>
   <223> hIgHJ6
<400> 23
   caggacaggg ccacggacag tc 22
<210> 24
   <211> 20
   <212> DNA
   <213> Artificial Sequence
<220>
   <223> hIgHJ6
<400> 24
   gccacccaga gaccttctgt 20
<210> 25
   <211> 20
   <212> DNA
   <213> Artificial Sequence
<220>
   <223> hIgH31
<400> 25
   atcacactca tcccatcccc 20
<210> 26
   <211> 29
   <212> DNA
   <213> Artificial Sequence
<220>
   <223> hIgH31
<400> 26
   cccttcccta agtaccacag agtgggctc 29
<210> 27
   <211> 20
   <212> DNA
   <213> Artificial Sequence
<220>
   <223> hIgH31
<400> 27
   cacagggaag caggaactgc 20
<210> 28
   <211> 17
   <212> DNA
   <213> Artificial Sequence
<220>
   <223> mIgHp2
<400> 28
   gccatgcaag gccaagc 17
<210> 29
   <211> 24
   <212> DNA
   <213> Artificial Sequence
<220>
   <223> mIgHp2
<400> 29
   ccaggaaaat gctgccagag cctg 24
<210> 30
   <211> 24
   <212> DNA
   <213> Artificial Sequence
<220>
   <223> mIgHp2
<400> 30
   agttcttgag ccttagggtg ctag 24
<210> 31
   <211> 20
   <212> DNA
   <213> Artificial Sequence
<220>
   <223> mADAM6-1
<400> 31
   ctgagcatgg tgtcccatct 20
<210> 32
   <211> 24
   <212> DNA
   <213> Artificial Sequence
<220>
   <223> mADAM6-1
<400> 32
   tgcagataag tgtcatctgg gcaa 24
<210> 33
   <211> 24
   <212> DNA
   <213> Artificial Sequence
<220>
   <223> mADAM6-1
<400> 33
   ccagagtact gtagtggctc taag 24
<210> 34
   <211> 20
   <212> DNA
   <213> Artificial Sequence
<220>
   <223> hIgk5
<400> 34
   ccccgtcctc ctcctttttc 20
<210> 35
   <211> 32
   <212> DNA
   <213> Artificial Sequence
<220>
   <223> hIgk5
<400> 35
   tcatgtccat taacccattt accttttgcc ca 32
<210> 36
   <211> 19
   <212> DNA
   <213> Artificial Sequence
<220>
   <223> hIgk5
<400> 36
   tgcaagtgct gccagcaag 19
<210> 37
   <211> 23
   <212> DNA
   <213> Artificial Sequence
<220>
   <223> hIgK21
<400> 37
   catttggcta catatcaaag ccg 23
<210> 38
   <211> 27
   <212> DNA
   <213> Artificial Sequence
<220>
   <223> hIgK21
<400> 38
   cctgagccag ggaacagccc actgata 27
<210> 39
   <211> 20
   <212> DNA
   <213> Artificial Sequence
<220>
   <223> hIgK21
<400> 39
   acatggctga ggcagacacc 20
<210> 40
   <211> 20
   <212> DNA
   <213> Artificial Sequence
<220>
   <223> mIgKd2
<400> 40
   gcaaacaaaa accactggcc 20
<210> 41
   <211> 37
   <212> DNA
   <213> Artificial Sequence
<220>
   <223> mIgKd2
<400> 41
   ctgttcctct aaaactggac tccacagtaa atggaaa 37
<210> 42
   <211> 19
   <212> DNA
   <213> Artificial Sequence
<220>
   <223> mIgKd2
<400> 42
   ggccacattc catgggttc 19
<210> 43
   <211> 17
   <212> DNA
   <213> Artificial Sequence
<220>
   <223> hyg
<400> 43
   tgcggccgat cttagcc 17
<210> 44
   <211> 21
   <212> DNA
   <213> Artificial Sequence
<220>
   <223> hyg
<400> 44
   acgagcgggt tcggcccatt c 21
<210> 45
   <211> 18
   <212> DNA
   <213> Artificial Sequence
<220>
   <223> hyg
<400> 45
   ttgaccgatt ccttgcgg 18
<210> 46
   <211> 20
   <212> DNA
   <213> Artificial Sequence
<220>
   <223> hIgHD-J.1
<400> 46
   tggcagctgt tcaaccatgt 20
<210> 47
   <211> 22
   <212> DNA
   <213> Artificial Sequence
<220>
   <223> hIgHD-J.1
<400> 47
   atccactgtc ccagacagca cc 22
<210> 48
   <211> 20
   <212> DNA
   <213> Artificial Sequence
<220>
   <223> hIgHD-J.1
<400> 48
   ggcggtttgt ggagtttcct 20
<210> 49
   <211> 20
   <212> DNA
   <213> Artificial Sequence
<220>
   <223> hIgHD-J.2
<400> 49
   tgttgcctcc ctgcttctag 20
<210> 50
   <211> 23
   <212> DNA
   <213> Artificial Sequence
<220>
   <223> hIgHD-J.2
<400> 50
   cccagaccct cccttgttcc tga 23
<210> 51
   <211> 18
   <212> DNA
   <213> Artificial Sequence
<220>
   <223> hIgHD-J.2
<400> 51
   ggcccacgca ttgttcag 18
<210> 52
   <211> 3263
   <212> DNA
   <213> Artificial Sequence
<220>
   <223> >p46458 (23-D3-3-12_delJ1-3 donor]
<400> 52
   tacgtaccct ggccaccgcc acccgggcct ctctgctgct gggaaaacaa gtcagaaagc      60
   aagtggatga gaggtggcgt gacagaccca gcttcagatc tgctctaccg gtataacttc     120
   gtataatgta tgctatacga agttatatgc atggcctccg cgccgggttt tggcgcctcc     180
   cgcgggcgcc cccctcctca cggcgagcgc tgccacgtca gacgaagggc gcagcgagcg     240
   tcctgatcct tccgcccgga cgctcaggac agcggcccgc tgctcataag actcggcctt     300
   agaaccccag tatcagcaga aggacatttt aggacgggac ttgggtgact ctagggcact     360
   ggttttcttt ccagagagcg gaacaggcga ggaaaagtag tcccttctcg gcgattctgc     420
   ggagggatct ccgtggggcg gtgaacgccg atgattatat aaggacgcgc cgggtgtggc     480
   acagctagtt ccgtcgcagc cgggatttgg gtcgcggttc ttgtttgtgg atcgctgtga     540
   tcgtcacttg gtgagtagcg ggctgctggg ctggccgggg ctttcgtggc cgccgggccg     600
   ctcggtggga cggaagcgtg tggagagacc gccaagggct gtagtctggg tccgcgagca     660
   aggttgccct gaactggggg ttggggggag cgcagcaaaa tggcggctgt tcccgagtct     720
   tgaatggaag acgcttgtga ggcgggctgt gaggtcgttg aaacaaggtg gggggcatgg     780
   tgggcggcaa gaacccaagg tcttgaggcc ttcgctaatg cgggaaagct cttattcggg     840
   tgagatgggc tggggcacca tctggggacc ctgacgtgaa gtttgtcact gactggagaa     900
   ctcggtttgt cgtctgttgc gggggcggca gttatggcgg tgccgttggg cagtgcaccc     960
   gtacctttgg gagcgcgcgc cctcgtcgtg tcgtgacgtc acccgttctg ttggcttata    1020
   atgcagggtg gggccacctg ccggtaggtg tgcggtaggc ttttctccgt cgcaggacgc    1080
   agggttcggg cctagggtag gctctcctga atcgacaggc gccggacctc tggtgagggg    1140
   agggataagt gaggcgtcag tttctttggt cggttttatg tacctatctt cttaagtagc    1200
   tgaagctccg gttttgaact atgcgctcgg ggttggcgag tgtgttttgt gaagtttttt    1260
   aggcaccttt tgaaatgtaa tcatttgggt caatatgtaa ttttcagtgt tagactagta    1320
   aattgtccgc taaattctgg ccgtttttgg cttttttgtt agacgtcgag ctagcctaga    1380
   ttgggaaccc gggtctctcg aattgttgac aattaatcat cggcatagta tatcggcata 1440
   gtataatacg acaaggtgag gaactaaacc atccaccatg attgaacaag atggattgca 1500
   cgcaggttct ccggccgctt gggtggagag gctattcggc tatgactggg cacaacagac 1560
   aatcggctgc tctgatgccg ccgtgttccg gctgtcagcg caggggcgcc cggttctttt 1620
   tgtcaagacc gacctgtccg gtgccctgaa tgaactgcag gacgaggcag cgcggctatc 1680
   gtggctggcc acgacgggcg ttccttgcgc agctgtgctc gacgttgtca ctgaagcggg 1740
   aagggactgg ctgctattgg gcgaagtgcc ggggcaggat ctcctgtcat ctcaccttgc 1800
   tcctgccgag aaagtatcca tcatggctga tgcaatgcgg cggctgcata cgcttgatcc 1860
   ggctacctgc ccattcgacc accaagcgaa acatcgcatc gagcgagcac gtactcggat 1920
   ggaagccggt cttgtcgatc aggatgatct ggacgaagag catcaggggc tcgcgccagc 1980
   cgaactgttc gccaggctca aggcgcgcat gcccgacggc gaggatctcg tcgtgaccca 2040
   tggcgatgcc tgcttgccga atatcatggt ggaaaatggc cgcttttctg gattcatcga 2100
   ctgtggccgg ctgggtgtgg cggaccgcta tcaggacata gcgttggcta cccgtgatat 2160
   tgctgaagag cttggcggcg aatgggctga ccgcttcctc gtgctttacg gtatcgccgc 2220
   tcccgattcg cagcgcatcg ccttctatcg ccttcttgac gagttcttct gaagatccgc 2280
   tgtaagtctg cagaaattga tgatctatta aacaataaag atgtccacta aaatggaagt 2340
   ttttcctgtc atactttgtt aagaagggtg agaacagagt acctacattt tgaatggaag 2400
   gattggagct acgggggtgg gggtggggtg ggattagata aatgcctgct ctttactgaa 2460
   ggctctttac tattgcttta tgataatgtt tcatagttgg atatcataat ttaaacaagc 2520
   aaaaccaaat taagggccag ctcattcctc ccactcatga tctatagatc tatagatctc 2580
   tcgtgggatc attgtttttc tcttgattcc cactttgtgg ttctaagtac tgtggtttcc 2640
   aaatgtgtca gtttcatagc ctgaagaacg agatcagcag cctctgttcc acatacactt 2700
   cattctcagt attgttttgc caagttctaa ttccatcaga cctcgacctg cagccggcgc 2760
   gccataactt cgtataatgt atgctatacg aagttatgtc gacctcgaga atttacaaaa 2820
   gaaaaggaaa aacacacttg gcagccttca gcactctaat gattcttaac agcagcaaat 2880
   tattggcaca agactccaga gtgactggca gggttgaggg ctggggtctc ccacgtgttt 2940
   tggggctaac agcggaaggg agagcactgg caaaggtgct gggggcccct ggacccgacc 3000
   cgccctggag accgcagcca catcagcccc cagccccaca ggccccctac cagccgcagg 3060
   gtttttgtgc accccttaat ggggcctccc acaatgtggt attacgattt ttggagtggt 3120
   tattataccc acagtgtcac agagtccatc aaaaaccaag atggctttcc ttctgcctcc 3180
   tttctctggg cccagcgtcc tctgtcctgg agctgggaga taatgtccgg gggctccttg 3240
   gtctgcgctg ggccatgtac gta 3263
<210> 53
   <211> 23
   <212> DNA
   <213> Artificial Sequence
<220>
   <223> DNA recognition site
<400> 53
   tcagaaagca agtggatgag agg 23
<210> 54
   <211> 42
   <212> DNA
   <213> Artificial Sequence
<220>
   <223> 5' D7.27 gRNA
<400> 54
   ucagaaagca aguggaugag guuuuagagc uaugcuguuu ug 42
<210> 55
   <211> 23
   <212> DNA
   <213> Artificial Sequence
<220>
   <223> DNA recognition site
<400> 55
   gctccaggac agaggacgct ggg 23
<210> 56
   <211> 42
   <212> DNA
   <213> Artificial Sequence
<220>
   <223> 3' JH1-3 del gRNA
<400> 56
   gcuccaggac agaggacgcu guuuuagagc uaugcuguuu ug 42
<210> 57
   <211> 19
   <212> DNA
   <213> Artificial Sequence
<220>
   <223> 5' up detect D7-27
<400> 57
   gtgaacaggt ggaaccaac 19
<210> 58
   <211> 21
   <212> DNA
   <213> Artificial Sequence
<220>
   <223> 3' ub pro-200
<400> 58
   ccagtgccct agagtcaccc a 21
<210> 59
   <211> 21
   <212> DNA
   <213> Artificial Sequence
<220>
   <223> 5' neo detect
<400> 59
   ctcccactca tgatctatag a 21
<210> 60
   <211> 20
   <212> DNA
   <213> Artificial Sequence
<220>
   <223> 3' down detect JH1-3 del
<400> 60
   gtcccagttc ccaaagaaag 20
<210> 61
   <211> 3263
   <212> DNA
   <213> Artificial Sequence
<220>
   <223> >p46459 (23-D3-3-12_delJ1-5 donor)
<400> 61
   tacgtaccct ggccaccgcc acccgggcct ctctgctgct gggaaaacaa gtcagaaagc 60
   aagtggatga gaggtggcgt gacagaccca gcttcagatc tgctctaccg gtataacttc 120
   gtataatgta tgctatacga agttatatgc atggcctccg cgccgggttt tggcgcctcc 180
   cgcgggcgcc cccctcctca cggcgagcgc tgccacgtca gacgaagggc gcagcgagcg 240
   tcctgatcct tccgcccgga cgctcaggac agcggcccgc tgctcataag actcggcctt 300
   agaaccccag tatcagcaga aggacatttt aggacgggac ttgggtgact ctagggcact 360
   ggttttcttt ccagagagcg gaacaggcga ggaaaagtag tcccttctcg gcgattctgc 420
   ggagggatct ccgtggggcg gtgaacgccg atgattatat aaggacgcgc cgggtgtggc 480
   acagctagtt ccgtcgcagc cgggatttgg gtcgcggttc ttgtttgtgg atcgctgtga 540
   tcgtcacttg gtgagtagcg ggctgctggg ctggccgggg ctttcgtggc cgccgggccg 600
   ctcggtggga cggaagcgtg tggagagacc gccaagggct gtagtctggg tccgcgagca 660
   aggttgccct gaactggggg ttggggggag cgcagcaaaa tggcggctgt tcccgagtct 720
   tgaatggaag acgcttgtga ggcgggctgt gaggtcgttg aaacaaggtg gggggcatgg 780
   tgggcggcaa gaacccaagg tcttgaggcc ttcgctaatg cgggaaagct cttattcggg 840
   tgagatgggc tggggcacca tctggggacc ctgacgtgaa gtttgtcact gactggagaa 900
   ctcggtttgt cgtctgttgc gggggcggca gttatggcgg tgccgttggg cagtgcaccc 960
   gtacctttgg gagcgcgcgc cctcgtcgtg tcgtgacgtc acccgttctg ttggcttata 1020
   atgcagggtg gggccacctg ccggtaggtg tgcggtaggc ttttctccgt cgcaggacgc 1080
   agggttcggg cctagggtag gctctcctga atcgacaggc gccggacctc tggtgagggg 1140
   agggataagt gaggcgtcag tttctttggt cggttttatg tacctatctt cttaagtagc 1200
   tgaagctccg gttttgaact atgcgctcgg ggttggcgag tgtgttttgt gaagtttttt 1260
   aggcaccttt tgaaatgtaa tcatttgggt caatatgtaa ttttcagtgt tagactagta 1320
   aattgtccgc taaattctgg ccgtttttgg cttttttgtt agacgtcgag ctagcctaga 1380
   ttgggaaccc gggtctctcg aattgttgac aattaatcat cggcatagta tatcggcata 1440
   gtataatacg acaaggtgag gaactaaacc atccaccatg attgaacaag atggattgca 1500
   cgcaggttct ccggccgctt gggtggagag gctattcggc tatgactggg cacaacagac 1560
   aatcggctgc tctgatgccg ccgtgttccg gctgtcagcg caggggcgcc cggttctttt 1620
   tgtcaagacc gacctgtccg gtgccctgaa tgaactgcag gacgaggcag cgcggctatc 1680
   gtggctggcc acgacgggcg ttccttgcgc agctgtgctc gacgttgtca ctgaagcggg 1740
   aagggactgg ctgctattgg gcgaagtgcc ggggcaggat ctcctgtcat ctcaccttgc 1800
   tcctgccgag aaagtatcca tcatggctga tgcaatgcgg cggctgcata cgcttgatcc 1860
   ggctacctgc ccattcgacc accaagcgaa acatcgcatc gagcgagcac gtactcggat 1920
   ggaagccggt cttgtcgatc aggatgatct ggacgaagag catcaggggc tcgcgccagc 1980
   cgaactgttc gccaggctca aggcgcgcat gcccgacggc gaggatctcg tcgtgaccca 2040
   tggcgatgcc tgcttgccga atatcatggt ggaaaatggc cgcttttctg gattcatcga 2100
   ctgtggccgg ctgggtgtgg cggaccgcta tcaggacata gcgttggcta cccgtgatat 2160
   tgctgaagag cttggcggcg aatgggctga ccgcttcctc gtgctttacg gtatcgccgc 2220
   tcccgattcg cagcgcatcg ccttctatcg ccttcttgac gagttcttct gaagatccgc 2280
   tgtaagtctg cagaaattga tgatctatta aacaataaag atgtccacta aaatggaagt 2340
   ttttcctgtc atactttgtt aagaagggtg agaacagagt acctacattt tgaatggaag 2400
   gattggagct acgggggtgg gggtggggtg ggattagata aatgcctgct ctttactgaa 2460
   ggctctttac tattgcttta tgataatgtt tcatagttgg atatcataat ttaaacaagc 2520
   aaaaccaaat taagggccag ctcattcctc ccactcatga tctatagatc tatagatctc 2580
   tcgtgggatc attgtttttc tcttgattcc cactttgtgg ttctaagtac tgtggtttcc 2640
   aaatgtgtca gtttcatagc ctgaagaacg agatcagcag cctctgttcc acatacactt 2700
   cattctcagt attgttttgc caagttctaa ttccatcaga cctcgacctg cagccggcgc 2760
   gccataactt cgtataatgt atgctatacg aagttatgtc gacctcgaga atttacaaaa 2820
   gaaaaggaaa aacacacttg gcagccttca gcactctaat gattcttaac agcagcaaat 2880
   tattggcaca agactccaga gtgactggca gggttgaggg ctggggtctc ccacgtgttt 2940
   tggggctaac agcggaaggg agagcactgg caaaggtgct gggggcccct ggacccgacc 3000
   cgccctggag accgcagcca catcagcccc cagccccaca ggccccctac cagccgcagg 3060
   gtttttgtgc accccttaat ggggcctccc acaatgtggt attacgattt ttggagtggt 3120
   tattataccc acagtgtcac agagtccatc aaaaacctcc tcaccacccc ctctctgagt 3180
   ccacttaggg agactcagct tgccagggtc tcagggtcag agtcttggag gcattttgga 3240
   ggtcaggaaa gaaagcctac gta 3263
<210> 62
   <211> 23
   <212> DNA
   <213> Artificial Sequence
<220>
   <223> DNA recognition site
<400> 62
   tcagaaagca agtggatgag agg 23
<210> 63
   <211> 42
   <212> DNA
   <213> Artificial Sequence
<220>
   <223> 5' D7.27 crRNA
<400> 63
   ucagaaagca aguggaugag guuuuagagc uaugcuguuu ug 42
<210> 64
   <211> 23
   <212> DNA
   <213> Artificial Sequence
<220>
   <223> DNA recognition site
<400> 64
   ttagggagac tcagcttgcc agg 23
<210> 65
   <211> 42
   <212> DNA
   <213> Artificial Sequence
<220>
   <223> 3' JH1-5 del crRNA
<400> 65
   uuagggagac ucagcuugcc guuuuagagc uaugcuguuu ug 42
<210> 66
   <211> 19
   <212> DNA
   <213> Artificial Sequence
<220>
   <223> 5' up detect D7-27
<400> 66
   gtgaacaggt ggaaccaac 19
<210> 67
   <211> 21
   <212> DNA
   <213> Artificial Sequence
<220>
   <223> 3' ub pro-200
<400> 67
   ccagtgccct agagtcaccc a 21
<210> 68
   <211> 21
   <212> DNA
   <213> Artificial Sequence
<220>
   <223> 5' neo detect
<400> 68
   ctcccactca tgatctatag a 21
<210> 69
   <211> 19
   <212> DNA
   <213> Artificial Sequence
<220>
   <223> 3' down detect JH1-5 del
<400> 69
   acgcaatcat cacgacagc 19
<210> 70
   <211> 3345
   <212> DNA
   <213> Artificial Sequence
<220>
   <223> >p51816 (12-D2-2-23 donor)
<400> 70
   actagtagaa gataagctcc agacccccaa gactgggacc tgccttcctg ccaccgcgcc      60
   ggcgatgcat ccgggtaggg gaggcgcttt tcccaaggca gtctggagtt gacaattaat 120
   catcggcata gtatatcggc atagtataat acgacaaggt gaggaactaa accatggaga 180
   aaaaaatcac tggatatacc accgttgata tatcccaatg gcatcgtaaa gaacattttg 240
   aggcatttca gtcagttgct caatgtacct ataaccagac cgttcagctg gatattacgg 300
   cctttttaaa gaccgtaaag aaaaataagc acaagtttta tccggccttt attcacattc 360
   ttgcccgcct gatgaatgct catccggaat tccgtatggc aatgaaagac ggtgagctgg 420
   tgatatggga tagtgttcac ccttgttaca ccgttttcca tgagcaaact gaaacgtttt 480
   catcgctctg gagtgaatac cacgacgatt tccggcagtt tctacacata tattcgcaag 540
   atgtggcgtg ttacggtgaa aacctggcct atttccctaa agggtttatt gagaatatgt 600
   ttttcgtctc agccaatccc tgggtgagtt tcaccagttt tgatttaaac gtggccaata 660
   tggacaactt cttcgccccc gttttcacca tgggcaaata ttatacgcaa ggcgacaagg 720
   tgctgatgcc gctggcgatt caggttcatc atgccgtttg tgatggcttc catgtcggca 780
   gaatgcttaa tgaattacaa cagtactgcg atgagtggca gggcggggcg taaggatcct 840
   gccatttcat tacctctttc tccgcacccg acatagataa agcttatcga taccgtcgac 900
   ctcgaggggg ggcccggtac ccgccggcgc ttgtagctcc agacctccgt gcctcccccg 960
   accacttact gttagatagg cattgatcta ggcacggggc gcgatgttga cacgggccag 1020
   ggagctgttc cacaaagatc aaccccaaac cgggaccgcc tggcactcgc gccgctgcca 1080
   cttccctctc catttgttcc cagcacctct gtgctccctc cctcctccct ccttcagggg 1140
   aacagcctgt gcagcccctc cctgcacccc acaccctggg gaggcccaac cctgcctcca 1200
   gccctttctc ccccgctgct cttcctgccc atccagacaa ccctggggtc ccatccctgc 1260
   agcctacacc ctggtctcca cccagacccc tgtctctccc tccagacacc cctcccaggc 1320
   caaccctgca cacgcaggcc ctcccctttt ctgctcccag agcctcaccc tctaccctct 1380
   gtgcctaccc cctgcctcct cctgcccaca actcgagctc ttcctctcct ggggcccctg 1440
   agccatggca ctgaccgtgc actcccaccc ccacactgcc catgccctca ccttcctcct 1500
   ggacactctg accccgctcc cctcttggac ccagccctgg tatttccagg acaaaggctc 1560
   acccaagtct tccccatgca ggcccttgcc ctcactgccc ggttacacgg cagcctcctg 1620
   tgcacagaag cagggagctc agcccttcca caggcagaag gcactgaaag aaatcggcct 1680
   ccagcaccct gatgcacgtc cgcctgtgtc tctcactgcc cgcacctgca gggaggctcg      1740
   gcactccctg taaagacgag ggatccaggc agcaacatca tgggagaatg cagggctccc      1800
   agacagccca gccctctcgc aggcctctcc tgggaagaga cctgcagcca ccactgaaca      1860
   gccacggagc ccgctggata gtaactgagt cagtgaccga cctggagggc aggggagcag      1920
   tgaaccggag cccagaccat agggacagag accagccgct gacatcccga gcccctcact      1980
   ggcggcccca gaacaccgcg tggaaacaga acagacccac attcccacct ggaaaagggc      2040
   agacactgct gagcccccag caccagccct gagaaacacc aggcaacggc atcagagggg      2100
   gctcctgaga aagaaaggag gggaggtctc cttcaccagc aagtacttcc cttgaccaaa      2160
   aacagggtcc acgcaactcc cccaggacaa aggaggagcc ccctgtacag cactgggctc      2220
   agagtcctct cccacacacc ctgagtttca gacaaaaacc ccctggaaat catagtatca      2280
   gcaggagaac tagccagaga cagcaagagg ggactcagtg actcccgcgg ggacaggagg      2340
   attttgtggg ggctcgtgtc actgtgagga tattgtagta gtaccagctg ctatacccac      2400
   agtgtgaaaa cccacatcct gagagtgaca caaacccctg ctgtaaacgc ttccacttct      2460
   ggagctgagg ggctgggggg agcgtctggg aagtagggcc taggggtggc catcaatgcc      2520
   caaaacgcac cagactcccc cccagacatc accccactgg ccagtgagca gagtaaacag      2580
   aaaatgagaa gcagctggga agcttgcaca ggccccaagg aaagagcttt ggcgggtgtg      2640
   caagagggga tgcgggcaga gcctgagcag ggccttttgc tgtttctgct ttcctgtgca      2700
   gatagttcca taaactggtg ttcaagatcg atggctggga gtgagcccag gaggacagtg      2760
   tgggaagggc acagggaagg agaagcagcc gctatcctac actgtcatct ttcaagagtt      2820
   tgccctgtgc ccacaatgct gcatcatggg atgcttaaca gctgatgtag acacagctaa      2880
   agagagaatc agtgaaatgg atttgcagca cagatctgaa taaattctcc agaatgtgga      2940
   gccacacaga agcaagcaca aggaaagtgc ctgatgcaag ggcaaagtac agtgtgtacc      3000
   ttcaggctgg gcacagacac tctgaaaagc cttggcagga actccctgca acaaagcaga      3060
   gccctgcagg caatgccagc tccagagccc tccctgagag cctcatgggc aaagatgtgc      3120
   acaacaggtg tttctcatag ccccaaactg agaatgaagc aaacagccat ctgaaggaaa      3180
   acaggcaaat aaacgatggc aggttcatgg cgcttgcagc gattcaacag ctaaccaata      3240
   ctaacgagga aatgcaaacc cagacagcca gaaggacaac agtgagggtt acaggtgact      3300
   ctgtggttga gttcatgaca atgctgagta attggagtaa ctagt                      3345
<210> 71
   <211> 2484
   <212> DNA
   <213> Artificial Sequence
<220>
   <223> >p53419 (12-D2-8-23 donor)
<400> 71
   actagtccca gtcctctcac aggcctctcc tgggaagaga cctgaagacg cccagtcgcc      60
   ggcgatgcat ccgggtaggg gaggcgcttt tcccaaggca gtctggagtt gacaattaat     120
   catcggcata gtatatcggc atagtataat acgacaaggt gaggaactaa accatggaga     180
   aaaaaatcac tggatatacc accgttgata tatcccaatg gcatcgtaaa gaacattttg     240
   aggcatttca gtcagttgct caatgtacct ataaccagac cgttcagctg gatattacgg     300
   cctttttaaa gaccgtaaag aaaaataagc acaagtttta tccggccttt attcacattc     360
   ttgcccgcct gatgaatgct catccggaat tccgtatggc aatgaaagac ggtgagctgg     420
   tgatatggga tagtgttcac ccttgttaca ccgttttcca tgagcaaact gaaacgtttt     480
   catcgctctg gagtgaatac cacgacgatt tccggcagtt tctacacata tattcgcaag     540
   atgtggcgtg ttacggtgaa aacctggcct atttccctaa agggtttatt gagaatatgt     600
   ttttcgtctc agccaatccc tgggtgagtt tcaccagttt tgatttaaac gtggccaata     660
   tggacaactt cttcgccccc gttttcacca tgggcaaata ttatacgcaa ggcgacaagg     720
   tgctgatgcc gctggcgatt caggttcatc atgccgtttg tgatggcttc catgtcggca     780
   gaatgcttaa tgaattacaa cagtactgcg atgagtggca gggcggggcg taaggatcct     840
   gccatttcat tacctctttc tccgcacccg acatagataa agcttatcga taccgtcgac     900
   ctcgaggggg ggcccggtac ccgccggcgc aacggagtct aacaccaaac ctccctggag     960
   gccgatgggc gagcaaaatc cgacacgtaa ctacaagtag ggcctgatct agtaacggag    1020
   tcattgccag acctggaggc aggggagcag tgagcccgag cccacaccat agggccagag    1080
   ggcacagcaa ctgacatccc aagccactca ctggtggtcc cacaacaccc catggaaaga    1140
   ggacagaccc acagtcccac ctggaccagg gcagagactg ctgagaccca gcaccagaac    1200
   caaccaagaa acaccaggca acagcatcag agggggctct ggcagaacag aggaggggag    1260
   gtctccttca ccagcaggcg cttcccttga ccgaagacag gatccatgca actcccccag    1320
   gacaaaggag gagccccttg ttcagcactg ggctcagagt cctctccaag acacccagag    1380
   tttcagacaa aaaccccctg gaatgcacag tctcagcagg agagccagcc agagccagca    1440
   agatggggct cagtgacacc cgcagggaca ggaggatttt gtgggggctc gtgtcactgt    1500
   gaggatattg tactaatggt gtatgctata cccacagtgt gaaaacccac atcctgagag    1560
   tgacacaaac ccctactgca aacgcattcc acttctgggg ctgaggggct gggggagcgt    1620
   ctgggaaata gggctcaggg gtgtccatca atgcccaaaa cgcaccagac tcccctccat    1680
   acatcacacc caccagccag cgagcagagt aaacagaaaa tgagaagcaa gctggggaag    1740
   cttgcacagg ccccaaggaa agagctttgg cgggtgtgta agaggggatg cgggcagagc    1800
   ctgagcaggg ccttttgctg tttctgcttt cctgtgcaga gagttccata aactggtgtt 1860
   cgagatcaat ggctgggagt gagcccagga ggacagcgtg ggaagagcac agggaaggag 1920
   gagcagccgc tatcctacac tgtcatcttt caaaagtttc ccttgtgccc acattgctgc 1980
   atcatgggat gcttaagagc tgatgtagac acagctaaag agagaatcag tgagatggat 2040
   ttgcagcaca gatctgaata aattctccag aatgtggagc agcacagaag caagcacaca 2100
   gaaagtgcct gatgcaagga caaagttcag tgggcacctt caggcattgc tgctgggcac 2160
   agacattctg aaaagccctg gcaggaactc cctgtgacaa agcagaaccc tcaggcaatg 2220
   ccagccccag agccctccct gagagcctca tgggcaaaga tgtgcacaac aggtgtttct 2280
   catagcccca aactgagagc aaagcaaacg tccatctgaa ggagaacagg caaataaacg 2340
   atggcaggct acacccgtgc atccaataag tcggttcggc aactcaactt catgaaatgc 2400
   aaacccagac agccacaagc acaaaagtac agggttataa gcgactctgg ttgagttcat 2460
   gacaatgctg agtaattgac tagt 2484
<210> 72
   <211> 2321
   <212> DNA
   <213> Artificial Sequence
<220>
   <223> >p51818 (12-D2-15-23 donor)
<400> 72
   actagtgaga ccagccgctg acatcccgag ctcctcaatg gtggccccat aacacacgcc      60
   ggcgatgcat ccgggtaggg gaggcgcttt tcccaaggca gtctggagtt gacaattaat     120
   catcggcata gtatatcggc atagtataat acgacaaggt gaggaactaa accatggaga     180
   aaaaaatcac tggatatacc accgttgata tatcccaatg gcatcgtaaa gaacattttg     240
   aggcatttca gtcagttgct caatgtacct ataaccagac cgttcagctg gatattacgg     300
   cctttttaaa gaccgtaaag aaaaataagc acaagtttta tccggccttt attcacattc     360
   ttgcccgcct gatgaatgct catccggaat tccgtatggc aatgaaagac ggtgagctgg     420
   tgatatggga tagtgttcac ccttgttaca ccgttttcca tgagcaaact gaaacgtttt     480
   catcgctctg gagtgaatac cacgacgatt tccggcagtt tctacacata tattcgcaag     540
   atgtggcgtg ttacggtgaa aacctggcct atttccctaa agggtttatt gagaatatgt     600
   ttttcgtctc agccaatccc tgggtgagtt tcaccagttt tgatttaaac gtggccaata     660
   tggacaactt cttcgccccc gttttcacca tgggcaaata ttatacgcaa ggcgacaagg     720
   tgctgatgcc gctggcgatt caggttcatc atgccgtttg tgatggcttc catgtcggca     780
   gaatgcttaa tgaattacaa cagtactgcg atgagtggca gggcggggcg taaggatcct     840
   gccatttcat tacctctttc tccgcacccg acatagataa agcttatcga taccgtcgac 900
   ctcgaggggg ggcccggtac ccgccggcgc ctaggaaaca taacacaccc acagccccac 960
   ctggaacaga tagatgcacc aatatctccc agtaagctca agctcgcggg gcagagactg 1020
   ctgagccccc agcaccagcc ccaagaaaca ccaggcaaca gtatcagagg gggctcccga 1080
   gaaagagagg aggggagatc tccttcacca tcaaatgctt cccttgacca aaaacagggt 1140
   ccacgcaact cccccaggac aaaggaggag ccccctatac agcactgggc tcagagtcct 1200
   ctctgagaca ccctgagttt cagacaacaa cccgctggaa tgcacagtct cagcaggaga 1260
   acagaccaaa gccagcaaaa gggacctcgg tgacaccagt agggacagga ggattttgtg 1320
   ggggctcgtg tcactgtgag gatattgtag tggtggtagc tgctactccc acagtgtgaa 1380
   aacccacatc ctgagagtga cacaaacccc tactgcaaac acccccactc ctggggctga 1440
   ggggctaggg gagcgtctgg gaagtagggt ccaggggtgt ctatcaatgt ccaaaatgca 1500
   ccagactccc cgccaaacac caccccacca gccagcgagc agggtaaaca gaaaatgaga 1560
   ggctctggga agcttgcaca ggccccaagg aaagagcttt ggcgggtgtg caagagggga 1620
   tgcaggcaga gcctgagcag ggccttttgc tgtttctgct ttcctgtgca gagagttcca 1680
   taaactggtg ttcaagatca gtggctggga atgagcccag gagggcagtc tgtgggaaga 1740
   gcacagggaa ggaggagcag ccgctatcct acactgtcat ctttcaaaag tttgccttgt 1800
   gaccacacta ttgcatcatg ggatgcttaa gagctgatgt agacacagct aaagagagaa 1860
   tcagtgagat gaatttgcag catagatctg aataaactct ccagaatgtg gagcagtaca 1920
   gaagcaaaca cacagaaagt gcctgatgca aggacaaagt tcagtgggca ccttcaggca 1980
   ttgctgctgg gcacagacac tctgaaaagc cctggcagga tctccctgcg acaaagcaga 2040
   accctcaggc aatgccagcc ccagagccct ccctgagagc gtcatgggga aagatgtgca 2100
   gaacagctga ttatcataga ctcaaactga gaacagagca aacgtccatc tgaagaacag 2160
   tcaaataagc aatggtaggt tcatgtccat ctcatcggtc agtaaaaata ccgccgctgg 2220
   tactccaatg caaacccaga cagccagggg acaacagtag agggctacag gcggctttgc 2280
   ggttgagttc atgacaatgc tgagtaattg gagtaactag t 2321
<210> 73
   <211> 40
   <212> DNA
   <213> Artificial Sequence
<220>
   <223> 1 P51816(D2-2)
<400> 73
   tgttagatag gcattgatct aggcacgggg cgcgatgttg 40
<210> 74
   <211> 40
   <212> DNA
   <213> Artificial Sequence
<220>
   <223> 2 P51816(D2-2)
<400> 74
   gcgcttgcag cgattcaaca gctaaccaat actaacgagg 40
<210> 75
   <211> 40
   <212> DNA
   <213> Artificial Sequence
<220>
   <223> 10 P53419(D2-8)
<400> 75
   cgagcaaaat ccgacacgta actacaagta gggcctgatc 40
<210> 76
   <211> 40
   <212> DNA
   <213> Artificial Sequence
<220>
   <223> 16 P53419(D2-8)
<400> 76
   ctacacccgt gcatccaata agtcggttcg gcaactcaac 40
<210> 77
   <211> 40
   <212> DNA
   <213> Artificial Sequence
<220>
   <223> 8 P51818(D2-15)
<400> 77
   atagatgcac caatatctcc cagtaagctc aagctcgcgg 40
<210> 78
   <211> 40
   <212> DNA
   <213> Artificial Sequence
<220>
   <223> 18 P51818(D2-15)
<400> 78
   tccatctcat cggtcagtaa aaataccgcc gctggtactc 40
<210> 79
   <211> 20
   <212> DNA
   <213> Artificial Sequence
<220>
   <223> 5' hIgHD HU2(h268)
<400> 79
   gcagtaaccc tcaggaagca 20
<210> 80
   <211> 31
   <212> DNA
   <213> Artificial Sequence
<220>
   <223> 3' hIgHD HU2 AgeI(h269)
<400> 80
   cttccaccgg tacagccaca ccaaggtcat c 31
<210> 81
   <211> 31
   <212> DNA
   <213> Artificial Sequence
<220>
   <223> 5' hIgHD HD2 XhoI(h271)
<400> 81
   cttccctcga ggaacactgt cagctcccac a 31
<210> 82
   <211> 19
   <212> DNA
   <213> Artificial Sequence
<220>
   <223> 3' hIgHD HD2(h272)
<400> 82
   agatcctcca tgcgtgctg 19
<210> 83
   <211> 20
   <212> DNA
   <213> Artificial Sequence
<220>
   <223> 5' hIgHD HU2 detect(h270)
<400> 83
   tctcctctcg tcgcctctac 20
<210> 84
   <211> 21
   <212> DNA
   <213> Artificial Sequence
<220>
   <223> 3' ub pro-200
<400> 84
   ccagtgccct agagtcaccc a 21
<210> 85
   <211> 21
   <212> DNA
   <213> Artificial Sequence
<220>
   <223> 5' neo detect
<400> 85
   ctcccactca tgatctatag a 21
<210> 86
   <211> 20
   <212> DNA
   <213> Artificial Sequence
<220>
   <223> 3' hIgHD HD2 detect(h273)
<400> 86
   gggtgcttgg gtcctgttag 20
<210> 87
   <211> 23
   <212> DNA
   <213> Artificial Sequence
<220>
   <223> 5' D2-2 Cas9 DNA target
<400> 87
   aggtctggag ctacaagcgg tgg 23
<210> 88
   <211> 42
   <212> DNA
   <213> Artificial Sequence
<220>
   <223> 5' D2-2 gRNA
<400> 88
   aggucuggag cuacaagcgg guuuuagagc uaugcuguuu ug 42
<210> 89
   <211> 23
   <212> DNA
   <213> Artificial Sequence
<220>
   <223> 3' D2-2 Cas9 DNA target
<400> 89
   aggacaacag tgagggttac agg 23
<210> 90
   <211> 42
   <212> DNA
   <213> Artificial Sequence
<220>
   <223> 3' D2-2 gRNA
<400> 90
   aggacaacag ugaggguuac guuuuagagc uaugcuguuu ug 42
<210> 91
   <211> 19
   <212> DNA
   <213> Artificial Sequence
<220>
   <223> 5' up detect D2-2(h274)
<400> 91
   gtcaaaggtg gaggcagtg 19
<210> 92
   <211> 21
   <212> DNA
   <213> Artificial Sequence
<220>
   <223> 3' cm up detect
<400> 92
   tccagctgaa cggtctggtt a 21
<210> 93
   <211> 19
   <212> DNA
   <213> Artificial Sequence
<220>
   <223> D2-2seqF2
<400> 93
   agcgattcaa cagctaacc 19
<210> 94
   <211> 20
   <212> DNA
   <213> Artificial Sequence
<220>
   <223> 3' down detect D2-2(h275)
<400> 94
   ccagtagaag taacgaccac 20
<210> 95
   <211> 80
   <212> DNA
   <213> Artificial Sequence
<220>
   <223> JO-1
<400> 95
   ctccagaccc ccaagactgg gacctgcctt cctgccaccg cttgtagctc cagacctccg 60
   tgcctccccc gaccacttac 80
<210> 96
   <211> 19
   <212> DNA
   <213> Artificial Sequence
<220>
   <223> 5' up detect D2-2(h274)
<400> 96
   gtcaaaggtg gaggcagtg 19
<210> 97
   <211> 19
   <212> DNA
   <213> Artificial Sequence
<220>
   <223> D2-2seqR1
<400> 97
   cccgtgccta gatcaatgc 19
<210> 98
   <211> 19
   <212> DNA
   <213> Artificial Sequence
<220>
   <223> D2-2seqF1
<400> 98
   aggcattgat ctaggcacg 19
<210> 99
   <211> 19
   <212> DNA
   <213> Artificial Sequence
<220>
   <223> D2-2seqR2
<400> 99
   ctgttgaatc gctgcaagc 19
<210> 100
   <211> 23
   <212> DNA
   <213> Artificial Sequence
<220>
   <223> 5' D2-8 Cas9 DNA target
<400> 100
   acctgaagac gcccagtcaa cgg 23
<210> 101
   <211> 42
   <212> DNA
   <213> Artificial Sequence
<220>
   <223> 5' D2-8 crRNA
<400> 101
   accugaagac gcccagucaa guuuuagagc uaugcuguuu ug 42
<210> 102
   <211> 23
   <212> DNA
   <213> Artificial Sequence
<220>
   <223> 3' D2-8 Cas9 DNA target
<400> 102
   gccacaagca caaaagtaca ggg 23
<210> 103
   <211> 42
   <212> DNA
   <213> Artificial Sequence
<220>
   <223> 3' D2-8 crRNA
<400> 103
   gccacaagca caaaaguaca guuuuagagc uaugcuguuu ug 42
<210> 104
   <211> 19
   <212> DNA
   <213> Artificial Sequence
<220>
   <223> 5' up detect D2-8(h276)
<400> 104
   gccttaccca agtctttcc 19
<210> 105
   <211> 21
   <212> DNA
   <213> Artificial Sequence
<220>
   <223> 3' cm up detect
<400> 105
   tccagctgaa cggtctggtt a 21
<210> 106
   <211> 19
   <212> DNA
   <213> Artificial Sequence
<220>
   <223> D2-8seqF2
<400> 106
   tccaataagt cggttcggc 19
<210> 107
   <211> 19
   <212> DNA
   <213> Artificial Sequence
<220>
   <223> 3' down detect D2-8(h277)
<400> 107
   gcagaagtaa ccaccactg 19
<210> 108
   <211> 80
   <212> DNA
   <213> Artificial Sequence
<220>
   <223> JO-2
<400> 108
   tcacaggcct ctcctgggaa gagacctgaa gacgcccagt caacggagtc taacaccaaa 60
   cctccctgga ggccgatggg 80
<210> 109
   <211> 19
   <212> DNA
   <213> Artificial Sequence
<220>
   <223> 5' up detect D2-8(h276)
<400> 109
   gccttaccca agtctttcc 19
<210> 110
   <211> 20
   <212> DNA
   <213> Artificial Sequence
<220>
   <223> D2-8seqR1
<400> 110
   gccctacttg tagttacgtg 20
<210> 111
   <211> 19
   <212> DNA
   <213> Artificial Sequence
<220>
   <223> D2-8seqF1
<400> 111
   gcaaaatccg acacgtaac 19
<210> 112
   <211> 20
   <212> DNA
   <213> Artificial Sequence
<220>
   <223> D2-8seqR2
<400> 112
   cgaaccgact tattggatgc 20
<210> 113
   <211> 23
   <212> DNA
   <213> Artificial Sequence
<220>
   <223> 5' D2-8 Cas9 DNA target
<400> 113
   ggtggcccca taacacacct agg 23
<210> 114
   <211> 42
   <212> DNA
   <213> Artificial Sequence
<220>
   <223> 5' D2-8 crRNA
<400> 114
   gguggcccca uaacacaccu guuuuagagc uaugcuguuu ug 42
<210> 115
   <211> 23
   <212> DNA
   <213> Artificial Sequence
<220>
   <223> 3' D2-8 Cas9 DNA target
<400> 115
   ggacaacagt agagggctac agg 23
<210> 116
   <211> 42
   <212> DNA
   <213> Artificial Sequence
<220>
   <223> 3' D2-8 crRNA
<400> 116
   ggacaacagu agagggcuac guuuuagagc uaugcuguuu ug 42
<210> 117
   <211> 20
   <212> DNA
   <213> Artificial Sequence
<220>
   <223> 5' up detect D2-15(h278)
<400> 117
   gcactccctc taaagacaag 20
<210> 118
   <211> 21
   <212> DNA
   <213> Artificial Sequence
<220>
   <223> 3' cm up detect
<400> 118
   tccagctgaa cggtctggtt a 21
<210> 119
   <211> 19
   <212> DNA
   <213> Artificial Sequence
<220>
   <223> D2-15seqF2
<400> 119
   gtaaaaatac cgccgctgg 19
<210> 120
   <211> 19
   <212> DNA
   <213> Artificial Sequence
<220>
   <223> 3' down detect D2-15(h279)
<400> 120
   gcattttcct gttcttgcg 19
<210> 121
   <211> 80
   <212> DNA
   <213> Artificial Sequence
<220>
   <223> JO-3
<400> 121
   gctgacatcc cgagctcctc aatggtggcc ccataacaca cctaggaaac ataacacacc 60
   cacagcccca cctggaacag 80
<210> 122
   <211> 20
   <212> DNA
   <213> Artificial Sequence
<220>
   <223> 5' up detect D2-15(h278)*
<400> 122
   gcactccctc taaagacaag 20
<210> 123
   <211> 20
   <212> DNA
   <213> Artificial Sequence
<220>
   <223> D2-15seqR1
<400> 123
   ctgggagata ttggtgcatc 20
<210> 124
   <211> 19
   <212> DNA
   <213> Artificial Sequence
<220>
   <223> D2-15seqF1
<400> 124
   tgcaccaata tctcccagt 19
<210> 125
   <211> 20
   <212> DNA
   <213> Artificial Sequence
<220>
   <223> D2-15seqR2
<400> 125
   cggcggtatt tttactgacc 20
<210> 126
   <211> 21
   <212> DNA
   <213> Artificial Sequence
<220>
   <223> 23D3.312-1
<400> 126
   tttttgtgca ccccttaatg g 21
<210> 127
   <211> 22
   <212> DNA
   <213> Artificial Sequence
<220>
   <223> 23D3.312-1
<400> 127
   atgtggtatt acgatttttg ga 22
<210> 128
   <211> 26
   <212> DNA
   <213> Artificial Sequence
<220>
   <223> 23D3.312-1
<400> 128
   tctgtgacac tgtgggtata ataacc 26
<210> 129
   <211> 22
   <212> DNA
   <213> Artificial Sequence
<220>
   <223> 23D3.312-2
<400> 129
   cccacagtgt cacagagtcc at 22
<210> 130
   <211> 24
   <212> DNA
   <213> Artificial Sequence
<220>
   <223> 23D3.312-2
<400> 130
   caagatggct ttccttctgc ctcc 24
<210> 131
   <211> 19
   <212> DNA
   <213> Artificial Sequence
<220>
   <223> 23D3.312-2
<400> 131
   tcccagctcc aggacagag 19
<210> 132
   <211> 24
   <212> DNA
   <213> Artificial Sequence
<220>
   <223> 23D3.312-3
<400> 132
   tgtcacagag tccatcaaaa acct 24
<210> 133
   <211> 14
   <212> DNA
   <213> Artificial Sequence
<220>
   <223> 23D3.312-3
<400> 133
   caccaccccc tctc 14
<210> 134
   <211> 19
   <212> DNA
   <213> Artificial Sequence
<220>
   <223> 23D3.312-3
<400> 134
   cctgagaccc tggcaagct 19
<210> 135
   <211> 19
   <212> DNA
   <213> Artificial Sequence
<220>
   <223> Jxn DH2-2
<400> 135
   atggcgcttg cagcgattc 19
<210> 136
   <211> 28
   <212> DNA
   <213> Artificial Sequence
<220>
   <223> Jxn DH2-2
<400> 136
   ccaatactaa cgaggaaatg caaaccca 28
<210> 137
   <211> 21
   <212> DNA
   <213> Artificial Sequence
<220>
   <223> Jxn DH2-2
<400> 137
   accctcactg ttgtccttct g 21
<210> 138
   <211> 23
   <212> DNA
   <213> Artificial Sequence
<220>
   <223> Jxn DH2-8
<400> 138
   gcaaacgtcc atctgaagga gaa 23
<210> 139
   <211> 27
   <212> DNA
   <213> Artificial Sequence
<220>
   <223> Jxn DH2-8
<400> 139
   caaataaacg atggcaggct acacccg 27
<210> 140
   <211> 22
   <212> DNA
   <213> Artificial Sequence
<220>
   <223> Jxn DH2-8
<400> 140
   gagttgccga accgacttat tg 22
<210> 141
   <211> 23
   <212> DNA
   <213> Artificial Sequence
<220>
   <223> Jxn DH2-15
<400> 141
   gcaatggtag gttcatgtcc atc 23
<210> 142
   <211> 20
   <212> DNA
   <213> Artificial Sequence
<220>
   <223> Jxn DH2-15
<400> 142
   accgccgctg gtactccaat 20
<210> 143
   <211> 19
   <212> DNA
   <213> Artificial Sequence
<220>
   <223> Jxn DH2-15
<400> 143
   ccctggctgt ctgggtttg 19
<210> 144
   <211> 7
   <212> DNA
   <213> Artificial Sequence
<220>
   <223> Artificial
<400> 144
   cacagtg 7
<210> 145
   <211> 9
   <212> DNA
   <213> Artificial Sequence
<220>
   <223> Artificial
<400> 145
   acaaaaacc 9
<210> 146
   <211> 29
   <212> DNA
   <213> Artificial Sequence
<220>
   <223> Template switching oligo
<400> 146
   agcagtggta tcaacgcaga gtacatggg 29
<210> 147
   <211> 54
   <212> DNA
   <213> Artificial Sequence
<220>
   <223> 1st round PCR primers IgM Constant
<400> 147
   acactctttc cctacacgac gctcttccga tctgggaaga catttgggaa ggac 54
<210> 148
   <211> 57
   <212> DNA
   <213> Artificial Sequence
<220>
   <223> 1st round PCR primers PE2-PIIA
<400> 148
   gtgactggag ttcagacgtg tgctcttccg atctaagcag tggtatcaac gcagagt 57
<210> 149
   <211> 68
   <212> DNA
   <213> Artificial Sequence
<220>
   <223> 2nd round PCR Primers
<220>
   <221> misc_feature
   <222> (30)..(35)
   <223> n is a, c, g, or t
<400> 149
   aatgatacgg cgaccaccga gatctacacn nnnnnacact ctttccctac acgacgctct 60
   tccgatct 68
<210> 150
   <211> 64
   <212> DNA
   <213> Artificial Sequence
<220>
   <223> 2nd round PCR Primers
<220>
   <221> misc_feature
   <222> (25)..(30)
   <223> n is a, c, g, or t
<400> 150
   caagcagaag acggcatacg agatnnnnnn gtgactggag ttcagacgtg tgctcttccg 60
   atct 64
<210> 151
   <211> 39
   <212> DNA
   <213> Artificial Sequence
<220>
   <223> 23-mer RSS from VH1-69
<400> 151
   cacagtgtga aaacccacat cctgagagtg acacaaacc 39

## Description

**[Deleted]**

### SEQUENCE LISTING

The specification encompasses the sequence listing submitted electronically via EFS-Web as an ASCII formatted sequence listing with a file named "10347_ST25.txt", which file was created on June 13, 2019, has a size of about 50 kilobytes, and was filed concurrently with the specification.

### BACKGROUND

Monoclonal antibody products have revolutionized the biopharmaceutical industry and achieved significant advances in the treatment of several diseases. Many of these monoclonal antibody products have leveraged the natural features of antibody molecules (i.e., traditional immunoglobulin gene segments) and, in some instances, incorporated other features such as labeling (e.g., pegylated, radiolabeled) or conjugation with other drugs. At the current rate of approval, approximately 70 monoclonal antibody products are expected to be on the market by 2020. Despite these advances and the knowledge gained by the use of monoclonal antibodies for therapeutic use, diseases linked to targets that are difficult for monoclonal antibodies to bind and/or access persist, which highlights the need for different approaches for developing effective treatments.

The following documents are referred to:
- Jung et al (2006) Annual Review Immunology, 24: 541-570 which is concerned with mechanism and control of V(D)J recombination at the immunoglobulin heavy chain locus;
- Kokubu et al (1988) The EMBO Journal, 7(11): 3413-3422 which is concerned with diverse organization of immunoglobulin VH gene loci in a primitive vertebrate;
- Tuaillon et al (2000) European Journal of Immunology, 30: 2998-3005 which is concerned with rearrangements in human immunoglobulin heavy chain mini locus transgenic mice;
- Meek et al (1989) Journal of Experimental Medicine, 170: 39-57 which is concerned with novel rearrangements at the immunoglobulin D locus; and
- Taylor et al (1992) Nucleic Acids Research, 20(23): 6287-6295 which is concerned with a transgenic mouse that expresses a diversity of human sequence heavy and light chain immunoglobulins.

### SUMMARY

The present invention stems from the recognition that it is desirable to engineer rodents (e.g., a rat, e.g., a mouse) to establish additional *in vivo* systems for identifying and developing new antibody-based therapeutics and, in some embodiments, antibody agents (e.g., monoclonal antibodies and/or fragments thereof), which can be used for the treatment of a variety of diseases. Specifically, the present invention focusses on rodents having an engineered heavy chain diversity (D_{H}) region within an immunoglobulin heavy chain variable region (e.g., a heterologous immunoglobulin heavy chain variable region, e.g., a human immunoglobulin heavy chain variable region) comprising one or more D_{H} segments engineered to be operably linked to a recombination signal sequence that permits D_{H}-to-D_{H} rearrangement, which may lead to the increased expression of antibodies containing complementary determining region three (CDR3s) that are characterized by a longer amino acid length as compared to wild-type (or reference) CDR3s and by diversity that, in some embodiments, directs binding to particular antigens. Rodents described herein provide ***in vivo*** systems for development of antibodies and/or antibody-based therapeutics for administration to humans.

### The invention

Accordingly, the present invention provides a nucleotide molecule comprising an engineered immunoglobulin heavy chain diversity (D_{H}) region ("engineered D_{H} region") comprising:
(i) an engineered D_{H} gene segment comprising a D_{H} gene segment immediately adjacent to a 23-mer RSS ("engineered D_{H} gene segment") and
(ii) an unrearranged D_{H} gene segment flanked on its 5' side by a first 12-mer RSS and on its 3' side by a second 12-mer RSS ("unrearranged D_{H} gene segment"),

wherein (i) the engineered D_{H} gene segment and (ii) the unrearranged D_{H} gene segment are operably linked such that (i) the engineered D_{H} gene segment and (ii) the unrearranged D_{H} gene segment are able to join in a D_{H}-D_{H} recombination event according to the 12/23 rule,
wherein the nucleotide molecule is suitable for obtaining rodents having the engineered D_{H} region, and
optionally wherein the engineered D_{H} region comprises only human D_{H} gene segments.

The present invention further provides a targeting vector comprising a nucleotide molecule of the present invention, comprising 5'- and 3'- homology arms configured to allow homologous recombination with an immunoglobulin heavy chain sequence at an endogenous rodent immunoglobulin heavy chain locus, optionally wherein the immunoglobulin heavy chain sequence comprises a human or humanized immunoglobulin heavy chain variable region, or a combination thereof.

The present invention also provides a method of modifying an immunoglobulin heavy chain variable region to engineer D_{H}-D_{H} recombination, the method comprising:
(a) obtaining an immunoglobulin heavy chain variable chain sequence comprising a D_{H} region comprising one or more unrearranged D_{H} gene segments, each of which unrearranged D_{H} gene segment is flanked on its 5' side by a first 12-mer RSS and on its 3' side by a second 12-mer RSS,
   optionally wherein the one or more unrearranged D_{H} gene segments comprises at least one unrearranged human D_{H} gene segment, further optionally wherein the at least one unrearranged human D_{H} gene segment comprises all the functional human D_{H} gene segments spanning between and including an unrearranged human D_{H}1-1 gene segment and an unrearranged human D_{H}7-27 gene segment, optionally wherein all the functional human D_{H} gene segments are in germline configuration, and
(b) engineering the D_{H} region to further comprise at least one engineered D_{H} gene segment, wherein the engineered D_{H} gene segment comprises a D_{H} gene segment immediately adjacent to a 23-mer RSS,

wherein, in the resulting engineered D_{H} region, the engineered D_{H} gene segment and at least one of the one or more unrearranged D_{H} gene segments are operably linked and able to join in a D_{H}-D_{H} recombination event according to the 12/23 rule,
optionally wherein the resulting engineered D_{H} region comprises only human D_{H} gene segments.

The present invention also provides an immunoglobulin heavy chain locus comprising the nucleotide molecule, the targeting vector, or the immunoglobulin heavy chain variable region modified of the present invention.

The present invention further provides a rodent genome comprising the nucleotide molecule of the present invention, the targeting vector of the present invention, the immunoglobulin heavy chain variable region modified according to the method of the present invention, or the immunoglobulin heavy chain locus of the present invention, wherein the rodent genome is optionally a rodent germline genome.

Also provided by the present invention is a rodent cell comprising the rodent germline genome of the present invention, optionally wherein the rodent cell is a rat, a mouse, a rat cell, or a mouse cell. In one embodiment, the rodent cell is an embryonic stem cell. The present invention further provides a rodent or rodent embryo generated from such a rodent embryonic stem cell of the present invention.

The present invention also provides a method of making a rodent whose genome comprises an engineered D_{H} region, the method comprising:
(A) generating a rodent from an embryonic stem cell of the present invention; or
(B)
   (a) modifying the genome of a rodent embryonic stem cell to comprise a DNA fragment comprising one or more engineered D_{H} gene segments each comprising a D_{H} gene segment immediately adjacent to a 23-mer RSS, optionally wherein the DNA fragment comprises the nucleotide molecule of the present invention, the targeting vector of the present invention, the immunoglobulin heavy chain variable region modified according to the present invention, or the immunoglobulin heavy chain of the present invention, and
   (b) generating a rodent using the modified rodent embryonic stem cell of (a).

The present invention further provides a method of producing an antibody or obtaining a nucleic acid encoding same, the method comprising
immunizing a rodent with an antigen, which rodent comprises a germline genome comprising an engineered D_{H} region that comprises one or more D_{H} segments that are each immediately adjacent to a 23-mer RSS, optionally wherein the rodent is the rodent of the present invention or made according to a method of the present invention, and
allowing the rodent to produce an immune response to the antigen including an antibody, or nucleic acid encoding same, that binds the antigen.
preferably wherein:
   (a) the method is one further comprising recovering the antibody, or nucleic acid encoding same, from the rodent or a rodent cell or wherein the rodent cell is a B cell or a hybridoma;
   (b) one or more D_{H} segments that are each immediately adjacent to a 23-mer RSS comprises a D_{H} gene segment operably linked to a 5' 23-mer RSS;
   (c) one or more D_{H} segments that are each immediately adjacent to a 23-mer RSS comprises a D_{H} gene segment operably linked to a 3' 23-mer RSS; and/or
   (d) the rodent is a rat or a mouse.

Also provided are targeting vectors, rodents (e.g., rats or mice) and rodent cells (e.g., rat cells or mouse cells) comprising a nucleotide molecule described herein, methods of using a nucleotide molecule described herein, etc.

### Specific embodiments and technical details

In some embodiments, a D_{H} gene segment operably linked to a 23-mer RSS comprises a human D_{H} gene segment operably linked to a 23-mer RSS. In some embodiments, the human D_{H} gene segment comprises at least 19 nucleotides and/or encodes two cysteines. In some embodiments, the human D_{H} gene segment comprises at least 20 nucleotides and/or encodes two cysteines. In some embodiments, the human D_{H} gene segment comprises at least 23 nucleotides and/or encodes two cysteines. In some embodiments, the human D_{H} gene segment comprises at least 28 nucleotides and/or encodes two cysteines. In some embodiments, the human D_{H} gene segment comprises at least 31 nucleotides and/or encodes two cysteines. In some embodiments, the human D_{H} gene segment comprises at least 37 nucleotides and/or encodes two cysteines. In some embodiments, the human D_{H} gene segment operably linked to a 23-mer RSS comprises a human D_{H}2 gene segment operably linked to a 23-mer RSS. In some embodiments, the D_{H}2 gene segment comprises at least 30 nucleotides and/or encodes two cysteines. In some embodiments, the human D_{H} gene segment operably linked to a 23-mer RSS comprises a D_{H} gene segment selected from the group consisting of a human D_{H}3-3 gene segment, a D_{H}3-9 gene segment, a D_{H}3-10 gene segment, a D_{H}3-16 gene segment, a D_{H}3-22 gene segment, a human D_{H}2-2 gene segment, a human D_{H}2-8 gene segment, or a human D_{H}2-15 gene segment. In some embodiments, the human D_{H} gene segment operably linked to a 23-mer RSS comprises a human D_{H} gene segment selected from the group consisting of a human D_{H}3-3 gene segment, a human D_{H}2-2 gene segment, a human D_{H}2-8 gene segment, and a human D_{H}2-15 gene segment. In some embodiments, the human D_{H} gene segment operably linked to a 23-mer RSS comprises a human D_{H} gene segment selected from the group consisting of a human D_{H}3-3 gene segment, a human D_{H}2-2 gene segment, a human D_{H}2-8 gene segment, and a human D_{H}2-15 gene segment. In some embodiments, the human D_{H} gene segment comprises a human D_{H}3-3 gene segment. In some embodiments, the human D_{H} gene segment comprises human D_{H}2-2 gene segment. In some embodiments, the human D_{H} gene segment comprises a human D_{H}2-8 gene segment. In some embodiments, the human D_{H} gene segment comprises a human D_{H}2-15 gene segment.

In some embodiments, a D_{H} gene segment operably linked to a 23-mer RSS comprises: (a) a human D_{H}3-3 gene segment operably linked to a 23-mer RSS, optionally wherein the 23-mer RSS is contiguous to the 5'-end of the human D_{H}3-3 gene segment, (b) a human D_{H}2-2 gene segment operably linked to a 23-mer RSS, optionally wherein the 23-mer RSS is contiguous to the 3'-end of the human D_{H}2-2 gene segment, (c) a human D_{H}2-8 gene segment operably linked to a 23-mer RSS, optionally wherein the 23-mer RSS is contiguous to the 3'-end of the human D_{H}2-8 gene segment, (d) a human 2-15 gene segment operably linked to a 23-mer RSS, optionally wherein the 23-mer RSS is contiguous to the 3'-end of the human D_{H}2-15 gene segment, or (e) any combination of (a)-(d)

In some embodiments, a nucleotide molecule described herein (e.g., a D_{H} gene segment operably linked to a 23-mer RSS, an engineered D_{H} region, an immunoglobulin heavy chain variable region, etc.) comprises a nucleotide sequence comprising the sequence set forth as SEQ ID NO:52.

In some embodiments, a nucleotide molecule described herein (e.g., a D_{H} gene segment operably linked to a 23-mer RSS, an engineered D_{H} region, an immunoglobulin heavy chain variable region, etc.) comprises a nucleotide sequence comprising the sequence set forth as SEQ ID NO:61.

In some embodiments, a nucleotide molecule described herein (e.g., a D_{H} gene segment operably linked to a 23-mer RSS, an engineered D_{H} region, an immunoglobulin heavy chain variable region, etc.) comprises a nucleotide sequence comprising the sequence set forth as SEQ ID NO:70.

In some embodiments, a nucleotide molecule described herein (e.g., a D_{H} gene segment operably linked to a 23-mer RSS, an engineered D_{H} region, an immunoglobulin heavy chain variable region, etc.) comprises a nucleotide sequence comprising the sequence set forth as SEQ ID NO:71.

In some embodiments, a nucleotide molecule described herein (e.g., a D_{H} gene segment operably linked to a 23-mer RSS, an engineered D_{H} region, an immunoglobulin heavy chain variable region, etc.) comprises a nucleotide sequence comprising the sequence set forth as SEQ ID NO:72.

In some embodiments, a D_{H} gene segment operably linked to a 23-mer RSS comprises from 5' to 3' the 23-mer RSS and the D_{H} gene segment, e.g., comprises from 5' to 3' a 23-mer RSS, a (human) D_{H} gene segment, and a 12-mer RSS, e.g., the 23-mer RSS is contiguous to the 5'-end of the D_{H} gene segment, e.g., the D_{H} gene segment is operably linked to a 5'-end 23-mer RSS. In some embodiments, the D_{H} gene segment operably linked to a 23-mer RSS comprises a human D_{H}3-3 gene segment operably linked to a 5' end 23-mer RSS, e.g., the nucleotide molecule comprises from 5' to 3' a 23-mer RSS, the human D_{H}3-3 gene segment, and a 12-mer RSS.

Engineered D_{H} regions comprising at least one D_{H} gene segment with a 5'-end 23-mer RSS may further comprise an unrearranged D_{H} gene segment that is flanked on its 5' side by a first 12-mer RSS and on its 3' side by a second 12-mer RSS (e.g., the unrearranged D_{H} gene segment that is flanked on its 5' side by a 12-mer RSS and on the 3' side by a second 12-mer RSS comprises a germline D_{H} gene segment, e.g., a D_{H} gene segment in its germline configuration, etc.) wherein the unrearranged D_{H} gene segment is upstream of and operably linked to the at least one D_{H} gene segment operably linked to a 5'-end 23-mer. In some embodiments, the unrearranged D_{H} gene segment flanked on its 5' side by a first 12-mer RSS and on its 3' side by a second 12-mer RSS comprises an unrearranged human D_{H} gene segment flanked on its 5'side by a 12-mer RSS and on its 3' side by a second 12-mer RSS.

In some embodiments, a D_{H} gene segment operably linked to a 23-mer RSS comprises from 5' to 3' the D_{H} gene segment and the 23-mer RSS, e.g., comprises from 5' to 3' a 12-mer RSS, a (human) D_{H} gene segment, and a 23-mer RSS, e.g., the 23-mer RSS is contiguous to the 3'-end of the D_{H} gene segment, e.g., the D_{H} gene segment is operably linked to a 3'-end 23-mer, etc. In some embodiments, the D_{H} gene segment operably linked to a 3' end 23-mer RSS comprises a human D_{H}2 gene segment operably linked to a 3'-end 23-mer RSS, wherein the D_{H}2 gene segment is selected from the group consisting of a human D_{H}2-2 gene segment, a human D_{H}2-8 gene segment, and a human D_{H}2-15 gene segment. In some embodiments, a D_{H} gene segment operably linked to a 23-mer RSS comprises from 5' to 3': a human D_{H}2-2 gene segment operably linked to a 3' end 23-mer RSS, a human D_{H}2-8 gene segment operably linked to a 3' end 23-mer RSS, and a human D_{H}2-15 gene segment operably linked to a 3' end 23-mer RSS. In some embodiments, a D_{H} gene segment operably linked to a 3' end 23-mer RSS comprises from 5' to 3': a first contiguous nucleotide sequence comprising a 12-mer RSS, a human D_{H}2-2 gene segment, and a 23-mer RSS, a second contiguous nucleotide sequence comprising a 12-mer RSS, a human D_{H}2-8 gene segment, and a 23-mer RSS, and a third contiguous nucleotide sequence comprising a 12-mer RSS, a human D_{H}2-15 gene segment, and a 23-mer RSS.

Engineered D_{H} regions comprising at least one D_{H} gene segment operably linked to a 3'-end 23-mer RSS may further comprise an unrearranged D_{H} gene segment that is flanked on its 5'side by a first 12-mer RSS and on its 3' other side by a second 12-mer RSS (e.g., the unrearranged D_{H} gene segment that is flanked on its 5' side by a first 12-mer RSS and on the 3' side by a second 12-mer RSS may comprise a germline D_{H} gene segment, e.g., a D_{H} gene segment in its germline configuration, etc.) wherein the unrearranged D_{H} gene segment flanked on its 5' side by a first 12-mer RSS and on its 3' side by a second 12-mer RSS is downstream of and operably linked to the at least one D_{H} gene segment operably linked to a 3'-end 23-mer RSS. In some embodiments, the unrearranged D_{H} gene segment flanked on its 5' side by a first 12-mer RSS and on its 3' side by a second 12-mer RSS comprises an unrearranged human D_{H} gene segment flanked on its 5' side by a first 12-mer RSS and on its 3' side by a second 12-mer RSS.

In some embodiments, an engineered D_{H} region as described herein comprises (i) one or more unrearranged human D_{H} gene segments, wherein each of the one or more of human D_{H} gene segments is flanked on its 5' and 3' ends by a 12-mer RSS, and (ii) at least one D_{H} gene segment operably linked to a 23-mer RSS comprising a human D_{H} gene segment, e.g., a human D_{H}3-3 gene segment) operably linked at its 5'-end to a 23-mer RSS. In some embodiments, an engineered DH region as described herein comprises from 5' to 3'(i) at least one D_{H} gene segment operably linked to a 23-mer RSS, e.g., at least one human D_{H}2 gene segment operably linked at its 3' end to a 23-mer RSS, optionally wherein the at least one human D_{H}2 gene segment operably linked at its 3'end to a 23-mer RSS comprises a human D_{H}2-2 gene segment operably linked at its 3' end to a 23-mer RSS, a human D_{H}2-8 gene segment operably linked at its 3' end to a 23-mer RSS, a human D_{H}2-15 gene segment operably linked at its 3' end to a 23-mer RSS, or any combination thereof, and (ii) one or a plurality of human D_{H} gene segments, wherein each of the one or plurality of human D_{H} gene segments is flanked on its 5' and 3' ends by a 12-mer RSS.

In some embodiments, an engineered D_{H} region as described herein comprises only human D_{H} gene segments.

In some embodiments, a nucleotide molecule described herein (e.g., engineered D_{H} regions, immunoglobulin heavy chain variable regions, etc.) comprises a D_{H} gene segment operably linked to a 23-mer RSS and an unrearranged D_{H} gene segment flanked on its 5 side by a first 12-mer RSS and on its 3' side by a second 12-mer RSS, wherein the D_{H} gene segment operably linked to a 23-mer RSS and the unrearranged D_{H} gene segment flanked on its 5' side by a first 12-mer RSS and on its 3' side by a second 12-mer RSS have not undergone recombination with (i) another D_{H} gene segment, (ii) a V_{H} gene segment, (iii) a J_{H} gene segment, or (iv) any combination thereof. In some embodiments, nucleotide molecules described herein include molecules comprising an engineered D_{H} regions comprising one or more D_{H} gene segments that have recombined with (i) another D_{H} gene segment, (ii) a V_{H} gene segment, (iii) a J_{H} gene segment, or any combination thereof, e.g., nucleotide molecules comprising a rearranged VDJ or VDDJ encoding sequence that encodes an immunoglobulin heavy chain variable region.

Accordingly, in some embodiments, a nucleotide molecule described herein comprising an engineered D_{H} region as described herein further comprises in operable linkage: (a) at least one unrearranged immunoglobulin heavy chain variable (V_{H}) gene segment (e.g., an unrearranged human V_{H}6-1 gene segment) that is upstream of and operably linked to an engineered D_{H} region, (b) at least one unrearranged immunoglobulin heavy chain joining (J_{H}) gene segment (e.g., an unrearranged human J_{H}6) gene segment that is downstream of and operably linked to the engineered D_{H} region, or a combination of (a) and (b).

In some embodiments, the at least one unrearranged V_{H} gene segment comprises the full repertoire of functional unrearranged human V_{H} gene segments spanning between and including the unrearranged human V_{H}3-74 and unrearranged human V_{H}1-6 gene segments. In some embodiments, the at least one unrearranged V_{H} gene segment comprises the full repertoire of functional unrearranged human V_{H} gene segments spanning between and including the unrearranged human V_{H}3-74 and unrearranged human V_{H}1-6 gene segments in germline configuration. In some embodiments, the at least one unrearranged J_{H} gene segment comprises an unrearranged human J_{H}4 gene segment, an unrearranged human J_{H}5 gene segment, and an unrearranged human J_{H}6 gene segment. In some embodiments, the at least one unrearranged J_{H} gene segment comprises an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment. In some embodiments, the at least one unrearranged J_{H} gene segment comprises an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment in germline configuration.

In some embodiments, a nucleotide molecule described herein comprises an immunoglobulin heavy chain variable (V_{H}) region comprising an engineered D_{H} region as described herein, e.g., comprises in operable linkage from 5' to 3':
(a) at least one unrearranged immunoglobulin heavy chain variable (V_{H}) gene segment,
(b) an engineered D_{H} region comprising at least one D_{H} gene segment operably linked to a 23-mer RSS, and
(c) at least one unrearranged immunoglobulin heavy chain joining (J_{H}) gene segment.

In some embodiments,
(a) the at least one unrearranged V_{H} gene segment comprises
   (i) an unrearranged human V_{H}6-1 gene segment,
   (ii) an unrearranged human V_{H}2-1 gene segment and an unrearranged human V_{H}6-1 gene segment and/or
   (iii) all functional unrearranged human V_{H} gene segments spanning between and including the unrearranged human V_{H}3-74 to unrearranged human V_{H}6-1 gene segment, e.g., all functional unrearranged human V_{H} gene segments in germline configuration, optionally, wherein a rodent Adam6 gene replaces a pseudogene between the unrearranged human V_{H}2-1 and V_{H}6-1 gene segments;
(b) the engineered D_{H} region comprises from 5' to 3':
   (i) one or more, e.g., a plurality of, unrearranged human D_{H} gene segments, wherein each of the plurality of unrearranged human D_{H} gene segments is flanked on its 5' and 3' ends by a 12-mer RSS, and a human D_{H} gene segment operably linked at its 5'-end to a 23-mer RSS, optionally wherein the plurality of unrearranged human D_{H} gene segments comprises the unrearranged human D_{H} gene segments spanning between and including the unrearranged human D_{H}1-1 gene segment and unrearranged human D_{H}1-26 gene segment in germline configuration and/or wherein human gene segment operably linked at its 5'-end to a 23-mer RSS is a human D_{H}3-3 gene segment, e.g., wherein the engineered D_{H} comprises the full repertoire of unrearranged human D_{H} gene segments in germline configuration with the exception of the unrearranged human D_{H}7-27 gene segment, which is replaced with the D_{H}3-3 gene segment operably linked to a 5'-end 23-mer RSS;
   (ii) at least one human D_{H} gene segment operably linked at its 3'-end with a 23-mer RSS, and one or more, e.g., a plurality of, human D_{H} gene segments, wherein each of the one or plurality of human D_{H} gene segment(s) is flanked on its 5' and 3' ends by a 12-mer RSS, optionally wherein the at least one human D_{H} gene segment operably linked at its 3'-end with a 23-mer RSS comprises a D_{H}2-2 gene segment operably linked at its 3' end to a 23-mer RSS, a human D_{H}2-8 gene segment operably linked at its 3' end to a 23-mer RSS, a human D_{H}2-15 gene segment operably linked at its 3' end to a 23-mer RSS and/or wherein the one or plurality of human D_{H} gene segment(s) comprises the D_{H} gene segments spanning between and including the unrearranged human D_{H}1-1 gene segment and the unrearranged human D_{H}7-27 gene segment, optionally wherein the engineered D_{H} comprises the full repertoire of unrearranged human D_{H} gene segments in germline configuration with the exception that the unrearranged human D_{H}2-2, D_{H}2-8, and D_{H}2-15 gene segments are respectively replaced with a D_{H}2-2 gene segment operably linked at its 3' end to a 23-mer RSS, a human D_{H}2-8 gene segment operably linked at its 3' end to a 23-mer RSS, and a human D_{H}2-15 gene segment operably linked at its 3' end to a 23-mer RSS;
   (iii) or a combination of (b)(i) and (b)(ii);and
(c) the at least one unrearranged J_{H} gene segment comprises
   (i) an unrearranged human J_{H}6 gene segment,
   (ii) an unrearranged human J_{H}4 gene segment, an unrearranged human J_{H}5 gene segment, and an unrearranged human J_{H}6 gene segment, and/or
   (iii) the full repertoire of unrearranged human J_{H} gene segments, e.g., an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment, optionally wherein the unrearranged human J_{H}1, J_{H}2, J_{H}3, J_{H}4, J_{H}5, and J_{H}6 gene segments are in germline configuration. In some embodiments, the immunoglobulin V_{H} region (comprising at least one functional V_{H} gene segment, the engineered D_{H} region, and at least one functional J_{H} gene segment) is a human immunoglobulin V_{H} region, e.g., each gene segment (e.g., each V_{H}, D_{H}, and J_{H} gene segment therein), including the D_{H} gene segment operably linked to a 23-mer RSS, is a human (V_{H}, D_{H}, or J_{H}) gene segment.

In some embodiments, a nucleotide molecule comprising an immunoglobulin V_{H} region as described herein comprises in operable linkage from 5' to 3'
(a) at least an unrearranged human V_{H}6-1 gene segment, e.g., all or a portion of all the functional unrearranged human V_{H} gene segments spanning between and including the unrearranged human V_{H}3-74 to unrearranged human V_{H}6-1 gene segment, e.g., the full repertoire of functional unrearranged human V_{H} gene segments, optionally including a rodent Adam6 gene between two unrearranged human V_{H} gene segments, e.g., (e.g., wherein the rodent Adam6 gene is located between a human V_{H}1-2 gene segment and a human V_{H}6-1 gene segment)
(b) a human engineered D_{H} region comprising from 5' to 3' the unrearranged human D_{H} gene segments spanning between and including the unrearranged human D_{H}1-1 gene segment and unrearranged human D_{H}1-26 gene segment in germline configuration and an unrearranged human D_{H}3-3 gene segment operably linked to a 5'-end 23-mer RSS, e.g., the full repertoire of unrearranged human D_{H} gene segments in germline configuration with the exception of the unrearranged human D_{H}7-27 gene segment, which is replaced with an unrearranged human D_{H}3-3 gene segment operably linked to a 5'-end 23-mer RSS, and
(c) at least an unrearranged human J_{H}6 gene segment.

In some embodiments, a nucleotide molecule comprising an immunoglobulin V_{H} region as described herein comprises in operable linkage from 5' to 3':
(a) at least an unrearranged human V_{H}6-1 gene segment, e.g., all or a portion of all the functional unrearranged human V_{H} gene segments spanning between and including the unrearranged human V_{H}3-74 to unrearranged human V_{H}6-1 gene segment, e.g., the full repertoire of functional unrearranged human V_{H} gene segments, optionally including a rodent Adam6 gene between two unrearranged human V_{H} gene segments, e.g., (e.g., wherein the rodent Adam6 gene is located between a human V_{H}1-2 gene segment and a human V_{H}6-1 gene segment)
(b) a human engineered D_{H} region comprising from 5' to 3' the unrearranged human D_{H} gene segments spanning between and including the unrearranged human D_{H}1-1 gene segment and unrearranged human D_{H}1-26 gene segment in germline configuration, and an unrearranged human D_{H}3-3 gene segment operably linked to a 5'-end 23-mer RSS, e.g., the full repertoire of unrearranged human D_{H} gene segments in germline configuration with the exception of the unrearranged human D_{H}7-27 gene segment, which is replaced with an unrearranged human D_{H}3-3 gene segment operably linked to a 5'-end 23-mer RSS, and
(c) an unrearranged human J_{H}4 gene segment, an unrearranged human J_{H}5 gene segment, and an unrearranged human J_{H}6 gene segment.

In some embodiments, a nucleotide molecule comprising an immunoglobulin V_{H} region as described herein comprises in operably linkage from 5' to 3'
(a) at least an unrearranged human V_{H}6-1 gene segment, e.g., all or a portion of all the functional unrearranged human V_{H} gene segments spanning between and including the unrearranged human V_{H}3-74 to unrearranged human V_{H}6-1 gene segment, e.g., the full repertoire of functional unrearranged human V_{H} gene segments, optionally including a rodent Adam6 gene between two unrearranged human V_{H} gene segments, e.g., (e.g., wherein the rodent Adam6 gene is located between a human V_{H}1-2 gene segment and a human V_{H}6-1 gene segment)
(b) a human engineered D_{H} region comprising from 5' to 3' an unrearranged human D_{H}1-1 gene segment, a D_{H}2-2 gene segment operably linked at its 3' end to a 23-mer RSS, a human D_{H}2-8 gene segment operably linked at its 3' end to a 23-mer RSS, a human D_{H}2-15 gene segment operably linked at its 3' end to a 23-mer RSS, and the unrearranged human D_{H} gene segments spanning between and including the unrearranged human D_{H}3-16 gene segment and the unrearranged human D_{H}7-27 gene segment, optionally wherein the engineered D_{H} comprises the full repertoire of unrearranged human D_{H} gene segments in germline configuration with the exception that the unrearranged human D_{H}2-2, D_{H}2-8, and D_{H}2-15 gene segments are respectively replaced with a D_{H}2-2 gene segment operably linked at its 3' end to a 23-mer RSS, a human D_{H}2-8 gene segment operably linked at its 3' end to a 23-mer RSS, and a human D_{H}2-15 gene segment operably linked at its 3' end to a 23-mer RSS, and
(c) the full repertoire of unrearranged human J_{H} gene segments, e.g., an unrearranged human J_{H}1 gene segment, an unrearranged J_{H}2 human gene segment, an unrearranged J_{H}3 human gene segment, an unrearranged J_{H}4 human gene segment, an unrearranged J_{H}5 human gene segment, and an unrearranged J_{H}6 human gene segment, optionally wherein the unrearranged human J_{H}1, J_{H}2, J_{H}3, J_{H}4, J_{H}5, and J_{H}6 gene segments are in germline configuration.

In some embodiments, a nucleotide molecule as described herein comprises from 5' to 3' (a) a (human) immunoglobulin heavy chain variable (V_{H}) region comprising at least one (human) V_{H} gene segment, a (human) engineered D_{H} region as described herein, and at least one (human) J_{H} gene segment operably linked to (b) a heavy chain immunoglobulin constant region (C_{H}) or a portion thereof, optionally wherein the C_{H} is a rodent C_{H} that comprises a rodent intronic enhancer region, a rodent IgM gene, a rodent IgD gene, a rodent IgG gene, a rodent IgA gene, a rodent IgE gene, or any combination thereof. In some embodiments, a nucleotide molecule as described herein comprises from 5' to 3' (a) a human immunoglobulin heavy chain V_{H} region comprising at least one human V_{H} gene segment, a human engineered D_{H} region as described herein, and at least one human J_{H} gene segment operably linked to (b) a rodent C_{H} region comprising at least a rodent intronic enhancer region, and optionally a rodent IgM gene. In some embodiments, a nucleotide molecule described herein comprises from 5' to 3' (a) human V_{H} region comprising at least one human V_{H} gene segment, a human engineered D_{H} region as described herein, and at least one human J_{H} gene segment operably linked to (b) a rodent C_{H} region comprising at least a rodent intronic enhancer region and a rodent IgM gene. In some embodiments, a nucleotide molecule described herein comprises from 5' to 3' (a) human V_{H} region comprising at least one human V_{H} gene segment, a human engineered D_{H} region as described herein, and at least one human J_{H} gene segment operably linked to (b) an endogenous rodent C_{H} region, e.g., at an endogenous rodent immunoglobulin heavy chain locus. In some embodiments, the rodent may be a rat. In some embodiments, the rodent may be a mouse.

In some embodiments, a nucleotide molecule as described herein further comprises a rodent Adam6 gene. In some embodiments, the rodent Adam6 gene is located between human V_{H}2-1 and V_{H}6-1 gene segments, e.g., replaces a human Adam6 gene located between a human V_{H}2-1 and V_{H}6-1 gene segment in germline configuration. In some embodiments, the rodent may be a rat. In some embodiments, the rodent may be a mouse.

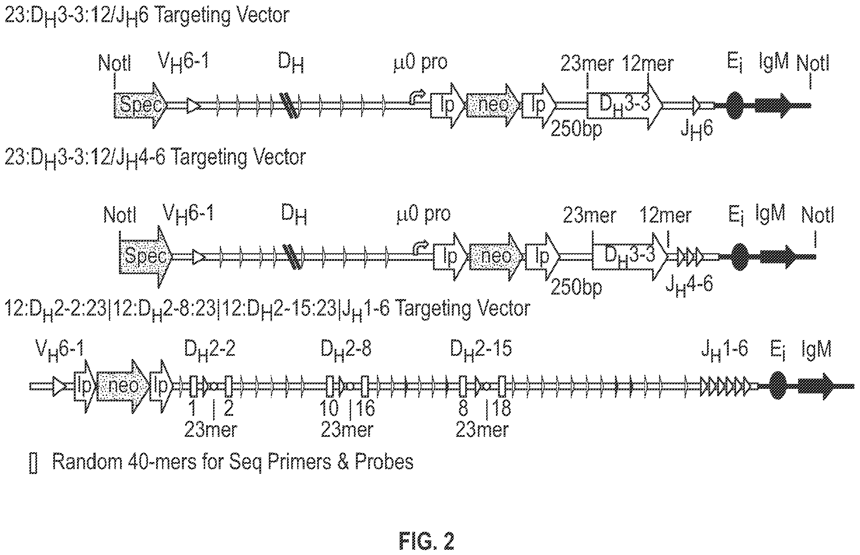

In some embodiments, a nucleotide molecule as described herein comprises one or more drug selection cassettes, e.g., a drug resistance gene flanked by one or more site-specific recombination sites, e.g., a neomycin drug resistance gene flanked by a *lox*P site-specific recombination recognition site, wherein at least one of the one or more drug resistance cassettes is optionally immediately upstream of the at least one D_{H} gene segment operably linked to a 23-mer RSS. In some embodiments the nucleotide molecule comprises a sequence illustrated in Figure 2.

Also described herein are targeting vectors, e.g., for modifying the genome (e.g., the germline genome) of a rodent, such as a rat or a mouse, to comprise an engineered D_{H} region as described herein. Generally, a targeting vector as described herein comprises any of the nucleotide molecules described herein, and optionally comprises 5'- and 3'- homology arms for homologous recombination within immunoglobulin heavy chain variable region, optionally wherein the immunoglobulin heavy chain variable region is a human or humanized immunoglobulin heavy chain variable region. In some embodiments, a targeting vector as described herein comprises a 5' homology arm comprising an unrearranged human gene segment, e.g., a V_{H}6-1 gene segment and/or a 3' homology arm comprising a rodent (e.g., mouse) C_{H} region or portion thereof, e.g., a rodent (mouse) C_{H} intronic enhancer region and/or rodent (mouse) IgM gene.

In some embodiments, a targeting vector comprises a nucleotide molecule as described herein, and 5'- and 3'- homology arms configured to allow homologous recombination with an immunoglobulin heavy chain sequence, which immunoglobulin heavy chain sequence may optionally be located at an endogenous rodent immunoglobulin heavy chain locus and/or comprise a human or humanized immunoglobulin heavy chain variable region. In some embodiments, a targeting vector as described herein comprising a 5' homology arm that comprises an unrearranged human gene segment (e.g., a V_{H}6-1 gene segment), a human engineered D_{H} region, at least one unrearranged human J_{H} gene segment, and a 3' homology arm comprising a rodent (mouse) C_{H} intronic enhancer region and/or rodent (mouse) may be useful in engineering a D_{H} region in a rodent comprising a humanized immunoglobulin heavy chain locus, e.g., a mouse comprising replacement of mouse immunoglobulin variable sequences with human immunoglobulin variable sequences, e.g., VELOCIMMUNE^{®} mice, which may optionally comprise a functional ADAM6 gene that restores their fertility, a modified endogenous heavy chain constant region gene sequence comprising an intact endogenous IgM gene and another endogenous modified constant region gene (e.g., IgG) for the production of reverse chimeric non-IgM antibodies lacking a functional CH1 domain, a sequence encoding a reverse chimeric humanized common light chain, a sequence encoding a reverse chimeric humanized kappa light chain, a sequence encoding a reverse chimeric humanized lambda light chain, a sequence encoding a hybrid kappa/lambda or kappa/lambda light chain, unrearranged germline human heavy gene segments and/or (un)rearranged germline light chain gene segments modified with a histidine codon for the expression of variable domains that have histidine amino acids and may exhibit pH sensitive antigen binding, and/or terminal deoxynucleotidyl transferase (TdT) for increased antigen receptor diversity. *See, e.g.,* U.S. Patent Nos. 9,035,128; 9,066,502; 9,163,092; 9,150,662; 9,334,333; 9,850,462; 9,844,212; 9,029,628; 9,006,511; 9,394,373; 9,206,261; 9,206,262; 9,206,263; 9,226,484; 9,399,683; 9,540,452; 9,012,717; 9,796,788, 8,697,940; 8,754,287; 9,334,334; 9,801,362; 9,332,742; 9,969,814; U.S. Patent Publications 2011/0195454, 2012/0021409, 2012/0192300, 2013/0185821, 2013/0302836, 2013/0045492, and 2018/0125043; PCT Publication Nos. WO2017210586, and WO2019/113065.

As such, described herein are methods of engineering a D_{H} region for D_{H}-D_{H} recombination in a rodent. In some embodiments, the method comprises modifying a D_{H} region comprising one or a plurality of unrearranged D_{H} gene segments to comprise at least one D_{H} gene segment operably linked to a 23-mer RSS. In some embodiments, a method of modifying an immunoglobulin heavy chain variable region to engineer D_{H}-D_{H} recombination comprises obtaining an immunoglobulin heavy chain variable region comprising a D_{H} region comprising one or more unrearranged D_{H} gene segments each of which unrearranged D_{H} gene segments is flanked on its 5' side by a first 12-mer RSS and on the 3' side by a second 12-mer RSS, and modifying the D_{H} region to further comprise at least one D_{H} gene segment operably linked to a 23-mer RSS. In some embodiments, the D_{H} region is a human D_{H} region comprising one or a plurality of unrearranged human D_{H} gene segments, e.g., wherein the plurality of unrearranged human D_{H} gene segments optionally comprises all functional unrearranged human D_{H} gene segments spanning between, and including, the D_{H}1-1 and the D_{H}7-27 gene segments, e.g., in germline configuration. In some embodiments, modifying comprises replacing one or more of the one or plurality of unrearranged D_{H} gene segment flanked on one side by a 12-mer RSS and on the other side by another12-mer RSS, e.g., a functional unrearranged human D_{H} gene segment, with the at least one D_{H} gene segment operably linked to a 23-mer RSS. In some embodiments, the most 3' unrearranged D_{H} gene segment flanked on one side by a 12-mer RSS and on the other side by another 12-mer RSS of the D_{H} region is replaced with the D_{H} gene segment operably linked to a 23-mer RSS, wherein the D_{H} gene segment operably linked to a 23-mer RSS comprises from 5' to 3' the 23-mer RSS, the D_{H} gene segment, and the 12-mer RSS. In some embodiments, an unrearranged D_{H} gene segment flanked on its 5' side by a first 12-mer RSS and on the 3' side by a second 12-mer RSS is replaced with a corresponding D_{H} gene segment engineered to be operably linked to a 23-mer RSS. In some embodiments, wherein the D_{H} region comprises an unrearranged human D_{H}7-27 gene segment (e.g., a germline D_{H}7-27 gene segment), the unrearranged human D_{H}7-27 gene segment is replaced by a human D_{H} gene segment (e.g., an unrearranged human D_{H}3-3 gene segment) operably linked to a 5'-end 23-mer RSS. In some embodiments, wherein the D_{H} region comprises an unrearranged D_{H}2-2 gene segment, an unrearranged D_{H}2-8 gene segment, and/or an unrearranged D_{H}2-15 gene segment (e.g., wherein the engineered D_{H} comprises the full repertoire of unrearranged human D_{H} gene segments in germline configuration) the unrearranged D_{H}2-2 gene segment, the unrearranged D_{H}2-8 gene segment, and/or the unrearranged D_{H}2-15 gene segment are respectively replaced with a D_{H}2-2 gene segment operably linked at its 3' end to a 23-mer RSS, a human D_{H}2-8 gene segment operably linked at its 3' end to a 23-mer RSS, and/or a human D_{H}2-15 gene segment operably linked at its 3' end to a 23-mer RSS. In some embodiments, modifying comprises replacing one of two 12-mer RSS flanking an unrearranged D_{H} gene segment (e.g., a human germline D_{H} gene segment) with a 23-mer RSS. In some embodiments, the immunoglobulin heavy chain variable region to be modified comprises, in addition to a D_{H} region, a J_{H} region (optionally comprising a full repertoire of human germline J_{H} gene segments comprising a human germline J_{H}1 gene segment, a human germline J_{H}2 gene segment, a human germline J_{H}3 gene segment, a human germline J_{H}4 gene segment, a human germline J_{H}5 gene segment, and a human germline J_{H}6 gene segment, optionally wherein the human germline J_{H}1, J_{H}2, J_{H}3, J_{H}4, J_{H}5, and J_{H}6 gene segments are in germline configuration) operably linked to the D_{H} region, and replacing an unrearranged D_{H} gene segment flanked on one side by a 12-mer RSS and on the other side by another12-mer RSS comprises deleting at least one unrearranged J_{H} gene segment comprised in the J_{H} region, e.g., results in deletion of unrearranged human J_{H}1, J_{H}2, J_{H}3, J_{H}4, and/or J_{H}5 gene segments contiguous with the D_{H} region. In some embodiments, deleting at least one germline J_{H} gene segment comprised in the J_{H} region comprises deleting the unrearranged human J_{H}1, J_{H}2, and J_{H}3 gene segments, and optionally further deleting the J_{H}4 and J_{H}5 gene segments. In some embodiments, replacing one or more of the all functional D_{H} gene segments, e.g., replacing a D_{H}7-27 gene segment with a D_{H} gene segment operably linked to a 5'-end 23-mer RSS, e.g., a D_{H}3-3 gene segment operably linked to a 5'-end 23-mer RSS, results in deletion of unrearranged human J_{H}1, J_{H}2, and J_{H}3 gene segments contiguous with the D_{H} region. In some embodiments, replacing one or more of the all functional D_{H} gene segments, e.g., replacing a D_{H}7-27 gene segment with a D_{H} gene segment operably linked to a 5'-end 23-mer RSS, e.g., a D_{H}3-3 gene segment operably linked to a 5'-end 23-mer RSS, results in deletion of unrearranged human J_{H}1, J_{H}2, J_{H}3, J_{H}4, and J_{H}5 gene segments contiguous with the D_{H} region. In some embodiments, a D_{H} region is modified to comprise at least one D_{H} gene segment operably linked to a 23-mer RSS, wherein the at least one D_{H} gene segment operably linked to a 23-mer RSS comprises (a) a human D_{H}3-3 gene segment operably linked to a 23-mer RSS, optionally wherein the 23-mer RSS is contiguous to the 5'-end of the D_{H}3-3 gene segment, (b) a human D_{H}2-2 gene segment operably linked to a 23-mer RSS, optionally wherein the 23-mer RSS is contiguous to the 3'-end of the D_{H}2-2 gene segment, (c) a human D_{H}2-8 gene segment operably linked to a 23-mer RSS, optionally wherein the 23-mer RSS is contiguous to the 3'-end of the D_{H}2-8 gene segment, (d) a human D_{H}2-15 gene segment operably linked to a 23-mer RSS, optionally wherein the 23-mer RSS is contiguous to the 3'-end of the D_{H}2-15 gene segment, or (e) any combination of (a)-(d).

Such methods may result in an immunoglobulin heavy chain variable region comprising an engineered D_{H} region, e.g., a nucleotide molecule comprising a (human) immunoglobulin heavy chain variable region as described herein, wherein the (un)rearranged (human) immunoglobulin heavy chain region may be optionally linked to a non-human immunoglobulin heavy chain constant region or portion thereof, e.g., a rodent immunoglobulin heavy chain constant region comprising at least a rodent C_{H} intronic enhancer region and/or a rodent IgM gene, e.g., optionally at an endogenous non-human immunoglobulin heavy chain locus. In some embodiments, upon recombination, such an immunoglobulin heavy chain locus, e.g., endogenous immunoglobulin heavy chain locus, comprises a rearranged immunoglobulin heavy chain variable region coding sequence encodes an immunoglobulin heavy chain variable domain, e.g., an immunoglobulin heavy chain variable domain having a complementarity determining region 3 (CDR3) amino acid length of more than 20 amino acids, which length may be the result of a V_{H}(D_{H}A-D_{H}B)J_{H}, a V_{H}D_{H}J_{H}6, or a V_{H}(D_{H}A-D_{H}B)J_{H}6 recombination. Accordingly, provided herein are rodents, rodent cells, loci and/or nucleotide molecules comprising a rearranged immunoglobulin heavy chain V_{H}(D_{H}A-D_{H}B)J_{H}, a V_{H}(D_{H})J_{H}6, or a V_{H}(D_{H}A-D_{H}B)J_{H}6 sequence encoding an immunoglobulin heavy chain variable domain comprising a CDR3, wherein the length of the CDR3 is at least 20 amino acids in length.

Also described herein are rodents comprising an engineered D_{H} region of the invention, e.g., the nucleic acids, targeting vectors and/or immunoglobulin heavy chain loci as described herein, e.g., in its genome, e.g., germline genome. Also described herein are such rodent genomes. In some embodiments, described herein is a rodent whose germline genome comprises, or a rodent germline genome comprising, an immunoglobulin heavy chain variable region as described, wherein the immunoglobulin heavy chain variable region comprises: (i) at least one unrearranged heavy chain variable (V_{H}) gene segment, (ii) an engineered heavy chain variable region diversity (D_{H}) region, wherein the engineered D_{H} region comprises one or more unrearranged D_{H} gene segments each flanked on the 5'side by a first 12-mer RSS and on the 3' side by a second 12-mer RSS and one or more D_{H} gene segments each operably linked to a 23-mer recombination signal sequence (RSS), and (iii) at least one unrearranged heavy chain joining (J_{H}) gene segment, wherein (i)-(iii) are in operable linkage such that, upon recombination, the immunoglobulin heavy chain variable region comprises a rearranged heavy chain variable region sequence encoding an immunoglobulin heavy chain variable domain, optionally wherein the rearranged heavy chain variable region sequence is formed after a V_{H}(D_{H}-D_{H})J_{H} recombination event, optionally wherein at least one of the one or more D_{H} gene segments each operably linked to a 23-mer recombination signal sequence (RSS) joins one of the one or more unrearranged D_{H} gene segments each flanked on the 5' side by a first 12-mer RSS and on the 3' side by a second 12-mer RSS during the V_{H}(D_{H}-D_{H}) recombination event. In some embodiments, the one or more D_{H} segments operably linked to a 23-mer RSS comprises a D_{H}gene segment operably linked to a 3' 23-mer RSS. In some embodiments, the one or more D_{H} segments operably linked to a 23-mer RSS comprises a D_{H} gene segment operably linked to a 5' 23-mer RSS. In some embodiments, the immunoglobulin heavy chain variable region is a human immunoglobulin heavy chain variable region comprising only human V_{H}, D_{H}, and J_{H} gene segments. In some embodiments, the human immunoglobulin heavy chain variable region is operably linked to an immunoglobulin heavy chain constant region. In some embodiments, the immunoglobulin heavy chain constant region is an endogenous immunoglobulin heavy chain constant region of the rodent or rodent genome, e.g., at an endogenous immunoglobulin heavy chain locus. In some embodiments, the immunoglobulin heavy chain variable region comprises human V_{H} gene segments spanning between from V_{H}3-74 to V_{H}6-1 in germline configuration. In some embodiments, the immunoglobulin heavy chain variable region comprises a human J_{H}6 gene segment. In some embodiments, the immunoglobulin heavy chain variable region comprises one or more nucleotide molecules encoding one or more rodent Adam6 polypeptides (e.g., a rodent Adam6 gene), which may optionally be inserted between two human V_{H} gene segment (e.g., inserted between a human V_{H}1-2 gene segment and a human V_{H}6-1 gene segment) and/or inserted in the place of a human Adam6 pseudogene. In some embodiments, the rodent genome is heterozygous for the engineered D_{H} region. In some embodiments, the rodent genome is homozygous for the engineered D_{H} region. In some embodiments, the rodent may be a rat. In some embodiments, the rodent may be a mouse. In some embodiments, the rodent, rodent genome, or rodent cell is a rat, a mouse, a rat genome, a mouse genome, a rat cell, or a mouse cell, e.g., a rodent (rat or mouse) embryonic stem cell.

In some embodiments, a rodent, rodent genome, or rodent cell described herein further comprises rearranged heavy chain VDJ and/or V_{H}(D_{H}A-D_{H}B)J_{H} coding sequences encoding immunoglobulin heavy chain variable domains. In some embodiments, described herein is a rodent, e.g., a rat or a mouse, that comprises (1) in its germline genome, e.g., in a germ cell, an immunoglobulin heavy chain locus comprising an engineered D_{H} region comprising a D_{H} gene segment operably linked to a 23-mer RSS, and (2) in its somatic genome, e.g., in a B cell, a rearranged heavy chain variable region V_{H}(D_{H}A-D_{H}B)J_{H} coding sequence, wherein the first or second D_{H} gene segment (D_{H}A or D_{H}B, respectively) is derived from the D_{H} gene segment operably linked to a 23-mer RSS, or a portion thereof (e.g., comprises a sequence identical to that of at least a portion the D_{H} gene segment operably linked to a 23-mer RSS, a somatically hypermutated variant thereof and/or a degenerate variant thereto). In some embodiments, wherein the B cell is a naive B cell and/or the rearranged heavy chain variable region coding sequence is operably linked to an IgM constant region sequence, at least one of D_{H}A and D_{H}B gene segment comprises at least 9 consecutive nucleotides that align with a nucleotide molecule encoded by the D_{H} gene segment operably linked to a 23-mer RSS, and each of the D_{H}A and D_{H}B gene segments comprises at least 5 consecutive nucleotides that align with a nucleotide molecule of a germline D_{H} gene segment. In some embodiments, where the B cell is a plasma or memory B cell and/or the rearranged heavy chain variable region coding sequence is somatically hypermutated and/or operably linked to an non-IgM constant region sequence (e.g., an IgG, IgA, IgE, etc.), each of D_{H}A and D_{H}B respectively shows 40% identity to a first and second germline D_{H} gene segment, with a maximum of 1 nucleotide mutation. In some embodiments, at least 95% of all rearranged heavy chain VDJ and/or V_{H}(D_{H}A-D_{H}B)J_{H} coding sequences in the rodent have a CDR3 length of at least 10 amino acids, optionally wherein at least 70% of all rearranged heavy chain VDJ and/or V_{H}(D_{H}A-D_{H}B)J_{H} coding sequences in the rodent have a CDR3 length of at least 11 amino acids, optionally at least 15% of all rearranged heavy chain VDJ and/or V_{H}(D_{H}A-D_{H}B)J_{H} coding sequences in the rodent have a CDR3 length of at least 14 amino acids. In some embodiments, a population of VDJ and/or V_{H}(D_{H}A-D_{H}B)J_{H} coding sequences in the rodent have a CDR3 length of at least 15 amino acids, optionally at least 16 amino acids, optionally at least 17 amino acids, and optionally at least 18 amino acids. In some embodiments, described herein is a rodent or a rodent cell that expresses, or a nucleic acid or immunoglobulin locus that comprises, a rearranged heavy chain V_{H}(D_{H}A-D_{H}B)J_{H} coding sequence, wherein the second germline D_{H} gene segment is D_{H}3-3, optionally wherein the rearranged heavy chain V_{H}(D_{H}A-D_{H}B)J_{H} coding sequence encodes a CDR3 length of over 20 amino acids. In some embodiments, described herein is a rodent or rodent cell that expresses, or a nucleic acid or immunoglobulin locus that comprises, a rearranged heavy chain V_{H}(D_{H}A-D_{H}B)J_{H} coding sequence, wherein the first germline D_{H} gene segment is D_{H}2-2, D_{H}2-8 or D_{H}2-15, optionally wherein the rearranged heavy chain V_{H}(D_{H}A-D_{H}B)J_{H} coding sequence encodes a CDR3 length of over 20 amino acids. In some embodiments, described herein is a rodent or rodent cell that expresses a rearranged heavy chain V_{H}(D_{H}A-D_{H}B)J_{H}6 coding sequence encoding CDR3 length of over 20 amino acids.

Also disclosed is a rodent genome, nucleic acid or immunoglobulin locus comprising a rearranged human immunoglobulin heavy chain V_{H}(D_{H}A-D_{H}B)J_{H} coding sequence operably linked to a rodent immunoglobulin heavy chain constant region sequence. In some instance, the D_{H}B gene segment is derived from a human germline D_{H}3-3 gene segment. In some instances the D_{H}A gene segment is derived from a human germline D_{H}2-2, D_{H}2-8 or D_{H}2-15 gene segment. In some instances the rearranged heavy chain is determined to be a V_{H}(D_{H}A-D_{H}B)J_{H} coding sequence since each of D_{H}A and D_{H}B respectively shows 40% identity to a first and second germline D_{H} gene segment, with a maximum of 1 nucleotide mutation. In some instances, the rearranged heavy chain V_{H}(D_{H}A-D_{H}B)J_{H} coding sequence encodes a CDR3 length of over 20 amino acids. In some instances, the J_{H} gene segment is derived from a human germline J_{H}6 gene segment. In some instances, a rodent or rodent cell comprising a V_{H}(D_{H}A-D_{H}B)J_{H} coding sequence is provided. In some instances, the rodent is a rat or a mouse or the rodent cell is a rat cell or a mouse cell. In some embodiments, the rodent cell is a rodent B cell. In some instances, a hybridoma comprising a rodent B cell expressing a rearranged heavy chain V_{H}(D_{H}A-D_{H}B)J_{H} coding sequence fused with a myeloma cell is provided. In some instances, a V_{H}(D_{H}A-D_{H}B)J_{H} sequence is confirmed to be the result of a D_{H}-D_{H} recombination event when the sequences identified as D_{H}A and D_{H}B each respectively shows 40% identity to a first and second germline D_{H} gene segment, with a maximum of 1 nucleotide mutation.

Also disclosed is a rodent or rodent cell, whose genome comprises an immunoglobulin heavy chain variable region that includes an engineered D_{H} region, wherein the engineered D_{H} region comprises at least one D_{H} segment that is operably linked to a first and second recombination signal sequences (RSS). In some embodiments, the first RSS is a 23-mer RSS and the second RSS is a 12-mer RSS. In some embodiments, the first RSS is a 12-mer RSS and the second RSS is a 23-mer RSS.

Also disclosed is a rodent, whose genome comprises an immunoglobulin heavy chain variable region that includes an engineered D_{H} region, which engineered D_{H} region comprises one or more D_{H} segments that are each operably linked to a 23-mer recombination signal sequence (RSS).

In some embodiments, a rodent cell comprising the rodent germline genome of the present invention is provided, preferably wherein the rodent cell is a rat, a mouse, a rat cell, or a mouse cell. In some embodiments, a cell is from a lymphoid or myeloid lineage. In some embodiments, a cell is a lymphocyte. In some embodiments, a cell is selected from a B cell, dendritic cell, macrophage, monocyte, and a T cell. In some embodiments, a tissue is selected from adipose, bladder, brain, breast, bone marrow, eye, heart, intestine, kidney, liver, lung, lymph node, muscle, pancreas, plasma, serum, skin, spleen, stomach, thymus, testis, ovum, or any combination thereof.

In some embodiments, an immortalized cell made from a rodent cell as described herein is provided, e.g., a hybridoma cell made from fusing a B cell isolated from a rodent as described herein with a myeloma cell.

In some embodiments, a rodent cell is a rodent embryonic stem (ES) cell. In some embodiments, a rodent embryonic stem cell is a mouse embryonic stem cell and is from a 129 strain, C57BL strain, or a mixture thereof. In some embodiments, a rodent embryonic stem cell is a mouse embryonic stem cell and is a mixture of 129 and C57BL strains.

In some embodiments, use of a rodent stem cell as described herein to make a non-human animal is provided. In some embodiments, a rodent embryonic stem cell is a mouse embryonic stem cell and is used to make a mouse of the present invention. In some embodiments, a rodent embryonic stem cell is a rat embryonic stem cell and is used to make a rat comprising an immunoglobulin heavy chain variable region that includes an engineered D_{H} region as described herein. In some embodiments, a non-limiting exemplary method for making the rat comprising an immunoglobulin heavy chain variable region that includes an engineered D_{H} region can include the methods disclosed in US20140309487.

In some embodiments, a rodent embryo comprising, made from, obtained from, or generated from a rodent embryonic stem cell as described herein is provided. In some embodiments, a rodent embryo is a mouse embryo; in some embodiments, a rat embryo.

In some embodiments, use of a rodent embryo described herein to make a rodent of the present invention is provided. In some embodiments, a rodent embryo is a mouse embryo and is used to make a mouse of the present invention comprising an immunoglobulin heavy chain variable region that includes an engineered D_{H} region as described herein. In some embodiments, a rodent embryo is a rat embryo and is used to make a rat of the present invention comprising an immunoglobulin heavy chain variable region that includes an engineered D_{H} region as described herein.

Provided herein is a method of making a rodent whose genome comprises an engineered D_{H} region, the method comprising:
(A) generating a rodent from the embryonic stem cell of the present invention, or
(B)
   (a) modifying the genome of a rodent embryonic stem cell to comprise a DNA fragment comprising one or more engineered D_{H} gene segments each comprising a D_{H} gene segment immediately adjacent and contiguous to a 23-mer RSS, optionally wherein the DNA fragment comprises the nucleotide molecule of the present invention, the targeting vector of the present invention, the immunoglobulin heavy chain variable region modified according to the method of the present invention, or the immunoglobulin heavy chain of the present invention, and
   (b) generating a rodent using the modified rodent embryonic stem cell of (a).
In some embodiments, modifying comprises replacing the one or more unrearranged human D_{H} gene segments, and optionally replacing or deleting one or more J_{H} gene segments, with the at least one D_{H} segments operably linked to a 23-mer RSS. In some embodiments, the immunoglobulin heavy chain variable region is a human immunoglobulin heavy chain variable region, e.g., wherein the human immunoglobulin heavy chain variable region comprises an unrearranged human immunoglobulin heavy chain V_{H} gene cluster comprising at least one V_{H} gene segment, an unrearranged human immunoglobulin heavy chain D_{H} region comprising one or more unrearranged human D_{H} gene segments, and an unrearranged human immunoglobulin heavy chain J_{H} gene cluster comprising at one unrearranged human J_{H} gene segment, wherein the unrearranged human immunoglobulin heavy chain D_{H} region is modified to comprise at least one D_{H} segment operably linked to a 23-mer RSS. In some embodiments, the modifying step results in the immunoglobulin heavy chain variable region comprising (i) the full repertoire of functional human V_{H} gene segments, e.g., all functional V_{H} gene segments spanning between and including V_{H}3-74 to V_{H}6-1, (ii) the full repertoire of unrearranged human D_{H} gene segments except for D_{H}7-27 which is replaced with the at least one D_{H} gene segment operably linked to a 23-mer RSS, and (iii) at least an unrearranged human J_{H}6 gene segment, and optionally at least an unrearranged J_{H}4 gene segment, an unrearranged J_{H}5 gene segment, and an unrearranged J_{H}6 gene segment. In some embodiments, the at least one D_{H} gene segment operably linked to a 23-mer RSS comprises a D_{H}3-3 gene segment operably linked to a 5' - 23-mer RSS. In some embodiments, the modifying step results in the immunoglobulin heavy chain variable region comprising (i) a full repertoire of functional human V_{H} gene segments, e.g., all functional V_{H} gene segments spanning between and including V_{H}3-74 to V_{H}6-1, (ii) the full repertoire of unrearranged human D_{H} gene segments except that the unrearranged human D_{H}2-2, D_{H}2-8, and D_{H}2-15 gene segments are respectively replaced with a D_{H}2-2 gene segment operably linked at its 3' end to a 23-mer RSS, a human D_{H}2-8 gene segment operably linked at its 3' end to a 23-mer RSS, and a human D_{H}2-15 gene segment operably linked at its 3' end to a 23-mer RSS, and (iii) the full repertoire of unrearranged human J_{H} gene segments, e.g., an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment. In some embodiments, the immunoglobulin heavy chain variable region (a) further comprises one or more rodent Adam6 genes, optionally wherein the one or more rodent Adam6 genes are located between two unrearranged V_{H} gene segments, e.g., between an unrearranged human V_{H}1-2 gene segment and an unrearranged human V_{H}6-1 gene segment, and/or (b) is operably linked to an immunoglobulin heavy chain constant region, optionally wherein the immunoglobulin heavy chain constant region is an endogenous rodent immunoglobulin heavy chain constant region, e.g., an endogenous rodent immunoglobulin heavy chain constant region at an endogenous immunoglobulin heavy chain locus. In some embodiments, the rodent is a rat or a mouse.

Also disclosed is a kit, comprising a rodent as described herein, a rodent cell or tissue as described herein, an immortalized cell as described herein, a rodent embryonic stem cell as described herein, or a rodent as described herein.

Also disclosed is a kit, for use in the manufacture and/or development of a drug (e.g., an antibody or antigen-binding fragment thereof) for therapy or diagnosis. In some instances, a kit as described herein is provided, for use in the manufacture and/or development of a drug (e.g., an antibody or antigen-binding fragment thereof) for the treatment, prevention or amelioration of a disease, disorder or condition.

In some embodiments, a transgene, nucleic acid construct, DNA construct, or targeting vector as described herein is provided that comprises an engineered D_{H} region as described herein. In some embodiments, a transgene, nucleic acid construct, DNA construct, or targeting vector further comprises one or more selection markers. In some embodiments, a transgene, nucleic acid construct, DNA construct, or targeting vector further comprises one or more site-specific recombination sites (e.g., *lox***P**, Frt, or combinations thereof). In some embodiments, a transgene, nucleic acid construct, DNA construct, or targeting vector is depicted in Figure 2.

In some embodiments, use of a transgene, nucleic acid construct, DNA construct, or targeting vector as described herein to make a rodent, rodent cell, rodent embryonic stem cell, and/or rodent embryo is provided.

In some embodiments, one or more D_{H} segments are each operably linked to a 3' 23-mer RSS. In some embodiments, one or more D_{H} segments are each operably linked to a 5' 23-mer RSS.

In some embodiments, an engineered D_{H} region comprises one D_{H} segment operably linked to a 5' 23-mer RSS. In some embodiments, an engineered D_{H} region comprises one D_{H} segment operably linked to a 3' RSS. In some embodiments of one D_{H} segment operably linked to a 5' 23-mer RSS, the one D_{H} segment is a synthetic D_{H} segment; in some embodiments, a synthetic human D_{H} segment; in some embodiments, a synthetic human D_{H} segment having a sequence that is identical or substantially identical to a human D_{H}3-3 segment. In some embodiments of one D_{H} segment operably linked to a 3' 23-mer RSS, the one D_{H} segment is a synthetic D_{H} segment; in some embodiments, a synthetic human D_{H} segment; in some embodiments, a synthetic human D_{H} segment having a sequence that is identical or substantially identical to a human D_{H}3-3 segment.

In some embodiments, an engineered D_{H} region comprises three D_{H} segments each operably linked to a 5' 23-mer RSS. In some embodiments of three D_{H} segments each operably linked to a 5' 23-mer RSS, the three D_{H} segments are synthetic D_{H} segments. In some embodiments of three D_{H} segments each operably linked to a 5' 23-mer RSS, the three D_{H} segments are human D_{H}2 family segments. In some embodiments of three D_{H} segments each operably linked to a 5' 23-mer RSS, the three D_{H} segments are selected from human D_{H}2-2, human D_{H}2-8, human D_{H}2-15, human D_{H}2-21 and combinations thereof. In some embodiments of three D_{H} segments each operably linked to a 5' 23-mer RSS, the three D_{H} segments are human D_{H}2-2, human D_{H}2-8 and human D_{H}2-15.

In some embodiments, an engineered D_{H} region comprises three D_{H} segments each operably linked to a 3' 23-mer RSS. In some embodiments of three D_{H} segments each operably linked to a 3' 23-mer RSS, the three D_{H} segments are synthetic D_{H} segments. In some embodiments of three D_{H} segments each operably linked to a 3' 23-mer RSS, the three D_{H} segments are human D_{H}2 family segments. In some embodiments of three D_{H} segments each operably linked to a 3' 23-mer RSS, the three D_{H} segments are selected from human D_{H}2-2, human D_{H}2-8, human D_{H}2-15, human D_{H}2-21 and combinations thereof. In some embodiments of three D_{H} segments each operably linked to a 3' 23-mer RSS, the three D_{H} segments are human D_{H}2-2, human D_{H}2-8 and human D_{H}2-15.

In some embodiments, an engineered D_{H} region as described herein includes a plurality of human D_{H} segments, wherein at least one of the plurality of human D_{H} gene segments is operably linked to a 5' or a 3' RSS; in some embodiments, a 5' 23-mer RSS. In some embodiments, an engineered D_{H} region as described herein includes a plurality of human D_{H} segment, wherein at least three of the plurality of human D_{H} gene segments are each operably linked to a 5' or a 3' RSS; in some embodiments, a 3' 23-mer RSS.

In some embodiments, the genome of a provided rodent, rodent cell or rodent tissue lacks one or more wild-type D_{H} segments. In some embodiments, the genome of a provided rodent, rodent cell or rodent tissue lacks all or substantially all wild-type D_{H} segments. In some embodiments, the genome of a provided rodent, rodent cell or rodent tissue contains only human D_{H} segments.

In some embodiments, an immunoglobulin heavy chain variable region is a human immunoglobulin heavy chain variable region. In some embodiments, a human immunoglobulin heavy chain variable region is operably linked to an immunoglobulin heavy chain constant region. In some embodiments, an immunoglobulin heavy chain constant region is an endogenous (e.g., non-human) immunoglobulin heavy chain constant region.

In some embodiments, a human immunoglobulin heavy chain variable region includes the human V_{H} gene segments from V_{H}3-74 to V_{H}6-1. In some embodiments, a human immunoglobulin heavy chain variable region includes at least human J_{H} gene segment J_{H}6. In some embodiments, a human immunoglobulin heavy chain variable region includes at least human J_{H} gene segments J_{H}4, J_{H}5 and J_{H}6. In some embodiments, a human immunoglobulin heavy chain variable region includes human J_{H} gene segments J_{H}1, J_{H}2, J_{H}3, J_{H}4, J_{H}5 and J_{H}6.

In some embodiments of a rodent, rodent cell or rodent tissue, the genome lacks an endogenous Adam6 gene. In some embodiments of a rodent animal, rodent cell or rodent tissue, the genome further comprises insertion of one or more nucleotide sequences encoding one or more rodent Adam6 polypeptides; in some embodiments, the one or more nucleotide sequences are inserted between a first and a second human V_{H} gene segment; in some embodiments, the one or more nucleotide sequences are inserted in the place of a human Adam6 pseudogene; in some embodiments, the one or more nucleotide sequences are inserted between a human V_{H} gene segment and a human D_{H} gene segment. In some embodiments, a first human V_{H} gene segment is human V_{H}1-2 and a second human V_{H} gene segment is human V_{H}6-1.

In some embodiments, a provided rodent, rodent cell or rodent tissue is homozygous, heterozygous or hemizygous for an engineered D_{H} region as described herein. In some embodiments, a provided rodent, rodent cell or rodent tissue is transgenic for an engineered D_{H} region as described herein.

In some embodiments, a method of making a rodent whose genome comprises an immunoglobulin heavy chain variable region that includes an engineered D_{H} region is provided, the method comprising (a) inserting a DNA fragment into a rodent embryonic stem cell, which DNA fragment comprises one or more D_{H} segments that are each operably linked to a 23-mer RSS; (b) obtaining the rodent embryonic stem cell generated in (a); and (c) creating a rodent using the rodent embryonic stem cell of (b).

In some embodiments, a DNA fragment comprises one or more D_{H} segments are each operably linked to a 3' 23-mer RSS. In some embodiments, a DNA fragment comprises one D_{H} segment operably linked to a 3' 23-mer RSS. In some embodiments, a DNA fragment comprises one synthetic human D_{H} segment operably linked to a 3' 23-mer RSS. In some embodiments, a DNA fragment comprises one synthetic human D_{H}3-3 segment operably linked to a 3' 23-mer RSS. In some embodiments, a DNA fragment comprises three D_{H} segments each operably linked to a 3' 23-mer RSS. In some embodiments, a DNA fragment comprises three human D_{H} segments each operably linked to a 3' 23-mer RSS. In some embodiments, a DNA fragment comprises three human D_{H} segments each operably linked to a 3' 23-mer RSS, which human D_{H} segments are human D_{H}2-2, human D_{H}2-8 and human D_{H}2-15.

In some embodiments, a DNA fragment comprises one or more D_{H} segments, each of which is operably linked to a 5' 23-mer RSS. In some embodiments, a DNA fragment comprises one D_{H} segment operably linked to a 5' 23-mer RSS. In some embodiments, a DNA fragment comprises one synthetic human D_{H} segment operably linked to a 5' 23-mer. In some embodiments, a DNA fragment comprises one synthetic human D_{H}3-3 segment operably linked to a 5' 23-mer RSS. In some embodiments, a DNA fragment comprises one synthetic human D_{H}3-3 segment operably linked to a 5' 23-mer RSS, which synthetic human D_{H}3-3 segment is positioned in a human D_{H} region in the place of a human D_{H}7-27 segment.

In some embodiments, a DNA fragment comprises one or more selection markers. In some embodiments, a DNA fragment comprises one or more site-specific recombination sites.

Also disclosed is a method of making a rodent whose genome comprises an immunoglobulin heavy chain variable region that includes an engineered D_{H} region, the method comprising a step of modifying the genome of a rodent or rodent cell so that it comprises an immunoglobulin heavy chain variable region that includes an engineered D_{H} region, which engineered D_{H} region comprises one or more D_{H} segments that are each operably linked to a 23-mer RSS, thereby making rodent.

In some embodiments of making a rodent, the genome of the rodent or rodent cell is modified to include one or more D_{H} segments that are each operably linked to a 5' 23-mer RSS. In some embodiments of making a rodent, the genome of the rodent or rodent cell is modified to include one or more D_{H} segments that are each operably linked to a 3' 23-mer RSS.

In some embodiments, a method of producing an antibody or obtaining a nucleic acid encoding same is provided, the method comprising:
immunizing a rodent with an antigen, which rodent comprises a germline genome comprising an engineered D_{H} region that comprises one or more D_{H} segments that are each immediately adjacent to a 23-mer RSS, optionally wherein the rodent is the rodent of the present invention or made according to the present invention, and
allowing the rodent to produce an immune response to the antigen including an antibody, or nucleic acid encoding same, that binds the antigen.
preferably wherein:
   (a) the method is one further comprising recovering the antibody, or nucleic acid encoding same, from the rodent or a rodent cell or wherein the rodent cell is a B cell or a hybridoma;
   (b) one or more D_{H} segments that are each immediately adjacent to a 23-mer RSS comprises a D_{H} gene segment operably linked to a 5' 23-mer RSS;
   (c) one or more D_{H} segments that are each immediately adjacent to a 23-mer RSS comprises a D_{H} gene segment operably linked to a 3' 23-mer RSS; and/or
   (d) the rodent is a rat or a mouse.

In some embodiments, the method further comprises recovering the antibody, or nucleic acid encoding same, from the rodent or a rodent cell, e.g., is a B cell or a hybridoma. In some embodiments, the one or more D_{H} segments are each operably linked to a 5' 23-mer RSS. In some embodiments, one or more D_{H} segments are each operably linked to a 3' 23-mer RSS.

In some embodiments, the method comprising the steps of (a) immunizing a rodent animal with an antigen, which rodent has a genome comprising an immunoglobulin heavy chain variable region that includes an engineered D_{H} region, which engineered D_{H} region comprises one or more D_{H} segments that are each operably linked to a 23-mer RSS; (b) maintaining the rodent under conditions sufficient that the rodent produces an immune response to the antigen; and (c) recovering an antibody from the non-human animal, or a non-human animal cell, that binds the antigen. In some embodiments, a rodent cell is a B cell. In some embodiments, a rodent cell is a hybridoma.

Also disclosed is a rodent whose genome comprises a human immunoglobulin heavy chain variable region that comprises one or more human V_{H} gene segments, an engineered D_{H} region that includes at least one human D_{H} segment operably linked to a 23-mer RSS, and at least one human J_{H} gene segment, wherein the human immunoglobulin heavy chain variable region is operably linked to one or more endogenous immunoglobulin constant region genes so that the rodent is characterized in that when it is immunized with an antigen, it generates antibodies comprising human heavy chain variable domains that include CDR3 regions generated by human D_{H}-D_{H} recombination and/or enhanced recombination to a J_{H}6 gene segment, and wherein the antibodies show specific binding to the antigen. In some instances, the at least one human D_{H} segment operably linked to a 23-mer RSS is positioned in the place of a human D_{H}7-27 segment. In some instances, a human immunoglobulin heavy chain variable region comprises less than all six human J_{H} gene segments. In some instances, a human immunoglobulin heavy chain variable region comprises only one of the six human J_{H} gene segment. In some instances, a human immunoglobulin heavy chain variable region comprises the human J_{H}6 gene segment and lacks a functional J_{H}1 gene segment, lacks a functional J_{H}2 gene segment, lacks a functional J_{H}3 gene segment, lacks a functional J_{H}4 gene segment, and lacks a functional J_{H}5 gene segment. In some instances, a human immunoglobulin heavy chain variable region comprises only three of the six human J_{H} gene segments. In some instances, a human immunoglobulin heavy chain variable region comprises only human J_{H}4 gene segment, the human J_{H}5 gene segment, and the human J_{H}6 gene segment, and lacks functional J_{H}1 gene segment, lacks a functional J_{H}2 gene segment, and lacks a functional J_{H}3 gene segment. In some instances, wherein a rodent whose genome comprises a human immunoglobulin heavy chain variable region comprising less than all six human J_{H} gene segments, e.g., a human immunoglobulin heavy chain variable region that comprises only one of the six human J_{H} gene segments (e.g., a human immunoglobulin heavy chain variable region that comprises the human J_{H}6 gene segment and lacks a functional J_{H}1 gene segment, lacks a functional J_{H}2 gene segment, lacks a functional J_{H}3 gene segment, lacks a functional J_{H}4 gene segment, and lacks a functional J_{H}5 gene segment), exhibits enhanced recombination to a J_{H}6 gene segment compared to a control rodent whose genome comprises a human immunoglobulin heavy chain variable region comprising all six human J_{H} gene segments, e.g., the rodent comprises a greater percentage of rearranged immunoglobulin heavy chain sequences comprising the J_{H}6 gene segment sequence or portion thereof and/or encoding a CDR3 length of at least 20 amino acids thereof than the control non-human animal. In some instances, wherein a rodent whose genome comprises a human immunoglobulin heavy chain variable region comprising less than all six human J_{H} gene segments, e.g., a human immunoglobulin heavy chain variable region that comprises only three of the six human J_{H} gene segment (e.g., a human immunoglobulin heavy chain variable region that comprises a human immunoglobulin heavy chain variable region comprises human J_{H}4 gene segment, the human J_{H}5 gene segment, and the human J_{H}6 gene segment, and lacks functional J_{H}1 gene segment, lacks a functional J_{H}2 gene segment, and lacks a functional J_{H}3 gene segment), exhibits enhanced recombination to a J_{H}6 gene segment compared to a control rodent whose genome comprises a human immunoglobulin heavy chain variable region comprising all six human J_{H} gene segments, e.g., the rodent comprises a greater percentage of rearranged immunoglobulin heavy chain sequences comprising the J_{H}6 gene segment sequence or portion thereof and/or encoding a CDR3 length of at least 20 amino acids compared than the control rodent.

Also disclosed is a rodent whose genome comprises a human immunoglobulin heavy chain variable region that comprises one or more human V_{H} gene segments, an engineered D_{H} region that includes at least one human D_{H} segment flanked 3' by a 23-mer RSS, and at least two human J_{H} gene segments, wherein the human immunoglobulin heavy chain variable region is operably linked to one or more endogenous immunoglobulin constant region genes so that the rodent is characterized in that when it is immunized with an antigen, it generates antibodies comprising human heavy chain variable domains that include CDR3 regions generated by human D_{H}-D_{H} recombination, and wherein the antibodies show specific binding to the antigen. In some instances, an engineered D_{H} region includes at least three human D_{H} segments that are each flanked 3' by a 23-mer RSS. In some instances, an engineered D_{H} region includes three human D_{H} segments that are each flanked 3' by a 23-mer RSS, which three human D_{H} segments are human D_{H}2-2, human D_{H}2-8 and human D_{H}2-15. In some instances, a human immunoglobulin heavy chain variable region includes the human V_{H} gene segments from V_{H}3-74 to V_{H}6-1.

In some embodiments, the antigen is a pathogen, e.g., a bacterial, fungal, or viral pathogen. In some embodiments, immunization of a rodent herein comprises infecting the rodent with a pathogen, e.g., a bacterial, fungal, or viral pathogen. In some embodiments, immunization of a rodent herein comprises administering to the rodent genomic or proteinaceous material isolated from the pathogen, e.g., bacterial, fungal, or viral pathogen. In some embodiments, the antigen is a receptor (e.g., a complement receptor, a chemokine receptor, etc.), or portion thereof. In some embodiments, the antigen is a nucleic acid encoding the receptor, or portion thereof. In some embodiments, the antigen is a cell expressing the receptor, or portion thereof. In some embodiments, the antigen is an ion channel, or portion thereof. In some embodiments, the antigen is a nucleic acid encoding the ion channel or portion thereof. In some embodiments, the antigen is a cell expressing the ion channel, or portion thereof.

In some embodiments, a provided rodent, rodent cell or rodent tissue has a genome that further comprises an insertion of one or more human V_{L} gene segments and one or more human J_{L} gene segments into an endogenous light chain locus. In some embodiments, human V_{L} and J_{L} segments are Vκ and Jκ gene segments and are inserted into an endogenous κ light chain locus. In some embodiments, human Vκ and Jκ gene segments are operably linked to a rodent Cκ gene (e.g. a mouse or a rat Cκ gene). In some embodiments, human V_{L} and J_{L} segments are Vλ and Jλ gene segments and are inserted into an endogenous λ light chain locus. In some embodiments, human Vλ and Jλ gene segments are operably linked to a rodent Cλ gene (e.g., a mouse or a rat Cλ gene).

Also disclosed is the use of a rodent, rodent cell or rodent tissue as described herein in the manufacture and/or development of a drug or vaccine for use in medicine, such as use as a medicament. In some embodiments, use of a rodent, rodent cell or rodent tissue as described herein in the manufacture and/or development of an antibody for administration to humans is provided. Also disclosed is the use of a rodent, rodent cell or rodent tissue as described herein in the manufacture of a medicament for the treatment, prevention or amelioration of a disease, disorder or condition.

Also disclosed is a rodent, rodent cell or rodent tissue as described herein for use in the manufacture and/or development of a drug for therapy or diagnosis. Also disclosed is a rodent animal, rodent cell or rodent tissue as described for use in the manufacture of a medicament for the treatment, prevention or amelioration of a disease, disorder or condition.

In some embodiments, a rodent provided is a mouse; in some embodiments, a rat. In many embodiments, a rodent cell is a mouse cell; in some embodiments, a rat cell. In many embodiments, a rodent tissue provided herein is a mouse tissue; in some embodiments, a rat tissue.

In some embodiments, a 23-mer RSS comprises a nucleotide sequence comprising a sequence set forth as SEQ ID NO: 151.

### BRIEF DESCRIPTION OF THE DRAWING

The Drawing included herein, which is composed of the following Figures, is for illustration purposes only and not for limitation.

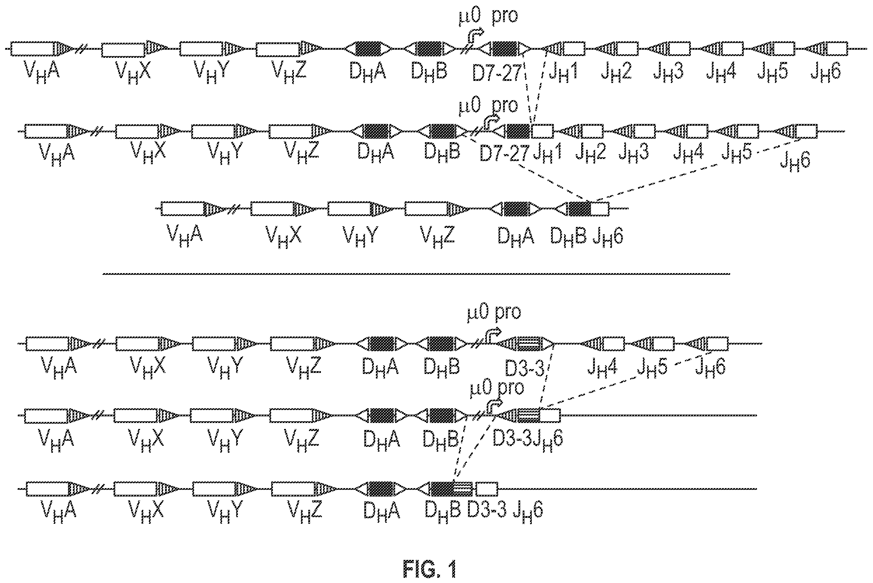

***Figure 1*** shows a general illustration, not to scale, of embodiments of the present invention showing ordered assembly of gene segments in a DJ recombination event for immunoglobulin heavy chain variable region gene segments with an unmodified D_{H} region (top panel) and engineered D_{H} region (bottom panel). 12-mer recombination signal sequences (RSS) are depicted as unfilled triangles. 23-mer RSS are depicted as triangles with vertical stripes. Illustrative unmodified V_{H} gene segments (unfilled boxes) and D_{H} gene segments (filled boxes) are respectively depicted as unfilled and filled boxes and are provided generic nomenclature using letters of the alphabet. D gene segments (e.g., D_{H}3-3) operably linked to a 23-mer RSS and unmodified J_{H} gene segments are respectively depicted as boxes filled with horizontal stripes and unfilled boxes and provided their proper nomenclature. Also shown is the µ0 promoter (µ 0 pro). Not depicted is recombination of a V_{H} gene segment to the recombined DJ gene segment.
***Figure 2*** shows illustrations, not to scale, of exemplary embodiments of targeting vectors made according to Example 1. Hash lines represent D_{H} gene segments that are included in the targeting vector but are not specifically depicted.

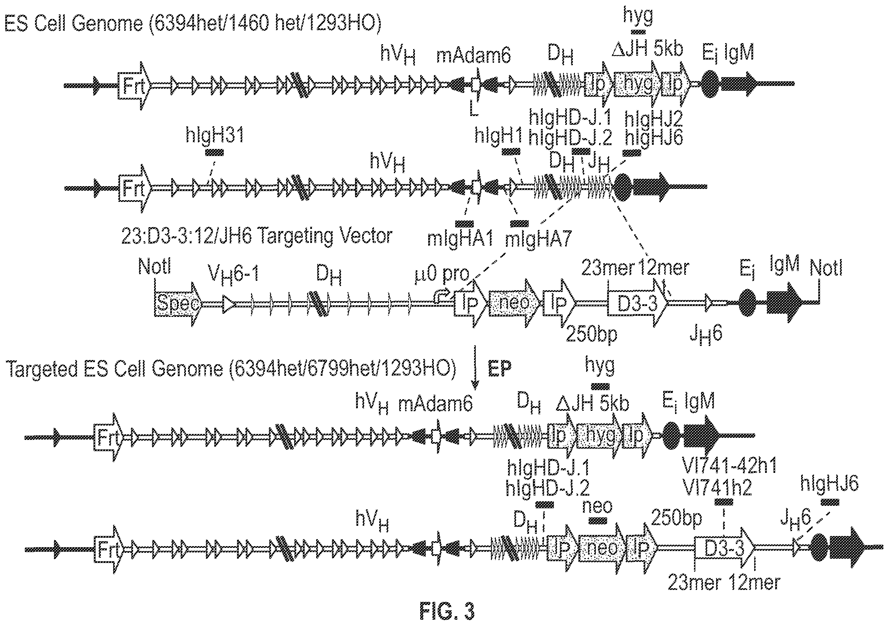

***Figure 3*** shows an illustration, not to scale, of a non-limiting exemplary embodiment of inserting the 23:D_{H}3-3:12/J_{H}6 targeting vector into a humanized immunoglobulin heavy chain variable region locus in the genome of mouse ES cells via electroporation (EP). Hash lines represent V_{H} gene segments or D_{H} gene segments that are included in the heavy chain variable region locus but are not specifically depicted.

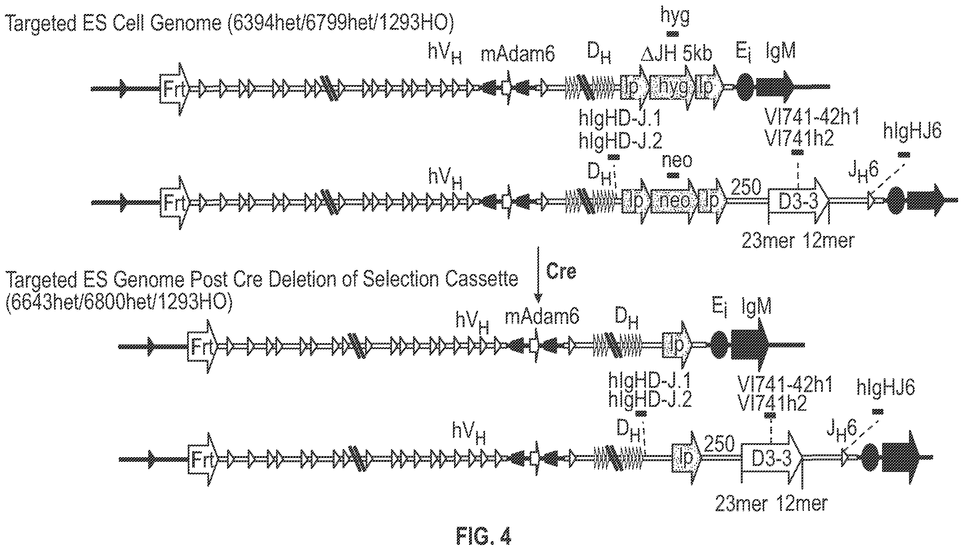

***Figure 4*** shows an illustration, not to scale, of a non-limiting exemplary embodiment of Cre-mediated deletion of selection cassettes in humanized immunoglobulin heavy chain loci after electroporation and integration of the 23:D_{H}3-3:12/J_{H}6 targeting vector as described in Examples 1 and 2. Hash lines represent V_{H} gene segments or D_{H} gene segments that are included in the heavy chain variable region locus but are not specifically depicted.

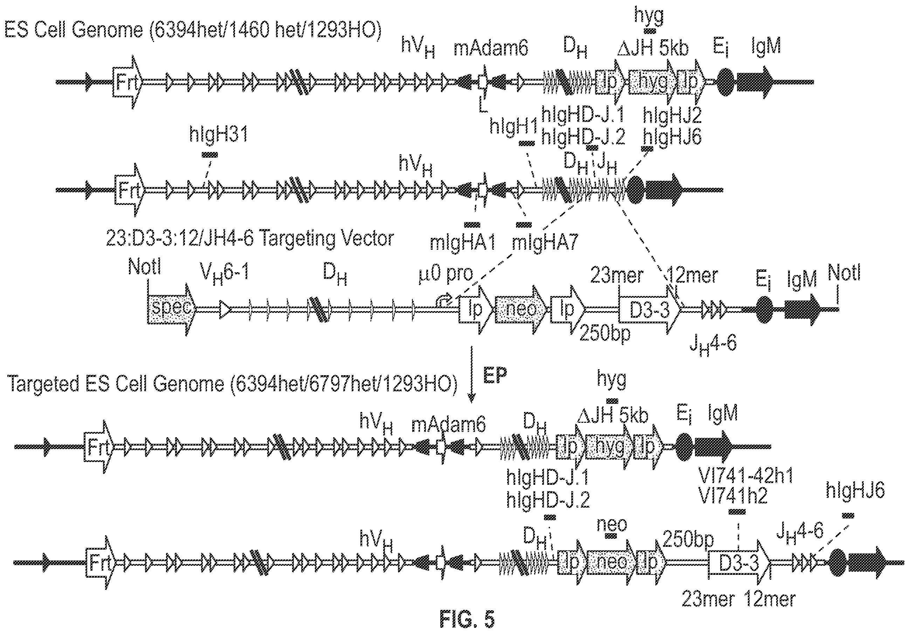

***Figure 5*** shows an illustration, not to scale, of a non-limiting exemplary embodiment of inserting the 23:D_{H}3-3:12/J_{H}4-6 targeting vector into a humanized immunoglobulin heavy chain variable region locus in the genome of mouse ES cells via electroporation (EP). Hash lines represent V_{H} gene segments or D_{H} gene segments that are included in the heavy chain variable region locus but are not specifically depicted.

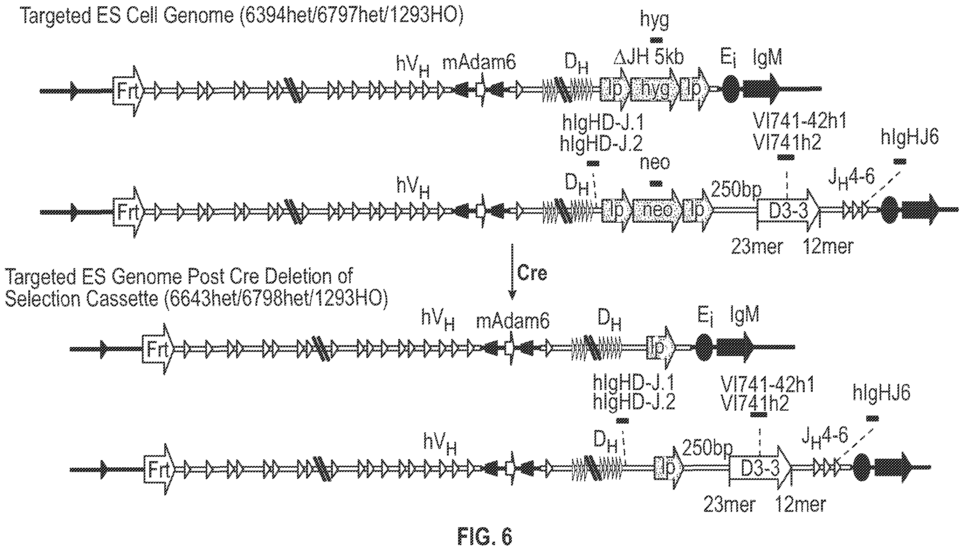

***Figure 6*** shows an illustration, not to scale, of a non-limiting exemplary embodiment of Cre-mediated deletion of selection cassettes in humanized immunoglobulin heavy chain loci after electroporation and integration of the 23:D_{H}3-3:12/J_{H}4-6 targeting vector as described in Examples 1 and 2. Hash lines represent V_{H} gene segments or D_{H} gene segments that are included in the heavy chain variable region locus but are not specifically depicted.

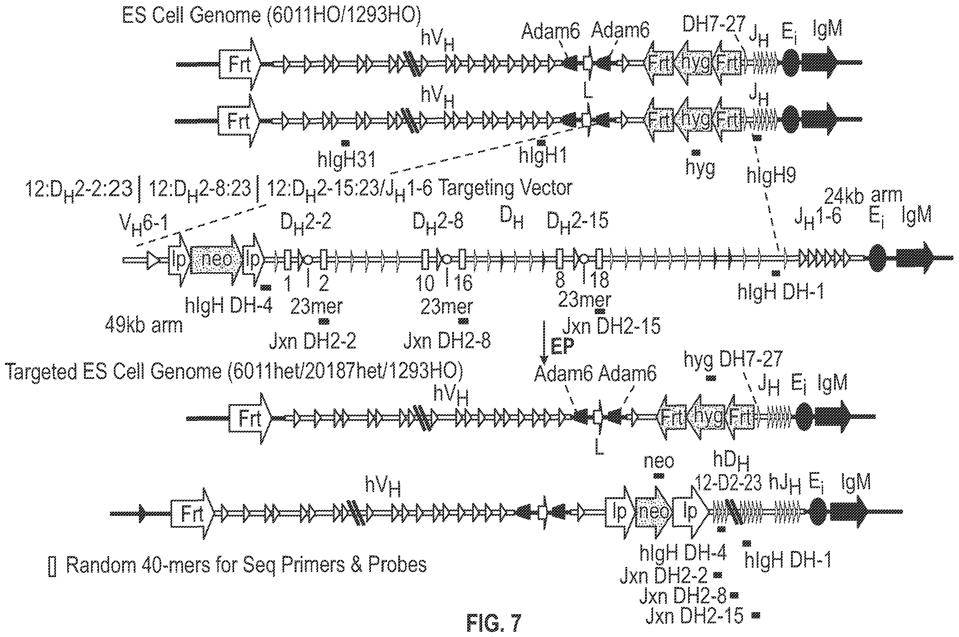

***Figure 7*** shows an illustration, not to scale, of a non-limiting exemplary embodiment of inserting the 12:D_{H}2-2:23|12:D_{H}2-8:23|12:D_{H}2-15:23/J_{H}1-6 targeting vector into a humanized immunoglobulin heavy chain variable region locus in the genome of mouse ES cells via electroporation (EP). Hash lines represent V_{H} gene segments or D_{H} gene segments that are included in the heavy chain variable region locus but are not specifically depicted. Filled triangles represent pseudogenes.

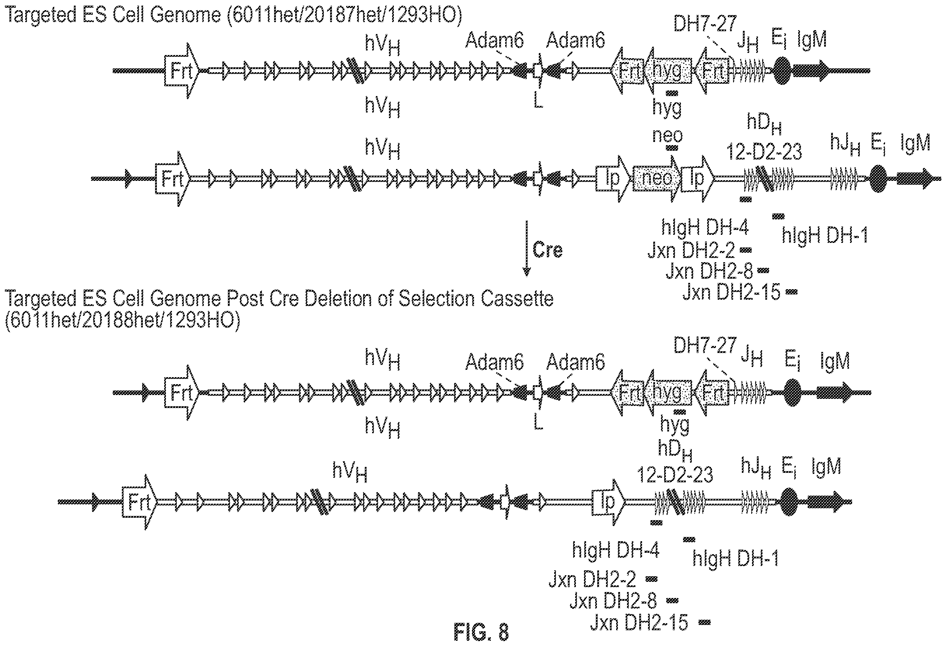

***Figure 8*** shows an illustration, not to scale, of an exemplary non-limiting embodiment of Cre-mediated deletion of selection cassettes in humanized immunoglobulin heavy chain loci after electroporation and integration of the 12:D_{H}2-2:23|12:D_{H}2-8:23|12:D_{H}2-15:23 targeting vector as described in Examples 1 and 2. Hash lines represent V_{H} gene segments or D_{H} gene segments that are included in the heavy chain variable region locus but are not specifically depicted

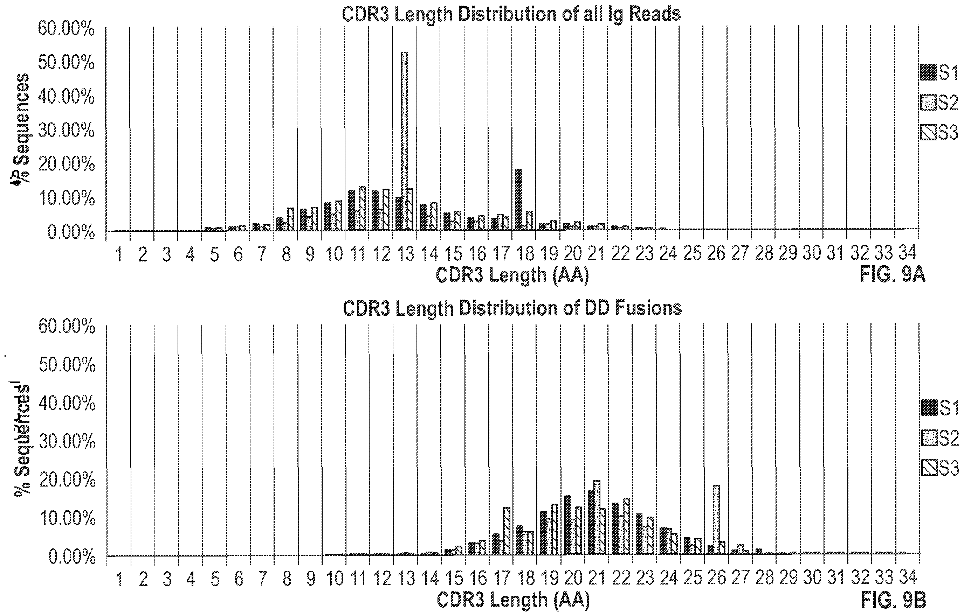

***Figure 9A*** shows results in connection with an embodiment of the invention, graphing the percentages (y-axes) of all functional immunoglobulin (Ig) reads resulting from a D_{H}-D_{H} recombination event (bottom panel) that have a CDR3 of a particular amino acid length (x-axes) isolated from animals modified with the 12:D_{H}2-2:23|12:D_{H}2-8:23|12:D_{H}2-15:23 targeting vector. "S1," "S2," and "S3" each represent a different experimental mouse.
***Figure 9B*** shows results in connection with an embodiment of the invention, graphing the percentages (y-axes) of all Ig reads resulting from a D_{H}-D_{H} recombination event (bottom panel) that have a CDR3 of a particular amino acid length (x-axes) isolated from animals modified with the 12:D_{H}2-2:23|12:D_{H}2-8:23|12:D_{H}2-15:23 targeting vector. "S1," "S2," and "S3" each represent a different experimental mouse.

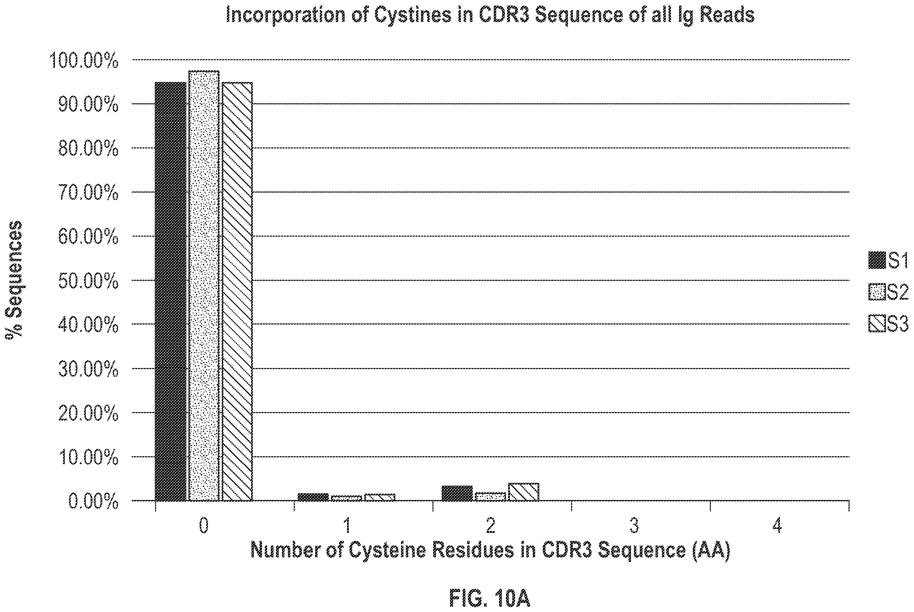

***Figure 10A*** shows results in connection with an embodiment of the invention, graphing the percentages (y-axes) of all functional immunoglobulin (Ig) reads resulting from a D_{H}-D_{H} recombination event that have a CDR3 comprising a certain number of cysteine residues (x-axes) isolated from animals modified with the 12:D_{H}2-2:23|12:D_{H}2-8:23|12:D_{H}2-15:23 targeting vector. "S1," "S2," and "S3" each represent a different experimental mouse.

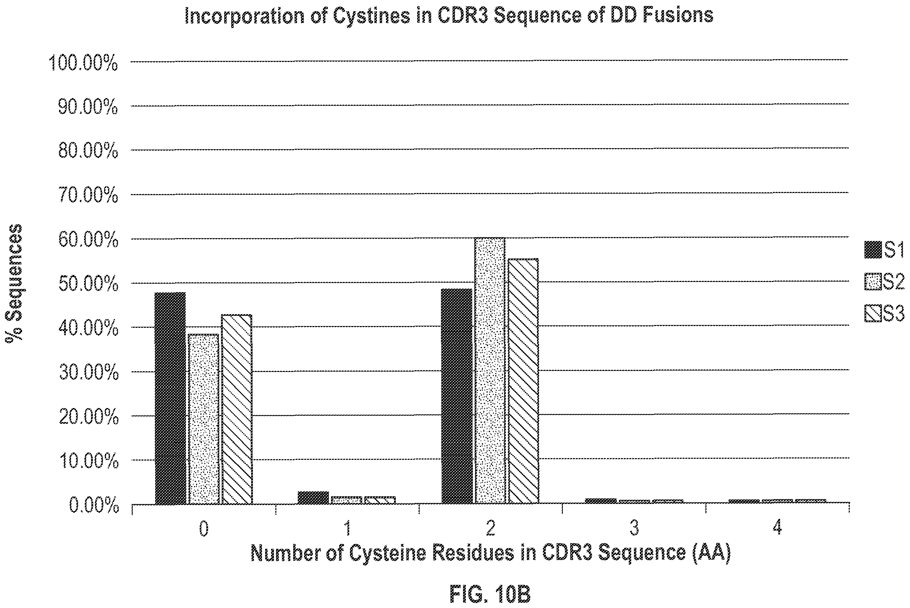

***Figure 10B*** results in connection with an embodiment of the invention, graphing the percentages (y-axes) of all immunoglobulin (Ig) reads resulting from a D_{H}-D_{H} recombination event that have a CDR3 comprising a certain number of cysteine residues (x-axes) isolated from animals modified with the 12:D_{H}2-2:23|12:D_{H}2-8:23|12:D_{H}2-15:23 targeting vector. "S1," "S2," and "S3" each represent a different experimental mouse.

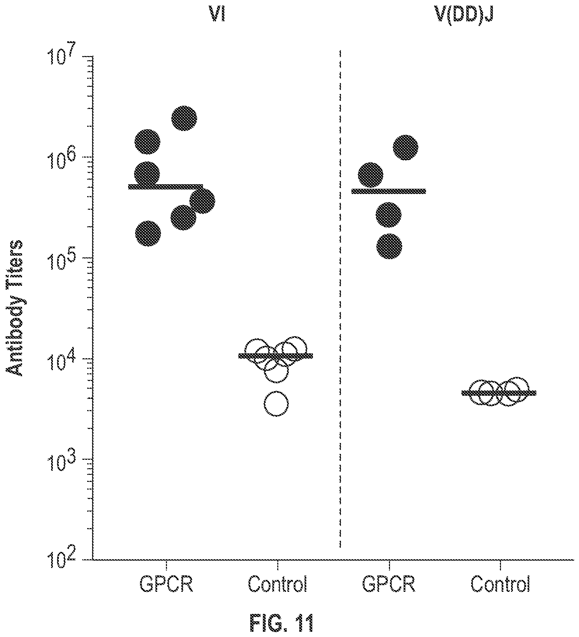

***Figure 11*** shows results in connection with an embodiment of the present invention, graphing representative antibody titers (y-axis) in individual mice having humanized immunoglobulin heavy and κ light chain variable region loci (VI; see, e.g., U.S. Patent Nos. 8,697,940 and 8,642,835) and mice modified with the 23:D_{H}3-3:12/J_{H}6 targeting vector as described herein (V(DD)J), both cohorts of which were immunized with a DNA immunogen encoding a G protein-coupled receptor (GPCR). Antibody titers were determined by MSD cell binding on 293 cells engineered to express the GPCR (GPCR; x-axis) and on cells that do not express GPCR (control; x-axis).

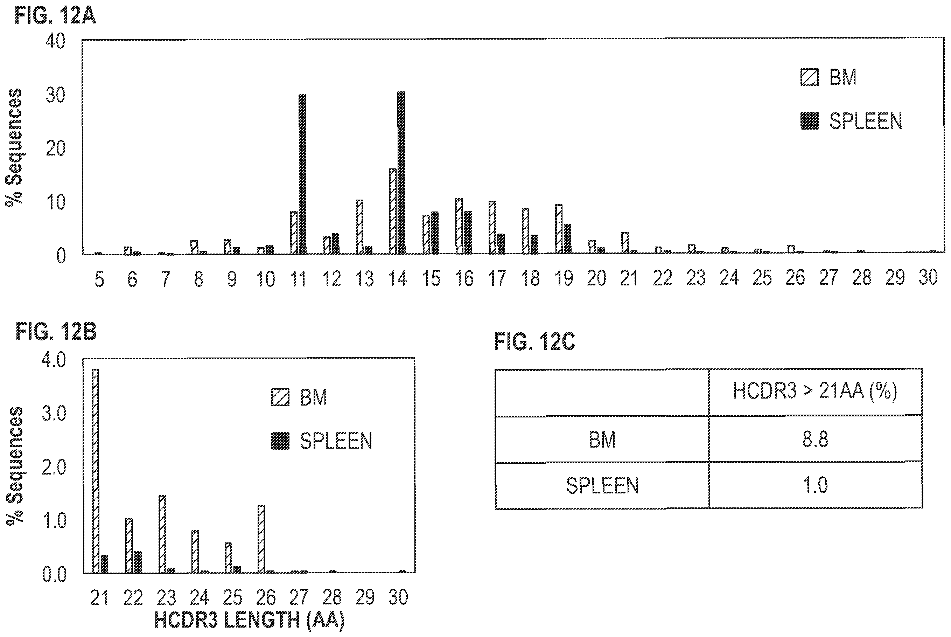

***Figure 12A*** shows results in connection with an embodiment of the present invention, showing the percentage (%; y-axes) of rearranged immunoglobulin heavy chain V_{H}D_{H}J_{H} gene sequences and gene sequences presumed to be the result of V_{H}D_{H}A-D_{H}BJ_{H} rearrangement according to the stringent criterion set forth in Table 7 isolated from the bone marrow (BM) or spleen of mice modified with the 23:D_{H}3-3:12/J_{H}6 targeting vector and encoding a heavy chain CDR3 (HCDR3) having an amino acid (AA) length of 5 to 30 amino acids (x-axes). BM and spleen cell numbers are not normalized to each other. n=1.
***Figure 12B*** shows results in connection with an embodiment of the present invention, providing an enlargement of the graph shown in panel Figure 12A. BM and spleen cell numbers are not normalized to each other. n=1.
***Figure 12C*** shows results in connection with an embodiment of the present invention, showing the percentage of reads having a CDR3 greater than 21 amino acids in length in both the bone marrow or spleen of mice modified with the 23:D_{H}3-3:12/J_{H}6 targeting vector. BM and spleen cell numbers are not normalized to each other. n=1.

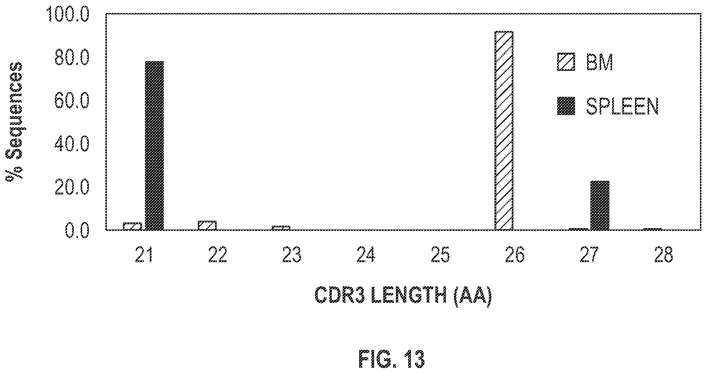

***Figure 13*** shows results in connection with an embodiment of the present invention, showing the percentage (%; y-axes) of presumed rearranged immunoglobulin heavy chain V_{H}D_{H}A-D_{H}BJ_{H} gene sequences (according to the stringent criterion set forth in Table 7) isolated from the bone marrow (BM) or spleen of mice modified with the 23:D_{H}3-3:12/J_{H}6 targeting vector and encoding a heavy chain CDR3 (HCDR3) having an amino acid (AA) length of, all of which were over 20 amino acids in length (x-axes). BM and spleen cell numbers are not normalized to each other. n=1.

### DEFINITIONS

In general, terminology is in accordance with its understood meaning in the art, unless clearly indicated otherwise. Explicit definitions of certain terms are provided herein and below; meanings of these and other terms in particular instances throughout this specification will be clear to those skilled in the art from context. Additional definitions for the following terms and other terms are set forth throughout the specification.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

The articles "a" and "an" in the specification and in the claims, unless clearly indicated to the contrary, should be understood to include the plural referents. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or the entire group members are present in, employed in, or otherwise relevant to a given product or process. Furthermore, it is to be understood that the invention encompasses all variations, combinations, and permutations in which one or more limitations, elements, clauses, descriptive terms, etc., from one or more of the listed claims is introduced into another claim dependent on the same base claim (or, as relevant, any other claim) unless otherwise indicated or unless it would be evident to one of ordinary skill in the art that a contradiction or inconsistency would arise. Where elements are presented as lists, (e.g., in Markush group or similar format) it is to be understood that each subgroup of the elements is also disclosed, and any element(s) can be removed from the group. It should be understood that, in general, where the invention, or aspects of the invention, is/are referred to as comprising particular elements, features, etc., embodiments of the invention or aspects of the invention consist, or consist essentially of, such elements, features, etc. For purposes of simplicity those embodiments have not in every case been specifically set forth in so many words herein. It should also be understood that any embodiment or aspect of the invention can be explicitly excluded from the claims, regardless of whether the specific exclusion is recited in the specification.

As used in this application, the terms "about" and "approximately" are used as equivalents. Any numerals used in this application with or without about/approximately are meant to cover any normal fluctuations, e.g., +/- 5%, appreciated by one of ordinary skill in the relevant art.

***Administration***: refers to the administration of a composition to a subject or system (e.g., to a cell, organ, tissue, organism, or relevant component or set of components thereof). Those of ordinary skill will appreciate that route of administration may vary depending, for example, on the subject or system to which the composition is being administered, the nature of the composition, the purpose of the administration, etc.

For example, in some embodiments, administration to an animal subject (e.g., to a human or a rodent) may be bronchial (including by bronchial instillation), buccal, enteral, intradermal, intra-arterial, intradermal, intragastric, intramedullary, intramuscular, intranasal, intraperitoneal, intrathecal, intravenous, intraventricular, mucosal, nasal, oral, rectal, subcutaneous, sublingual, topical, tracheal (including by intratracheal instillation), transdermal, vaginal and/or vitreal. In some embodiments, administration may involve intermittent dosing. In some embodiments, administration may involve continuous dosing (e.g., perfusion) for at least a selected period of time. In some embodiments, an antibody produced by a rodent disclosed herein can be administered to a subject (e.g., a human subject or rodent). In some embodiments, a pharmaceutical composition includes an antibody produced by a rodent disclosed herein. In some embodiments, a pharmaceutical composition can include a buffer, a diluent, an excipient, or any combination thereof. In some embodiments, a pharmaceutical composition including an antibody produced by a rodent disclosed herein can be included in a container for storage or administration, for example, a vial, a syringe (e.g., an IV syringe), or a bag (e.g., an IV bag).

***Biologically active***: refers to a characteristic of any agent that has activity in a biological system, *in vitro* or *in vivo* (e.g., in an organism). For instance, an agent that, when present in an organism, has a biological effect within that organism is considered to be biologically active.

In particular embodiments where a protein or polypeptide is biologically active, a portion of that protein or polypeptide that confers at least one biological activity of the protein or polypeptide is typically referred to as a "*biologically active*" portion.

***Comparable***: refers to two or more agents, entities, situations, sets of conditions, etc. that may not be identical to one another but that are sufficiently similar to permit comparison there between so that conclusions may reasonably be drawn based on differences or similarities observed. Those of ordinary skill in the art will understand, in context, what degree of identity is required in any given circumstance for two or more such agents, entities, situations, sets of conditions, etc. to be considered comparable.

***Conservative***: refers to a conservative amino acid substitution, i.e., a substitution of an amino acid residue by another amino acid residue having a side chain R group with similar chemical properties (e.g., charge or hydrophobicity). In general, a conservative amino acid substitution will not substantially change the functional properties of interest of a protein, for example, the ability of a receptor to bind to a ligand. Examples of groups of amino acids that have side chains with similar chemical properties include: aliphatic side chains such as glycine, alanine, valine, leucine, and isoleucine; aliphatic-hydroxyl side chains such as serine and threonine; amide-containing side chains such as asparagine and glutamine; aromatic side chains such as phenylalanine, tyrosine, and tryptophan; basic side chains such as lysine, arginine, and histidine; acidic side chains such as aspartic acid and glutamic acid; and sulfur-containing side chains such as cysteine and methionine. Conservative amino acids substitution groups include, for example, valine/leucine/isoleucine, phenylalanine/tyrosine, lysine/arginine, alanine/valine, glutamate/aspartate, and asparagine/glutamine.

In some embodiments, a conservative amino acid substitution can be a substitution of any native residue in a protein with alanine, as used in, for example, alanine scanning mutagenesis. In some embodiments, a conservative substitution is made that has a positive value in the PAM250 log-likelihood matrix disclosed in Gonnet, G. H. et al., 1992, Science 256:1443-1445. In some embodiments, a substitution is a moderately conservative substitution wherein the substitution has a nonnegative value in the PAM250 log-likelihood matrix.

***Control***: refers to the art-understood meaning of a "*control*" being a standard against which results are compared. Typically, controls are used to augment integrity in experiments by isolating variables in order to make a conclusion about such variables. In some embodiments, a control is a reaction or assay that is performed simultaneously with a test reaction or assay to provide a comparator. A "*control*" may refer to a "*control animal*." A "*control animal*" may have a modification as described herein, a modification that is different as described herein, or no modification (i.e., a wild-type animal). In one experiment, a *"test"* (i.e., a variable being tested) is applied. In a second experiment, the *"control,"* the variable being tested is not applied. A control may be a positive control or a negative control.

In some embodiments, a control is a historical control (i.e., of a test or assay performed previously, or an amount or result that is previously known). In some embodiments, a control is or comprises a printed or otherwise saved record.

***Disruption***: refers to the result of a homologous recombination event with a DNA molecule (e.g., with an endogenous homologous sequence such as a gene or gene locus).

In some embodiments, a disruption may achieve or represent an insertion, deletion, substitution, replacement, missense mutation, or a frame-shift of a DNA sequence(s), or any combination thereof. Insertions may include the insertion of entire genes, fragments of genes, e.g., exons, which may be of an origin other than the endogenous sequence (e.g., a heterologous sequence), or coding sequences derived or isolated from a particular gene of interest. In some embodiments, a disruption may increase expression and/or activity of a gene or gene product (e.g., of a protein encoded by a gene). In some embodiments, a disruption may decrease expression and/or activity of a gene or gene product. In some embodiments, a disruption may alter the sequence of a gene or an encoded gene product (e.g., an encoded protein). In some embodiments, a disruption may alter sequence of a chromosome or chromosome position in a genome. In some embodiments, a disruption may truncate or fragment a gene or an encoded gene product (e.g., an encoded protein). In some embodiments, a disruption may extend a gene or an encoded gene product. In some such embodiments, a disruption may achieve assembly of a fusion protein. In some embodiments, a disruption may affect level, but not activity, of a gene or gene product. In some embodiments, a disruption may affect activity, but not level, of a gene or gene product. In some embodiments, a disruption may have no significant effect on level of a gene or gene product. In some embodiments, a disruption may have no significant effect on activity of a gene or gene product. In some embodiments, a disruption may have no significant effect on either level or activity of a gene or gene product. In some embodiments, a significant effect can be measured by, e.g., but not limited to, a Student's T-test.

***Endogenous locus*** or ***endogenous gene***: refers to a genetic locus found in a parent or reference organism (or cell) prior to introduction of an alteration, disruption, deletion, insertion, modification, substitution or replacement as described herein.

In some embodiments, an endogenous locus comprises a sequence, in whole or in part, found in nature. In some embodiments, the endogenous locus is a wild-type locus. In some embodiments, a reference organism is a wild-type organism. In some embodiments, a reference organism is an engineered organism. In some embodiments, a reference organism is a laboratory-bred organism (whether wild-type or engineered).

***Endogenous promoter***: refers to a promoter that is naturally associated, e.g., in a wild-type organism, with an endogenous gene or genetic locus.

***Engineered**:* refers, in general, to the aspect of having been manipulated by the hand of man. As is common practice and is understood by those in the art, progeny of an engineered polynucleotide or cell are typically still referred to as *"engineered* even though the actual manipulation was performed on a prior entity. Furthermore, as will be appreciated by those skilled in the art, a variety of methodologies are available through which *"engineering"* as described herein may be achieved.

In some embodiments, a polynucleotide may be considered to be *"engineered"* when two or more sequences that are not linked together in that order in nature are manipulated by the hand of man to be directly linked to one another in the engineered polynucleotide. In some embodiments, an engineered polynucleotide may comprise a regulatory sequence, which is found in nature in operative linkage with a first coding sequence but not in operative linkage with a second coding sequence, linked by the hand of man so that it is operatively linked with the second coding sequence. In engineered D_{H} gene segment embodiments herein, a D_{H} gene segment is manipulated by the hand of man to be immediately adjacent to a 23-mer RSS. In some embodiments a D_{H} gene segment engineered to be operably linked to a 23-mer RSS is derived from another D_{H} gene segment, e.g., the D_{H} gene segment operably linked to a 23-mer RSS comprises a nucleotide sequence identical to that of the other D_{H} gene segment but for differences due to the degeneracy of the genetic code and/or the replacement of a 12-mer RSS with a 23-mer RSS. Any other D_{H} gene segment and an engineered D_{H} gene segment derived therefrom (e.g., a D_{H} gene segment operably linked to a 23-mer RSS) may be considered corresponding gene segments. For example a human D_{H}3-3 gene segment operably linked to a 23-mer RSS may be considered to correspond to a D_{H}3-3 gene segment flanked on one side by a 12-mer RSS and on the other side by another 12-mer RSS, wherein the D_{H}3-3 gene segment operably linked to a 23-mer RSS and the D_{H}3-3 gene segment flanked on one side by a 12-mer RSS and on the other side by another 12-mer RSS share an identical nucleotide sequence except for differences due to degeneracy of the genetic code and/or the replacement of one of the two 12-mer RSS with the 23-mer RSS. Alternatively, or additionally, in some embodiments, first and second nucleic acid sequences that each encodes polypeptide elements or domains that in nature are not linked to one another may be linked to one another in a single engineered polynucleotide. Comparably, in some embodiments, a cell or organism may be considered to be *"engineered*ʺ if it has been manipulated so that its genetic information is altered (e.g., new genetic material not previously present has been introduced, or previously present genetic material has been altered or removed).

In some embodiments, *"engineering"* may involve selection or design (e.g., of nucleic acid sequences, polypeptide sequences, cells, tissues, and/or organisms) through use of computer systems programmed to perform analysis or comparison, or otherwise to analyze, recommend, and/or select sequences, alterations, etc.). Alternatively, or additionally, in some embodiments, *"engineering"* may involve use of *in vitro* chemical synthesis methodologies and/or recombinant nucleic acid technologies such as, for example, for example, nucleic acid amplification (e.g., via the polymerase chain reaction) hybridization, mutation, transformation, transfection, etc., and/or any of a variety of controlled mating methodologies. As will be appreciated by those skilled in the art, a variety of established such techniques (e.g., for recombinant DNA, oligonucleotide synthesis, and tissue culture and transformation (e.g., electroporation, lipofection, etc.) are well known in the art and described in various general and more specific references that are cited and/or discussed throughout the present specification. See e.g., Sambrook et al., Molecular Cloning: A Laboratory Manual (2d ed., Cold Spring Harbor Laboratory Press, Cold Spring Harbor, N.Y., 1989).

***Gene***: refers to a DNA sequence in a chromosome that codes for a product (e.g., an RNA product and/or a polypeptide product). For the purpose of clarity, the term *"gene"* generally refers to a portion of a nucleic acid that encodes a polypeptide; the term may optionally encompass regulatory sequences, as will be clear from context to those of ordinary skill in the art. This definition is not intended to exclude application of the term *"gene"* to non-protein-coding expression units but rather to clarify that, in most cases, the term as used in this document refers to a polypeptide-coding nucleic acid.

In some embodiments, a gene includes coding sequence (i.e., sequence that encodes a particular product). In some embodiments, a gene includes non-coding sequence. In some embodiments, a gene may include both coding (e.g., exonic) and non-coding (e.g., intronic) sequence. In some embodiments, a gene may include one or more regulatory sequences (e.g., promoters, enhancers, *etc*.) and/or intron sequences that, for example, may control or impact one or more aspects of gene expression (e.g., cell-type-specific expression, inducible expression, etc.).

***Heterologous***: refers to an agent or entity from a different source. For example, when used in reference to a polypeptide, gene, or gene product present in a particular cell or organism, the term clarifies that the relevant polypeptide or fragment thereof, gene or fragment thereof, or gene product or fragment thereof: (1) was engineered by the hand of man; (2) was introduced into the cell or organism (or a precursor thereof) through the hand of man (e.g., via genetic engineering); and/or (3) is not naturally produced by or present in the relevant cell or organism (e.g., the relevant cell type or organism type). Another example includes a polypeptide or fragment thereof, gene or fragment thereof, or gene product or fragment thereof that is normally present in a particular native cell or organism, but has been modified, for example, by mutation or placement under the control of non-naturally associated and, in some embodiments, non-endogenous regulatory elements (e.g., a promoter).

***Host cell***: refers to a cell into which a heterologous (e.g., exogenous) nucleic acid or protein has been introduced. Persons of skill upon reading this disclosure will understand that such terms refer not only to the particular subject cell, but also is used to refer to the progeny of such a cell. Because certain modifications may occur in succeeding generations due to either mutation or environmental influences, such progeny may not, in fact, be identical to the parent cell, but are still included within the scope of the term *"host cell".*

In some embodiments, a host cell is or comprises a prokaryotic or eukaryotic cell. In embodiments, a host cell is or comprises a mammalian cell. In general, a host cell is any cell that is suitable for receiving and/or producing a heterologous nucleic acid or protein, regardless of the Kingdom of life to which the cell is designated. Exemplary cells include those of prokaryotes and eukaryotes (single-cell or multiple-cell), bacterial cells (e.g., strains of *Escherichia coli, Bacillus* spp., *Streptomyces* spp., etc.), mycobacteria cells, fungal cells, yeast cells (e.g., *Saccharomyces cerevisiae, Schizosaccharomyces pombe, Pichia pastoris, Pichia methanolica,* etc.), plant cells, insect cells (e.g., SF-9, SF-21, baculovirus-infected insect cells, *Trichoplusia ni,* etc.), non-human animal cells, human cells, or cell fusions such as, for example, hybridomas or quadromas.

In some embodiments, the cell is a human, monkey, ape, hamster, rat, or mouse cell. In some embodiments, the cell is eukaryotic and is selected from the following cells: CHO (e.g., CHO K1, DXB-11 CHO, Veggie-CHO), COS (e.g., COS-7), retinal cell, Vero, CV1, kidney (e.g., HEK293, 293 EBNA, MSR 293, MDCK, HaK, BHK), HeLa, HepG2, WI38, MRC 5, Colo205, HB 8065, HL-60, (e.g., BHK21), Jurkat, Daudi, A431 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In some embodiments, the cell comprises one or more viral genes, e.g., a retinal cell that expresses a viral gene (e.g., a PER.C6^{®} cell). In some embodiments, a host cell is or comprises an isolated cell. In some embodiments, a host cell is part of a tissue. In some embodiments, a host cell is part of an organism.

***Humanized:*** refers to a molecule (e.g., a nucleic acid, protein, etc.) that was non-human in origin and for which a portion has been replaced with a corresponding portion of a corresponding human molecule in such a manner that the modified (e.g., humanized) molecule retains its biological function and/or maintains the structure that performs the retained biological function. In contrast "human" and the like encompasses molecules having only a human origin, e.g., human nucleotides or protein comprising only human nucleotide and amino acid sequences respectively. The term "human(ized)" is used to reflect that the human(ized) molecule may be (a) a human molecule or (b) a humanized molecule.

***Identity***: in connection with a comparison of sequences, refers to identity as determined by a number of different algorithms known in the art that can be used to measure nucleotide and/or amino acid sequence identity.

In some embodiments, identities as described herein are determined using a ClustalW v. 1.83 (slow) alignment employing an open gap penalty of 10.0, an extend gap penalty of 0.1, and using a Gonnet similarity matrix (MACVECTOR^{™} 10.0.2, MacVector Inc., 2008).

***In vitro***: refers to events that occur in an artificial environment, e.g., in a test tube or reaction vessel, in cell culture, etc., rather than within a multi-cellular organism.

***In vivo***: refers to events that occur within a multi-cellular organism, such as a human and/or a non-human animal. In the context of cell-based systems, the term may be used to refer to events that occur within a living cell (as opposed to, for example, *in vitro* systems).

***Isolated***: refers to a substance and/or entity that has been (1) separated from at least some of the components with which it was associated when initially produced (whether in nature and/or in an experimental setting), and/or (2) designed, produced, prepared, and/or manufactured by the hand of man. Isolated substances and/or entities may be separated from about 10 or more of the other components with which they were initially associated. In some embodiments, isolated agents are at least about 80% or more pure. A substance is *"pure"* if it is substantially free of other components. In some embodiments, as will be understood by those skilled in the art, a substance may still be considered *"isolated"* or even *"pure",* after having been combined with certain other components such as, for example, one or more carriers or excipients (e.g., buffer, solvent, water, etc.); in such embodiments, percent isolation or purity of the substance is calculated without including such carriers or excipients.

To give but one example, in some embodiments, a biological polymer such as a polypeptide or polynucleotide that occurs in nature is considered to be *"isolated"* when: (a) by virtue of its origin or source of derivation is not associated with some or all of the components that accompany it in its native state in nature; (b) it is substantially free of other polypeptides or nucleic acids of the same species from the species that produces it in nature; or (c) is expressed by or is otherwise in association with components from a cell or other expression system that is not of the species that produces it in nature. Thus, for instance, in some embodiments, a polypeptide that is chemically synthesized or is synthesized in a cellular system different from that which produces it in nature is considered to be an *"isolated"* polypeptide. Alternatively, or additionally, in some embodiments, a polypeptide that has been subjected to one or more purification techniques may be considered to be an *"isolated"* polypeptide to the extent that it has been separated from other components: a) with which it is associated in nature; and/or b) with which it was associated when initially produced.

***Non-human animal*:** refers to any vertebrate organism that is not a human.

A non-human animal may be a cyclostome, a bony fish, a cartilaginous fish (e.g., a shark or a ray), an amphibian, a reptile, a mammal, and a bird. In some embodiments, a non-human mammal is a primate, a goat, a sheep, a pig, a dog, a cow, or a rodent. The non-human animal of the present invention is a rodent. In some embodiments, the rodent is a rat or a mouse.

***Nucleic acid***: in its broadest sense, refers to any compound and/or substance that is or can be incorporated into an oligonucleotide chain and is generally interchangeable with nucleic acid molecule, nucleic acid sequence, nucleotide molecule, nucleotide molecule, which terms are also interchangeable with each other.

In some embodiments, a *"nucleic acid*ʺ is a compound and/or substance that is or can be incorporated into an oligonucleotide chain via a phosphodiester linkage. As will be clear from context, in some embodiments, *"nucleic acid*ʺ refers to individual nucleic acid residues (e.g., nucleotides and/or nucleosides); in some embodiments, *"nucleic acid*ʺ refers to an oligonucleotide chain comprising individual nucleic acid residues. In some embodiments, a *"nucleic acid*ʺ is or comprises RNA; in some embodiments, a *"nucleic acid*ʺ is or comprises DNA. In some embodiments, a *"nucleic acid*ʺ is, comprises, or consists of one or more natural nucleic acid residues. In some embodiments, a *"nucleic acid*ʺ is, comprises, or consists of one or more nucleic acid analogs. In some embodiments, a nucleic acid analog differs from a *"nucleic acid*ʺ in that it does not utilize a phosphodiester backbone. For example, in some embodiments, a *"nucleic acid*ʺ is, comprises, or consists of one or more *"peptide nucleic acids",* which are known in the art and have peptide bonds instead of phosphodiester bonds in the backbone, are considered within the scope of the present invention. Alternatively, or additionally, in some embodiments, a *"nucleic acid*ʺ has one or more phosphorothioate and/or 5'-N-phosphoramidite linkages rather than phosphodiester bonds. In some embodiments, a *"nucleic acid*ʺ is, comprises, or consists of one or more natural nucleosides (e.g., adenosine, thymidine, guanosine, cytidine, uridine, deoxyadenosine, deoxythymidine, deoxyguanosine, and deoxycytidine). In some embodiments, a *"nucleic acid*ʺ is, comprises, or consists of one or more nucleoside analogs (e.g., 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, 2-thiocytidine, methylated bases, intercalated bases, and combinations thereof). In some embodiments, a *"nucleic acid*ʺ comprises one or more modified sugars (e.g., 2'-fluororibose, ribose, 2'-deoxyribose, arabinose, and hexose) as compared with those in natural nucleic acids. In some embodiments, a *"nucleic acid*ʺ has a nucleotide sequence that encodes a functional gene product such as an RNA or protein. In some embodiments, a *"nucleic acid*ʺ has a nucleotide sequence that encodes polypeptide fragment (e.g., a peptide). In some embodiments, a *"nucleic acid*ʺ includes one or more introns. In some embodiments, a *"nucleic acid*ʺ includes one or more exons. In some embodiments, a *"nucleic acid*ʺ includes one or more coding sequences. In some embodiments, a *"nucleic acid*ʺ is prepared by one or more of isolation from a natural source, enzymatic synthesis by polymerization based on a complementary template (*in vivo* or *in vitro*), reproduction in a recombinant cell or system, and chemical synthesis. In some embodiments, a *"nucleic acid*ʺ is at least 3 or more residues long. In some embodiments, a *"nucleic acid*ʺ is single stranded; in some embodiments, a *"nucleic acid*ʺ is double stranded. In some embodiments, a *"nucleic acid*ʺ has a nucleotide sequence comprising at least one element that encodes, or is the complement of a sequence that encodes, a polypeptide or fragment thereof. In some embodiments, a *"nucleic acid*ʺ has enzymatic activity.

***Operably linked***: refers to a juxtaposition wherein the components described are in a relationship permitting them to function in their intended manner.

In some embodiments, operably linked nucleotide sequences are contiguous with one another, e.g., a nucleic acid sequence comprising an immunoglobulin gene segment operably linked to an RSS comprises the immunoglobulin gene segment nucleotide sequence contiguous with the RSS nucleotide sequence, e.g., the immunoglobulin gene segment is flanked at least on one side by (e.g., abuts) the RSS nucleotide sequence in a contiguous manner such that the immunoglobulin gene segment is immediately adjacent to the RSS nucleotide sequence.

In other embodiments, operable linkage does not require contiguity. For example, unrearranged variable region gene segments "operably linked" to each other are capable of rearranging to form a rearranged variable region gene, which unrearranged variable region gene segments may not necessarily be contiguous with one another. Unrearranged variable region gene segments operably linked to each other and to a contiguous constant region gene are capable of rearranging to form a rearranged variable region gene that is expressed in conjunction with the constant region gene as a polypeptide chain of an antigen binding protein. A control sequence "*operably linked*" to a coding sequence is ligated in such a way that expression of the coding sequence is achieved under conditions compatible with the control sequences. "*Operably linked*" sequences include both expression control sequences that are contiguous with the gene of interest and expression control sequences that act in trans or at a distance to control the gene of interest.

The term "*expression control sequence*"*,* refers to polynucleotide sequences, which are necessary to affect the expression and processing of coding sequences to which they are ligated. "*Expression control sequences*" include: appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance protein stability; and when desired, sequences that enhance protein secretion. The nature of such control sequences differs depending upon the host organism. For example, in prokaryotes, such control sequences generally include promoter, ribosomal binding site and transcription termination sequence, while in eukaryotes typically, such control sequences include promoters and transcription termination sequence. The term "*control sequences*" is intended to include components whose presence is essential for expression and processing and can also include additional components whose presence is advantageous, for example, leader sequences and fusion partner sequences.

Generally, each unrearranged immunoglobulin V gene segment, D gene segment, or J gene segment is operably linked to (e.g., associated with, flanked on by one or both sides with, contiguous with, etc.) a recombination signal sequence (RSS), which may be a 12-mer RSS or a 23-mer RSS. Any gene segment flanked on each side by an RSS (including a D_{H} gene segment operably linked to a 23-mer RSS) has not undergone recombination, and thus, may be considered an "unrearranged" gene segment. In some embodiments, an unrearranged gene segment herein may comprise a gene segment in its germline (e.g., wildtype) configuration, e.g., a germline V_{H} gene segment and a germline J_{H} gene segment are each flanked on both sides by 23-mer RSS. In contrast, a germline D_{H} gene segment, e.g., an unrearranged D_{H} gene segment, is flanked on each side by a 12-mer RSS. A D_{H} gene segment operably linked to a 23-mer RSS, e.g., an engineered D_{H} gene segment, also has not undergone recombination, and thus, comprises (i) a 23-mer RSS and (ii) a 12-mer RSS. An engineered D_{H} gene segment (e.g., a D_{H} gene segment operably linked to a 23-mer RSS) is able to rearrange with another D_{H} gene segment operably linked with a 12-mer RSS in accordance with the 12/23 rule of recombination.

***Physiological conditions*:** has its art-understood meaning referencing conditions under which cells or organisms live and/or reproduce. In some embodiments, the term refers to conditions of the external or internal milieu that may occur in nature for an organism or cell system. In some embodiments, physiological conditions are those conditions present within the body of a human or non-human animal, especially those conditions present at and/or within a surgical site. Physiological conditions typically include, e.g., a temperature range of 20-40°C, atmospheric pressure of 1, pH of 6-8, glucose concentration of 1-20 mM, oxygen concentration at atmospheric levels, and gravity as it is encountered on earth. In some embodiments, conditions in a laboratory are manipulated and/or maintained at physiological conditions. In some embodiments, physiological conditions are encountered in an organism (e.g., non-human animal).

***Polypeptide***: refers to any polymeric chain of amino acids.

In some embodiments, a polypeptide has an amino acid sequence that occurs in nature. In some embodiments, a polypeptide has an amino acid sequence that does not occur in nature. In some embodiments, a polypeptide has an amino acid sequence that contains portions that occur in nature separately from one another (i.e., from two or more different organisms, for example, human and non-human portions). In some embodiments, a polypeptide has an amino acid sequence that is engineered in that it is designed and/or produced through action of the hand of man. In some embodiments, a polypeptide may comprise or consist of a plurality of fragments, each of which is found in the same parent polypeptide in a different spatial arrangement relative to one another than is found in the polypeptide of interest (e.g., fragments that are directly linked in the parent may be spatially separated in the polypeptide of interest or vice versa, and/or fragments may be present in a different order in the polypeptide of interest than in the parent), so that the polypeptide of interest is a derivative of its parent polypeptide.

***Recombinant***: refers to polypeptides that are designed, engineered, prepared, expressed, created or isolated by recombinant means, such as polypeptides expressed using a recombinant expression vector transfected into a host cell, polypeptides isolated from a recombinant, combinatorial human polypeptide library (Hoogenboom H. R., 1997 TIB Tech. 15:62-70; Hoogenboom H., and Chames P., 2000, Immunology Today 21:371-378; Azzazy H., and Highsmith W. E., 2002, Clin. Biochem. 35:425-445; Gavilondo J. V., and Larrick J. W., 2002, BioTechniques 29:128-145), antibodies isolated from an animal (e.g., a mouse) that is transgenic for human immunoglobulin genes (see e.g., Taylor, L. D., et al., 1992, Nucl. Acids Res. 20:6287-6295; Little M. et al., 2000, Immunology Today 21:364-370; Kellermann S. A. and Green L. L., 2002, Current Opinion in Biotechnology 13:593-597; Murphy, A.J., et al., 2014, Proc. Natl. Acad. Sci. U. S. A. 111(14):5153-5158) or polypeptides prepared, expressed, created or isolated by any other means that involves splicing selected sequence elements to one another.

In some embodiments, one or more of such selected sequence elements is found in nature. In some embodiments, one or more of such selected sequence elements is designed *in silico.* In some embodiments, one or more such selected sequence elements result from mutagenesis (e.g., *in vivo* or *in vitro*) of a known sequence element, e.g., from a natural or synthetic source. For example, in some embodiments, a recombinant polypeptide comprises sequences found in the genome (or polypeptide) of a source organism of interest (e.g., human, mouse, etc.). In some embodiments, a recombinant polypeptide comprises sequences that occur in nature separately from one another (i.e., from two or more different organisms, for example, human and non-human portions) in two different organisms (e.g., a human and a non-human organism). In some embodiments, a recombinant polypeptide has an amino acid sequence that resulted from mutagenesis (e.g., *in vitro* or *in vivo,* for example in a non-human animal), so that the amino acid sequences of the recombinant polypeptides are sequences that, while originating from and related to polypeptide sequences, may not naturally exist within the genome of a non-human animal *in vivo.*

***Reference***: refers to a standard or control agent, animal, cohort, individual, population, sample, sequence or value against which an agent, animal, cohort, individual, population, sample, sequence or value of interest is compared. A "*reference*" may refer to a "*reference animal*". A "*reference animal*" may have a modification as described herein, a modification that is different as described herein or no modification (i.e., a wild-type animal). Typically, as would be understood by those skilled in the art, a reference agent, animal, cohort, individual, population, sample, sequence or value is determined or characterized under conditions comparable to those utilized to determine or characterize the agent, animal (e.g., a mammal), cohort, individual, population, sample, sequence or value of interest.

In some embodiments, a reference agent, animal, cohort, individual, population, sample, sequence or value is tested and/or determined substantially simultaneously with the testing or determination of the agent, animal, cohort, individual, population, sample, sequence or value of interest. In some embodiments, a reference agent, animal, cohort, individual, population, sample, sequence or value is a historical reference, optionally embodied in a tangible medium. In some embodiments, a reference may refer to a control.

***Substantially***: refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "*substantially*" is therefore used to capture the potential lack of completeness inherent in many biological and chemical phenomena.

***Substantial homology***: refers to a comparison between amino acid or nucleic acid sequences. As will be appreciated by those of ordinary skill in the art, two sequences are generally considered to be "*substantially homologous*" if they contain homologous residues in corresponding positions. Homologous residues may be identical residues. Alternatively, homologous residues may be non-identical residues with appropriately similar structural and/or functional characteristics. For example, as is well known by those of ordinary skill in the art, certain amino acids are typically classified as "*hydrophobic*" or "*hydrophilic*" amino acids, and/or as having "*polar*" or "*non-polar*" side chains. Substitution of one amino acid for another of the same type may often be considered a "*homologous*" substitution. Typical amino acid categorizations are summarized below:

| | | | | | |
|---|---|---|---|---|---|
| Alanine | Ala | A | Nonpolar | Neutral | 1.8 |
| Arginine | Arg | R | Polar | Positive | -4.5 |
| Asparagine | Asn | N | Polar | Neutral | -3.5 |
| Aspartic acid | Asp | D | Polar | Negative | -3.5 |
| Cysteine | Cys | C | Nonpolar | Neutral | 2.5 |
| Glutamic acid | Glu | E | Polar | Negative | -3.5 |
| Glutamine | Gln | Q | Polar | Neutral | -3.5 |
| Glycine | Gly | G | Nonpolar | Neutral | -0.4 |
| Histidine | His | H | Polar | Positive | -3.2 |
| Isoleucine | Ile | I | Nonpolar | Neutral | 4.5 |
| Leucine | Leu | L | Nonpolar | Neutral | 3.8 |
| Lysine | Lys | K | Polar | Positive | -3.9 |
| Methionine | Met | M | Nonpolar | Neutral | 1.9 |
| Phenylalanine | Phe | F | Nonpolar | Neutral | 2.8 |
| Proline | Pro | P | Nonpolar | Neutral | -1.6 |
| Serine | Ser | S | Polar | Neutral | -0.8 |
| Threonine | Thr | T | Polar | Neutral | -0.7 |
| Tryptophan | Trp | W | Nonpolar | Neutral | -0.9 |
| Tyrosine | Tyr | Y | Polar | Neutral | -1.3 |
| Valine | Val | V | Nonpolar | Neutral | 4.2 |
| Ambiguous Amino Acids | | | 3-Letter | | 1-Letter |
| Asparagine or aspartic acid | | | Asx | | B |
| Glutamine or glutamic acid | | | Glx | | Z |
| Leucine or Isoleucine | | | Xle | | J |
| Unspecified or unknown amino acid | | | Xaa | | X |

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, S. F. et al., 1990, J. Mol. Biol., 215(3): 403-410; Altschul, S. F. et al., 1996, Methods in Enzymol. 266:460-80; Altschul, S. F. et al., 1997, Nucleic Acids Res., 25:3389-402; Baxevanis, A.D., and B. F. F. Ouellette (eds.) Bioinformatics: A Practical Guide to the Analysis of Genes and Proteins, Wiley, 1998; and Misener et al. (eds.) Bioinformatics Methods and Protocols (Methods in Molecular Biology, Vol. 132), Humana Press, 1998. In addition to identifying homologous sequences, the programs mentioned above typically provide an indication of the degree of homology.

In some embodiments, two sequences are considered to be substantially homologous if at least 95% or more of their corresponding residues are homologous over a relevant stretch of residues. In some embodiments, the relevant stretch is a complete sequence. In some embodiments, the relevant stretch is at least 9 or more residues. In some embodiments, the relevant stretch includes contiguous residues along a complete sequence. In some embodiments, the relevant stretch includes discontinuous residues along a complete sequence, for example, noncontiguous residues brought together by the folded conformation of a polypeptide or a portion thereof. In some embodiments, the relevant stretch is at least 10 or more residues.

***Substantial identity***: refers to a comparison between amino acid or nucleic acid sequences. As will be appreciated by those of ordinary skill in the art, two sequences are generally considered to be "*substantially identical*" if they contain identical residues in corresponding positions. As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, S. F. et al., 1990, J. Mol. Biol., 215(3): 403-410; Altschul, S. F. et al., 1996, Methods in Enzymol. 266:460-80; Altschul, S. F. et al., 1997, Nucleic Acids Res., 25:3389-3402; Baxevanis, A.D., and B. F. F. Ouellette (eds.) Bioinformatics: A Practical Guide to the Analysis of Genes and Proteins, Wiley, 1998; and Misener et al. (eds.) Bioinformatics Methods and Protocols (Methods in Molecular Biology, Vol. 132), Humana Press, 1998. In addition to identifying identical sequences, the programs mentioned above typically provide an indication of the degree of identity.

In some embodiments, two sequences are considered to be substantially identical if at least 95% or more of their corresponding residues are identical over a relevant stretch of residues. In some embodiments, the relevant stretch is a complete sequence. In some embodiments, the relevant stretch is at least 10 or more residues.

***Targeting vector*** or ***targeting construct***: refers to a polynucleotide molecule that comprises a targeting region. A targeting region comprises a sequence that is identical or substantially identical to a sequence in a target cell, tissue or animal and provides for integration of the targeting construct into a position within the genome of the cell, tissue or animal via homologous recombination. Targeting regions that target using site-specific recombinase recognition sites (e.g., *lox*P or Frt sites) are also included.

In some embodiments, a targeting construct as described herein further comprises a nucleic acid sequence or gene of particular interest, a selectable marker, control and or regulatory sequences, and other nucleic acid sequences that allow for recombination mediated through exogenous addition of proteins that aid in or facilitate recombination involving such sequences. In some embodiments, a targeting construct as described herein further comprises a gene of interest in whole or in part, wherein the gene of interest is a heterologous gene that encodes a polypeptide, in whole or in part, that has a similar function as a protein encoded by an endogenous sequence. In some embodiments, a targeting construct as described herein further comprises a humanized gene of interest, in whole or in part, wherein the humanized gene of interest encodes a polypeptide, in whole or in part, that has a similar function as a polypeptide encoded by an endogenous sequence. In some embodiments, a targeting construct (or targeting vector) may comprise a nucleic acid sequence manipulated by the hand of man. For example, in some embodiments, a targeting construct (or targeting vector) may be constructed to contain an engineered or recombinant polynucleotide that contains two or more sequences that are not linked together in that order in nature yet manipulated by the hand of man to be directly linked to one another in the engineered or recombinant polynucleotide.

***Transgene*** or ***transgene construct***: refers to a nucleic acid sequence (encoding e.g., a polypeptide of interest, in whole or in part) that has been introduced into a cell by the hand of man such as by the methods described herein. A transgene could be partly or entirely heterologous, i.e., foreign, to the transgenic animal or cell into which it is introduced. A transgene can include one or more transcriptional regulatory sequences and any other nucleic acid, such as introns or promoters, which may be necessary for expression of a selected nucleic acid sequence. A transgene can include one or more selectable markers that allow for subsequent selection of progeny (e.g., cells) that have taken up the transgene.

***Transgenic animal, transgenic non-human animal*** or ***Tg***⁺: are used interchangeably herein and refer to any non-naturally occurring non-human animal in which one or more of the cells of the non-human animal contain heterologous nucleic acid and/or gene encoding a polypeptide of interest, in whole or in part. The non-human animal of the present invention is a rodent.

In some embodiments, a heterologous nucleic acid sequence and/or gene is introduced into the cell, directly or indirectly by introduction into a precursor cell, by way of deliberate genetic manipulation, such as by microinjection or by infection with a recombinant virus. The term genetic manipulation does not include classic breeding techniques, but rather is directed to introduction of recombinant DNA molecule(s). This molecule may be integrated within a chromosome, or it may be extrachromosomally replicating DNA. The term "*Tg*⁺" includes animals that are heterozygous or homozygous for a heterologous nucleic acid and/or gene, and/or animals that have single or multicopies of a heterologous nucleic acid and/or gene.

***Vector***: refers to a nucleic acid molecule capable of transporting another nucleic acid to which it is associated.

In some embodiment, vectors are capable of extra-chromosomal replication and/or expression of nucleic acids to which they are linked in a host cell such as a eukaryotic and/or prokaryotic cell. Vectors capable of directing the expression of operably linked genes are referred to herein as "*expression vectors.*"

***Wild-type***: has its art-understood meaning that refers to an entity having a structure and/or activity as found in nature in a "*normal*" (as contrasted with mutant, diseased, engineered, altered, etc.) state or context. Those of ordinary skill in the art will appreciate that wild-type genes and polypeptides often exist in multiple different forms (e.g., alleles).

**[Deleted].**

### DETAILED DESCRIPTION

The present invention provides among other things, transgenic or engineered rodents having heterologous genetic material encoding one or more portions (functional fragments, binding portions, etc.) of human immunoglobulins, which heterologous genetic material is inserted into an immunoglobulin heavy chain variable region locus so that the heterologous genetic material is operably linked with heavy chain constant (C_{H}) genes. It is contemplated that such rodents demonstrate a capacity to generate antibodies encoded by rearranged V(DD)J sequences. It is also contemplated that such rodents demonstrate an antibody population characterized by heavy chain variable regions having an increase in CDR3 diversity as compared to an antibody population having immunoglobulin heavy chain variable CDR3 diversity generated from a wild-type immunoglobulin heavy chain variable region locus (or an immunoglobulin heavy chain variable region locus that appears in nature). Therefore, provided rodents are particularly useful for the development of antibody-based therapeutics that bind particular antigens, in particular, antigens associated with low and/or poor immunogenicity or antigens that are characterized by one or more epitopes that are unfavorable for binding by traditional (or wild-type) antibodies. In particular, the present invention encompasses the introduction of a 23-mer recombination signal sequence adjacent to (e.g., 5' or 3' of) one or more D_{H} segments of a D_{H} region in an immunoglobulin heavy chain variable region resulting in the capacity for D_{H}-to-D_{H} segment rearrangement during VDJ recombination, and expression of antibodies having heavy chain variable regions and, in particular, CDR3 regions, which may be characterized by a longer amino acid length as compared to antibodies generated from VDJ recombination involving single D_{H} segments. Such transgenic rodents provide an *in vivo* system for identifying and developing antibodies and/or antibody-based therapeutics that bind disease targets beyond the targeting capabilities of established drug discovery technologies. Further, such transgenic rodents provide a useful animal model system for the development of antibodies and/or antibody-based therapeutics centered on or designed for disrupting protein-protein interactions that are central to various diseases and/or disease pathologies that affect humans.

As shown in ***Figure 1*****, top panel,** recombination between immunoglobulin gene segments follows a rule commonly referred to as the 12/23 rule, in which gene segments flanked by recombination signal sequences (RSS) are joined by an ordered process. Each RSS consists of a conserved block of seven nucleotides (heptamer; 5'-CACAGTG-3'; SEQ ID NO: 144) that is contiguous with a coding sequence (e.g., a V_{H}, D_{H} or J_{H} segment) followed by a non-conserved region, known as the spacer which is either 12bp or 23bp in length, and a second conserved block of nine nucleotides (nonamer; 5'-ACAAAAACC-3'; SEQ ID NO:145). The spacer may vary in sequence, but its conserved length corresponds to one or two turns of the DNA double helix. This brings the heptamer and nonamer sequences to the same side of the DNA helix, where they can be bound by a complex of proteins that catalyzes recombination. An RSS with a 23bp spacer is a 23-mer RSS and an RSS with a 12bp spacer is a 12-mer RSS. The 12/23 rule of recombination generally promotes recombination between a 12-mer RSS and a 23-mer RSS and prohibits recombination between a 23-mer RSS and another 23-mer RSS, or between a 12-mer RSS and another 12-mer RSS, e.g., prohibits direct germline V_{H}-to-germline J_{H} recombination (i.e., 23-mer-to-23-mer joining) or germline D_{H}-germline D_{H} recombination (i.e., 12-mer-to-12-mer joining), although exceptions have been reported.

In some embodiments, rodents as described herein comprise an engineered immunoglobulin heavy chain diversity (D_{H}) region ("engineered D_{H} region") comprising:
(i) an engineered D_{H} gene segment comprising a D_{H} gene segment immediately adjacent to a 23-mer RSS ("engineered D_{H} gene segment") and
(ii) an unrearranged D_{H} gene segment flanked on its 5' side by a first 12-mer RSS and on its 3' side by a second 12-mer RSS ("unrearranged D_{H} gene segment"),

wherein (i) the engineered D_{H} gene segment and (ii) the unrearranged D_{H} gene segment are operably linked such that (i) the engineered D_{H} gene segment and (ii) the unrearranged D_{H} gene segment are able to join in a D_{H}-D_{H} recombination event according to the 12/23 rule,
wherein the nucleotide molecule is suitable for obtaining rodents having the engineered D_{H} region, and
optionally wherein the engineered D_{H} region comprises only human D_{H} gene segments. In some embodiments, antibodies containing CDR3s generated from such recombination may be characterized as having increased diversity resulting from longer amino acid length to direct binding to particular antigens (e.g., viruses, membrane channels, etc.). In some embodiments, rodents described herein comprise human heavy chain variable (V_{H}) and joining (J_{H}) gene segments operably linked with the engineered D_{H} region so that VDJ recombination occurs between said V_{H}, J_{H} and more than one D_{H} segment to create a heavy chain variable region that binds an antigen of interest. In some embodiments, an engineered D_{H} region as described herein contains one or more (e.g., 1, 2, 3, 4, 5, 10 or more) human D_{H} segments engineered to allow for (or promote) D_{H}-to-D_{H} recombination at an increased frequency as compared to a reference immunoglobulin heavy chain variable region locus.

In some embodiments, rodents as described herein further comprise a human or humanized immunoglobulin light chain locus (e.g., κ and/or λ) such that the non-human animals produce antibodies comprising human variable regions (i.e., heavy and light) and non-human constant regions. In some embodiments, said human or humanized immunoglobulin light chain locus comprises human V_{L} and J_{L} gene segments operably linked to a rodent light chain constant region (e.g., a rodent Cκ or Cλ). In some embodiments, rodents described herein further comprise an immunoglobulin light chain locus as described in U.S. Patent Nos. 9,796,788; 9,969,814; U.S. Patent Application Publication Nos. 2011/0195454 A1, 2012/0021409 A1, 2012/0192300 A1, 2013/0045492 A1, 2013/0185821 A1, 2013/0302836 A1, 2018/0125043; International Patent Application Publication Nos. WO 2011/097603, WO 2012/148873, WO 2013/134263, WO 2013/184761, WO 2014/160179, WO 2014/160202; and WO2019/113065).

Various aspects of the invention are described in detail in the following sections. The use of sections is not meant to be limiting to embodiments described herein. Each section can apply to any aspect or embodiment described herein. In this application, the use of "or" means "and/or" unless stated otherwise.

### VDJ Recombination

Genes responsible for immunoglobulin synthesis are found in all cells of an animal and are arranged in gene segments, sequentially situated along the chromosome. The configuration of human gene segments that are inherited, e.g., the germline configuration of human gene segments, e.g., the order of human gene segments in the germline genome (e.g., the genome passed down to the next generation) of a human, may be found at Lefranc, M.-P., Exp. Clin. Immunogenet., 18, 100-116 (2001), which also shows functional gene segments and pseudogenes found within the human immunoglobulin heavy chain locus in germline configuration. A series of recombination events, involving several genetic components, serves to assemble immunoglobulins from ordered arrangement of gene segments (e.g., V, D and J). This assembly of gene segments is known to be imprecise and, therefore, immunoglobulin diversity is achieved both by combination of different gene segments and formation of unique junctions through imprecise joining. Further diversity is generated through a process known as somatic hypermutation in which the variable region sequence of immunoglobulins is altered to increase affinity and specificity for antigen. The immunoglobulin molecule is a Y-shaped polypeptide composed of two identical heavy and two identical light chains, each of which have two structural components: one variable domain and one constant domain. It is the variable domains of heavy and light chains that are formed by the assembly of gene segments, while constant domains are fused to variable domains through RNA splicing. Although the mechanism of assembling (or joining) gene segments is similar for heavy and light chains, only one joining event is required for light chains (i.e., V to J) while two are required for heavy chains (i.e., D to J and V to DJ).

The assembly of gene segments for heavy and light chain variable regions (referred to respectively as VDJ recombination and VJ recombination) is guided by conserved noncoding DNA sequences that flank each gene segment, termed recombination signal sequences (RSSs), which ensure DNA rearrangements at precise locations relative to V, D and J coding sequences (see, e.g., Ramsden, D.A. et al., 1994, Nuc. Acids Res. 22(10):1785-96). A representative schematic of the sequences involved in VDJ recombination of heavy chain gene segments as understood by a skilled artisan is set forth in Figure 1. Each RSS consists of a conserved block of seven nucleotides (heptamer) that is contiguous with a coding sequence (e.g., a V, D or J segment) followed by a spacer (either 12bp or 23bp) and a second conserved block of nine nucleotides (nonamer). Although considerable sequence divergence in the 12bp or 23Bp spacer among individuals is tolerated, the length of these sequences typically does not vary. Recombination between immunoglobulin gene segments follows a rule commonly referred to as the 12/23 rule, in which gene segments flanked by an RSS with a 12bp spacer (or 12-mer) are typically joined to a gene segment flanked by a 23bp spacer (or 23-mer; see, e.g., Hiom, K. and M. Gellert, 1998, Mol. Cell. 1(7):1011-9). The sequence of an RSS has been reported to influence the efficiency and/or the frequency of recombination with a particular gene segment (see, e.g., Ramsden, D.A and G.E. Wu, 1991, Proc. Natl. Acad. Sci. U.S.A. 88:10721-5; Boubnov, N.V. et al., 1995, Nuc. Acids Res. 23:1060-7; Ezekiel, U.R. et al., 1995, Immunity 2:381-9; Sadofsky, M. et al., 1995, Genes Dev. 9:2193-9; Cuomo, C.A. et al., 1996, Mol. Cell Biol. 16:5683-90; Ramsden, D.A. et al., 1996, EMBO J 15:3197-3206). Indeed, many reports point to a highly biased and variable usage of gene segments, in particular, D_{H} segments, among individuals. Unless otherwise indicated or unless it would be evident to one of ordinary skill in the art that a contradiction or inconsistency would arise, an unrearranged gene segment without reference to an RSS is presumed to comprise the two RSS with which the gene segment is naturally associated, e.g., flanked by, operably linked to, etc. In some embodiments, an unrearranged gene segment herein may comprise a gene segment in its germline (e.g., wildtype) configuration, e.g., a germline V_{H} gene segment and a germline J_{H} gene segment are each flanked on both sides by 23-mer RSS. In contrast, a germline D_{H} gene segment, e.g., an unrearranged D_{H} gene segment, is flanked on each side by a 12-mer RSS.

As such, an unrearranged gene segment may also refer to a gene segment in its germline configuration, including any RSS associated with such germline configuration. Moreover, a plurality of gene segments in their germline configuration generally refers to the not only each individual gene segment being in its germline (e.g., unrearranged) configuration, but also the order and/or location of the functional gene segments. See, e.g., Lefranc, M.-P., Exp. Clin. Immunogenet., 18, 100-116 (2001).

The assembly of gene segments to form heavy and light chain variable regions results in the formation of antigen-binding regions (or sites) of immunoglobulins. Such antigen-binding regions are characterized, in part, by the presence of hypervariable regions, which are commonly referred to as complementary determining regions (CDRs). There are three CDRs for both heavy and light chains (i.e., for a total of six CDRs) with both CDR1 and CDR2 being entirely encoded by the V gene segment. CDR3, however, is encoded by the sequence resulting from the joining of the V and J segments for light chains, and the V, D and J segments for heavy chains. Thus, the additional gene segment employed during recombination to form a heavy chain variable region coding sequence significantly increases the diversity of the antigen-binding sites of heavy chains. Thus, provided non-human animals containing an engineered D_{H} region as described herein generate CDR3 diversity characterized by D_{H}-to-D_{H} recombination and have an increased amino acid length as compared to CDR3 regions of heavy chain variable regions generated by traditional VDJ recombination.

Without being bound by theory, it is thought that further diversity in heavy chain CDR3 repertoire may possible through increasing the number of junctions forming the rearranged heavy chain variable region gene sequence and/or increasing the length of the CDR3 region. One mechanism to increase the number of junctions and/or increase the length of the CDR3 region may be through the joining of a first D_{H} segment (which may be generically referred to D_{H}"A") to another D_{H} segment (which may be generically referred to as D_{H}"B") in a D_{H}-D_{H} recombination event prior to subsequent D_{H}-J_{H} and V-D_{H}J_{H} recombination events, leading to V_{H}(D_{H}A-D_{H}B)J_{H} gene sequence. In nature D_{H}-D_{H} recombination events were long thought to be prohibited by the 12/23 rule (Alt, F.W. et al., 1984, EMBO J. 3(6):1209-19). However, it appears that D_{H}-D_{H} recombination events do occur at very low frequency in humans in contravention of the 12/23 rule, (1 in 800 or ~0.125% naive B cells; see, e.g., Ollier, P.J. et al. 1985, EMBO J. 4(13B):3681-88; Milner, E.C.B. et al. 1986, Immunol. Today 7 :36-40; Liu, Z. et al., 1987, Nucleic Acids Res. 15(11):4688; Liu, Z. et al., 1987, Nucleic Acids Res. 15(15):6296; Meek, K.D. et al., 1989, J. Exp. Med. 169(2):519-33; Meek, K.D. et al., 1989, J. Exp. Med. 170:39-57; Baskin, B. et al. 1998, Clin. Exp. Immunol. 112:44-7; Briney, B.S. et al., 2012 Immunol. 137: 56-64) and are thought to be the primary mechanism for generating the unusually long CDR3s observed in some heavy chains (Janeway's Immunobiology. 9th Edition. Kenneth Murphy, Casey Weaver. Chapter 5, 2017). However, some potential therapeutic targets (e.g. but not limited to, viruses, cell surface receptors, type IV transmembrane proteins like GPCRs, ion channels) have masked or recessed epitopes that are inaccessible to normal antibodies but may be recognized by antibodies having long HCDR3 sequences. For example, antibodies with very long HCDR3s are often found in patients with chronic viral infections and in some cases have broadly neutralizing activity (e.g. broadly neutralizing antibodies to HIV-1 or influenza). To select antibodies capable of reaching these hidden epitopes, it would be useful to increase the frequency of heavy chains with very long HCDR3s. Without wishing to be bound by theory, one way to achieve this is to increase the frequency of heavy chain V_{H}(D_{H}A-D_{H}B)J_{H} rearrangements by engineering a D_{H} segments to comprise a 23-mer RSS and/or to increase the frequency of recombination of a D_{H} gene segment to the longer J_{H}6 gene segment. In mouse, D_{H}-J_{H} joining is thought to occur in two ordered steps: (1) primary rearrangement of the proximal DQ52 segment (referred to as D_{H}7-27 in humans) to one of the nearest J_{H} segments (J_{H}1 or J_{H}2). The strong µ0 promoter upstream of DQ52 then allows for the remaining J_{H} segments (J_{H}3 and J_{H}4) to be more accessible to recombination activating genes (RAGs); (2) secondary rearrangement of a distal D_{H} segment to one of the remaining J_{H} segments (J_{H}3 or J_{H}4). *See,* *Figure 1**, top panel.* This is consistent with the more frequent usage of downstream J_{H} segments observed in both mice (J_{H}3-J_{H}4) and humans (J_{H}4-J_{H}6) (Nitschke et al. 2001 J. Immunol. 166:2540-52). Without wishing to be bound by theory, it is hypothesized that replacing D_{H}7-27 and J_{H}1-J_{H}3 (or J_{H}1-J_{H}5) with a synthetic D_{H} gene (e.g., a synthetic D_{H}3-3 segment) having a 5' 23-mer RSS and 3' 12-mer RSS would result in a high frequency of V_{H}(D_{H}-D_{H})J_{H} recombination and may occur by a three-step mechanism: (1) 23-D_{H}-12 rearrangement to J_{H}4, J_{H}5, or J_{H}6 to yield a 23(D_{H})J_{H}, (2) rearrangement of a distal D_{H} (D_{H}1-1 to D_{H}1-26) to the 23(D_{H})J_{H} to yield a 12(D_{H}-D_{H})J_{H} (V_{H} rearrangement to the 23(D_{H})J_{H} would be prohibited by the 12/23 rule), (3) V_{H} to 12(D_{H}-D_{H})J_{H} rearrangement to yield a VDDJ coding sequence encoding an immunoglobulin heavy chain variable domain. *See, e.g.,* *Figure 1**, bottom panel.* It is also hypothesized that replacing D_{H}2-2, D_{H}2-8, and D_{H}2-15 gene segments, which are long D_{H} gene segments (comprising more than 31 nucleotides encoding two cysteines that form can form a disulfide bond thought to stabilize long HCDR3 regions [see, e.g., Wang et al. 2013 Cell 153:1379-93], respectively with those gene segments operably linked to a 5'end 12-mer RSS and 3'end 23-mer RSS would also result in a high frequency of V_{H}(D_{H}-D_{H})J_{H} recombination that may occur by a three-step mechanism: (1) 12:D_{H}-12 rearrangement to J_{H}4, J_{H}5, or J_{H}6 to yield a 12(D_{H})J_{H}, (2) rearrangement of a distal 12:D_{H}2-2:23, 12:D_{H}2-8:23, or 12:D_{H}2-15-23 to the 12(D_{H})J_{H} to yield a 12(D_{H}-D_{H})J_{H}, and (3) V_{H} to 12(D_{H}-D_{H})J_{H} rearrangement to yield a VDDJ coding sequence encoding an immunoglobulin heavy chain variable domain. As described herein, an engineered D_{H} region was constructed by placing a 23-mer spacer at either a 5' or 3' flanking position adjacent to one or more D_{H} segments, thereby enabling Dr_{H} to D_{H} recombination in a humanized immunoglobulin heavy chain variable region locus.

In some embodiments rodents described herein comprise an immunoglobulin heavy chain variable region locus that demonstrates VDJ recombination that does not follow the 12/23 rule as compared to a reference rodent. In some embodiments, rodents described herein comprise one or more RSSs adjacent to or flanking one or more D_{H} segments that are modified as compared to wild-type D_{H} segments. In some embodiments, one or more D_{H} gene segments of an immunoglobulin heavy chain variable region of a non-human animal as described herein are each operably linked to either a 5' or 3' 23-mer RSS so that the frequency of D_{H}-D_{H} recombination is increased in the rodent as compared to a reference rodent. Recombination efficiency and/or frequency may be determined, in some embodiments, by usage frequencies of gene segments in a population of antibody sequences (e.g., from an individual or group of individuals; see e.g., Arnaout, R. et al., 2011, PLoS One 6(8):e22365; Glanville, J. et al., 2011, Proc. Natl. Acad. Sci. U.S.A. 108(50):20066-71). Thus, rodents described herein may, in some embodiments, comprise one or more D_{H} segments that are each operably linked to or flanked by a 23-mer RSS so that D_{H}-D_{H} recombination occurs at an increased frequency as compared to D_{H}-D_{H} recombination in a reference non-human animal. In some embodiments, a rodent comprising an engineered D_{H} region as described herein exhibits at least a 3-fold increased frequency of D_{H}-D_{H} recombination as compared to D_{H}-D_{H} recombination in a reference non-human animal. In some embodiments, a rodent comprising an engineered D_{H} region as described herein exhibits at least a 4-fold increased frequency of D_{H}-D_{H} recombination as compared to D_{H}-D_{H} recombination in a reference rodent. In some embodiments, a rodent comprising an engineered D_{H} region as described herein exhibits at least a 5-fold increased frequency of D_{H}-D_{H} recombination as compared to D_{H}-D_{H} recombination in a reference rodent. In some embodiments, a rodent comprising an engineered D_{H} region as described herein exhibits at least a 10-fold increased frequency of D_{H}-D_{H} recombination as compared to D_{H}-D_{H} recombination in a reference rodent. In some embodiments, a rodent comprising an engineered D_{H} region as described herein exhibits at least a 20-fold increased frequency of D_{H}-D_{H} recombination as compared to D_{H}-D_{H} recombination in a reference non-human animal. In some embodiments, a rodent comprising an engineered D_{H} region as described herein exhibits at least a 30-fold increased frequency of D_{H}-D_{H} recombination as compared to D_{H}-D_{H} recombination in a reference rodent. In some embodiments, a rodent comprising an engineered D_{H} region as described herein exhibits at least a 40-fold increased frequency of D_{H}-D_{H} recombination as compared to D_{H}-D_{H} recombination in a reference rodent. In some embodiments, a rodent comprising an engineered D_{H} region as described herein exhibits at least a 50-fold increased frequency of D_{H}-D_{H} recombination as compared to D_{H}-D_{H} recombination in a reference rodent.

### Provided in vivo systems

The present invention is based on the recognition that particular antigens are associated with low and/or poor immunogenicity and, therefore, are poor targets for antibody-based therapeutics. Indeed, many disease targets (e.g., viruses, channel proteins) have been characterized as intractable or undruggable. Thus, the present invention is based on the creation of an *in vivo* system for the development of antibodies and antibody-based therapeutics that overcome deficiencies associated with established drug discovery technologies and/or approaches. In some embodiments, the present invention provides an *in vivo* system characterized by the presence of immunoglobulin loci, in particular, humanized immunoglobulin heavy chain variable region loci, that include an engineered D_{H} region in which one or more D_{H} RSS are altered, modified or engineered from a 12-mer-D_{H}-12-mer format to a 12-mer-D_{H}-23-mer or 23-mer-D_{H}-12-mer format, thereby enabling D_{H}-to-D_{H} recombination at an increased frequency as compared D_{H}-to-D_{H} recombination observed in a reference *in vivo* system. The present disclosure specifically demonstrates the construction of a transgenic rodent whose genome comprises an immunoglobulin heavy chain variable region that includes an engineered D_{H} region, which engineered D_{H} region includes one or more D_{H} segments that are each operably linked to a 23-mer RSS positioned relative to each of the one or more D_{H} segment to allow for an increased frequency of D_{H}-D_{H} recombination. The methods described herein can be adapted to achieve any number of D_{H} segments (e.g., traditional or synthetic) that each are operably linked to a 5' or 3' 23-mer RSS for creating an engineered D_{H} region as described herein. The engineered D_{H} region, once integrated into an immunoglobulin heavy chain variable region (i.e., placed in operable linkage with V_{H} and J_{H} gene segments and/or one or more constant regions), provides for recombination of V_{H} and J_{H} gene segments with more than one D_{H} segment, e.g., to generate antibodies characterized by heavy chains having added diversity (i.e., long CDR3s, e.g., wherein at least 95% of the heavy chain CDR3 sequences are at least 14 amino acids in length) to direct binding to particular antigens. In some embodiments, such heavy chain variable regions have the capability to access difficult-to-reach epitopes in viruses, channel proteins, GPCRs, etc.

Without wishing to be bound by any particular theory, we note that data provided herein demonstrate that, in some embodiments, rodents whose genome comprises an immunoglobulin heavy chain variable locus that includes an engineered D_{H} region characterized by the inclusion of a D_{H} segment operably linked to a 5' 23-mer RSS effectively generate antibodies produced by V(DD)J recombination. We also note that data provided herein demonstrate that, in some embodiments, rodents whose genome comprises an immunoglobulin heavy chain variable locus that includes an engineered D_{H} region characterized by the inclusion of three D_{H} segments each operably linked to a 3' 23-mer RSS effectively generate antibodies produced by V(DD)J recombination. Also noted herein is that deletion of some or all five J_{H} gene segments upstream of the J_{H}6 gene segment results in preferential recombination to the J_{H}6 gene segment. Notably, the J_{H}6 gene segment comprises 63 nucleotides, e.g., 10 more nucleotides than the other J_{H}1, J_{H}2, J_{H}3, J_{H}4, and J_{H}5 gene segments, which respectively comprise 52, 53, 50, 40, and 51 nucleotides. Thus, the present disclosure, in at least some embodiments, embraces the development of an *in vivo* system for generating antibodies and/or antibody-based therapeutics to intractable disease targets, e.g., by providing rodents that generate rearranged immunoglobulin heavy chain variable region gene sequences, e.g., V_{H}D_{H}J_{H} and V_{H}(D_{H}A-D_{H}B)J_{H}, e.g., V_{H}(D_{H}A-D_{H}B)J_{H}6, sequences encoding heavy chain variable domains with CDR3 lengths of at least 20 amino acids, e.g., CDR3 lengths between 20-30 amino acids in length. In some embodiments, at least 8-10% of the rearranged immunoglobulin heavy chain variable region gene sequences, e.g., V_{H}D_{H}J_{H} and V_{H}(D_{H}A-D_{H}B)J_{H} gene sequences of a non-human animal described herein encode CDR3 regions that are at least 21 amino acids in length.

In some embodiments, described herein is a., a rodent, e.g., a rat or a mouse, that comprises (1) in its germline genome, e.g., in a germ cell, an immunoglobulin heavy chain locus comprising an engineered D_{H} region comprising a D_{H} gene segment operably linked to a 23-mer RSS, and (2) in its somatic genome, e.g., in a B cell, a rearranged heavy chain V_{H}(D_{H}A-D_{H}B)J_{H} coding sequence, wherein the first or second D_{H} gene segment (i.e., D_{H}A or D_{H}B of the V_{H}(D_{H}A-D_{H}B)J_{H} coding sequence, respectively) comprises the D_{H} gene segment operably linked to a 23-mer RSS, or a portion thereof, e.g., wherein the first or second D_{H} gene segment has at least 9 consecutive nucleotides aligning with a D_{H} gene segment operably linked to a 23-mer RSS, and wherein each of the first and second D_{H} gene segments comprises at least 5 consecutive nucleotides aligning with a corresponding germline D_{H} gene segment irrespective of any overlap.

In some embodiments, provided rodents comprise an immunoglobulin heavy chain locus characterized by the presence of a plurality of human V_{H}, D_{H} and J_{H} gene segments arranged in germline configuration and operably linked to non-human immunoglobulin heavy chain constant region genes, enhancers and regulatory regions. In some embodiments, provided rodents comprise one or more human V_{H} gene segments, one or more human D_{H} gene segments and one or more human J_{H} gene segments operably linked to a non-human immunoglobulin heavy chain constant region.

In some embodiments, provided rodents comprise at least human V_{H} gene segments V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2 and V_{H}6-1.

In some embodiments, provided rodents comprise human D_{H} gene segments D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}5-18, D_{H}6-19, D_{H}1-20, D_{H}2-21, D_{H}3-22, D_{H}6-25 and D_{H}1-26. In some embodiments, provided rodents further comprise D_{H}1-14, D_{H}4-11, D_{H}4-23, D_{H}5-24, or a combination thereof. In some embodiments, provided rodents further comprise human D_{H}7-27.

In some embodiments, described herein is a rodent, e.g., a rat or a mouse, that comprises (1) in its germline genome, e.g., in a germ cell, an immunoglobulin heavy chain locus comprising an engineered D_{H} region comprising a D_{H} gene segment operably linked to a 23-mer RSS, and (2) in its somatic genome, e.g., in a B cell, a rearranged heavy chain V_{H}(D_{H}A-D_{H}B)J_{H} coding sequence, wherein the first or second D_{H} gene segment (i.e., D_{H}A or D_{H}B of the V_{H}(D_{H}A-D_{H}B)J_{H} coding sequence, respectively) comprises the D_{H} gene segment operably linked to a 23-mer RSS, or a portion thereof, e.g., wherein the first or second D_{H} gene segment has at least 9 consecutive nucleotides aligning with a D_{H} gene segment operably linked to a 23-mer RSS, and wherein each of the first and second D_{H} gene segments comprises at least 5 consecutive nucleotides aligning with a corresponding germline D_{H} gene segment, irrespective of any overlap. In some embodiments, provided rodents comprise a human D_{H} gene segments operably linked to a 23-mer RSS. In some embodiments, the human D_{H} gene segment operably linked to a 23-mer RSS comprises two cysteine codons. In some embodiments, the human D_{H} gene segment operably linked to a 23-mer RSS contains at least 37 nucleotides. In some embodiments, the human D_{H} gene segment operably linked to a 23-mer RSS contains at least 19 nucleotides. In some embodiments, the human D_{H} gene segment operably linked to a 23-mer RSS contains at least 20 nucleotides. In some embodiments, the human D_{H} gene segment operably linked to a 23-mer RSS contains at least 23 nucleotides. In some embodiments, the human D_{H} gene segment operably linked to a 23-mer RSS contains at least 28 nucleotides. In some embodiments, the human D_{H} gene segment operably linked to a 23-mer RSS contains at least 31 nucleotides. In some embodiments, the human D_{H} gene segment operably linked to a 23-mer RSS contains at least 37 nucleotides.

In some embodiments, the human D_{H} gene segment operably linked to a 23-mer RSS comprises the human D_{H}1 gene segment. In some embodiments, the human D_{H} gene segment operably linked to a 23-mer RSS comprises the human D_{H}2 gene segment. In some embodiments, the human D_{H} gene segment operably linked to a 23-mer RSS comprises the human D_{H}3 gene segment. In some embodiments, the human D_{H} gene segment operably linked to a 23-mer RSS comprises the human D_{H}4 gene segment. In some embodiments, the human D_{H} gene segment operably linked to a 23-mer RSS comprises the human D_{H}5 gene segment. In some embodiments, the human D_{H} gene segment operably linked to a 23-mer RSS comprises the human D_{H}6 gene segment. In some embodiments, the human D_{H} gene segment operably linked to a 23-mer RSS comprises the human D_{H}7gene segment.

In some embodiments, the human D_{H} gene segment operably linked to a 23-mer RSS comprises the human D_{H}1-1 gene segment. In some embodiments, the human D_{H} gene segment operably linked to a 23-mer RSS comprises the human D_{H}2-2 gene segment. In some embodiments, the human D_{H} gene segment operably linked to a 23-mer RSS comprises the human D_{H}3-3 gene segment. In some embodiments, the human D_{H} gene segment operably linked to a 23-mer RSS comprises the human D_{H}4-4 gene segment. In some embodiments, the human D_{H} gene segment operably linked to a 23-mer RSS comprises the human D_{H}5-5 gene segment. In some embodiments, the human D_{H} gene segment operably linked to a 23-mer RSS comprises the human D_{H}6-6 gene segment. In some embodiments, the human D_{H} gene segment operably linked to a 23-mer RSS comprises the human D_{H}1-7 gene segment. In some embodiments, the human D_{H} gene segment operably linked to a 23-mer RSS comprises the human D_{H}2-8 gene segment. In some embodiments, the human D_{H} gene segment operably linked to a 23-mer RSS comprises the human D_{H}3-9 gene segment. In some embodiments, the human D_{H} gene segment operably linked to a 23-mer RSS comprises the human D_{H}3-10 gene segment. In some embodiments, the human D_{H} gene segment operably linked to a 23-mer RSS comprises the human D_{H}5-12 gene segment. In some embodiments, the human D_{H} gene segment operably linked to a 23-mer RSS comprises the human D_{H}6-13 gene segment. In some embodiments, the human D_{H} gene segment operably linked to a 23-mer RSS comprises the human D_{H}2-15 gene segment. In some embodiments, the human D_{H} gene segment operably linked to a 23-mer RSS comprises the human D_{H}3-16 gene segment. In some embodiments, the human D_{H} gene segment operably linked to a 23-mer RSS comprises the human D_{H}4-17 gene segment. In some embodiments, the human D_{H} gene segment operably linked to a 23-mer RSS comprises the human D_{H}5-18 gene segment. In some embodiments, the human D_{H} gene segment operably linked to a 23-mer RSS comprises the human D_{H}6-19 gene segment. In some embodiments, the human D_{H} gene segment operably linked to a 23-mer RSS comprises the human D_{H}1-20 gene segment. In some embodiments, the human D_{H} gene segment operably linked to a 23-mer RSS comprises the human D_{H}2-21 gene segment. In some embodiments, the human D_{H} gene segment operably linked to a 23-mer RSS comprises the human D_{H}3-22 gene segment. In some embodiments, the human D_{H} gene segment operably linked to a 23-mer RSS comprises the human D_{H}6-25 gene segment. In some embodiments, the human D_{H} gene segment operably linked to a 23-mer RSS comprises the human D_{H}1-26 gene segment. In some embodiments, the human D_{H} gene segment operably linked to a 23-mer RSS comprises the human D_{H}1-14 gene segment. In some embodiments, the human D_{H} gene segment operably linked to a 23-mer RSS comprises the human D_{H}4-11 gene segment. In some embodiments, the human D_{H} gene segment operably linked to a 23-mer RSS comprises the human D_{H}4-23 gene segment. In some embodiments, the human D_{H} gene segment operably linked to a 23-mer RSS comprises the human D_{H}5-24 gene segment. In some embodiments, the human D_{H} gene segment operably linked to a 23-mer RSS comprises the human D_{H}7-27 gene segment.

In some embodiments, provided rodents comprise the full or substantially full repertoire of human D_{H} gene segments generally arranged in the order found in an unrearranged human genomic variable locus, wherein one of the human D_{H} gene segments of the full or substantially full repertoire is replaced with a D_{H} gene segment engineered to be operably linked to a 23-mer RSS.
In some embodiments one of the human D_{H} gene segments of the full or substantially full repertoire is replaced with corresponding a D_{H} gene segment engineered to be operably linked to a 23-mer RSS, e.g., a wildtype D_{H}2-2 gene segment is replaced with a D_{H}2-2 gene segment engineered to be operably linked to a 23-mer RSS. In some embodiments, one of the human D_{H} gene segments of the full or substantially full repertoire is replaced with another D_{H} gene segment engineered to be operably linked to a 23-mer RSS, e.g., a wildtype D_{H}7-27 gene segment is replaced with a D_{H}3-3 gene segment engineered to be operably linked to a 23-mer RSS. In some embodiments, the D_{H}1-1 gene segment of a full or substantially full repertoire human D_{H} gene segments generally arranged in the order found in an unrearranged human genomic variable locus is replaced with a corresponding or another D_{H} gene segment engineered to be operably linked to a 23-mer RSS, e.g., a 3' 23-mer RSS. In some embodiments, the D_{H}1-1, D_{H}2-2, D_{H}2-8, D_{H}2-15, D_{H}2-21 gene segment(s) or any combination thereof of a full or substantially full repertoire human D_{H} gene segments generally arranged in the order found in an unrearranged human genomic variable locus is replaced with a corresponding or another D_{H} gene segment engineered to be operably linked to a 23-mer RSS, e.g., a 3' 23-mer RSS. In some embodiments, each of the D_{H}2-2, D_{H}2-8, and D_{H}2-15 gene segments of a full or substantially full repertoire human D_{H} gene segments generally arranged in the order found in an unrearranged human genomic variable locus is replaced with a corresponding or another D_{H} gene segment engineered to be operably linked to a 23-mer RSS, e.g., a 3' 23-mer RSS.

In some embodiments, provided rodents comprise at least human J_{H} gene segment J_{H}6. In some embodiments, provided rodents comprise at least human J_{H} gene segments J_{H}4, J_{H}5 and J_{H}6. In some embodiments, provided rodents comprise human J_{H} gene segments J_{H}1, J_{H}2, J_{H}3, J_{H}4, J_{H}5 and J_{H}6.

In some embodiments, a non-human immunoglobulin heavy chain constant region includes one or more non-human immunoglobulin heavy chain constant region genes such as, for example, immunoglobulin M (IgM), immunoglobulin D (IgD), immunoglobulin G (IgG), immunoglobulin E (IgE) and immunoglobulin A (IgA). In some embodiments, a non-human immunoglobulin heavy chain constant region includes a rodent IgM, rodent IgD, rodent IgG3, rodent IgG1, rodent IgG2b, rodent IgG2a, rodent IgE and rodent IgA constant region genes. In some embodiments, said human V_{H}, D_{H} and J_{H} gene segments are operably linked to one or more non-human immunoglobulin heavy chain enhancers (i.e., enhancer sequences or enhancer regions). In some embodiments, said human V_{H}, D_{H} and J_{H} gene segments are operably linked to one or more non-human immunoglobulin heavy chain regulatory regions (or regulatory sequences). In some embodiments, said human V_{H}, D_{H} and J_{H} gene segments are operably linked to one or more non-human immunoglobulin heavy chain enhancers (or enhancer sequence) and one or more non-human immunoglobulin heavy chain regulatory regions (or regulatory sequence).

In some embodiments, provided rodents do not contain (or lack) an endogenous Adam6 gene. In some embodiments, provided rodents do not contain or lack an endogenous Adam6 gene (or Adam6-encoding sequence) in the same germline genomic position as found in a germline genome of a wild-type rodent of the same species. In some embodiments, provided rodents do not contain or lack a human Adam6 pseudogene. In some embodiments, provided rodents comprise insertion of at least one nucleotide sequence that encodes one or more rodent Adam6 polypeptides. Said insertion may be outside of an engineered immunoglobulin heavy chain locus as described herein (e.g., upstream of a 5' most V_{H} gene segment), within an engineered immunoglobulin heavy chain locus or elsewhere in the germline genome of a rodent (e.g., a randomly introduced non-human Adam6-encoding sequence), cell or tissue. In some embodiments, provided rodents do not contain or lack a functional endogenous Adam6 pseudogene.

In some embodiments, a provided rodent, rodent cell or rodent tissue as described herein does not detectably express, in whole or in part, an endogenous rodent V_{H} region in an antibody molecule. In some embodiments, a provided rodent, rodent cell or rodent tissue as described herein does not contain (or lacks or contains a deletion of) one or more nucleotide sequences that encode, in whole or in part, an endogenous non-human V_{H} region (e.g., V_{H}, D_{H} and/or J_{H}) in an antibody molecule. In some embodiments, a provided rodent, rodent cell or rodent tissue as described herein has a germline genome that includes a deletion of endogenous non-human V_{H}, D_{H} and J_{H} gene segments, in whole or in part. In some embodiments, a provided rodent is fertile.

In some embodiments, provided rodents further comprise an engineered immunoglobulin κ light chain locus characterized by the presence of a plurality of human Vκ and Jκ gene segments arranged in germline configuration and inserted upstream of, and operably linked to, a non-human Cκ gene. In some embodiments, an engineered immunoglobulin κ light chain locus comprises at least human Vκ gene segments that appear in the proximal variable cluster (or proximal arm, or proximal duplication) of a human immunoglobulin κ light chain locus. In some embodiments, an engineered immunoglobulin κ light chain locus comprises at least human Vκ gene segments Yκ2-40, Vκ1-39, Vκ1-33, Vκ2-30, Vκ2-8, Vκ1-27, Vκ2-24, Vκ6-21, Vκ3-20, Vκ1-17, Vκ1-16, Vκ3-15, Vκ1-12, Vκ3-11, Vκ1-9, Vκ1-8, Vκ1-6, Vκ1-5, Vκ5-2 and Vκ4-1. In some embodiments, an engineered immunoglobulin κ light chain locus comprises at least 1, 2, 3, 4, 5, 10, or 15 human Vκ gene segments selected from Vκ2-40, Vκ1-39, Vκ1-33, Vκ2-30, Vκ2-8, Vκ1-27, Vκ2-24, Vκ6-21, Vκ3-20, Vκ1-17, Vκ1-16, Vκ3-15, Vκ1-12, Vκ3-11, Vκ1-9, Vκ1-8, Vκ1-6, Vκ1-5, Vκ5-2 and Vκ4-1. In some embodiments, an engineered immunoglobulin κ light chain locus comprises human Jκ gene segments Jκ1, Jκ2, Jκ3, Jκ4 and Jκ5. In some embodiments, an engineered immunoglobulin κ light chain locus comprises at least 1, 2, 3, or 4 human Jκ gene segments selected from Jκ1, Jκ2, Jκ3, Jκ4 and Jκ5.

In many embodiments, said human Vκ and Jκ gene segments are operably linked to one or more non-human immunoglobulin κ light chain enhancers (i.e., enhancer sequences or enhancer regions). In some embodiments, said human Vκ and Jκ gene segments are operably linked to a murine Igκ light chain intronic enhancer region (Igκ Ei or Eiκ). In many embodiments, said human Vκ and Jκ gene segments are operably linked to one or more non-human immunoglobulin κ light chain regulatory regions (or regulatory sequences). In some embodiments, said human Vκ and Jκ gene segments are operably linked to a murine Igκ light chain 3' enhancer region (Igκ 3'E or 3'Eκ). In some embodiments, said human Vκ and Jκ gene segments are operably linked to a murine Eiκ and operably linked to a murine 3'Eκ. In some embodiments, said human Vκ and Jκ gene segments are operably linked to one or more non-human immunoglobulin κ light chain enhancers (or enhancer sequences or enhancer regions) and one or more non-human immunoglobulin κ light chain regulatory regions (or regulatory sequences). In some embodiments, an engineered immunoglobulin κ light chain locus contains the same non-human immunoglobulin κ light chain enhancer regions (or enhancer sequences) that appear in a wild-type immunoglobulin κ light chain locus. In some embodiments, an engineered immunoglobulin κ light chain locus contains non-human Igκ light chain enhancer regions (or enhancer sequences) that appear in a wild-type immunoglobulin κ light chain locus of a different species (e.g., a different rodent species).

In some embodiments, an engineered immunoglobulin κ light chain locus as described herein does not contain (i.e., lacks) a human VpreB gene (or human VpreB gene-encoding sequence).

In some embodiments, a non-human Cκ gene of an engineered immunoglobulin κ light chain locus includes a rodent Cκ gene such as, for example, a mouse Cκ gene or a rat Cκ gene. In some embodiments, a non-human Cκ gene of an engineered immunoglobulin κ light chain locus is or comprises a mouse Cκ gene from a genetic background that includes a 129 strain, a BALB/c strain, a C57BL/6 strain, a mixed 129xC57BL/6 strain or combinations thereof.

In some embodiments, a provided rodent, rodent cell or rodent tissue as described herein does not detectably express, in whole or in part, an endogenous rodent Vκ region in an antibody molecule. In some embodiments, a provided rodent, rodent cell or rodent tissue as described herein does not contain (or lacks or contains a deletion of) one or more nucleotide sequences that encode, in whole or in part, an endogenous rodent Vκ region in an antibody molecule. In some embodiments, a provided rodent, rodent cell or rodent tissue as described herein has a germline genome that includes a deletion of endogenous rodent Vκ and Jκ gene segments, in whole or in part.

In some embodiments, provided rodent further comprise a wild-type or inactivated (e.g., by gene targeting) immunoglobulin λ light chain locus.

In some embodiments, a provided rodent, rodent cell or rodent tissue as described herein does not detectably express, in whole or in part, an endogenous rodent Vλ region in an antibody molecule. In some embodiments, a provided rodent, rodent cell or rodent tissue as described herein does not contain (or lacks, or contains a deletion of) one or more nucleotide sequences that encode, in whole or in part, an endogenous rodent Vλ region in an antibody molecule. In some embodiments, a provided rodent, rodent cell or rodent tissue as described herein has a germline genome that includes a deletion of endogenous rodent Vλ and Jλ gene segments, in whole or in part. In some embodiments, a provided rodent, rodent cell or rodent tissue as described herein as a germline genome that includes a deletion of endogenous non-human Vλ, Jλ and Cλ gene segments, in whole or in part.

Guidance for the creation of targeting vectors, non-human cells and animals harboring such engineered immunoglobulin loci can be found in U.S. Patent Nos. 8,642,835, 8,697,940, 9,006,511, 9,012,717, 9,029,628, 9,035,128, 9,066,502, 9,150,662 and 9,163,092. Persons skilled in the art are aware of a variety of technologies, known in the art, for accomplishing such genetic engineering and/or manipulation of non-human (e.g., mammalian) genomes or for otherwise preparing, providing, or manufacturing such sequences for introducing into the germline genome of non-human animals.

### DNA constructs

Typically, a polynucleotide molecule containing immunoglobulin gene segments as described herein, in particular, one or more D_{H} segments that each are operably linked to a 5' or 3' 23-mer RSS is inserted into a vector, preferably a DNA vector, in order to replicate the polynucleotide molecule in a suitable host cell.

Due to their size, D_{H} segments can be cloned directly from genomic sources available from commercial suppliers or designed *in silico* based on published sequences available from GenBank. Alternatively, bacterial artificial chromosome (BAC) libraries can provide immunoglobulin sequences. BAC libraries contain an average insert size of 100-150kb and are capable of harboring inserts as large as 300kb (Shizuya, et al., 1992, Proc. Natl. Acad. Sci., USA 89:8794-8797; Swiatek, et al., 1993, Genes and Development 7:2071-2084; Kim, et al., 1996, Genomics 34 213-218). For example, human and mouse genomic BAC libraries have been constructed and are commercially available (e.g., Invitrogen, Carlsbad Calif.). Genomic BAC libraries can also serve as a source of immunoglobulin sequences as well as transcriptional control regions.

Alternatively, immunoglobulin sequences may be isolated, cloned and/or transferred from yeast artificial chromosomes (YACs). An entire immunoglobulin locus, or substantial portion thereof, can be cloned and contained within one or a few YACs. If multiple YACs are employed and contain regions of overlapping homology, they can be recombined within yeast host strains to produce a single construct representing an entire locus. YAC arms can be additionally modified with mammalian selection cassettes by retrofitting to assist in introducing the constructs into non-human embryonic stems cells or non-human embryos by methods known in the art and/or described herein.

DNA constructs can be prepared using methods known in the art. For example, a DNA construct can be prepared as part of a larger plasmid. Such preparation allows the cloning and selection of the correct constructions in an efficient manner as is known in the art. DNA fragments containing one or more D_{H} segments that are each operably linked to a 5' or 3' 23-mer RSS as described herein can be located between convenient restriction sites on the plasmid so that they can be easily isolated from the remaining plasmid sequences for incorporation into the desired animal.

In some embodiments, methods employed in preparation of plasmids and transformation of host organisms are known in the art. For other suitable expression systems for both prokaryotic and eukaryotic cells, as well as general recombinant procedures, see Molecular Cloning: A Laboratory Manual, 2nd Ed., ed. by Sambrook, J. et al., Cold Spring Harbor Laboratory Press: 1989.

### Production of Rodents

Rodents are provided that express antibodies having heavy chain CDR3 diversity characterized by long amino acid length resulting from integration of 23-mer RSSs adjacent to one or more D_{H} segments that allow for D_{H}-to-D_{H} recombination within an immunoglobulin heavy chain variable region in the genome of the rodent. Suitable examples described herein include r in particular, mice. One or more D_{H} segments, in many embodiments, include heterologous D_{H} segments (e.g., human D_{H} segments). Rodent animals, embryos, cells and targeting constructs for making rodents, rodent embryos, and cells containing said D_{H} segments that are capable of D_{H}-D_{H} recombination at an increased frequency as compared to wild-type or reference rodents are also provided.

In some embodiments, one or more D_{H} segments are modified to be immediately adjacent to a 5' or 3' 23-mer RSS within a diversity cluster (i.e., a D_{H} region) of an immunoglobulin heavy chain variable region in the genome of a rodent. In some embodiments, a D_{H} region (or portion thereof) of an immunoglobulin heavy chain variable region is not deleted (i.e., intact). In some embodiments, a D_{H} region (or portion thereof) of an immunoglobulin heavy chain variable region is altered, disrupted, deleted, engineered or replaced with one or more D_{H} segments that are each operably linked to a 5' or 3' 23-mer RSS. In some embodiments, all or substantially all of a D_{H} region is replaced with one or more synthetic D_{H} segments that are each operably linked to a 5' or 3' 23-mer RSS; in some embodiments, one or more traditional D_{H} gene segments are not deleted or replaced in a D_{H} region of an immunoglobulin heavy chain variable region. In some embodiments, a D_{H} region contains both traditional D_{H} segments (i.e., one or more D_{H} segments each operably linked to a 5' 12-mer RSS and a 3' 12-mer RSS) and engineered D_{H} gene segments (i.e., one or more D_{H} segments each operably linked to a 5' or 3' 23-mer RSS. In some embodiments, a D_{H} region as described herein is a synthetic D_{H} region. In some embodiments, a D_{H} region is a human D_{H} region. In some embodiments, a D_{H} region is a murine D_{H} region. In some embodiments, an engineered D_{H} region (or portion thereof) as described herein is inserted into an immunoglobulin heavy chain variable region so that said engineered D_{H} region (or portion thereof) is operably linked with one or more V_{H} gene segments and/or one or more J_{H} gene segments. In some embodiments, said engineered D_{H} region is inserted into one of the two copies of an immunoglobulin heavy chain variable region, giving rise to a rodent that is heterozygous with respect to the said engineered D_{H} region. In some embodiments, a rodent is provided that is homozygous for an engineered D_{H} region. In some embodiments, a rodent is provided that is heterozygous for an engineered D_{H} region.

In some embodiments, a rodent as described herein contains a randomly integrated human immunoglobulin heavy chain variable region that includes a D_{H} region that contains one or more D_{H} segments that are each operably linked to a 5' or 3' 23-mer RSS within its genome. Thus, such rodents can be described as having a human immunoglobulin heavy chain transgene containing an engineered D_{H} region. An engineered D_{H} region can be detected using a variety of methods including, for example, PCR, Western blot, Southern blot, restriction fragment length polymorphism (RFLP), or a gain of allele (GOA) or loss of allele (LOA) assay. In some such embodiments, a rodent as described herein is heterozygous with respect to an engineered D_{H} region as described herein. In some such embodiments, a rodent as described herein is homozygous with respect to an engineered D_{H} region as described herein. In some such embodiments, a rodent as described herein is hemizygous with respect to an engineered D_{H} region as described herein. In some such embodiments, a rodent as described herein contains one or more copies of an engineered D_{H} region as described herein.

In some embodiments, an engineered D_{H} region of a rodent as described herein includes at least one D_{H} segment that is associated with (or operably linked to) a 5' 23-mer RSS. In some embodiments, an engineered D_{H} region of a rodent as described herein includes at least one D_{H} segment that is associated with (or operably linked to) a 3' 23-mer RSS. In some embodiments, an engineered D_{H} region of a rodent as described herein includes at least a human D_{H}3-3 segment that is associated with (or operably linked to) a 5' 23-mer RSS. In some embodiments, an engineered D_{H} region of a rodent as described herein includes at least a human D_{H}2 segment that is associated with (or operably linked to) a 3' 23-mer RSS, wherein the human D_{H}2 segment is selected from the group consisting of human D_{H}2-2, human D_{H}2-8, human D_{H}2-15 and human D_{H}2-21.

In some embodiments, an engineered D_{H} region of a rodent as described herein includes more than one D_{H} segment that are each associated with (or operably linked to) a 5' 23-mer RSS. In some embodiments, an engineered D_{H} region of a rodent as described herein includes more than one D_{H} segment that are each associated with (or operably linked to) a 3' 23-mer RSS. In some embodiments, an engineered D_{H} region of a rodent as described herein includes human D_{H}2-2, human D_{H}2-8, and human D_{H}2-15 segments that are each associated with (or operably linked to) a 3' 23-mer RSS.

Compositions and methods for making rodents whose genome comprises an immunoglobulin heavy chain variable region that includes an engineered D_{H} region, wherein the engineered D_{H} region includes one or more D_{H} segments that are each associated with (or operably linked to) a 5' or 3' 23-mer RSS, are provided, including compositions and methods for making rodents that express antibodies comprising a heavy chain variable region that includes a CDR3 region having an amino acid sequence encoded by more than one D_{H} segment from an immunoglobulin heavy chain locus that contains human V_{H} and J_{H} gene segments operably linked to one or more non-human heavy chain constant region genes. In some embodiments, compositions and methods for making rodents that express such antibodies under the control of an endogenous enhancer(s) and/or an endogenous regulatory sequence(s) are also provided. In some embodiments, compositions and methods for making rodents that express such antibodies under the control of a heterologous enhancer(s) and/or a heterologous regulatory sequence(s) are also provided. Methods include inserting one or more D_{H} segments and other sequences that that allow for D_{H}-D_{H} recombination at an increased frequency as compared to wild-type D_{H} segments, in the genome of a rodent so that an antibody is expressed which includes immunoglobulin heavy chains resulting from V(DD)J recombination.

In some embodiments, methods include inserting DNA that includes a D_{H} gene segment with a 5' 23-mer RSS and a 3' 12-mer RSS operably linked to one or more J_{H} gene segments. As described herein, the D_{H} gene segment is positioned downstream of a µ0 promoter sequence. In some embodiments, methods include inserting a D_{H} segment with a 5' 23-mer RSS and a 3' 12-mer RSS in a position relative to a µ0 promoter sequence so that said J_{H} gene segment is accessible to RAG genes (e.g., RAG-1 and/or RAG-2) during recombination. Genetic material that includes the D_{H} segment and flanking RSSs described above may be inserted into the genome of a rodent, thereby creating a non-human animal having an engineered D_{H} region that contains said D_{H} segment and necessary RSSs to allow for recombination with an adjacent D_{H} gene segment and V_{H} and J_{H} gene segments.

In some embodiments, methods include inserting DNA that includes three D_{H} gene segments each associated with a 5' 12-mer RSS and a 3' 23-mer RSS operably linked to six J_{H} gene segments. As described herein, the D_{H} gene segments are positioned amongst a plurality of D_{H} gene segments that are each associated with 5' and 3' 12-mer RSS. In some embodiments, methods include inserting D_{H}2-2, D_{H}2-8 and D_{H}2-15 gene segments each associated with a 5' 12-mer RSS and a 3' 23-mer RSS in a diversity cluster with a plurality of other DH gene segments each associated with traditional or wild-type RSS. Genetic material that includes the D_{H} gene segments and flanking RSSs described above may be inserted into the genome of a rodent, thereby creating a rodent having an engineered D_{H} region that contains said D_{H} segments and necessary RSSs to allow for recombination with adjacent D_{H} gene segments and V_{H} and J_{H} gene segments.

Where appropriate, sequences corresponding to (or encoding) D_{H} segments may be modified to include codons that are optimized for expression in the non-human animal (e.g., see U.S. Patent Nos. 5,670,356 and 5,874,304). Codon optimized sequences are synthetic sequences, and preferably encode the identical polypeptide (or a biologically active fragment of a full-length polypeptide which has substantially the same activity as the full-length polypeptide) encoded by the non-codon optimized parent polynucleotide. In some embodiments, sequences corresponding to (or encoding) D_{H} segments may include an altered sequence to optimize codon usage for a particular cell type (e.g., a rodent cell). For example, the codons of sequences corresponding to D_{H} segments to be inserted into the genome of a rodent may be optimized for expression in a cell of the non-human animal. Such a sequence may be described as a codon-optimized sequence.

Insertion of D_{H} segments operably linked to a 5' or 3' 23-mer RSS into a D_{H} region so that said D_{H} segments are operably linked to V_{H} and J_{H} gene segments (e.g., a plurality of V_{H} and J_{H} gene segments) employs a relatively minimal modification of the genome and results in expression of antibodies comprising heavy chains characterized by CDR3s having longer amino acid lengths.

Methods for generating transgenic non-human animals, including knockouts and knockins, are well known in the art (see, e.g., Gene Targeting: A Practical Approach, Joyner, ed., Oxford University Press, Inc. (2000)). For example, generation of transgenic rodents may optionally involve disruption of the genetic loci of one or more endogenous rodent genes (or gene segments) and introduction of one or more D_{H} segments each operably linked to a 23-mer RSS into the rodent genome, in some embodiments, at the same location as an endogenous rodent gene (or gene segments). In some embodiments, one or more D_{H} segments each operably linked to a 23-mer RSS are introduced into a D_{H} region of a randomly inserted immunoglobulin heavy chain locus in the genome of a rodent. In some embodiments, one or more D_{H} segments each operably linked to a 23-mer RSS are introduced into a D_{H} region of an endogenous immunoglobulin heavy chain locus in the genome of a rodent; in some embodiments, an endogenous immunoglobulin heavy chain locus is altered, modified, or engineered to contain human gene segments (e.g., V and/or J) operably linked to one or more constant region genes (e.g., human or murine).

A schematic illustration (not to scale) of representative targeting vectors for constructing an engineered D_{H} region and integration into rodent embryonic stem (ES) cells to create a rodent whose genome comprises an immunoglobulin heavy chain variable region that includes an engineered diversity cluster (i.e., D_{H} region), which diversity cluster includes one or more D_{H} segments each operably linked to a 5' or 3' 23-mer RSS is provided in Figure 2. Exemplary strategies and methods for inserting such vectors into immunoglobulin heavy chain variable regions in the genome of rodent ES cells are provided in Figures 3-8. In each of Figures 2-8, NotI restriction enzyme recognition sites are indicated were appropriate, names and approximate locations (small dash) of various primer/probe sets (see Table 4) are indicated for various alleles shown (not to scale), and unless otherwise noted, open symbols and lines represent human sequence, while closed symbols and dark lines represent mouse sequence. The following abbreviations are used for each of the figures, spec: spectinomycin resistance gene; neo: neomycin resistance gene; hyg: hygromycin resistance gene; lp: *lox*P site-specific recombination recognition site; Ei: murine heavy chain intronic enhancer; IgM: murine immunoglobulin M constant region gene; L: *lox*P site sequence; Frt: Flippase recognition target sequence; µ0 pro: µ0 promoter sequence.

As illustrated in Figure 2, DNA fragments containing a plurality of D_{H} segments with one D_{H} segments operably linked to a 5' 23-mer RSS (Figure 2, top and middle) or with three D_{H} segments each operably linked to a 3' 23-mer RSS (Figure 2, bottom) are made using VELOCIGENE^{®} technology (see, e.g., U.S. Patent No. 6,586,251 and Valenzuela et al., 2003, Nature Biotech. 21(6):652-659) and molecular biology techniques known in the art. In ***Figure 2****,* unless otherwise noted, open symbols and lines represent human sequence, while closed symbols and dark lines represent mouse sequence. The not-to-scale relative locations of the human V_{H}6-1, D_{H}2-2, D_{H}2-8, D_{H}2-15, D_{H}3-3, and J_{H}1, J_{H}2, J_{H}3, J_{H}4, J_{H}5 and J_{H}6 gene segments are depicted, when present and any gene segment remaining unnamed is a D_{H} gene segment. Generally, the D_{H} region as depicted with the hash marks comprises the full repertoire of unrearranged human D_{H} gene segments (see, e.g., www.imgt.org/IMGTrepertoire/index.php?section=LocusGenes&repertoire=locus&species=human &group=IGH) with the following exceptions: the top two targeting vectors lacks the last D_{H}7-27 gene, which is replaced by a sequence comprising the D_{H}3-3 gene segment flanked by a 5'-end 23-mer RSS and a 3'-end 12-mer RSS (depicted as an open arrow); the bottom targeting vector lacks the unrearranged human D_{H}2-2 gene segment, the unrearranged human D_{H}2-8 gene segment, and D_{H}2-15 gene segments, which are respectively replaced by the D_{H}2-2 gene segment flanked by a 5'-end 12-mer RSS and a 3'-end 23-mer RSS, the D_{H}2-8 gene segment flanked by a 5'-end 12-mer RSS and a 3'-end 23-mer RSS, and the D_{H}2-15 gene segment flanked by a 5'-end 12-mer RSS and a 3'-end 23-mer RSS (each of which engineered D_{H}2-2, D_{H}2-8, and D_{H}2-15 gene segment is depicted as an open arrow). Unfilled vertical rectangles represent locations of unique artificial 40-mers homologous to sequence primers and probes placed in the 12:D_{H}2-2:23|12:D_{H}2-8:23|12:D_{H}2-15:23 targeting vector to help insure correct modification of the targeting vector/ES cell. Sequences for the unique artificial 40-mers denoted as "1," "2," "10," "16," "8," and "18" are set forth as SEQ ID NO:73, SEQ ID NO:74, SEQ ID NO:75, SEQ ID NO:76, SEQ ID NO:77, and SEQ ID NO:78, respectively.

DNA fragments are assembled with homology arms for precise targeted insertion into a humanized immunoglobulin heavy chain variable region locus (Figures 3, 5, 7). Selection cassette (e.g., neomycin) flanked by site-specific recombination recognition sites (e.g., *lox*P) are included in the targeting vectors for facilitate screening of proper insertion in ES cells clones and may be removed by transient expression of a recombinase (e.g., Cre) in positive ES cell clones (Figures 4, 6, 8). The DNA fragments include the necessary sequences for proper assembly (i.e., recombination), transcription and expression of heavy chain variable regions once integrated into an immunoglobulin heavy chain locus. The targeting vectors are designed so that the engineered D_{H} region is flanked 5' by human V_{H} genomic DNA and 3' by human J_{H} genomic DNA and non-human (e.g., rodent) genomic heavy chain constant region DNA (e.g., intronic enhancer and a IgM constant region gene). The final targeting vectors for incorporation into the genome of a non-human cell (e.g., a rodent embryonic stem cell) contain V_{H} genomic DNA (e.g., containing one or more V_{H} gene segments), an engineered D_{H} region, 3' J_{H} genomic DNA, and non-human (e.g., rodent) genomic heavy chain constant region DNA, all of which are operably linked to allow for recombination between V_{H} gene segments, the engineered D_{H} region and a J_{H} gene segment once integrated into the genome of a non-human animal. Once assembled, the targeting vectors are linearized and electroporated into rodent ES cells.

The targeting vectors are introduced into rodent (e.g., mouse) embryonic stem cells so that the sequence contained in the targeting vector (i.e., an engineered D_{H} region) results in the capacity of a non-human cell or non-human animal (e.g., a mouse) that expresses antibodies that contain CDR3s having amino acids encoded by more than one D_{H} segment.

As described herein, transgenic rodents are generated where an engineered D_{H} region has been introduced into an immunoglobulin heavy chain locus of the rodent genome (e.g., an immunoglobulin heavy chain locus engineered to contain human variable region gene segments, which may be an endogenous immunoglobulin heavy chain locus so engineered).

Immunoglobulin loci comprising human variable region gene segments are known in the art and can be found, for example, in U. S. Pat. Nos. 5,633,425; 5,770,429; 5,814,318; 6,075,181; 6,114,598; 6,150,584; 6,998,514; 7,795,494; 7,910,798; 8,232,449; 8,502,018; 8,697,940; 8,703,485; 8,754,287; 8,791,323; 8,809,051; 8,907,157; 9,035,128; 9,145,588;9,206,263; 9,447,177; 9,551,124; 9,580,491 and 9,475,559, as well as in U.S. Pat. Pub. Nos. 20100146647, 20110195454, 20130167256, 20130219535, 20130326647, 20130096287, and 2015/0113668, and in PCT Pub. Nos. WO2007117410, WO2008151081, WO2009157771, WO2010039900, WO2011004192, WO2011123708 and WO2014093908.

In some embodiments, rodents as disclosed herein comprise exogenous fully human immunoglobulin transgenes comprising an engineered D_{H} region, which are able to rearrange in precursor B cells in mice (Alt et al., 1985, Immunoglobulin genes in transgenic mice, Trends Genet 1:231-236). In these embodiments, fully human immunoglobulin transgenes comprising an engineered D_{H} region may be (randomly) inserted and endogenous immunoglobulin genes may also be knocked-out (Green et al., 1994, Antigen-specific human monoclonal antibodies from mice engineered with human Ig heavy and light chain YACs, Nat Genet 7:13-21; Lonberg et al., 1994, Antigen-specific human antibodies from mice comprising four distinct genetic modifications, Nature 368:856-859; Jakobovits et al., 2007, From XenoMouse technology to panitumumab, the first fully human antibody product from transgenic mice, Nat Biotechnol 25:1134-1143) e.g., wherein endogenous immunoglobulin heavy chain and κ light chain loci are inactivated, e.g., by targeted deletion of small but critical portions of each endogenous locus, followed by introduction of human immunoglobulin gene loci as randomly integrated large transgenes, or mini chromosomes (Tomizuka et al., 2000, Double trans-chromosomic mice: maintenance of two individual human chromosome fragments containing Ig heavy and kappa loci and expression of fully human antibodies, PNAS USA 97:722-727.

In some embodiments, human or humanized immunoglobulin heavy and light chain loci comprising an engineered D_{H} region are at endogenous immunoglobulin heavy and light chain loci, respectively. A method for a large in situ genetic replacement of the mouse germline immunoglobulin variable gene loci with human germline immunoglobulin variable gene loci while maintaining the ability of the mice to generate offspring has been previously described. *See*, *e*.*g*., U.S. Patent Nos. 6,596,541 and 8,697,940. Specifically, the precise replacement of six megabases of both the mouse heavy chain and κ light chain immunoglobulin variable gene loci with their human counterparts while leaving the mouse constant regions intact is described. As a result, mice have been created that have a precise replacement of their entire germline immunoglobulin variable repertoire with equivalent human germline immunoglobulin variable sequences, while maintaining mouse constant regions. The human variable regions are linked to mouse constant regions to form chimeric human-mouse immunoglobulin loci that rearrange and express at physiologically appropriate levels. The antibodies expressed are "reverse chimeras," i.e., they comprise human variable region sequences and mouse constant region sequences. These mice having humanized immunoglobulin variable regions that express antibodies having human or humanized variable regions and mouse constant regions are called VELOCIMMUNE^{®} mice.

VELOCIMMUNE^{®} humanized mice exhibit a fully functional humoral immune system that is essentially indistinguishable from that of wild-type mice. They display normal cell populations at all stages of B cell development. They exhibit normal lymphoid organ morphology. Antibody sequences of VELOCIMMUNE^{®} mice exhibit normal V(D)J rearrangement and normal somatic hypermutation frequencies. Antibody populations in these mice reflect isotype distributions that result from normal class switching (e.g., normal isotype cis-switching). Immunizing VELOCIMMUNE^{®} mice results in robust humoral immune responses that generate large, diverse antibody repertoires having human immunoglobulin variable domains suitable for use as therapeutic candidates. This platform provides a plentiful source of naturally affinity-matured human immunoglobulin variable region sequences for making pharmaceutically acceptable antibodies and other antigen-binding proteins. It has also been shown that replacement of even a single endogenous V_{H} gene segment with a human V_{H} gene segment can result in an immune response comprising humanized immunoglobulin variable domain. *See*, *e*.*g*., Tien et al. (2016) Cell 166:1471-84. It is the precise replacement of mouse immunoglobulin variable sequences with human immunoglobulin variable sequences such that the human immunoglobulin variable sequences are operably linked with endogenous non-human constant region gene sequence(s) in a reverse chimeric manner that allows for making VELOCIMMUNE^{®} mice.

Mice modified in a reverse chimeric manner include mice modified to comprise at an endogenous immunoglobulin locus a human(ized) variable region (e.g., comprising (D), J, and one or more human V gene segments) operably linked to an endogenous constant region, e.g.,
(a) at an endogenous heavy chain locus:
   (i) an unrearranged human(ized) immunoglobulin heavy chain variable region in operable linkage to an endogenous heavy chain constant region, wherein the unrearranged human(ized) immunoglobulin heavy chain variable region comprises a plurality of unrearranged human heavy chain variable region V_{H} gene segments (e.g., all functional human unrearranged human V_{H} gene segments), one or more unrearranged immunoglobulin heavy chain D_{H} gene segments and one or more unrearranged immunoglobulin heavy chain J_{H} gene segments,
      optionally wherein the one or more unrearranged immunoglobulin heavy chain D_{H} gene segments and one or more unrearranged immunoglobulin heavy chain J_{H} gene segments are one or more unrearranged human immunoglobulin heavy chain D_{H} gene segments (e.g., all functional human D_{H} gene segments) and/or one or more unrearranged human immunoglobulin heavy chain J_{H} gene segments (e.g., all functional human J_{H} gene segments);
   (ii) a restricted unrearranged human(ized) heavy chain variable region in operable linkage to an endogenous heavy chain constant region, wherein the restricted unrearranged human(ized) heavy chain variable region consists essentially of a single unrearranged human heavy chain variable region V_{H} gene segment operably linked with one or more unrearranged immunoglobulin heavy chain D_{H} gene segments and one or more unrearranged immunoglobulin heavy chain J_{H} gene segments, optionally wherein the one or more unrearranged immunoglobulin heavy chain D_{H} gene segments and one or more unrearranged immunoglobulin heavy chain J_{H} gene segments are one or more unrearranged human immunoglobulin heavy chain D_{H} gene segments and/or one or more unrearranged human immunoglobulin heavy chain J_{H} gene segments, respectively;
   (iii) a histidine modified unrearranged human(ized) heavy chain variable region in operable linkage to an endogenous heavy chain constant region, wherein the histidine modified unrearranged human(ized) heavy chain variable region comprises an unrearranged immunoglobulin heavy chain variable gene sequence comprising in a complementarity determining region 3 (CDR3) encoding sequence a substitution of at least one non-histidine codon with a histidine codon or an insertion of at least one histidine codon; or
   (iv) a heavy chain only immunoglobulin encoding sequence comprising an unrearranged human(ized) heavy chain variable region in operable linkage to an endogenous heavy chain constant region, wherein the endogenous heavy chain constant region comprises (1) an intact endogenous IgM gene that encodes an IgM isotype that associates with light chain and (2) a non-IgM gene, e.g., an IgG gene, lacking a sequence that encodes a functional CH1 domain, wherein the non-IgM gene encodes a non-IgM isotype lacking a CH1 domain capable of covalently associating with a light chain constant domain;
      and/or
(b) at an endogenous light chain locus:
   (i) an unrearranged human(ized) immunoglobulin light chain variable region in operable linkage to an endogenous light chain constant region, wherein the unrearranged human(ized) immunoglobulin light chain variable region comprises a plurality of unrearranged human light chain variable region V_{L} gene segments (e.g., all functional human unrearranged human V_{L} gene segments) and one or more unrearranged immunoglobulin light chain J_{L} gene segments,
      optionally wherein the one or more unrearranged immunoglobulin light chain J_{L} gene segments are one or more unrearranged human immunoglobulin light chain J_{L} gene segments (e.g., all functional human J_{H}L gene segments),
      optionally wherein the endogenous immunoglobulin light chain locus is an endogenous immunoglobulin light chain kappa (κ) locus, the unrearranged human(ized) immunoglobulin light chain variable region comprises human variable κ (V_{κ}) and joining κ (JK) gene segments, and wherein the endogenous light chain constant region is an endogenous κ chain constant region sequence and/or wherein the endogenous immunoglobulin light chain locus is an endogenous immunoglobulin light chain lambda (λ), the unrearranged human(ized) immunoglobulin light chain variable region comprises human variable λ (V_{λ}) and joining λ (J_{λ}) gene segments, and the endogenous light chain constant region is an endogenous λ chain constant region sequence, optionally wherein the endogenous immunoglobulin light chain λ locus comprises (a) one or more human V_{λ} gene segments, (b) one or more human J_{λ} gene segments, and (c) one or more human C_{λ} gene segments, wherein (a) and (b) are operably linked to (c) and a rodent immunoglobulin light chain constant (C_{λ}) gene segment, and wherein the endogenous immunoglobulin λ light chain locus further comprises: one or more rodent immunoglobulin λ light chain enhancers (Eλ), and one or more human immunoglobulin λ light chain enhancers (Eλ), optionally comprising three human Eλs;
   (ii) a common light chain encoding sequence comprising a rearranged human(ized) light chain variable region sequence in operable linkage to an endogenous light chain constant region, wherein the rearranged human(ized) light chain variable region sequence comprises a human light chain variable region V_{L} gene segment rearranged with an immunoglobulin light chain J_{L} gene segment;
   (iii) a restricted unrearranged human(ized) light chain variable region in operable linkage to an endogenous light chain constant region, wherein the restricted unrearranged human(ized) light chain variable region comprises no more than two unrearranged human immunoglobulin light chain variable (V_{L}) gene segments operably linked to one or more unrearranged human immunoglobulin light chain joining (J_{L}) gene segments;
   (iv) a histidine modified unrearranged human(ized) light chain variable region in operable linkage to an endogenous light chain constant region, wherein the histidine modified unrearranged human(ized) light chain variable region comprises an unrearranged human(ized) immunoglobulin light chain variable gene sequence comprising in a complementarity determining region 3 (CDR3) encoding sequence a substitution of at least one non-histidine codon with a histidine codon or an insertion of at least one histidine codon; or
   (v) a histidine modified rearranged human(ized) light chain variable region in operable linkage to an endogenous light chain constant region, wherein the histidine modified rearranged human(ized) light chain variable region comprises a rearranged human(ized) immunoglobulin light chain variable gene sequence comprising in a complementarity determining region 3 (CDR3) encoding sequence a substitution of at least one non-histidine codon with a histidine codon or an insertion of at least one histidine codon,
   optionally wherein the mouse further comprises
   (i) a human(ized) immunoglobulin heavy chain locus comprising a functional ADAM6 gene such that the mouse exhibits wildtype fertility of the non-human animal; and/or
   (ii) an exogenous terminal deoxynucleotidyl transferase (TdT) gene for increased antigen receptor diversity, optionally such that at least 10% of the rearranged variable region genes comprise non-template additions,
   which mice have been previously described. *See*, *e*.*g*., U.S. Patent Nos. 8,697,940; 8,754,287; 9,204,624; 9,334,334; 9,801,362; 9,332,742; and 9,516,868; U.S. Patent Publications 20110195454, 20120021409, 20120192300, 20130045492; 20150289489; 20180125043; 20180244804; PCT Publication No. WO2019/113065, WO2017210586, and WO2011163314; Lee et al. (2014) Nature Biotechnology 32:356.

In some embodiments, the present invention includes a genetically modified non-human animal whose genome, e.g., germline genome, comprises:
an endogenous immunoglobulin locus comprising an immunoglobulin heavy chain variable region comprising a human V_{H} gene segment, a human engineered D_{H} gene region of the invention, and a human J_{H} gene segment, wherein the immunoglobulin heavy chain variable region is operably linked to a constant region, and/or
an endogenous chain locus comprising an immunoglobulin light chain variable region comprising a human VL gene segment and a human JL gene segments, wherein the immunoglobulin light chain variable region is operably linked to a constant region.

In some embodiments, a rodent, e.g., a rat or a mouse, comprises in its genome a replacement of one or more endogenous V_{H}, D_{H}, and J_{H} segments at an endogenous immunoglobulin heavy chain locus with one or more human V_{H}, D_{H}, and J_{H} segments, wherein the one or more human V_{H}, D_{H}, and J_{H} segments comprises a human D_{H} gene segment operably linked to a 23-mer RSS and are operably linked to an endogenous immunoglobulin heavy chain gene; and optionally an unrearranged or rearranged human V_{L} and human J_{L} segment operably linked to a rodent, e.g., a mouse or rat, or human immunoglobulin light chain constant (C_{L}) region gene, e.g., at an endogenous non-human light chain locus.

In certain embodiments, the genetically modified rodents comprise in their genome, e.g., germline genome, an immunoglobulin locus (exogenous or endogenous) containing an immunoglobulin variable region comprising one or more unrearranged human immunoglobulin variable region gene segments including an engineered D_{H} region and an immunoglobulin constant region comprising an immunoglobulin constant region gene and in which the one or more unrearranged human immunoglobulin variable region gene segments are operably linked to the immunoglobulin constant region gene.

Generally, a genetically modified immunoglobulin locus comprises an immunoglobulin variable region (comprising immunoglobulin variable region gene segments) operably linked to an immunoglobulin constant region. In some embodiments, the genetically modified immunoglobulin locus comprises one or more human unrearranged immunoglobulin heavy chain variable region gene segments, including an engineered D_{H} region, operably linked to a heavy chain constant region gene. In some embodiments, the genetically modified immunoglobulin locus comprises human unrearranged immunoglobulin variable region κ gene segments operably linked to a κ chain constant region gene. In some embodiments, the genetically modified immunoglobulin locus comprises human unrearranged immunoglobulin variable region λ gene segments operably linked to a κ chain constant region gene. In some embodiments, the genetically modified immunoglobulin locus comprises human unrearranged immunoglobulin variable region λ gene segments operably linked to a λ chain constant region gene.

In certain embodiments, the rodent comprises at an endogenous heavy chain locus an unrearranged human(ized) immunoglobulin heavy chain variable region comprising an engineered D_{H} region in operable linkage to an endogenous heavy chain constant region, wherein immunoglobulin variable region contains one or more unrearranged human Ig heavy chain variable region gene segments. In some embodiments, the one or more unrearranged human Ig variable region gene segments comprises at least one human immunoglobulin heavy chain variable (V_{H}) segment, one or more immunoglobulin heavy chain diversity (D_{H}) segments (e.g., one or more unrearranged human D_{H} segments operably linked to a 23-mer RSS), and one or more immunoglobulin heavy chain joining (J_{H}) segments (optionally one or more unrearranged human J_{H} segments). In some embodiments, the unrearranged human Ig variable region gene segments comprise a plurality of unrearranged human V_{H} segments, one or more unrearranged (human) D_{H} segments (e.g., one or more unrearranged (human) D_{H} segments operably linked to a 23-mer RSS) and one or more unrearranged (human) J_{H} segments. In some embodiments, the unrearranged human Ig variable region gene segments comprise at least 3 V_{H} gene segments, at least 18 V_{H} gene segments, at least 20 V_{H} gene segments, at least 30 V_{H} gene segments, at least 40 V_{H} gene segments, at least 50 V_{H} gene segments, at least 60 V_{H} gene segments, at least 70 V_{H} gene segments, or at least 80 V_{H} gene segments. In some embodiments, the unrearranged human Ig gene segments include all of the functional human D_{H} gene segments, wherein at least one of the functional human D_{H} gene segments is modified to be operably linked to a 23-mer RSS. In some embodiments, the unrearranged human Ig gene segments include all of the functional human J_{H} gene segments. Exemplary variable regions comprising Ig heavy chain gene segments are provided, for example, in Macdonald et al, Proc. Natl. Acad. Sci. USA 111 :5147-52.

In some embodiments, the rodents provided herein comprise at an endogenous heavy chain locus a restricted unrearranged human(ized) heavy chain variable region in operable linkage to an endogenous heavy chain constant region comprising at least a non-human IgM gene, wherein the restricted unrearranged human(ized) heavy chain variable region is characterized by a single human V_{H} gene segment, a plurality of D_{H} gene segments (e.g., human D_{H} gene segments, including one or more unrearranged (human) D_{H} segments operably linked to a 23-mer RSS) and a plurality of J_{H} gene segments (e.g. human J_{H} gene segments), wherein the restricted immunoglobulin heavy chain locus is capable of rearranging and forming a plurality of distinct rearrangements, wherein each rearrangement is derived from the single human V_{H} gene segment, one of the D_{H} segments, and one of the J_{H} segments, and wherein each rearrangement encodes a different heavy chain variable domain (e.g., as described in U.S. Pat. Pub. No. 20130096287). In some embodiments the single human V_{H} gene segment is V_{H}1-2 or V_{H}1-69.

In certain embodiments, a rodent comprises at an endogenous light chain locus an unrearranged human(ized) immunoglobulin light chain variable region in operable linkage to an endogenous light chain constant region. In some embodiments the unrearranged human(ized) immunoglobulin light chain variable region contains unrearranged human Ig κ variable region gene segments. In some embodiments, the unrearranged human(ized) immunoglobulin variable region comprises a plurality of unrearranged human Vκ segments and one or more unrearranged human J_{K} segments. In some embodiments, the unrearranged human immunoglobulin variable region gene segments comprise all of the human Jκ segments. In some embodiments, the immunoglobulin variable region gene segments comprise four functional Vκ segments and all human Jκ segments. In some embodiments, the immunoglobulin variable region gene segments comprise 16 functional Vκ segments and all human Jκ segments (e.g., all functional human Vκ segments and Jκ segments). In some embodiments, the unrearranged human immunoglobulin variable region gene segments comprise all of the human Vκ segments and all human Jκ segments. Exemplary variable regions comprising Ig κ gene segments are provided, for example, in Macdonald et al, Proc. Natl. Acad. Sci. USA 1 11 :5147-52 and supplemental information.

In some embodiments, a restricted unrearranged human(ized) light chain variable region in operable linkage to an endogenous light chain constant region is characterized in that the unrearranged human(ized) light chain variable region comprises no more than two human V_{L} gene segments and a plurality of J_{L} gene segments (e.g., dual light chain mice, or DLC, as described in U. S. Pat. No. 9,796,788). In some embodiments the V_{L} gene segments are Vκ gene segments. In some embodiments the V_{L} gene segments are Vλ gene segments. In some embodiments the Vκ gene segments are IGKV3-20 and IGKV1 -39. In some embodiments, a rodent comprises exactly two unrearranged human Vκ gene segments and five unrearranged human Jκ gene segments operably linked to a mouse light chain constant region at the endogenous κ light chain loci of the mouse, optionally wherein the exactly two unrearranged human Vκ gene segments are a human Vκ1-39 gene segment and a human Vκ3-20 gene segment, wherein the five unrearranged human Jκ gene segments are a human Jκ1 gene segment, a human Jκ2 gene segment, a human Jκ3 gene segment, a human Jκ4 gene segment, and a human Jκ5 gene segment, wherein the unrearranged human kappa light chain gene segments are capable of rearranging and encoding human variable domains of an antibody, and optionally further wherein the non-human animal does not comprise an endogenous Vκ gene segment that is capable of rearranging to form an immunoglobulin light chain variable region.

In certain embodiments, the unrearranged human(ized) immunoglobulin light chain variable region in operable linkage to an endogenous light chain constant region contains unrearranged human Igλ variable region gene segments. In some embodiments, the unrearranged human immunoglobulin variable region gene segments comprise a plurality of human Vλ segments and one or more human Jλ segments. In some embodiment, the unrearranged human immunoglobulin variable region gene segments comprise one or more human Vλ segments, one or more human Jλ segments, and one or more human Cλ constant region sequences. In some embodiments, the unrearranged human immunoglobulin variable region gene segments comprise all of the human Vλ segments. In some embodiments, the unrearranged human immunoglobulin variable region gene segments comprise all of the human Jλ segments. Exemplary variable regions comprising Ig λ gene segments are provided, for example, U. S. Pat. Nos. 9,035,128 and 6,998,514. In some embodiments, the unrearranged human(ized) immunoglobulin light chain variable region in operable linkage to an endogenous light chain constant region comprises (a) one or more human Vλ gene segments, (b) one or more human Jλ gene segments, and (c) one or more human Cλ gene segments, wherein (a) and (b) are operably linked to (c) and an endogenous (e.g., rodent) Cλ gene segment, and wherein the endogenous immunoglobulin λ light chain locus further comprises: one or more rodent immunoglobulin λ light chain enhancers (Eλ), and one or more human immunoglobulin λ light chain enhancers (Eλ), optionally comprising three human Eλ.

In certain embodiments, the unrearranged human(ized) immunoglobulin light chain variable region in operable linkage to an endogenous light chain constant region comprises an unrearranged human Igλ variable region gene segments operably linked to an endogenous (e.g., rodent, e.g., rat or mouse) Cκ gene such that the non-human animal expresses an immunoglobulin light chain that comprises a human λ variable domain sequence derived from the Vλ and Jλ gene segments fused with an endogenous κ constant domain, see, e.g., US Patent No. 9,226,484.

In some embodiments, the immunoglobulin variable region comprising unrearranged human immunoglobulin variable region gene segments also includes human immunoglobulin variable region intergenic sequences. In some embodiments, the immunoglobulin variable region includes non-human (e.g., rodent, rat, mouse) Ig variable region intergenic sequences. In some embodiments, the intergenic sequence is of endogenous species origin.

In some embodiments, the immunoglobulin variable region is a rearranged light variable region (a universal light chain variable region). In some embodiments, the rearranged Ig light chain variable region gene is a human rearranged Ig light chain variable region gene. Exemplary rearranged Ig light chain variable regions are provided in, e.g., U.S. Patent Nos.: 9,969,814; 10,130,181, and 10,143,186 and U.S. Patent Pub. Nos. 20120021409, 20120192300, 20130045492, 20130185821, 20130302836, and 20150313193. In some embodiments, the rodent ("universal light chain" organism) comprising a universal light chain variable region is used to produce bispecific antibodies. In some embodiments, a common light chain encoding sequence comprises a single rearranged human immunoglobulin light chain Yκ/Jκ sequence operably linked to an endogenous light chain constant region, wherein the single rearranged human immunoglobulin light chain Yκ/Jκ sequence is either (i) a human Vκ1-39/Jκ5 sequence comprising a human Vκ1-39 gene segment fused to a human Jκ5 gene segment, or (ii) a human Vκ3-20/Jκ1 sequence comprising a human Vκ3-20 gene segment fused to a human Jκ1 gene segment.

In some embodiments, the immunoglobulin variable region is a light chain and/or a heavy chain immunoglobulin variable region that includes insertions and/or replacements of histidine codons designed to introduce pH- dependent binding properties to the antibodies generated in such non-human organism. In some of such embodiments, the histidine codons are inserted and/or replaced in the nucleic acid sequences encoding CDR3. Various such light and/or heavy immunoglobulin loci are provided in U.S. Patent Nos. 9,301,510; 9,334,334; and 9,801,362 and U.S. Patent Application Publication No. 20140013456. In some embodiments, the histidine modified rearranged human(ized) light chain variable region in operable linkage to an endogenous light chain constant region comprises a single rearranged human immunoglobulin light chain variable region gene sequence comprising human Vκ and Jκ segment sequences, optionally wherein the Vκ segment sequence is derived from a human Vκ1-39 or Vκ3-20 gene segment, and wherein the single rearranged human immunoglobulin light chain variable region gene sequence comprises a substitution of at least one non-histidine codon of the Vκ segment sequence with a histidine codon that is expressed at a position selected from the group consisting of 105, 106, 107, 108, 109, 111 and a combination thereof (according to IMGT numbering). In some embodiments, the histidine modified unrearranged human(ized) heavy chain variable region in operable linkage to an endogenous heavy chain constant region comprises an unrearranged human(ized) immunoglobulin heavy chain variable gene sequence comprising in a complementarity determining region 3 (CDR3) encoding sequence (e.g., in a (human) D_{H} gene segment modified to be operably linked to a 23-mer RSS) a substitution of at least one non-histidine codon with a histidine codon or an insertion of at least one histidine codon. In some embodiments, the unrearranged human(ized) immunoglobulin heavy chain variable gene sequence comprises unrearranged human V_{H}, unrearranged human D_{H} or synthetic D_{H} including a D_{H} gene segment operably linked to a 23-mer RSS, and unrearranged human J_{H} gene segments, optionally wherein the unrearranged human D_{H} or synthetic D_{H} gene segment or D_{H} gene segment operably linked to a 23-mer RSS comprises the substitution of at least one non-histidine codon with a histidine codon or an insertion of at least one histidine codon. In some embodiments, the histidine modified unrearranged human(ized) light chain variable region in operable linkage to an endogenous heavy chain constant region comprises unrearranged V_{L} and unrearranged J_{L} gene segments. In some embodiments, the histidine modified unrearranged human(ized) light chain variable region comprises no more than two unrearranged human V_{L} (e.g., no more than two Vκ gene segments) and one or more unrearranged human J_{L} (e.g., Jκ) gene segment(s), wherein each of the no more than two human V_{L} gene segments comprises in a CDR3 encoding sequence a substitution of at least one non-histidine codon with a histidine codon or an insertion of at least one histidine codon. In some embodiments, the no more than two unrearranged human Vκ gene segments are human Vκ1-39 and Vκ3-20 gene segments each comprising one or more substitutions of a non-histidine codon with a histidine codon, and wherein the human Vκ and Jκ gene segments are capable of rearranging and the human Vκ and Jκ gene segments encode a human light chain variable domain comprising one or more histidines at a position selected from the group consisting of 105, 106, 107, 108, 109, 111 (according to IGMT numbering), and a combination thereof, wherein the one or more histidines are derived from the one or more substitutions.

In some embodiments, the immunoglobulin constant region comprises a heavy chain constant region gene. In some embodiments, the heavy chain constant region gene is a human heavy chain constant region gene. In some embodiments, the heavy chain constant region gene is of endogenous species origin. In some embodiments, the heavy chain constant region gene is a mouse constant region gene or a rat constant region gene. In some embodiments, the constant region gene is a mixture of human and non-human sequence. For example, in some embodiments, the constant region gene encodes a human CH1 region and a non-human (e.g., endogenous species origin, mouse, rat) CH2 and/or CH3 region. In some embodiments, the heavy chain constant region gene is an Cµ, Cδ, Cγ (Cγ1, Cγ2, Cγ3, Cγ4), Cα or Cε constant region gene. In some embodiments, the constant region gene is an endogenous constant region gene. In some embodiments, the constant region gene encodes a mutated CH1 region so that the non-human animal expresses heavy chain only antibodies (see., e.g., U.S. Patent No. 8,754,287, U.S. Patent Application Publication No. 2015/0289489). In some embodiments, e.g., where the goal is to generate heavy chains to make bispecific antibodies (e.g., in universal or dual light chain organisms), the Fc domains of the heavy chains comprise modifications to facilitate heavy chain heterodimer formation and/or to inhibit heavy chain homodimer formation. Such modifications are provided, for example, in U.S. Pat. Nos. 5,731,168; 5,807,706; 5,821,333; 7,642,228 and 8,679,785 and in U.S. Pat. Pub. No. 2013/0195849.

In some embodiments, the immunoglobulin constant region comprises a light chain constant region gene. In some embodiments, the light chain constant region gene is a κ constant region gene. In some embodiments, the light chain constant region gene is a λ constant region gene. In some embodiments, the light chain constant region gene is of endogenous species origin. In some embodiments, the light chain constant region gene is a mouse constant region gene or a rat constant region gene. In some embodiments, the light chain constant region gene is a mixture of human and non-human sequence.

In some embodiments, the immunoglobulin variable region comprising human variable region gene segments and the immunoglobulin constant region gene to which the variable region gene segments are operably linked are located at an endogenous immunoglobulin locus. In some embodiments, the endogenous immunoglobulin locus is an endogenous heavy chain locus. In some embodiments, the endogenous immunoglobulin locus is an endogenous κ locus. In some embodiments, the endogenous immunoglobulin locus is an endogenous λ locus. In some embodiments, the constant region gene to which the human variable region gene segments are operably linked is an endogenous constant region gene.

In some embodiments, one or more of the endogenous immunoglobulin loci or a portion of the one or more endogenous loci (e.g., a variable region and/or a constant region) in the genome of the rodent provided herein is inactivated. Endogenous immunoglobulin variable region gene loci and portions thereof can be inactivated using any method known in the art, including, but not limited to, the deletion of the locus or a portion thereof from the genome of the organism, the replacement of a locus or a portion thereof with a different nucleic acid sequence, the inversion of a portion of the locus and/or the displacement of a portion of the locus to another position in the genome of the non-human organism. In some embodiments the inactivation of the locus is only a partial inactivation. In some embodiments, the variable region of the locus is inactivated but the constant region remains functional (e.g., because it is operably linked to non-endogenous variable region gene segments).

In some embodiments, the genetically modified rodent includes an inactivated endogenous immunoglobulin heavy chain locus. In some embodiments, the endogenous immunoglobulin heavy chain locus or a portion thereof is inactivated by deletion, replacement, displacement and/or inversion of at least part of the endogenous variable region of the endogenous heavy chain locus. In some embodiments, the at least part of the variable region of the endogenous heavy chain locus that is deleted, replaced, displaced, and/or inverted comprises the J segments of the variable region. In some embodiments, the endogenous immunoglobulin heavy chain locus or portion thereof is inactivated by deletion, replacement, displacement and/or inversion of at least part of the endogenous constant region of the endogenous heavy chain locus. In some embodiments, the at least part of the constant region of the endogenous heavy chain locus that is deleted, replaced, displaced, and/or inverted comprises the Oµ gene of the endogenous constant region.

In some embodiments, the genetically modified rodent includes an inactivated endogenous immunoglobulin κ chain locus. In some embodiments, the endogenous immunoglobulin κ chain locus or a portion thereof is inactivated by deletion, replacement, displacement and/or inversion of at least part of the endogenous variable region of the endogenous κ chain locus. In some embodiments, the at least part of the variable region of the endogenous κ chain locus that is deleted, replaced, displaced, and/or inverted comprises the J segments of the variable region. In some embodiments, the endogenous immunoglobulin κ chain locus or portion thereof is inactivated by deletion, replacement, displacement and/or inversion of at least part of the endogenous constant region of the endogenous κ chain locus. In some embodiments, the at least part of the constant region of the endogenous κ chain locus that is deleted, replaced, displaced, and/or inverted comprises the Cκ gene of the endogenous constant region.

In some embodiments, the genetically modified rodent includes an inactivated endogenous immunoglobulin λ chain locus. In some embodiments, the endogenous immunoglobulin λ chain locus or a portion thereof is inactivated by deletion, replacement, displacement and/or inversion of at least part of an endogenous variable region of the endogenous λ chain locus. In some embodiments, the at least part of at least one V-J-C gene cluster in the endogenous λ chain locus is deleted, replaced, displaced, and/or inverted. In some embodiments, the endogenous immunoglobulin λ chain locus or portion thereof is inactivated by deletion, replacement, displacement and/or inversion of at least part of an endogenous constant region of the endogenous λ chain locus. In some embodiments, the at least part of the constant region of the endogenous λ chain locus that is deleted, replaced, displaced, and/or inverted comprises a C gene of the endogenous constant region.

In various embodiments, the immunoglobulin locus modifications do not affect fertility of the rodent. In some embodiments, the heavy chain locus comprises a functional, e.g., endogenous ADAM6a gene, ADAM6b gene, or both, and the genetic modification does not affect the expression and/or function of the endogenous ADAM6a gene, ADAM6b gene, or both. In some embodiments, the genome of the genetically modified rodent further comprises an ectopically located functional, e.g., endogenous ADAM6a gene, ADAM6b gene, or both. Exemplary rodents expressing exogenous ADAM6a and/or ADAM6b are described in U.S. Pat. Nos. 8,642,835 and 8,697,940.

In some embodiments, the genetically modified rodent further comprises and expresses an exogenous terminal deoxynucleotidyl transferase (TdT) for increased antigen receptor diversity. Exemplary non-human animals expressing exogenous TdT are described in PCT Publication WO 2017210586.

In some embodiments, the genome of a provided rodent further comprises one or more human immunoglobulin heavy and/or light chain genes (see, e.g., U.S. Patent No. 8,502,018; U.S.

Patent No. 8,642,835; U.S. Patent No. 8,697,940; U.S. Patent No: 8,791,323; and U.S. Patent Application Publication Nos. 2013/0096287 A1 and 2018/0125043 A1; and PCT Publication No. WO2019/113065). Alternatively, an engineered D_{H} region can be introduced into an embryonic stem cell of a different modified strain such as, e.g., a VELOCIMMUNE^{®} strain (see, e.g., U.S. Patent No. 8,502,018 or U.S. Patent No. 8,642,835). In some embodiments, rodents as described herein may be prepared by introducing a targeting vector as described herein, into a cell from a modified strain. To give but one example, a targeting vector as described herein, may be introduced into a rodent as described in U.S. Patent Nos. 8,642,835 and 8,697,940, which rodent expresses antibodies that have fully human variable regions and mouse constant regions. In some embodiments, rodents as described herein are prepared to further comprise human immunoglobulin genes (variable and/or constant region genes). In some embodiments, rodents as described herein comprise an engineered D_{H} region as described herein, and genetic material from a heterologous species (e.g., humans), wherein the genetic material encodes, in whole or in part, one or more human heavy and/or light chain variable regions.

The rodents as described herein may be prepared as described above, or using methods known in the art, to comprise additional human or humanized genes, oftentimes depending on the intended use of the rodent. Genetic material of such additional human or humanized genes may be introduced through the further alteration of the genome of cells (e.g., embryonic stem cells) having the genetic modifications as described above or through breeding techniques known in the art with other genetically modified strains as desired.

For example, as described herein, rodents comprising an engineered D_{H} region may further comprise (e.g., via cross-breeding or multiple gene targeting strategies) one or more modifications as described U.S. Patent Application Publication Nos. 2011-0195454 A1, 2012-0021409 A1, 2012-0192300 A1, 2013-0045492 A1, 2013-0185821 A1, 2013-0198880 A1, 2013-0302836 A1, 2015-0059009 A1; International Patent Application Publication Nos. WO 2011/097603, WO 2012/148873, WO 2013/134263, WO 2013/184761, WO 2014/160179, WO 2014/160202.

A transgenic founder rodent can be identified based upon the presence of an engineered D_{H} region in its genome and/or expression of antibodies that include CDR3 regions containing amino acids resulting from D_{H}-D_{H} recombination in tissues or cells of the non-human animal. A transgenic founder rodent can then be used to breed additional rodents carrying the engineered D_{H} region thereby creating a series of non-rodents each carrying one or more copies of an engineered D_{H} region. Moreover, transgenic rodents s carrying an engineered D_{H} region can further be bred to other transgenic rodents carrying other transgenes (e.g., human immunoglobulin genes) as desired.

Rodents may also be produced to contain selected systems that allow for regulated or directed expression of the transgene. Exemplary systems include the Cre/*lox*P recombinase system of bacteriophage PI (see, e.g., Lakso, M. et al., 1992, Proc. Natl. Acad. Sci. USA 89:6232-6236) and the FLP/Frt recombinase system of S. cerevisiae (O'Gorman, S. et al, 1991, Science 251:1351-1355). Such animals can be provided through the construction of *"double"* transgenic animals, e.g., by mating two transgenic animals, one containing a transgene comprising a selected modification (e.g., an engineered D_{H} region) and the other containing a transgene encoding a recombinase (e.g., a Cre recombinase).

Although embodiments employing an engineered D_{H} region in a mouse (i.e., a mouse with an engineered D_{H} region operably linked with human V_{H} and J_{H} gene segments, all of which are operably linked with one or more murine heavy chain constant region genes) are extensively discussed herein, other rodents that comprise an engineered D_{H} region are also provided. In some embodiments, such rodents comprise an engineered D_{H} region operably linked to endogenous V_{H} and J_{H} gene segments. In some embodiments, such non-rodents comprise an engineered D_{H} region operably linked to humanized V_{H} and J_{H} gene segments. Such rodents include any of those which can be genetically modified to express antibodies having CDR3s that include amino acids resulting from D_{H}-D_{H} recombination at an increased frequency as compared to a wild-type immunoglobulin heavy chain locus as disclosed herein, including, e.g., mouse, rat, etc. For example, for those rodents for which suitable genetically modifiable ES cells are not readily available, other methods are employed to make a non-human animal comprising the genetic modification. Such methods include, e.g., modifying a non-ES cell genome (e.g., a fibroblast or an induced pluripotent cell) and employing somatic cell nuclear transfer (SCNT) to transfer the genetically modified genome to a suitable cell, e.g., an enucleated oocyte, and gestating the modified cell (e.g., the modified oocyte) in a rodent under suitable conditions to form an embryo.

Methods for modifying a non-human animal genome (e.g., a pig, cow, rodent, chicken, etc.) include, e.g., employing a zinc finger nuclease (ZFN), a transcription activator-like effector nuclease (TALEN) or a Cas protein (i.e., a CRISPR/Cas system) to modify a genome to include an engineered D_{H} region as described herein. Guidance for methods for modifying the germline genome of a non-human animal can be found in, e.g., U.S. Patent Application Publication Nos. 2015-0376628 A1, US 2016-0145646 A1 and US 2016-0177339 A1.

In some embodiments, a rodent as described herein is selected from a mouse, a rat, and a hamster. In some embodiments, a rodent as described herein is selected from the superfamily Muroidea. In some embodiments, a genetically modified animal as described herein is from a family selected from Calomyscidae (e.g., mouse-like hamsters), Cricetidae (e.g., hamster, New World rats and mice, voles), Muridae (true mice and rats, gerbils, spiny mice, crested rats), Nesomyidae (climbing mice, rock mice, with-tailed rats, Malagasy rats and mice), Platacanthomyidae (e.g., spiny dormice), and Spalacidae (e.g., mole rates, bamboo rats, and zokors). In some embodiments, a genetically modified rodent as described herein is selected from a true mouse or rat (family Muridae), a gerbil, a spiny mouse, and a crested rat. In some embodiments, a genetically modified mouse as described herein is from a member of the family Muridae. In some embodiments, a rodent as described herein is selected from a mouse and a rat. In some embodiments, rodet as described herein is a mouse.

In some embodiments, a rodent as described herein is a rodent that is a mouse of a C57BL strain selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, and C57BL/Ola. In some embodiments, a mouse of the present invention is a 129-strain selected from the group consisting of a strain that is 129P1, 129P2, 129P3, 129X1, 129S1 (e.g., 129S1/SV, 129S1/SvIm), 129S2, 129S4, 129S5, 129S9/SvEvH, 129/SvJae, 129S6 (129/SvEvTac), 129S7, 129S8, 129T1, 129T2 (see, e.g., Festing et al., 1999, Mammalian Genome 10:836; Auerbach, W. et al., 2000, Biotechniques 29(5):1024-1028, 1030, 1032). In some embodiments, a genetically modified mouse as described herein is a mix of an aforementioned 129 strain and an aforementioned C57BL/6 strain. In some embodiments, a mouse as described herein is a mix of aforementioned 129 strains, or a mix of aforementioned BL/6 strains. In some embodiments, a 129 strain of the mix as described herein is a 129S6 (129/SvEvTac) strain. In some embodiments, a mouse as described herein is a BALB strain, e.g., BALB/c strain. In some embodiments, a mouse as described herein is a mix of a BALB strain and another aforementioned strain.

In some embodiments, a rodent as described herein is a rat. In some embodiments, a rat as described herein is selected from a Wistar rat, an LEA strain, a Sprague Dawley strain, a Fischer strain, F344, F6, and Dark Agouti. In some embodiments, a rat strain as described herein is a mix of two or more strains selected from the group consisting of Wistar, LEA, Sprague Dawley, Fischer, F344, F6, and Dark Agouti.

### Methods

Several *in vitro* and *in vivo* technologies have been developed for the production of antibody-based therapeutics. In particular, *in vivo* technologies have featured the production of transgenic animals (i.e., rodents) containing human immunoglobulin genes either randomly incorporated into the genome of the animal (e.g., see U.S. Patent No. 5,569,825) or precisely placed at an endogenous immunoglobulin locus in operable linkage with endogenous immunoglobulin constant regions of the animal (e.g., see U.S. Patent Nos. 8,502,018; 8,642,835; 8,697,940; and 8,791,323). Both approaches have been productive in producing promising antibody therapeutic candidates for use in humans. Further, both approaches have the advantage over *in vitro* approaches in that antibody candidates are chosen from antibody repertoires generated *in vivo,* which includes selection for affinity and specificity for antigen within the internal milieu of the host's immune system. In this way, antibodies bind to naturally presented antigen (within relevant biological epitopes and surfaces) rather than artificial environments or *in silico* predictions that can accompany *in vitro* technologies. Despite the robust antibody repertoires produced from *in vivo* technologies, antibodies to complex (e.g., viruses, channel polypeptides) or cytoplasmic antigens remains difficult. Further, generating antibodies to polypeptides that share a high degree of sequence identity between species (e.g., human and mouse) remains a challenge due to immune tolerance.

Thus, the present invention is, among other things, based on the recognition that the construction of an *in vivo* system characterized by the production of antibodies having added diversity in CDRs, in particular, CDR3s, generated from non-traditional gene segment rearrangement (i.e., D_{H} to D_{H} recombination) can be made using one or more D_{H} segments that are each operably linked to a 5' or 3' 23-mer RSS. By having the 5' or 3' 23-mer RSS, recombination between D_{H} segments is increased as compared to an immunoglobulin heavy chain locus that lacks such engineered D_{H} gene segments. Such added diversity can direct binding to particular antigens (e.g., viruses, channel polypeptides). Upon recombination of a V_{H} gene segment, at least two D_{H} gene segments, and a J_{H} gene segment, a heavy chain variable region coding sequence is formed that contains a CDR3 region having an amino acid sequence resulting from D_{H}-to-D_{H} recombination. Furthermore, this CDR3 resulting from D_{H} to D_{H} recombination contains an added diversity due to increased amino acid length. Furthermore, such a CDR3 region has the capability to direct binding to a particular antigen (or epitope) that is otherwise unable to be bound by an antibody generated by traditional VDJ recombination.

Provided rodents may be employed for making a human antibody, where the human antibody comprises variable domains derived from one or more variable region nucleic acid sequences encoded by genetic material of a cell of a rodent as described herein. For example, a provided rodent is immunized with an antigen of interest (e.g., virus or channel polypeptide, in whole or in part) under conditions and for a time sufficient that the rodent develops an immune response to said antigen of interest. Antibodies are isolated from the rodent (or one or more cells, for example, one or more B cells) and characterized using various assays measuring, for example, affinity, specificity, epitope mapping, ability for blocking ligand-receptor interaction, inhibition receptor activation, etc. In some embodiments, antibodies produced by provided rodents comprise one or more human variable domains that are derived from one or more human variable region nucleotide sequences isolated from the rodent. In some embodiments, anti-drug antibodies (e.g., anti-idiotype antibody) may be raised in provided rodents.

Rodents as described herein provide an improved *in vivo* system and source of biological materials (e.g., cells) for producing human antibodies that are useful for a variety of assays. In some embodiments, provided rodents are used to develop therapeutics that target one or more viruses and/or modulate viral activity and/or modulate viral interactions with other binding partners (e.g., a cell surface receptor). In some embodiments, provided rodents are used to develop therapeutics that target one or more channel proteins and/or modulate channel protein activity and/or modulate channel protein interactions with other binding partners. In some embodiments, provided rodents are used to identify, screen and/or develop candidate therapeutics (e.g., antibodies, siRNA, etc.) that bind one or more virus, channel or G-protein-coupled receptor (GPCR) polypeptides. In some embodiments, provided rodents are used to screen and develop candidate therapeutics (e.g., antibodies, siRNA, etc.) that block activity of one or more virus polypeptides, one or more human channel polypeptides or one or more human GPCR polypeptides. In some embodiments, provided rodents are used to determine the binding profile of antagonists and/or agonists of one or more human GPCR polypeptides or of one or more human channel polypeptides. In some embodiments, provided rodents are used to determine the epitope or epitopes of one or more candidate therapeutic antibodies that bind one or more human GPCR polypeptides or that bind one or more human channel polypeptides.

In some embodiments, provided rodents are used to determine the pharmacokinetic profiles of antibodies. In some embodiments, one or more provided rodents and one or more control or reference rodents are each exposed to one or more candidate therapeutic antibodies at various doses (e.g., 0.1 mg/kg, 0.2 mg/kg, 0.3 mg/kg, 0.4 mg/kg, 0.5 mg/kg, 1 mg/kg, 2 mg/kg, 3 mg/kg, 4 mg/kg, 5 mg/mg, 7.5 mg/kg, 10 mg/kg, 15 mg/kg, 20 mg/kg, 25 mg/kg, 30 mg/kg, 40 mg/kg, or 50 mg/kg or more). Candidate therapeutic antibodies may be dosed via any desired route of administration including parenteral and non-parenteral routes of administration. Parenteral routes include, e.g., intravenous, intraarterial, intraportal, intramuscular, subcutaneous, intraperitoneal, intraspinal, intrathecal, intracerebroventricular, intracranial, intrapleural or other routes of injection. Non-parenteral routes include, e.g., oral, nasal, transdermal, pulmonary, rectal, buccal, vaginal, ocular. Administration may also be by continuous infusion, local administration, sustained release from implants (gels, membranes or the like), and/or intravenous injection, e.g., using an intravenous fluid bag. Blood is isolated from non-human animals (humanized and control) at various time points (e.g., 0 hr, 6 hr, 1 day, 2 days, 3 days, 4 days, 5 days, 6 days, 7 days, 8 days, 9 days, 10 days, 11 days, or up to 30 or more days). Various assays may be performed to determine the pharmacokinetic profiles of administered candidate therapeutic antibodies using samples obtained from rodents as described herein including, but not limited to, total IgG, anti-therapeutic antibody response, agglutination, etc.

In some embodiments, provided rodents as are used to measure the therapeutic effect of blocking or modulating activity of a target antigen and the effect on gene expression as a result of cellular changes or cell surface density of the target antigen (in the case of a cell surface receptor) of cells of rodents as described herein. In some embodiments, a provided rodent or cells isolated therefrom are exposed to a candidate therapeutic that binds an antigen of interest and, after a subsequent period of time, analyzed for effects on target antigen-dependent processes (or interactions), for example, ligand-receptor interactions or antigen related signaling.

In some embodiments, provided rodents are used to measure the therapeutic effect of blocking or modulating channel activity (or channel signaling, or channel-mediated interactions, or channel action potentials) and the effect on gene expression as a result of cellular changes or the channel density of cells of rodents as described herein. In some embodiments, a provided rodent or cells isolated therefrom are exposed to a candidate therapeutic that binds a human channel polypeptide (or a portion thereof) and, after a subsequent period of time, analyzed for effects on channel-dependent processes (or interactions), for example, ligand-receptor interactions or channel action potentials.

In some embodiments, provided rodents express antibodies, thus cells, cell lines, and cell cultures can be generated to serve as a source of antibodies for use in binding and functional assays, e.g., to assay for binding or function of an antagonist or agonist, particularly where the antagonist or agonist is specific for a human polypeptide sequence or epitope or, alternatively, specific for a human polypeptide sequence or epitope that functions in ligand-receptor interaction (binding). In some embodiments, epitopes bound by candidate therapeutic antibodies or siRNAs can be determined using cells isolated from provided rodents.

In some embodiments, cells from provided rodents can be isolated and used on an ad hoc basis or can be maintained in culture for many generations. In some embodiments, cells from a provided rodent are immortalized (e.g., via use of a virus) and maintained in culture indefinitely (e.g., in serial cultures).

In some embodiments, rodents as described herein provide an *in vivo* system for the generation of antibody variants that binds a human target antigen. Such variants include antibodies having a desired functionality, specificity, low cross-reactivity to a common epitope shared by two or more human target antigens. In some embodiments, provided rodents are employed to generate panels of antibodies to generate a series of antibody variants that are screened for a desired or improved functionality.

In some embodiments, rodents as described herein provide an *in vivo* system for generating antibody libraries. Such libraries provide a source for heavy and light chain variable region sequences that may be grafted onto different Fc regions based on a desired effector function and/or used as a source for affinity maturation of the variable region sequence using techniques known in the art (e.g., site-directed mutagenesis, error-prone PCR, etc.).

In some embodiments, rodents as described herein provide an *in vivo* system for the analysis and testing of a drug or vaccine. In some embodiments, a candidate drug or vaccine may be delivered to one or more provided rodents, followed by monitoring of the rodents to determine one or more of the immune responses to the drug or vaccine, the safety profile of the drug or vaccine, or the effect on a disease or condition and/or one or more symptoms of a disease or condition. Exemplary methods used to determine the safety profile include measurements of toxicity, optimal dose concentration, antibody (i.e., anti-drug) response, efficacy of the drug or vaccine, and possible risk factors. Such drugs or vaccines may be improved and/or developed in such rodents.

Vaccine efficacy may be determined in a number of ways. Briefly, rodents described herein are vaccinated using methods known in the art and then challenged with a vaccine or a vaccine is administered to already-infected rodents. The response of a non-human animal(s) to a vaccine may be measured by monitoring of, and/or performing one or more assays on, the rodent(s) (or cells isolated therefrom) to determine the efficacy of the vaccine. The response of a rodent(s) to the vaccine is then compared with control animals, using one or more measures known in the art and/or described herein.

Vaccine efficacy may further be determined by viral neutralization assays. Briefly, non-human animals as described herein are immunized and serum is collected on various days post-immunization. Serial dilutions of serum are pre-incubated with a virus during which time antibodies in the serum that are specific for the virus will bind to it. The virus/serum mixture is then added to permissive cells to determine infectivity by a plaque assay or microneutralization assay. If antibodies in the serum neutralize the virus, there are fewer plaques or lower relative luciferase units compared to a control group.

Rodents as described herein provide an improved *in vivo* system for development and characterization of antibody-based therapeutics for use in cancer and/or inflammatory diseases. Inflammation has long been associated with cancer (reviewed in, e.g., Grivennikov, S.I. et al., 2010, Cell 140:883-99; Rakoff-Nahoum, S., 2006, Yale J. Biol. Med. 79:123-30). Indeed, a developing tumor environment is characterized, in part, by infiltration of various inflammatory mediators. Also, persistent inflammation can lead to a higher probability of developing cancer. Thus, in some embodiments, a rodent as described herein provides for an *in vivo* system for the development and/or identification of anti-cancer and/or anti-inflammatory therapeutics. In some embodiments, provided rodents or control rodents (e.g., having a genetic modification different than described herein or no genetic modification, i.e., wild-type) may be implanted with a tumor (or tumor cells), followed by administration of one or more candidate therapeutics. In some embodiments, candidate therapeutics may include a multi-specific antibody (e.g., a bi-specific antibody) or an antibody cocktail. In some embodiments, candidate therapeutics include combination therapy such as, for example, administration of two or more mono-specific antibodies dosed sequentially or simultaneously. The tumor may be allowed sufficient time to be established in one or more locations within the non-human animal prior to administration of one or more candidate therapeutics. Tumor cell proliferation, growth, survival, etc. may be measured both before and after administration with the candidate therapeutic(s). Cytotoxicity of candidate therapeutics may also be measured in the rodent as desired.

### Kits

Also disclosed us a pack or kit comprising one or more containers filled with at least one non-human animal, non-human cell, DNA fragment, and/or targeting vector as described herein. Kits may be used in any applicable method (e.g., a research method). Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both, or a contract that governs the transfer of materials and/or biological products (e.g., a rodent or rodent cell as described herein) between two or more entities.

Other features of the invention will become apparent in the course of the following descriptions of exemplary embodiments, which are given for illustration and are not intended to be limiting thereof.

### EXAMPLES

The following examples are provided so as to describe to those of ordinary skill in the art how to make and use methods and compositions of the invention, and are not intended to limit the scope of what the inventors regard as their invention. Unless indicated otherwise, temperature is indicated in Celsius, and pressure is at or near atmospheric.

### Example 1. Targeting vector design and construction

This example illustrates the construction of targeting vectors for inserting into the genome of a non-human animal such as a rodent (e.g., a mouse). In particular, the methods described in this example demonstrate the production of targeting vectors for inserting into the genome of rodent (e.g., a mouse) embryonic stem (ES) cells to produce a rodent whose genome comprises an immunoglobulin heavy chain variable region that includes a engineered heavy chain diversity (D_{H}) region, which engineered D_{H} region includes one or more D_{H} segments that are each operably linked to a 5' or 3' 23-mer RSS. In this example, three targeting vectors are described: a first targeting vector containing an engineered D_{H} region that includes a proximal (or 3') D_{H} segment operably linked to a 5' 23-mer RSS and operably linked to one human J_{H} segment, a second targeting vector containing an engineered D_{H} region that includes a proximal (or 3') D_{H} segment operably linked to a 5' 23-mer RSS and operably linked to three human J_{H} segments, and a third targeting vector containing an engineered D_{H} region that includes three D_{H} segments each associated with a 3' 23-mer RSS and operably linked to six human J_{H} segments (Figure 2). Each of these targeting vectors were generated and separately inserted into a humanized immunoglobulin heavy chain variable region (Figures 3, 5, 7). As described below, the engineered D_{H} region was placed in operable linkage with heavy chain variable (V_{H}) and heavy chain joining (J_{H}) segments so that, upon VDJ recombination, antibodies having CDR3s resulting from D_{H}-D_{H} recombination are expressed.

Targeting vectors containing one or more human D_{H} segments each associated with a 5' or 3' 23-mer RSS for insertion into an immunoglobulin heavy chain variable region were created using VELOCIGENE^{®} technology (see, e.g., U.S. Patent No. 6,586,251 and Valenzuela et al., 2003, Nature Biotech. 21(6):652-659) and molecular biology techniques known in the art. The methods described in this example can be employed to utilize any D_{H} segment, set of D_{H} segments, or combination of D_{H} segments as desired.

### A. 23:D_{H}3-3:12/J_{H}6 targeting vector (Figure 2)

Briefly, a first targeting vector was constructed using a genomic DNA fragment containing a plurality of human D_{H} segments and a synthetic human D_{H}3-3 segment positioned in the place of human D_{H}7-27. A donor for *in vitro* Cas9/GA modification was made by *de novo* synthesis (Blue Heron Bio) and comprised from 5' to 3: (a) a 100 bp homology arm starting 350 bp upstream of the 5' 12-mer RSS of D_{H}7-27 (b) AgeI and XhoI sites for insertion of a neomycin-resistance cassette, (c) the 250bp region upstream of the 5'-end 12 RSS of D_{H}7-27, (d) a synthetic human D_{H}3-3 segment engineered with a 5'end 23-mer RSS (from J_{H}4) and a 3'end 12-mer RSS (from D_{H}3-3), and (3) a 100bp homology box starting 3bp downstream of J_{H}5. A loxp-UbC-Em7-Neo-loxp cassette, e.g., a neomycin resistance gene flanked by *loxP* site-specific recombination recognition sites, was positioned ~250bp upstream of the synthetic D_{H} segment and downstream by ligation into the AgeI and XhoI sites to allow selection in E. coli and mouse ES cells. This **23:D_{H}3-3:12/J_{H}6** donor (SEQ ID NO:61) including the neomycin cassette was used to modify a BAC by *in vitro* Cas9/GA using two Cas9:gRNA complexes. Prior to the modification, the BAC was identical in sequence to the chimeric IgH locus of a VELOCIMMUNE^{®} mouse (see, e.g., Figures 3 and 4, showing an exemplary non-limiting endogenous immunoglobulin heavy chain locus of a VELOCIMMUNE^{®} mouse that is heterozygous for a 6394 allele comprising a humanized variable region that lacks J_{H} gene segments and is operably linked to and endogenous mouse immunoglobulin heavy chain constant region sequence and a 1460 allele comprising a humanized variable region operably linked to an endogenous mouse immunoglobulin heavy chain constant region sequence.). Specifically, the BAC was identical to the 1460 allele starting from the most proximal human V_{H} gene (V_{H}6-1) and including all 27 human D_{H} genes, all 6 human J_{H} genes, the mouse IgH intronic enhancer (Eµ), the mouse IgM switch region (Sµ), and the first 4 exons of the mouse IgM gene. The BAC also included a spectinomycin (spec)-resistance cassette and ~29kb of human intergenic sequence upstream from the V_{H}6-1 gene. The human-mouse junction is 222bp 3' of the human J_{H}6 gene and 490 bp 5' of the mouse Eµ enhancer. Insertion of the 23:D3-3:12/J_{H}6 donor into the BAC via GA resulted in the final 23:D3-3:12/J_{H}6 targeting vector comprising a replacement of the D_{H}7-7 gene segment with the engineered 23:D3-3:12 segment and deletion of J_{H}1, J_{H}2, J_{H}3, J_{H}4, and J_{H}5 gene segments(Figure 2). Table 1 provides the sequence of the gRNA, primers and probes used to determine accurate construction of the 23:D_{H}3-3:12/J_{H}6 targeting vector.

**Table 1: Primer and gRNA sequences for construction of23:D_{H}3-3:12/J_{H}6 targeting vector**

| ***Cas9 sites and gRNAs*** | ***Sequence (protospacer adjacent motif)*** |
|---|---|
| DNA recognition site | TCAGAAAGCAAGTGGATGAG(AGG) ***SEQ ID NO:62*** |
| 5' D7.27 crRNA | |
| DNA recognition site | TTAGGGAGACTCAGCTTGCC(AGG); ***SEQ ID NO:64*** |
| 3' JH1-5 del crRNA | |
| | |
| ***PCR*/*Sequencing Primers*** | ***Sequence*** |
| 5' up detect D7-27 | GTGAACAGGTGGAACCAAC; ***SEQ ID NO:66*** |
| 3' ub pro-200 | CCAGTGCCCTAGAGTCACCCA; ***SEQ ID NO:67*** |
| | |
| 5' neo detect | CTCCCACTCATGATCTATAGA; ***SEQ ID NO:68*** |
| 3' down detect JH1-5 del | ACGCAATCATCACGACAGC; ***SEQ ID NO:69*** |

The 23 :D3-3:12/JH6 targeting vector was linearized with NotI and electroporated into mouse embryonic stem cells having a genome that was heterozygous for a humanized immunoglobulin heavy chain variable region (i.e., a first heavy chain allele (1460het) containing a plurality of human V_{H}, D_{H}, and J_{H} segments operably linked to a rodent immunoglobulin heavy chain constant region including rodent heavy chain enhancers and regulatory regions, and containing an inserted nucleotide sequence encoding one or more murine Adam6 genes (e.g., U.S. Patent Nos. 8,642,835 and 8,697,940); and a second heavy chain allele (6394het) containing a plurality of human V_{H} and D_{H} segments, a J_{H} region deletion, and a rodent immunoglobulin heavy chain constant region including rodent heavy chain enhancers and regulatory regions, and containing an inserted nucleotide sequence encoding one or more murine Adam6 genes (e.g., U.S. Patent Nos. 8,642,835 and 8,697,940)) and that was homozygous (HO) for a humanized endogenous κ locus comprising the full repertoire of human immunoglobulin light chain Vκ and Jκ gene segments operably linked to an endogenous mouse immunoglobulin heavy chain Cκ region (1293) (see Figure 3). The 6799 allele is created upon electroporation and proper homologous recombination of the 1460 allele with the 23 :D3-3: 12/J_{H}6 targeting vector comprising the neomycin cassette. (Figure 3). These engineered mouse ES cells heterozygous for the 6394 and 679 alleles were employed to facilitate efficient screening of positive ES clones (see below) and subsequent cre-mediated removal of the drug resistance cassettes, after which deletion the 6394 and 6799 alleles are respectively referred to as 6643 and 6800 (Figure 4).

### B. 23:D_{H}3-3:12/J_{H}4-6 targeting vector (Figure 2)

In a similar manner, another targeting vector (23:D_{H}3-3:12/J_{H}4-6 ) was constructed using the same a genomic DNA fragment containing a plurality of human D_{H} segments and a synthetic human D_{H}3-3 segment positioned in the place of human D_{H}7-27. However, this second targeting vector included three of the six human J_{H} segments (i.e., J_{H}4, J_{H}5, J_{H}6). To create the final 23:D_{H}3-3:12/J_{H}4-6 targeting vector, a donor was used to modify the BAC identical in sequence to the chimeric IgH locus of a VELOCIMMUNE^{®} mouse (see, e.g., Figures 3 and 4, showing an exemplary non-limiting endogenous immunoglobulin heavy chain locus of a VELOCIMMUNE^{®} mouse that is heterozygous for a 6394 allele comprising a humanized variable region that lacks J_{H} gene segments and is operably linked to and endogenous mouse immunoglobulin heavy chain constant region sequence and a 1460 allele comprising a humanized variable region operably linked to an endogenous mouse immunoglobulin heavy chain constant region sequence). The donor comprised, from 5' to 3': (a) a 100bp homology box starting 350bp upstream of the 5'-end 12 RSS of D7-27, (b) AgeI and XhoI sites for inserting a neomycin-resistance cassette, (c) the 250bp region upstream of the 5'-end 12 RSS of D7-27, (d) a synthetic human D_{H}3-3 segment flanked 5' by a 23-mer RSS (from J_{H}4) and 3' by a 12-mer RSS (from D_{H}3-3), and (e) a 100bp homology box starting 3bp downstream of J_{H}3. A loxp-UbC-Em7-Neo-loxp cassette, e.g., a neomycin resistance gene flanked by *loxP* site-specific recombination recognition sites, was positioned ~250bp upstream of the synthetic D_{H} segment by ligation into the AgeI and XhoI sites to allow selection in E. coli and mouse ES cells. This 23:D3-3:12/J_{H}4-6 donor comprising a nucleotide sequence set forth as SEQ ID NO:52, including the neomycin cassette, was used to modify the BAC identical in sequence to the 1460 allele (Figures 3 and 4). Specifically, the BAC was identical to the 1460 allele starting from the most proximal human V_{H} gene (V_{H}6-1) and including all 27 human D_{H} genes, all 6 human J_{H} genes, the mouse IgH intronic enhancer (Eµ), the mouse IgM switch region (Sµ), and the first 4 exons of the mouse IgM gene. The BAC also included a spectinomycin (spec)-resistance cassette and ~29kb of human intergenic sequence upstream from the V_{H}6-1 gene. The human-mouse junction is 222bp 3' of the human J_{H}6 gene and 490 bp 5' of the mouse Eµ enhancer. Insertion of the 23:D3-3:12/J_{H}4-6 donor into the BAC via *in vitro* Cas9/GA using two Cas9:gRNA complexes resulted in the final 23:D3-3:12/J_{H}4-6 targeting vector (Figure 2) comprising a replacement of the D_{H}7-7 gene segment with the engineered 23:D3-3:12 segment and deletion of J_{H}1, J_{H}2, and J_{H}3 gene segments. Table 2 provides the sequence of the gRNA, primers and probes used to determine accurate construction of the **23:D_{H}3-3:12/J_{H}6** targeting vector.

**Table 2: Primer and gRNA sequences for construction of 23:D_{H}3-3:12/JH4-6 targeting vector**

| ***Cas9 sites and gRNAs*** | ***Sequence (protospacer adjacent motif)*** |
|---|---|
| DNA recognition site | TCAGAAAGCAAGTGGATGAG(AGG); ***SEQ ID NO:53*** |
| 5' D7.27 gRNA | |
| | |
| DNA recognition site | GCTCCAGGACAGAGGACGCT(GGG); ***SEQ ID NO:55*** |
| 3' J_{H}1-3 del gRNA | |
| | |
| ***PCR*/*Sequencing Primers*** | ***Sequence*** |
| 5' up detect D7-27 | GTGAACAGGTGGAACCAAC; ***SEQ ID NO:57*** |
| 3' ub pro-200 | CCAGTGCCCTAGAGTCACCCA; ***SEQ ID NO:58*** |
| | |
| 5' neo detect | CTCCCACTCATGATCTATAGA; ***SEQ ID NO:59*** |
| 3' down detect J_{H}1-3 del | GTCCCAGTTCCCAAAGAAAG; ***SEQ ID NO:60*** |

The 23:D3-3:12/J_{H}4-6 targeting vector was also linearized with NotI and electroporated into mouse embryonic stem cells having a genome that was heterozygous for a humanized immunoglobulin heavy chain variable region (i.e., a first heavy chain allele (1460het) containing a plurality of human V_{H}, D_{H}, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]; and a second heavy chain allele (6394het) containing a plurality of human V_{H} and D_{H} segments, a J_{H} region deletion, and a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]) and that was homozygous (HO) for a humanized endogenous κ locus comprising the full repertoire of human immunoglobulin light chain Vκ and Jκ gene segments operably linked to an endogenous mouse immunoglobulin heavy chain Cκ region (1293) (see, Figure 5). The 6797 allele is created upon electroporation and proper homologous recombination of the 1460 allele with the 23:D3-3:12/J_{H}4-6 targeting vector comprising the neomycin cassette. (Figure 5). These engineered mouse ES cells heterozygous for the 6394 and 6797 alleles were employed to facilitate efficient screening of positive ES clones (see below) and subsequent cre-mediated removal of the drug resistance cassettes, after which deletion the 6394 and 6797 alleles are respectively referred to as 6643 and 6798 (Figure 6).

### C. 12:D_{H}2-2:23|12:D_{H}2-8:23|12:D_{H}2-15:23 /J_{H}1-6 targeting vector (Figure 2)

Briefly, a third targeting vector was constructed using a DNA fragment containing a plurality of human D_{H} segments that included three D_{H}2 family gene segments (D_{H}2-2, D_{H}2-8 and D_{H}2-15) each having a 12-mer 5' RSS and a 23-mer 3' RSS. Firstly, the BAC identical in sequence to the chimeric IgH locus of a VELOCIMMUNE^{®} mouse (see, e.g., Figures 3 and 4, showing an exemplary non-limiting endogenous immunoglobulin heavy chain locus of a VELOCIMMUNE^{®} mouse that is heterozygous for a 6394 allele comprising a humanized variable region that lacks J_{H} gene segments and is operably linked to and endogenous mouse immunoglobulin heavy chain constant region sequence and a 1460 allele comprising a humanized variable region operably linked to an endogenous mouse immunoglobulin heavy chain constant region sequence). Specifically, the BAC was identical to the 1460 allele starting from the most proximal human V_{H} gene (V_{H}6-1) and including all 27 human D_{H} genes, all 6 human J_{H} genes, the mouse IgH intronic enhancer (Eµ), the mouse IgM switch region (Sµ), and the first 4 exons of the mouse IgM gene was modified using bacterial homologous recombination (BHR) to insert a loxp-UbC-Em7-Neo-loxp cassette, e.g., a neomycin resistance gene flanked by *lox*P site-specific recombination recognition sites, 512 bp upstream of the first human D_{H} segment (D_{H}1-1) for selection in *E. coli* and mouse ES cells. This resulting BAC was subsequently modified with three donors using *in vitro* Cas9/GA.
1. The first donor for modification of D_{H}2-2 contained, from 5' to 3', a 50bp homology box starting 1419bp upstream of the 5'-end 12 RSS of D_{H}2-2, a multiple cloning site (MreI-NsiI-EcoRI-KpnI-MreI), a modified D2-2 gene with the 3'-end 12-mer RSS replaced by an optimized 3'-end 23-mer RSS derived from human V_{H}1-69 (CACAGTGTGA AAACCCACAT CCTGAGAGTG ACACAAACC; T->A, G->C; SEQ ID NO:151), and a 50bp homology box ending 863bp downstream of the 3'-end 23-mer RSS of D2-2. The nucleotide sequence of this first donor for modification of D_{H}2-2 is set forth as SEQ ID NO:70. Since the D_{H} region consists of four direct repeats of ~10kb, designing unique primers and probes for screening is extremely difficult. To overcome this problem, two unique 40bp sequences were inserted: one 74bp downstream of the 5' homology box and one 40bp upstream of the 3' homology box. These unique 40-mers were used as binding sites for PCR/sequencing primers and Taqman probes, are denoted by unfilled vertical rectangles "1" and "2" of Figure 2, and comprise a nucleotide sequence set forth as SEQ ID NO:73 and SEQ ID NO:74, respectively
2. The second donor for modification of D_{H}2-8 contained, from 5' to 3', a 50bp homology box starting 552bp upstream of the 5'-end 12 RSS of D_{H}2-8, a multiple cloning site (MreI-NsiI-EcoRI-KpnI-MreI), a modified D2-8 gene with the 3'-end 12-mer RSS replaced by an optimized 3'-end 23-mer RSS derived from human VH1-69 (CACAGTGTGA AAACCCACAT CCTGAGAGTG ACACAAACC; T->A, G->C SEQ ID NO: 151), and a 50bp homology box ending 867bp downstream of the 3'-end 23-mer RSS of D2-8. The nucleotide sequence of this second donor for modification of D_{H}2-8 is set forth as SEQ ID NO:71. Since the D_{H} region consists of four direct repeats of ~10kb, designing unique primers and probes for screening is extremely difficult. To overcome this problem, two unique 40bp sequences were inserted: one 74bp downstream of the 5' homology box and one 40bp upstream of the 3' homology box. These unique 40-mers were used as binding sites for PCR/sequencing primers and Taqman probes, are denoted by unfilled vertical rectangles "10" and "16" of Figure 2 and comprise a nucleotide sequence set forth as SEQ ID NO:75 and SEQ ID NO:76, respectively.
3. The third donor for modification of D_{H}2-15 contained, from 5' to 3', a 50bp homology box starting 391bp upstream of the 5'-end 12 RSS of D_{H}2-15, a multiple cloning site (MreI-NsiI-EcoRI-KpnI-MreI), a modified D2-15 gene with the 3'-end 12-mer RSS replaced by an optimized 3'-end 23-mer RSS derived from human V_{H}1-69 (CACAGTGTGA AAACCCACAT CCTGAGAGTG ACACAAACC; T->A, G->C SEQ ID NO: 151), and a 50bp homology box ending 867bp downstream of the 3'-end 23-mer RSS of D2-15. The nucleotide sequence of this third donor for modification of D_{H}2-2 is set forth as SEQ ID NO:72. Since the D_{H} region consists of four direct repeats of ~10kb, designing unique primers and probes for screening is extremely difficult. To overcome this problem, two unique 40bp sequences were inserted: one 74bp downstream of the 5' homology box and one 40bp upstream of the 3' homology box. These unique 40-mers were used as binding sites for PCR/sequencing primers and Taqman probes, are denoted by unfilled vertical rectangles "8" and "18" of Figure 2, and comprise a nucleotide sequence set forth as SEQ ID NO:77 and SEQ ID NO:78, respectively.

After *in vitro* Cas9/GA modification with the three donors, the final targeting vector contained, from 5' to 3', a 49kb 5' homology arm containing human variable region DNA, a Neomycin selection cassette, an engineered human D_{H} region including three human DH segments each flanked 5' by a 12-mer RSS and 3' by a 23-mer RSS, six human J_{H} segments, and a 24kb 3' homology arm containing a mouse heavy chain intronic enhancer (Ei) and a mouse IgM constant region gene. (Figure 2). Table 3 provides the sequence of the gRNA, primers and probes used to determine accurate construction of the **12:D_{H}2-2:23|12:D_{H}2-8:23|12:D_{H}2-15:23 /J_{H}1-6** targeting vector.

**Table 3: Primer and gRNA sequences for construction of 12:DH2-2:23|12:DH2-8:23|12:DH2-15:23/JH1-6**

| ***Step*** | ***Name*** | ***Sequence (protospacer adjacent motif)*** |
|---|---|---|
| 1. BHR: VI433-pLMa0298= VI826 | 5' hIgHD HU2(h268) | GCAGTAACCCTCAGGAAGCA; ***SEQ ID NO:79*** |
| | 3' hIgHD HU2 AgeI(h269) | CTTCC**ACCGGT**ACAGCCACACCAAGGTCATC; ***SEQ ID NO:80*** |
| | | |
| | 5' hIgHD HD2 XhoI(h271) | CTTCC**CTCGAG**GAACACTGTCAGCTCCCACA; ***SEQ ID NO:81*** |
| | 3' hIgHD HD2(h272) | AGATCCTCCATGCGTGCTG; ***SEQ ID NO:82*** |
| | | |
| Jxn PCR | 5' hIgHD HU2 detect(h270) | TCTCCTCTCGTCGCCTCTAC; ***SEQ ID NO:83*** |
| | 3' ub pro-200 | CCAGTGCCCTAGAGTCACCCA; ***SEQ ID NO:84*** |
| | | |
| Jxn PCR | 5' neo detect | CTCCCACTCATGATCTATAGA; ***SEQ ID NO:85*** |
| | 3' hIgHD HD2 detect(h273) | GGGTGCTTGGGTCCTGTTAG; ***SEQ ID NO:86*** |
| 2. Cas9/GA: VI826-p51816= VI835 | | |
| gRNAs | 5' D2-2 Cas9 DNA target | AGGTCTGGAGCTACAAGCGG(TGG); ***SEQ ID NO:87*** |
| | 5' D2-2 gRNA | |
| | | |
| | 3' D2-2 Cas9 DNA target | AGGACAACAGTGAGGGTTAC(AGG); ***SEQ ID NO:89*** |
| | 3' D2-2 gRNA | |
| Jxn PCR | 5' up detect D2-2(h274) | GTCAAAGGTGGAGGCAGTG; ***SEQ ID NO:91*** |
| | 3' cm up detect | TCCAGCTGAACGGTCTGGTTA; ***SEQ ID NO:92*** |
| | | |
| Jxn PCR | D2-2seqF2 | AGCGATTCAACAGCTAACC; ***SEQ ID NO:93*** |
| | 3' down detect D2-2(h275) | CCAGTAGAAGTAACGACCAC; ***SEQ ID NO:94*** |
| 3. MreI/JO-1 GA: VI8352-delCM= VI841 | | |
| GA | JO-1 | |
| | | |
| Jxn PCR | 5' up detect D2-2(h274) | GTCAAAGGTGGAGGCAGTG; ***SEQ ID NO:96*** |
| | D2-2seqR1 | CCCGTGCCTAGATCAATGC; ***SEQ ID NO:97*** |
| | | |
| Additional D2-2 sequencing primers | D2-2seqF 1 | AGGCATTGATCTAGGCACG; ***SEQ ID NO:98*** |
| | D2-2seqR2 | CTGTTGAATCGCTGCAAGC; ***SEQ ID NO:99*** |
| 4. Cas9/GA: VI841-p53419= VI853 | | |
| crRNAs | 5' D2-8 Cas9 DNA target | ACCTGAAGACGCCCAGTCAA(CGG) ***SEQ ID NO:100*** |
| | 5' D2-8 crRNA | |
| | | |
| | 3' D2-8 Cas9 DNA target | GCCACAAGCACAAAAGTACA(GGG); ***SEQ ID NO:102*** |
| | 3' D2-8 crRNA | |
| | | |
| Jxn PCR | 5' up detect D2-8(h276) | GCCTTACCCAAGTCTTTCC; ***SEQ ID NO:104*** |
| | 3' cm up detect | TCCAGCTGAACGGTCTGGTTA; ***SEQ ID NO:105*** |
| Jxn PCR | D2-8seqF2 | TCCAATAAGTCGGTTCGGC; ***SEQ ID NO:106*** |
| | 3' down detect D2-8(h277) | GCAGAAGTAACCACCACTG; ***SEQ ID NO:107*** |
| 5. MreI/JO-2 GA= VI853-delCM= VI872 | | |
| GA | JO-2 | |
| | | |
| Jxn PCR | 5' up detect D2-8(h276) | GCCTTACCCAAGTCTTTCC; ***SEQ ID NO:109*** |
| | D2-8seqR1 | GCCCTACTTGTAGTTACGTG; ***SEQ ID NO:110*** |
| | | |
| Additional D2-8 sequencing primers | D2-8seqF1 | GCAAAATCCGACACGTAAC; ***SEQ ID NO:111*** |
| | D2-8seqR2 | CGAACCGACTTATTGGATGC; ***SEQ ID NO:112*** |
| 6. Cas9/GA: VI872-p51818= VI886 | | |
| crRNAs | 5' D2-8 Cas9 DNA target | GGTGGCCCCATAACACACCT(AGG); ***SEQ ID NO:113*** |
| | 5' D2-8 crRNA | |
| | | |
| | 3' D2-8 Cas9 DNA target | GGACAACAGTAGAGGGCTAC(AGG); ***SEQ ID NO:115*** |
| | 3' D2-8 crRNA | |
| | | |
| Jxn PCR | 5' up detect D2-15(h278) | GCACTCCCTCTAAAGACAAG; ***SEQ ID NO:117*** |
| | 3' cm up detect | TCCAGCTGAACGGTCTGGTTA; ***SEQ ID NO:118*** |
| | | |
| Jxn PCR | D2-15seqF2 | GTAAAAATACCGCCGCTGG; ***SEQ ID NO:119*** |
| | 3' down detect D2-15(h279) | GCATTTTCCTGTTCTTGCG; ***SEQ ID NO:120*** |
| 7. MreI/JO-2 GA= VI886-delCM= VI896/MAI D20187 | | |
| GA | JO-3 | |
| | | |
| Jxn PCR | 5' up detect D2-15(h278)^{∗} | GCACTCCCTCTAAAGACAAG; ***SEQ ID NO:122*** |
| | D2-15seqR1 | CTGGGAGATATTGGTGCATC; ***SEQ ID NO:123*** |
| | | |
| Additional D2-15 sequencing primers | D2-15seqF1 | TGCACCAATATCTCCCAGT; ***SEQ ID NO:124*** |
| | D2-15seqR2 | CGGCGGTATTTTTACTGACC; ***SEQ ID NO:125*** |

| Name | Forward Primer; Probe; Revere Primer | Type of Probe | Type of Assay | Specificity |
|---|---|---|---|---|
| 23:D3-3:12/1 | Fwd -TTTTTGTGCACCCCTTAATGG; ***SEQ ID NO:126*** Probe - ATGTGGTATTACGATTTTTGGA; ***SEQ ID NO:127*** Reverse-TCTGTGACACTGTGGGTATAATAAC C; ***SEQ ID NO:128*** | Taqman MGB | GOA | 23:D3-3:12 segment |
| 23:D3-3:12/2 | Fwd - CCCACAGTGTCACAGAGTCCAT; ***SEQ ID NO:129*** Probe - CAAGATGGCTTTCCTTCTGCCTCC; ***SEQ ID NO:130*** Reverse - TCCCAGCTCCAGGACAGAG; ***SEQ ID NO:131*** | BHQ1 | GOA | MAID6797 3'jxn |
| 23:D3-3:12/3 | Fwd - TGTCACAGAGTCCATCAAAAACCT; ***SEQ ID NO:132*** | Taqman MGB | GOA | MAID6799 3' jxn |
| | Probe - CACCACCCCCTCTC; ***SEQ ID*** | | | |
| | ***NO:133*** Reverse - CCTGAGACCCTGGCAAGCT; ***SEQ ID NO:134*** | | | |

| Name | Forward Primer, Probe, Reverse Primer | Type of Probe | Type of Assay | Specificity |
|---|---|---|---|---|
| Jxn D_{H}2-2 | Fwd -ATGGCGCTTGCAGCGATTC; ***SEQ ID NO:135*** Probe - CCAATACTAACGAGGAAATGCAAACCCA; ***SEQ ID NO:136*** Reverse - ACCCTCACTGTTGTCCTTCTG; ***SEQ ID NO:137*** | BHQ1 | GOA | 40bp-2 jxn of D2-2 |
| Jxn D_{H}2-8 | Fwd -GCAAACGTCCATCTGAAGGAGAA; ***SEQ ID NO:138*** Probe - CAAATAAACGATGGCAGGCTACACCCG; ***SEQ ID NO:139*** Reverse - GAGTTGCCGAACCGACTTATTG; ***SEQ ID NO:140*** | BHQ1 | GOA | 40bp-16 jxn of D2-8 |
| Jxn D_{H}2-15 | Fwd -GCAATGGTAGGTTCATGTCCATC; ***SEQ ID NO:141*** Probe- ACCGCCGCTGGTACTCCAAT; ***SEQ ID NO:142*** Reverse- CCCTGGCTGTCTGGGTTTG; ***SEQ ID NO:143*** | BHQ1 | GOA | 40bp-18 jxn of D2-15 |

The 12:D_{H}2-2:23:|12:D_{H}2-8:23|12:D_{H}2-15:23×3/J_{H}1-6 targeting vector was also linearized with NotI and electroporated into mouse embryonic stem cells having a genome that was homozygous for a humanized immunoglobulin heavy chain variable region that contained the full repertoire of functional human V_{H} gene segments, a deletion of the D_{H} region except for D_{H}7-27, and a full repertoire of functional human J_{H} gene segments (6011) and that was homozygous (HO) for a humanized endogenous κ locus comprising the full repertoire of human immunoglobulin light chain Vκ and Jκ gene segments operably linked to an endogenous mouse immunoglobulin heavy chain Cκ region (1293) (see, Figure 7). The 20187 allele is created upon electroporation and proper homologous recombination of a 6011 allele with the **12:D_{H}2-2:23|12:D_{H}2-8:23|12:D_{H}2-15:23 /J_{H}1-6** targeting vector comprising the neomycin cassette. (Figure 7). These engineered mouse ES cells were employed to facilitate efficient screening of positive ES clones (see below) and subsequent cre-mediated removal of the neomycin drug resistance cassette, after which removal the 20187 allele is to as 20188 (Figure 8).

### Example 2. ES cell screening

This example demonstrates the production of non-human animals (e.g., rodents) whose genome comprises an immunoglobulin heavy chain variable region that includes an engineered heavy chain diversity (D_{H}) region, wherein the engineered D_{H} region includes one or more D_{H} segments that are each operably linked to a 23-mer RSS.

Correct assembly of the targeting vectors described in Example 1 and targeted insertion of the DNA fragments into the diversity cluster of a humanized immunoglobulin heavy chain locus contained with BAC DNA clones was confirmed by sequencing and polymerase chain reaction during the construction each targeting vector. Targeted BAC DNA, confirmed by polymerase chain reaction, was then introduced into mouse embryonic stem (ES) cells via electroporation followed by culturing in selection medium. The genome of the ES cells used for electroporation of each targeting vector is depicted in Figures 3, 5 and 7, respectively. Drug-resistant colonies were picked 10 days after electroporation and screened by TAQMAN^{™} and karyotyping for correct targeting as previously described (Valenzuela et al., *supra;* Frendewey, D. et al., 2010, Methods Enzymol. 476:295-307) using primer/probe sets that detected proper integration of the engineered D_{H} gene segments (Table 4; F: forward primer, P: probe, R: reverse primer).

The VELOCIMOUSE^{®} method (DeChiara, T.M. et al., 2010, Methods Enzymol. 476:285-294; DeChiara, T.M., 2009, Methods Mol. Biol. 530:311-324; Poueymirou et al., 2007, Nat. Biotechnol. 25:91-99) was used, in which targeted ES cells were injected into uncompacted 8-cell stage Swiss Webster embryos, to produce healthy fully ES cell-derived F0 generation mice heterozygous for the engineered D_{H} region and that express antibodies containing heavy chain variable regions that include CDR3 regions generated from D_{H}-D_{H} recombination. F0 generation heterozygous male were crossed with C57B16/NTac females to generate F1 heterozygotes that were intercrossed to produce F2 generation homozygotes and wild-type mice.

The drug selection cassette may optionally be removed by the subsequent addition of a recombinase (e.g., by Cre treatment) or by breeding to a Cre deleter mouse strain (see, e.g., International Patent Application Publication No. WO 2009/114400) in order to remove any loxed selected cassette introduced by the targeting construct that is not removed, e.g., at the ES cell stage or in the embryo. Optionally, the selection cassette is retained in the mice. Selection cassettes engineered into targeting vectors described herein were removed in positive ES cells by transient expression of Cre recombinase (see Figures 4, 6 and 8, respectively).

**Table 4. Primer/probe sets for ES cell screening**

| Name | Sequence (5'-3') | | SEQ ID NO |
|---|---|---|---|
| | F | GAGGCTGTGCTACTGGTACTTC | 1 |
| hIgHJ2 | P | CCGTGGCACCCTGGTCACTGT | 2 |
| | R | TGGTGCCTGGACAGAGAAG | 3 |
| | | | |
| | F | TTTTTGTGCACCCCTTAATGG | 4 |
| VI741-42h1 | P | ATGTGGTATTACGATTTTTGGA | 5 |
| | R | CTCTGTGACACTGTGGGTATAATAACC | 6 |
| | | | |
| | F | TGTCACAGAGTCCATCAAAAACCT | 7 |
| VI742h2 | P | CACCACCCCCTCTC | 8 |
| | R | CCTGAGACCCTGGCAAGCT | 9 |
| | | | |
| | F | GGTGGAGAGGCTATTCGGC | 10 |
| Neo | P | TGGGCACAACAGACAATCGGCTG | 11 |
| | R | GAACACGGCGGCATCAG | 12 |
| | | | |
| | F | ATGGCGCTTGCAGCGATTC | 13 |
| Jxn D_{H}2-2 | P | CCAATACTAACGAGGAAATGCAAACCCA | 14 |
| | R | ACCCTCACTGTTGTCCTTCTG | 15 |
| | | | |
| | F | GCAAACGTCCATCTGAAGGAGAA | 16 |
| Jxn D_{H}2-8 | P | CAAATAAACGATGGCAGGCTACACCCG | 17 |
| | R | GAGTTGCCGAACCGACTTATTG | 18 |
| | F | GCAATGGTAGGTTCATGTCCATC | 19 |
| Jxn D_{H}2-15 | P | ACCGCCGCTGGTACTCCAAT | 20 |
| | R | CCCTGGCTGTCTGGGTTTG | 21 |
| | | | |
| | F | CAGCAGAGGGTTCCATGAGAA | 22 |
| hIgHJ6 | P | CAGGACAGGGCCACGGACAGTC | 23 |
| | R | GCCACCCAGAGACCTTCTGT | 24 |
| | | | |
| | F | ATCACACTCATCCCATCCCC | 25 |
| hIgH31 | P | CCCTTCCCTAAGTACCACAGAGTGGGCTC | 26 |
| | R | CACAGGGAAGCAGGAACTGC | 27 |
| | | | |
| | F | GCCATGCAAGGCCAAGC | 28 |
| mIgHp2 | P | CCAGGAAAATGCTGCCAGAGCCTG | 29 |
| | R | AGTTCTTGAGCCTTAGGGTGCTAG | 30 |
| | | | |
| | F | CTGAGCATGGTGTCCCATCT | 31 |
| mADAM6-1 | P | TGCAGATAAGTGTCATCTGGGCAA | 32 |
| | R | CCAGAGTACTGTAGTGGCTCTAAG | 33 |
| | | | |
| | F | CCCCGTCCTCCTCCTTTTTC | 34 |
| hIgk5 | P | TCATGTCCATTAACCCATTTACCTTTTGCCCA | 35 |
| | R | TGCAAGTGCTGCCAGCAAG | 36 |
| | | | |
| | F | CATTTGGCTACATATCAAAGCCG | 37 |
| hIgK21 | P | CCTGAGCCAGGGAACAGCCCACTGATA | 38 |
| | R | ACATGGCTGAGGCAGACACC | 39 |
| | | | |
| | F | GCAAACAAAAACCACTGGCC | 40 |
| mIgKd2 | P | CTGTTCCTCTAAAACTGGACTCCACAGTAAATGGAAA | 41 |
| | R | GGCCACATTCCATGGGTTC | 42 |
| | | | |
| | F | TGCGGCCGATCTTAGCC | 43 |
| hyg | P | ACGAGCGGGTTCGGCCCATTC | 44 |
| | R | TTGACCGATTCCTTGCGG | 45 |
| | | | |
| | F | TGGCAGCTGTTCAACCATGT | 46 |
| hIgHD-J.1 | P | ATCCACTGTCCCAGACAGCACC | 47 |
| | R | GGCGGTTTGTGGAGTTTCCT | 48 |
| | | | |
| | F | TGTTGCCTCCCTGCTTCTAG | 49 |
| hIgHD-J.2 | P | CCCAGACCCTCCCTTGTTCCTGA | 50 |
| | R | GGCCCACGCATTGTTCAG | 51 |

### Example 3. Characterization of mice modified with the 23:D_{H}3-3:12/J_{H}6, 23:D_{H}3-3:12/J_{H}4-6, or 12:D_{H}2-2:23|12:D_{H}2-8:23|12:D_{H}2-15:23 /J_{H}1-6 targeting vectors

### Immunophenotyping

Immunophenotypic analysis of animals modified with 23:D_{H}3-3:12/J_{H}6 or 23:D_{H}3-3:12/J_{H}4-6 targeting vectors was performed for mice heterozygous for the respective 6800 or 6795 modified alleles (see Figures 4 and 6). Immunophenotypic analysis of animals modified with the 12:D_{H}2-2:23|12:D_{H}2-8:2-23| 12:D_{H}2-15:23/J_{H}1-6 targeting vector was performed for mice bred to homozygosity for the 20188 modified allele. B cell development was analyzed in these animals by fluorescence activated cell sorting (FACS). Briefly, spleens and leg bones (femur and tibia) were harvested from:
VELOCIMMUNE^{®} mice (n=3, 26% C57BL/6 23% 129S6/SvEvTac 51% Balb/cAnNTac; see, e.g., U.S. Patent Nos. 8,502,018 and 8,642,835),
6643het/6800het//1293ho mice (25% C57BL/6NTac 25% 129S6/SvEvTac 50% Balb/cAnNTac modified with the 23:D_{H}3-3:12/J_{H}6 targeting vector; see Figure 4; n=3;
6643het/6798het//1293ho mice (25% C57BL/6NTac 25% 129S6/SvEvTac 50% Balb/cAnNTac modified with the 23:D_{H}3-3:12/J_{H}4-6 targeting vector; see Figure 6; n=3); or
20188ho/1293ho mice (25% C57BL/6NTac 25% 129S6/SvFvTac 50% Balb/cAnNTac modified with the 12:D_{H}2-2:23|12:D_{H}2-8:23|12:D_{H}2.1523/J_{H}1-6 targeting vector; n= 3).

Bone marrow was collected from femurs by centrifugation. Red blood cells from spleen and bone marrow preparations were lysed with ACK lysis buffer (Gibco) followed by washing with 1xPBS with 2% FBS. Isolated cells (1×10⁶) were incubated with selected antibody cocktails for 30 min at +4°C: Stain 1: rat anti-mouse CD43-FITC (Biolegend 121206, clone 1B11), rat anti-mouse c-kit-PE (Biolegend 105808, clone 2B8), rat anti-mouse IgM-PeCy (eBiosciences 25-5790-82, clone II/41), rat anti-mouse IgD-PerCP-Cy5.5 (Biolegend 405710, clone 11-26c.2a), rat anti-mouse CD3-PB (Biolegend 100214, clone 17-A2), rat anti-mouse B220-APC (eBiosciences 17-0452-82, clone RA3-6B2), and rat anti-mouse CD19-APC-H7 (BD 560143 clone 1D3). Stain 2: rat anti-mouse kappa-FITC (BD 550003, clone 187.1), rat anti-mouse lambda-PE (Biolegend 407308, clone RML-42), rat anti-mouse IgM-PeCy (eBiosciences 25-5790-82, clone II/41), rat anti-mouse IgD-PerCP-Cy5.5 (Biolegend 405710, clone 11-26c.2a), rat anti-mouse CD3-PB (Biolegend 100214, clone 17-A2), rat anti-mouse B220-APC (eBiosciences 17-0452-82, clone RA3-6B2), and rat anti-mouse CD19-APC-H7 (BD 560143 clone 1D3). Following staining, cells were washed and then fixed in 2% formaldehyde. Data acquisition was performed on a BD LSRFORTESSA^{™} flow cytometer (BD Biosciences) and analyzed with FLOWJO^{™} software (BD Biosciences).

In mice heterozygous for the 6800 or 6798 alleles, total B cell numbers were decreased in both the spleen and bone marrow, and there was an increase in pro-B cells observed in the bone marrow when compared to control VELOCIMMUNE^{®} mice (data not shown). Overall, the B cells had similar κ and λ usage (data not shown). In mice homozygous for the 20188 allele, a slightly higher level of λ⁺ B cells in the spleen were seen (data not shown). None of the differences observed in the B cell populations in mice expressing the 6800, 6798, or 20188 alleles compared to control VELOLCIMMUNE^{®} mice were considered to affect B cell development of these animals (data not shown).

### Frequencies of D_{H}-D_{H} rearrangements and characteristics

Gene rearrangements in unimmunized naive mice modified with the 23:D_{H}3-3:12/J_{H}6 targeting vector, the 23:D_{H}3-3:12/J_{H}4-6 targeting vector, or the 12:D_{H}2-2:23|12:D_{H}2-8:23|12:D_{H}2-15:23 targeting vector were analyzed by IgM repertoire sequencing. IgM repertoire library from spleen B cells was prepared using a SMARTer^{™} RACE (rapid amplification of cDNA ends) cDNA Amplification Kit (Clontech) with primers specific to the mouse constant IgM (Table 5, "nnnnnn" represents a 6bp index sequences to enable multiplexing samples for sequencing). Prepared repertoire library was then sequenced on a MiSeq sequencer (Illumina).

Illumina MiSeq paired-end sequences (2x300 cycles) were merged and filtered based on quality and perfect match to the heavy chain IgM constant region primer. Rearranged heavy chain sequences were aligned to germline V, D, and J reference databases (IMGT). Subsequent analyses only included productive rearrangements, defined as sequences with no stop codons within the open reading frame and an in-frame VDJ junction.

**Table 5: Primers used in library preparation for IgM repertoire sequencing**

| **RT primers** | **Oligo-dT (SEQ ID NO)** | **SEQUENCE** |
|---|---|---|
| **Template switching oligo** | **(SEQ ID NO:146)** | 5' -AGCAGTGGTATCAACGCAGAGTACATGGG -3' |
| **1^{st} round PCR primers** | **IgM Constant (SEQ ID NO:147)** | |
| | **PE2-PIIA (SEQ ID NO:148)** | |
| **2^{nd} round PCR Primers** | **Forward (SEQ ID NO:149)** | |
| | **Reverse (SEQ ID NO: 150)** | |

In unimmunized animals modified with the 23:D_{H}3-3:12/J_{H}6 targeting vector or the 23:D_{H}3-3:12/J_{H}4-6 targeting vector, no expression of D_{H}7-27 was detected, and J_{H} usage was respectively limited to J_{H}6, or J_{H}4, J_{H}5, and J_{H}6, as expected (data not shown). In unimmunized animals modified with the 12:D_{H}2-2:23|12:D_{H}2-8:23|12:D_{H}2-15:23 targeting vector, recombination with all J_{H}1-6 gene segments was detected, although recombination with J_{H}4 was seen at higher frequencies (about 50%) in both the modified and control animals (data not shown). In all modified animals, a variety of V_{H} gene segments were used (e.g., but not limited to V_{H}4-39, V_{H}3-23, etc.) and a variety of D_{H} gene segments (e.g., but not limited to D_{H}1-7, D_{H}3-10, and D_{H}5-12) recombined with the engineered D_{H} gene segments. Interestingly, usage of D_{H}2-2, D_{H}2-8, and D_{H}2-15 gene segments trended higher in animals modified with the 12:D_{H}2-2:23|12:D_{H}2-8:23|12:D_{H}2-15:23 targeting vector (2.41%; 6.34%; 6.91%; n=3) compared to control animals (2.57%, 3.44%; 3.58%; n=3). (Table 6).

Functional sequences with a minimum of two consecutive D_{H} segments identified within the junction region were further confirmed as resulting from a D_{H}-D_{H} recombination event using the following criteria. In sequences isolated from mice modified with the 23:D_{H}3-3:12/J_{H}6 targeting vector or the 23:D_{H}3-3:12/J_{H}4-6 targeting vector, a D_{H}-D_{H} recombination event was confirmed when (1) the identified "successive" or 3' D_{H} segment aligned with a minimum of 9 consecutive base pairs to a germline sequence of the IGHD3-3 D_{H} gene segment and (2) both the identified leading 5' and successive 3' D_{H} gene segments aligned with a minimum of 5 consecutive base pairs to a germline D_{H} gene segment, irrespective of any overlap. In sequences isolated from mice modified with the 12:D_{H}2-2:23|12:D_{H}2-8:23|12:D_{H}2-15:23 targeting vector, a D_{H}-D_{H} recombination event was confirmed when (1) the identified "leading" or 5' D_{H} segment aligned with a minimum of 9 consecutive base pairs to a germline sequence of the IGHD2-2 D_{H} gene segment, the IGHD2-8 D_{H} gene segment, or the IGHD2-15 D_{H} gene segment, and (2) both the identified leading 5' and successive 3' D_{H} gene segments aligned with a minimum of 5 consecutive nucleotides to a germline D_{H} gene segment, irrespective of any overlap.

Table 6 provides the number of functional Ig reads as an absolute amount and percent of total reads, the percentage of all functional reads derived from a D_{H}2-2 gene segment, a D_{H}2-8 gene segment, or a D_{H}2-15 gene segment, and the percentage of all reads derived from a D_{H}2-2 gene segment, a D_{H}2-8 gene segment, or a D_{H}2-15 gene segment that satisfy the criteria to be confirmed as the result of a D_{H}-D_{H} recombination event.

**Table 6**

| Mouse | % D2-2/D2-8/D2-15 usage | % D_{H}-D_{H} fusions |
|---|---|---|
| Control | 3.44 | 0.20^{∗} |
| Control | 3.58 | 0.10^{∗} |
| Control | 2.57 | 0.08^{∗} |
| 20188 S1 | 6.34 | 3.80 |
| 20188 S2 | 2.41 | 1.69 |
| 20188 S3 | 6.91 | 4.16 |

| | | |
|---|---|---|
| ^{∗}D_{H}-D_{H} fusion in each of these control animals were confirmed using the same criteria set forth for those animals modified with the 12:D_{H}2-2:23\|12:D_{H}2-8:23\|12:D_{H}2-15:23 targeting vector. | | |

As shown in Table 6, all unimmunized modified animals showed an increase in D_{H}-D_{H} recombination events compared to the control animals. In animals modified with engineered 2-2, 2-8 and 2-15 gene segments, e.g., homozygous for the 20188 allele, D_{H}-D_{H} recombination events in 1.69% to 4.16% of all rearrangements comprising one of the three engineered 2-2, 2-8, 2-15 D_{H} gene segments. In contrast, D_{H}-D_{H} recombination was confirmed in less than 0.2% of all rearrangements derived from 2-2, 2-8, or 2-15 gene segment in control animals. In a separate experiment, the frequency of confirmed D_{H}-D_{H} recombination events involving an engineered D_{H}3-3 gene segment in mice heterozygous for the 6800 mice (Figure 4) ranged from 0.6 to 1.2% of all functional reads derived from a D_{H}3-3 gene segment.

In all modified animals, the rearranged V_{H}(D_{H}A-D_{H}B)J_{H} rearrangements encoded longer CDR3 lengths (e.g. but not limited to CDR3 lengths of about 21 amino acids) and incorporated more cysteine residues. (e.g., Figures 9 and 10).

In summary, mice modified with an engineered D_{H} gene segment exhibit a relatively normal B cell phenotype with an increased frequency of D_{H}-D_{H} recombination events due to the utilization of the engineered D_{H} gene segment. Additionally, the D_{H}-D_{H} recombined sequences encoded for CDR3 that were longer in length and had a higher rate of cysteine incorporation.

### Example 4. Production of antibodies

This example demonstrates production of antibodies in a rodent whose genome comprises an immunoglobulin heavy chain variable region that includes an engineered D_{H} region as described herein. The methods described in this example, and/or immunization methods well known in the art, can be used to immunize rodents containing an engineered D_{H} region as described herein with polypeptides or fragments thereof (e.g., peptides derived from a desired epitope), or a combination of polypeptides or fragments thereof, as desired.

Briefly, cohorts of mice that include an engineered D_{H} region as described herein are immunized with an antigen of interest using immunization methods known in the art. The antibody immune response (i.e., serum titer) is monitored by an ELISA immunoassay and/or on engineered cells by MSD. When a desired immune response is achieved, splenocytes (and/or other lymphatic tissue) are harvested and fused with mouse myeloma cells to preserve their viability and form immortal hybridoma cell lines. The hybridoma cell lines are screened (e.g., by an ELISA assay or MSD) and selected to identify hybridoma cell lines that produce antigen-specific antibodies. Hybridomas may be further characterized for relative binding affinity and isotype as desired. Using this technique, several antigen-specific chimeric antibodies (i.e., antibodies possessing human variable domains and rodent constant domains) are obtained.

DNA encoding the variable regions of heavy chain and light chains may be isolated and linked to desirable isotypes (constant regions) of the heavy chain and light chain for the preparation of fully-human antibodies. Such an antibody may be produced in a cell, such as a CHO cell. Fully human antibodies are then characterized for relative binding affinity and/or neutralizing activity of the antigen of interest.

DNA encoding the antigen-specific chimeric antibodies, or the variable domains of light and heavy chains, may be isolated directly from antigen-specific lymphocytes. Initially, high affinity chimeric antibodies are isolated having a human variable region and a rodent constant region and are characterized and selected for desirable characteristics, including, but not limited to, affinity, selectivity, epitope, etc. Rodent (e.g., but not limited to, rat, mouse, etc.) constant regions are replaced with a desired human constant region to generate fully-human antibodies. While the constant region selected may vary according to specific use, high affinity antigen-binding and target specificity characteristics reside in the variable region. Antigen-specific antibodies are also isolated directly from antigen-positive B cells (from immunized mice) without fusion to myeloma cells, as described in, e.g., U.S. Patent No. 7,582,298,. Using this method, several fully human antigen-specific antibodies (i.e., antibodies possessing human variable domains and human constant domains) are made.

In one experiment, control mice (e.g., VELOCIMMUNE^{®} mice with humanized immunoglobulin heavy and κ light chain variable region loci (n= 4-6; see, e.g., U.S. Patent Nos. 8,697,940 and 8,642,835)) and test mice (e.g., mice modified to comprise an engineered 23:D_{H}3-3:12/J_{H}4-6 or 23:D_{H}3-3:12/J_{H}6 region as described herein (n= 4-6)) were immunized with different antigens and/or nucleic acids encoding same (a G protein-coupled receptor (GPCR), an ion channel, a chemokine receptor, or a pathogen).

Mice were able to generate responses to each of the tested antigens, with responses ranging from low titers to high specific titers (data not shown). Mice having an engineered 23:D_{H}3-3:12/J_{H}6 region as described herein demonstrated titers to a GPCR expressing cell line comparable to control mice having humanized immunoglobulin heavy and κ light chain variable region loci (Figure 11; filled circles represent an engineered cell line that expresses GPCR; unfilled circles are control cells that do not express GPCR).

Additionally, 100% of 23 tested antibodies specific to a G protein-coupled receptor (GPCR) that were generated in mice comprising an engineered D_{H} region had a heavy chain CDR3 length of 10 or more amino acids; 20% had a CDR3 length of 14 amino acids and 1 antibody had a CDR3 length of 18 amino acids (data not shown). In contrast, only about 60% of the 8 tested antibodies specific to the GPCR that were generated in mice having humanized immunoglobulin heavy and κ light chain variable region loci, but not an engineered D_{H} region, had CDR3 lengths of 10 amino acids or more, none of which had CDR3 lengths of 14 or more (data not shown).

A plurality of antiviral antibodies was isolated from a mouse having an engineered 23:D_{H}3-3:12/J_{H}4-6 region, with at least one demonstrating a neutralization activity superior to that of the comparator molecule (data not shown).

Antibodies from a mouse having an engineered 23:D_{H}3-3:12/J_{H}6 and immunized with an ion channel comprised a population of antibodies having HCDR3 lengths of greater than 21 amino acids stemming from both V_{H}D_{H}J_{H} and V_{H}D_{H}A-D_{H}BJ_{H} rearrangement (as determined by stringent criteria described for this example), with the sequences encoding longer HCDR3 lengths being found more in the bone marrow compared to the spleen (Figure 12). Analysis of CDR3 lengths encoded by sequences determined to be the result of V_{H}D_{H}A-D_{H}BJ_{H} rearrangement according to the stringent criteria of this example (when using the stringent criteria of this example each D_{H} gene segment detected must display a minimum of 40% identity to a germline D gene segment, the 3' D_{H} gene segment must be derived from the D_{H}3-3 gene segment, and a maximum of 1 nucleotide mutation in each D_{H} gene segment) is shown in Figure 13. Table 7 below provides a comparison of the frequency of V_{H}D_{H}A-D_{H}BJ_{H} recombination in the bone marrow or spleen using the stringent criteria described in this example, or non-stringent criteria in this example (when using the non-stringent criteria of this example each D_{H} gene segment detected must display a minimum of 5 nucleotide identity to a germline D gene segment, the 3' D_{H} gene segment must be derived from the D_{H}3-3 gene segment).

**Table 7: V_{H}D_{H}A-D_{H}BJ_{H} recombination frequency**

| | Non-stringent Criteria | Stringent Criteria |
|---|---|---|
| Bone Marrow | 3.54 | 1.7 |
| Spleen | 0.38 | 0.04 |

## Claims

1. A nucleotide molecule comprising an engineered immunoglobulin heavy chain diversity (D_{H}) region ("engineered D_{H} region") comprising:
(i) an engineered D_{H} gene segment comprising a D_{H} gene segment immediately adjacent to a 23-mer RSS ("engineered D_{H} gene segment") and
(ii) an unrearranged D_{H} gene segment flanked on its 5' side by a first 12-mer RSS and on its 3' side by a second 12-mer RSS ("unrearranged D_{H} gene segment"),
wherein (i) the engineered D_{H} gene segment and (ii) the unrearranged D_{H} gene segment are operably linked such that (i) the engineered D_{H} gene segment and (ii) the unrearranged D_{H} gene segment are able to join in a D_{H}-D_{H} recombination event according to the 12/23 rule,
wherein the nucleotide molecule is suitable for obtaining rodents having the engineered D_{H} region, and
optionally wherein the engineered D_{H} region comprises only human D_{H} gene segments.

2. The nucleotide molecule of claim 1, wherein the nucleotide molecule further comprises:
(a) at least one unrearranged immunoglobulin heavy chain variable (V_{H}) gene segment ("unrearranged V_{H} gene segment") that is upstream of and operably linked to the engineered D_{H} region;
(b) at least one unrearranged immunoglobulin heavy chain joining (J_{H}) gene segment ("unrearranged J_{H} gene segment") that is downstream of and operably linked to the engineered D_{H} region; or
(c) a combination of (a) and (b).

3. The nucleotide molecule of claim 2, wherein:
(a) the at least one unrearranged V_{H} gene segment comprises an unrearranged human V_{H}6-1 gene segment, the at least one unrearranged J_{H} gene segment comprises an unrearranged human J_{H}6 gene segment, or a combination thereof;
(b) the at least one unrearranged J_{H} gene segment comprises an unrearranged human J_{H}4 gene segment, an unrearranged human J_{H}5 gene segment, and an unrearranged human J_{H}6 gene segment;
(c) the at least one unrearranged J_{H} gene segment comprises an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment, optionally wherein the unrearranged human J_{H}1 gene segment, the unrearranged human J_{H}2 gene segment, the unrearranged human J_{H}3 gene segment, the unrearranged human J_{H}4 gene segment, the unrearranged human J_{H}5 gene segment, and the unrearranged human J_{H}6 gene segment are in germline configuration; and/or
(d) the at least one unrearranged V_{H} gene segment comprises the full repertoire of functional unrearranged human V_{H} gene segments spanning between and including an unrearranged human V_{H}3-74 gene segment and an unrearranged human V_{H}1-6 gene segment ("full repertoire of functional unrearranged human V_{H} gene segments"), optionally wherein the full repertoire of functional unrearranged human V_{H} gene segments is in germline configuration.

4. The nucleotide molecule of any one of the preceding claims, wherein:
(a) the nucleotide molecule further comprises one or more functional rodent Adam6 genes, optionally wherein the one or more functional rodent Adam6 genes is located between a human V_{H}1-2 gene segment and a human V_{H}6-1 gene segment and/or replaces a human Adam6 gene;
(b) the engineered D_{H} gene segment comprises the 23-mer RSS immediately adjacent to the 5'-end of the D_{H} gene segment;
(c) the engineered D_{H} gene segment comprises the 23-mer RSS immediately adjacent to the 3'-end of the D_{H} gene segment;
(d) the engineered D_{H} gene segment comprises:
(i) a human D_{H}3-3 gene segment immediately adjacent to the 23-mer RSS, optionally wherein the 23-mer RSS is immediately adjacent to the 5'-end of the D_{H}3-3 gene segment;
(ii) a human D_{H}2-2 gene segment immediately adjacent to the 23-mer RSS, optionally wherein the 23-mer RSS is immediately adjacent to the 3'-end of the D_{H}2-2 gene segment;
(iii) a human D_{H}2-8 gene segment immediately adjacent to the 23-mer RSS, optionally wherein the 23-mer RSS is immediately adjacent to the 3'-end of the D_{H}2-8 gene segment;
(iv) a human D_{H}2-15 gene segment immediately adjacent to the 23-mer RSS, optionally wherein the 23-mer RSS is immediately adjacent to the 3'-end of the D_{H}2-15 gene segment; or
(v) any combination of (i)-(iv);
(e) the nucleotide molecule comprises the nucleotide sequence set forth as SEQ ID NO:52 or comprises the nucleotide sequence set forth as SEQ ID NO:61;
(f) the nucleotide molecule comprises the nucleotide sequence set forth as SEQ ID NO:70;
(g) the nucleotide molecule comprises the nucleotide sequence set forth as SEQ ID NO:71; and/or
(h) the nucleotide molecule comprises the nucleotide sequence set forth as SEQ ID NO:72.

5. The nucleotide molecule of any one of claims 1-4, comprising in operable linkage from 5' to 3':
(a) at least one unrearranged human V_{H} gene segment,
(b) the engineered D_{H} region, wherein the engineered D_{H} gene segment comprises a human D_{H}3-3 gene segment immediately adjacent to the 23-mer RSS, wherein the 23-mer RSS is immediately adjacent to the 5' end of the human D_{H}3-3 gene segment, and
(c) at least one unrearranged human J_{H} gene segment.

6. The nucleotide molecule of any one of claims 1-4, comprising in operable linkage from 5' to 3':
(a) at least one unrearranged human V_{H} gene segment,
(b) the engineered D_{H} region, wherein the engineered D_{H} gene segment comprises a human D_{H}2 gene segment immediately adjacent to the 23-mer RSS, wherein the 23-mer RSS is immediately adjacent to the 3'end of the human D_{H}2 gene segment, optionally wherein:
(i) the engineered D_{H} gene segment comprises a human D_{H}2-2 gene segment immediately adjacent to 23-mer RSS;
(ii) the engineered D_{H} gene segment comprises a human D_{H}2-8 gene segment immediately adjacent to 23-mer RSS;
(iii) the engineered D_{H} gene segment comprises a human D_{H}2-15 gene segment immediately adjacent to 23-mer RSS; or
(iv) any combination thereof, and
(c) at least one unrearranged human J_{H} gene segment.

7. The nucleotide molecule of claim 5 or claim 6, wherein:
(a) the at least one unrearranged human V_{H} gene segment comprises from 5' to 3' an unrearranged human V_{H}1-2 gene segment and an unrearranged human V_{H}6-1 gene segment, wherein the nucleotide further comprises one or more functional rodent Adam6 genes between the unrearranged human V_{H}1-2 gene segment and the unrearranged human V_{H}6-1 gene segment, and wherein the unrearranged human V_{H}1-2 gene segment, the one or more functional rodent Adam6 genes, and the unrearranged human V_{H}6-1 gene segment are contiguous;
(b) the at least one unrearranged human V_{H} gene segment comprises the full repertoire of functional unrearranged human V_{H} gene segments, optionally wherein the full repertoire of functional unrearranged human V_{H} gene segments is in germline configuration; and/or
(c) the at least one unrearranged human J_{H} gene segment comprises an unrearranged human J_{H}6 gene segment, optionally wherein:
(i) the at least one unrearranged human J_{H} gene segment comprises an unrearranged human J_{H}4 gene segment, an unrearranged human J_{H}5 gene segment, and the unrearranged human J_{H}6 gene segment; and/or
(ii) wherein the at least one unrearranged human J_{H} gene segment comprises an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment, further optionally wherein the unrearranged human J_{H}1 gene segment, the unrearranged J_{H}2 gene segment, the unrearranged J_{H}3 gene segment, the unrearranged J_{H}4 gene segment, the unrearranged J_{H}5 gene segment, and the unrearranged J_{H}6 gene segment are in germline configuration.

8. The nucleotide molecule of any one of claims 1-7, further comprising:
(a) at its 3'-end, a heavy chain immunoglobulin constant (C_{H}) region or a portion thereof;
(b) at its 3'-end, a rodent C_{H} region or a portion thereof;
(c) at its 3'-end, a rodent C_{H} region or a portion thereof comprising a rodent intronic enhancer region and a rodent IgM gene; and/or
(d) a drug selection cassette located upstream of the engineered D_{H} gene segment, optionally wherein the drug selection cassette is flanked by one or more site-specific recombination sites.

9. A targeting vector comprising a nucleotide molecule of any one of claims 1-8, comprising 5'- and 3'- homology arms configured to allow homologous recombination with an immunoglobulin heavy chain sequence at an endogenous rodent immunoglobulin heavy chain locus, optionally wherein the immunoglobulin heavy chain sequence comprises a human or humanized immunoglobulin heavy chain variable region.

10. A method of modifying an immunoglobulin heavy chain variable region to engineer D_{H}-D_{H} recombination, the method comprising:
(a) obtaining an immunoglobulin heavy chain variable chain sequence comprising a D_{H} region comprising one or more unrearranged D_{H} gene segments, each of which unrearranged D_{H} gene segment is flanked on its 5' side by a first 12-mer RSS and on its 3' side by a second 12-mer RSS,
optionally wherein the one or more unrearranged D_{H} gene segments comprises at least one unrearranged human D_{H} gene segment, further optionally wherein the at least one unrearranged human D_{H} gene segment comprises all the functional human D_{H} gene segments spanning between and including an unrearranged human D_{H}1-1 gene segment and an unrearranged human D_{H}7-27 gene segment, optionally wherein all the functional human D_{H} gene segments are in germline configuration, and
(b) engineering the D_{H} region to further comprise at least one engineered D_{H} gene segment, wherein the engineered D_{H} gene segment comprises a D_{H} gene segment immediately adjacent to a 23-mer RSS,
wherein, in the resulting engineered D_{H} region, the engineered D_{H} gene segment and at least one of the one or more unrearranged D_{H} gene segments are operably linked and able to join in a D_{H}-D_{H} recombination event according to the 12/23 rule,
optionally wherein the resulting engineered D_{H} region comprises only human D_{H} gene segments.

11. The method of claim 10, wherein the D_{H} region of claim 10(a) comprises two or more unrearranged D_{H} gene segments, each of which is flanked on its 5' side by a first 12-mer RSS and on its 3' side by a second 12-mer RSS, and
wherein engineering comprises
(a) replacing at least one of the two or more unrearranged D_{H} gene segments with the engineered D_{H} gene segment as defined in claim 10(b),
(b) replacing the first 12-mer RSS or the second 12-mer RSS of at least one of the two or more unrearranged D_{H} gene segments with a 23-mer RSS, or
(c) a combination of (a) and (b),
optionally wherein the immunoglobulin heavy chain variable chain sequence further comprises a J_{H} region comprising at least one unrearranged J_{H} gene segment,
wherein the J_{H} region is operably linked to the D_{H} region, and
wherein replacing at least one of the two or more unrearranged D_{H} gene segments with the engineered D_{H} gene segment as defined in claim 10(b) comprises deleting the at least one unrearranged J_{H} gene segment,
further optionally wherein the J_{H} region comprises a full repertoire of human germline J_{H} gene segments comprising an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment, optionally wherein the unrearranged human J_{H}1 gene segment, the unrearranged human J_{H}2 gene segment, the unrearranged human J_{H}3 gene segment, the unrearranged human J_{H}4 gene segment, the unrearranged human J_{H}5 gene segment, and the unrearranged human J_{H}6 gene segment are in germline configuration, and
wherein deleting at least one germline J_{H} gene segment comprises deleting the unrearranged human J_{H}1 gene segment, the unrearranged human J_{H}2 gene segment, and the unrearranged human J_{H}3 gene segment, and optionally further deleting the unrearranged human J_{H}4 gene segment and the unrearranged human J_{H}5 gene segment.

12. The method of claim 10 or 11, wherein:
(a) the engineered D_{H} gene segment as defined in claim 10(b) comprises
(i) a human D_{H}3-3 gene segment immediately adjacent to the 23-mer RSS, optionally wherein the 23-mer RSS is immediately adjacent to the 5'-end of the D_{H}3-3 gene segment,
(ii) a human D_{H}2-2 gene segment immediately adjacent to the 23-mer RSS, optionally wherein the 23-mer RSS is immediately adjacent to the 3'-end of the D_{H}2-2 gene segment,
(iii) a human D_{H}2-8 gene segment immediately adjacent to the 23-mer RSS, optionally wherein the 23-mer RSS is immediately adjacent to the 3'-end of the D_{H}2-8 gene segment,
(iv) a human D_{H}2-15 gene segment immediately adjacent to the 23-mer RSS, optionally wherein the 23-mer RSS is immediately adjacent to the 3'-end of the D_{H}2-15 gene segment, or
(v) any combination of (i)-(iv);
(b) engineering comprises replacing the most 3' unrearranged D_{H} gene segment of the D_{H} region as defined in claim 10(a) with the engineered D_{H} gene segment as defined in claim 10(b); and/or
(c) engineering comprises replacing at least one of the one or more unrearranged D_{H} gene segments that corresponds to the engineered D_{H} gene segment as defined in claim 10(b) with the engineered D_{H} gene segment as defined in claim 10(b).

13. An immunoglobulin heavy chain locus comprising the nucleotide molecule of any one of claims 1-8, the targeting vector of claim 9, or the immunoglobulin heavy chain variable region modified according to the method of any one of claims 10-12.

14. A rodent genome comprising the nucleotide molecule of any one of claims 1-8, the targeting vector of claim 9, the immunoglobulin heavy chain variable region modified according to the method of any one of claims 10-12, or the immunoglobulin heavy chain locus of claim 13, wherein the rodent genome is optionally a rodent germline genome.

15. The rodent genome of claim 14, wherein the rodent genome comprises an immunoglobulin heavy chain variable region that comprises:
(a) unrearranged V_{H} gene segments,
(b) the engineered D_{H} region, optionally wherein the engineered D_{H} region comprises only human D_{H} gene segments, and
(c) unrearranged J_{H} gene segments,
wherein (a)-(c) are in operable linkage such that, upon recombination, the immunoglobulin heavy chain variable region comprises a rearranged heavy chain variable region sequence encoding an immunoglobulin heavy chain variable domain, optionally wherein the rearranged heavy chain variable region sequence is formed after a V_{H}(D_{H}-D_{H})J_{H} recombination event,
optionally wherein:
(aa) the engineered D_{H} gene segment comprises a D_{H} segment immediately adjacent to a 3' 23-mer RSS;
(bb) the engineered D_{H} gene segments comprises a D_{H} gene segment immediately adjacent to a 5' 23-mer RSS;
(cc) the immunoglobulin heavy chain variable region is a human immunoglobulin heavy chain variable region comprising only human V_{H} gene segments, human D_{H} gene segments, and human J_{H} gene segments, optionally wherein the human immunoglobulin heavy chain variable region is operably linked to a C_{H} region, further optionally wherein the C_{H} region is an endogenous C_{H} region, optionally at an endogenous immunoglobulin heavy chain locus;
(dd) the unrearranged V_{H} gene segments comprise the full repertoire of functional unrearranged human V_{H} gene segments, optionally wherein the full repertoire of functional unrearranged human V_{H} gene segments is in germline configuration;
(ee) the unrearranged J_{H} gene segments comprise a human J_{H}6 gene segment; and/or
(ff) (i) the rodent genome lacks an endogenous Adam6 gene; or
(ii) the rodent genome further comprises one or more rodent Adam6 genes, optionally wherein:
the one or more rodent Adam6 genes comprises an endogenous rodent Adam6 gene;
the one or more rodent Adam6 genes are inserted between two human V_{H} gene segments, optionally wherein the one or more rodent Adam6 genes are inserted between an unrearranged human V_{H}1-2 gene segment and an unrearranged human V_{H}6-1 gene segment
the one or more one or more rodent Adam6 genes are inserted in the place of a human Adam6 pseudogene; and/or
the one or more one or more rodent Adam6 genes are inserted between an unrearranged human V_{H} gene segment and an unrearranged human D_{H} gene segment.

16. The rodent genome of claim 14 or 15, wherein:
(a) the rodent genome further comprises a terminal deoxynucleotidyl transferase gene;
(b) the rodent genome is **characterized in that** it is homozygous for the engineered D_{H} region or the rodent genome is **characterized in that** it is heterozygous for the engineered D_{H} region; and/or
(c) the rodent genome is a rat genome or a mouse genome.

17. A rodent cell comprising the rodent genome of any one of claims 14 to 16, optionally wherein the rodent cell is a rat cell or a mouse cell.

18. The rodent cell of claim 17, wherein the rodent cell is a rodent embryonic stem cell.

19. A rodent generated from the rodent embryonic stem cell of claim 18, wherein the rodent comprises the rodent genome in its germline.

20. The rodent of claim 19, wherein:
(a) the rodent further comprises a somatic genome that comprises a rearranged heavy chain V_{H}(D_{H}A-D_{H}B)J_{H} coding sequence,
optionally wherein the rearranged heavy chain V_{H}(D_{H}A-D_{H}B)J_{H} coding sequence is operably linked to an IgM constant region gene sequence and/or wherein D_{H}A or D_{H}B is derived from the engineered D_{H} gene segment or a portion thereof and at least 9 base pairs of D_{H}A or D_{H}B is identical to at least 9 base pairs of the engineered D_{H} gene segment; and/or
(b) at least 95% of all rearranged heavy chain VDJ coding sequences in the rodent have a CDR3 length of at least 10 amino acids, optionally wherein at least 70% of all rearranged heavy chain VDJ gene sequences in the rodent have a CDR3 length of at least 11 amino acids, optionally wherein at least 15% of all rearranged heavy chain VDJ gene sequences in the rodent have a CDR3 length of at least 14 amino acids, further optionally **characterized in that** the rodent exhibits at least a at least a 3-fold increased frequency of D_{H}-D_{H} recombination as compared to the frequency of D_{H}-D_{H} recombination in a reference rodent.

21. A method of making a rodent whose genome comprises an engineered D_{H} region, the method comprising:
(A) generating the rodent from the embryonic stem cell of claim 18, or
(B)
(a) modifying the genome of a rodent embryonic stem cell to comprise a DNA fragment comprising one or more engineered D_{H} gene segments, each comprising a D_{H} gene segment immediately adjacent to a 23-mer RSS, optionally wherein the DNA fragment comprises the nucleotide molecule of any one of claims 1-8, the targeting vector of claim 9, the immunoglobulin heavy chain variable region modified according to the method of any one of claims 10-12, or the immunoglobulin heavy chain of claim 13, and
(b) generating the rodent using the modified rodent embryonic stem cell of (B)(a).

22. The method of claim 21, wherein the rodent is a rat or a mouse.

23. A method of producing an antibody or obtaining a nucleic acid encoding same, the method comprising
immunizing a rodent with an antigen, which rodent comprises a germline genome comprising an engineered D_{H} region that comprises one or more engineered D_{H} gene segments that are each immediately adjacent to a 23-mer RSS, optionally wherein the rodent is the rodent according to any one of claims 19 to 20 or made according to claim 21, and
allowing the rodent to produce an immune response to the antigen including an antibody, or nucleic acid encoding same, that binds the antigen,
optionally wherein:
(a) the method is one further comprising recovering the antibody, or nucleic acid encoding same, from the rodent or a rodent cell, wherein the rodent cell is a B cell or a hybridoma;
(b) one or more engineered D_{H} gene segments that are each immediately adjacent to a 23-mer RSS comprises a D_{H} gene segment operably linked to a 5' 23-mer RSS;
(c) one or more engineered D_{H} gene segments that are each immediately adjacent to a 23-mer RSS comprises a D_{H} gene segment operably linked to a 3' 23-mer RSS; and/or
(d) the rodent is a rat or a mouse.

## Patentansprüche

1. Nukleotidmolekül, umfassend eine konstruierte Diversitätsregion der schweren Immunglobulinkette (D_{H}) (konstruierte D_{H}-Region"), umfassend:
(i) ein konstruiertes D_{H}-Gensegment, umfassend ein D_{H}-Gensegment unmittelbar benachbart zu einer 23-mer-RSS ("konstruiertes D_{H}-Gensegment") und
(ii) ein nicht neugeordnetes D_{H}-Gensegment, flankiert auf seiner 5'-Seite von einer ersten 12-mer-RSS und auf seiner 3'-Seite von einer zweiten 12-mer-RSS ("nicht neugeordnetes D_{H}-Gensegment"),
wobei (i) das konstruierte D_{H}-Gensegment und (ii) das nicht neugeordnete D_{H}-Gensegment operabel verknüpft sind, sodass (i) das konstruierte D_{H}-Gensegment und (ii) das nicht neugeordnete D_{H}-Gensegment in der Lage sind, sich in einem D_{H}-D_{H}-Rekombinationsereignis gemäß der 12/23-Regel zu verbinden,
wobei das Nukleotidmolekül zum Erhalten von Nagetieren geeignet ist, die die konstruierte D_{H}-Region haben, und
wobei optional die konstruierte D_{H}-Region nur menschliche D_{H}-Gensegmente umfasst.

2. Nukleotidmolekül nach Anspruch 1, wobei das Nukleotidmolekül ferner Folgendes umfasst:
(a) mindestens ein nicht neugeordnetes variables Gensegment der schweren Immunglobulinkette (V_{H}) ("nicht neugeordnetes V_{H}-Gensegment"), das stromaufwärts der konstruierten D_{H}-Region ist und mit dieser operabel verknüpft ist;
(b) mindestens ein nicht neugeordnetes verbindendes Gensegment der schweren Immunglobulinkette (J_{H}) ("nicht neugeordnetes J_{H}-Gensegment"), das stromabwärts der konstruierten D_{H}-Region ist und mit dieser operabel verknüpft ist; oder
(c) eine Kombination aus (a) und (b).

3. Nukleotidmolekül nach Anspruch 2, wobei:
(a) das mindestens eine nicht neugeordnete V_{H}-Gensegment ein nicht neugeordnetes, humanes V_{H}6-1-Gensegment umfasst, das mindestens eine nicht neugeordnete J_{H}-Gensegment ein nicht neugeordnetes, humanes J_{H}6-Gensegment umfasst, oder eine Kombination davon;
(b) das mindestens eine nicht neugeordnete J_{H}-Gensegment ein nicht neugeordnetes, humanes J_{H}4-Gensegment, ein nicht neugeordnetes, humanes J_{H}5-Gensegment und ein nicht neugeordnetes, humanes J_{H}6-Gensegment umfasst;
(c) das mindestens eine nicht neugeordnete J_{H}-Gensegment ein nicht neugeordnetes, humanes J_{H}1-Gensegment, ein nicht neugeordnetes, humanes J_{H}2-Gensegment, ein nicht neugeordnetes, humanes J_{H}3-Gensegment, ein nicht neugeordnetes, humanes J_{H}4-Gensegment, ein nicht neugeordnetes J_{H}5-Gensegment und ein nicht neugeordnetes, humanes J_{H}6-Gensegment umfasst, wobei optional das nicht neugeordnete, humane J_{H}1-Gensegment, das nicht neugeordnete, humane J_{H}2-Gensegment, das nicht neugeordnete, humane J_{H}3-Gensegment, das nicht neugeordnete, humane J_{H}4-Gensegment, das nicht neugeordnete, humane J_{H}5-Gensegment und das nicht neugeordnete, humane J_{H}6-Gensegment in Keimbahnkonfiguration sind; und/oder
(d) das mindestens eine nicht neugeordnete V_{H}-Gensegment das vollständige Repertoire an funktionellen, nicht neugeordneten, humanen V_{H}-Gensegmenten umfasst, die sich zwischen einem nicht neugeordneten, humanen V_{H}3-74-Gensegment und einem nicht neugeordneten, humanen V_{H}1-6-Gensegment ("vollständiges Repertoire an funktionellen, nicht neugeordneten, humanen V_{H}-Gensegmenten") erstrecken und diese beinhalten, wobei optional das vollständige Repertoire an funktionellen, nicht neugeordneten, humanen V_{H}-Gensegmenten in Keimbahnkonfiguration ist.

4. Nukleotidmolekül nach einem der vorhergehenden Ansprüche, wobei:
(a) das Nukleotidmolekül ferner ein oder mehrere funktionelle Nagetier-Adam6-Gene umfasst, wobei optional das eine oder die mehreren funktionelle Nagetier-Adam6-Gene zwischen einem humanen V_{H}1-2-Gensegment und einem humanen V_{H}6-1-Gensegment angeordnet sind und/oder ein humanes Adam6-Gen ersetzen;
(b) das konstruierte D_{H}-Gensegment die 23-mer-RSS unmittelbar benachbart zu dem 5'-Ende des D_{H}-Gensegment umfasst;
(c) das konstruierte D_{H}-Gensegment die 23-mer-RSS unmittelbar benachbart zu dem 3'-Ende des D_{H}-Gensegments umfasst;
(d) das konstruierte D_{H}-Gensegment Folgendes umfasst:
(i) ein humanes D_{H}3-3-Gensegment unmittelbar benachbart zu der 23-mer-RSS, wobei optional die 23-mer-RSS unmittelbar benachbart zu dem 5'-Ende des D_{H}3-3-Gensegments ist;
(ii) ein humanes D_{H}2-2-Gensegment unmittelbar benachbart zu der 23-mer RSS, wobei optional die 23-mer-RSS unmittelbar benachbart zu dem 3'-Ende des D_{H}2-2-Gensegments ist;
(iii) ein humanes D_{H}2-8-Gensegment unmittelbar benachbart zu der 23-mer RSS, wobei optional die 23-mer-RSS unmittelbar benachbart zu dem 3'-Ende des D_{H}2-8-Gensegments ist;
(iv) ein humanes D_{H}2-15-Gensegment unmittelbar benachbart zu der 23-mer RSS, wobei optional die 23-mer-RSS unmittelbar benachbart zu dem 3'-Ende des D_{H}2-15-Gensegments ist; oder
(v) jegliche Kombination aus (i)-(iv);
(e) das Nukleotidmolekül die Nukleotidsequenz umfasst, die als SEQ ID NO: 52 dargelegt ist oder die Nukleotidsequenz umfasst, die als SEQ ID NO: 61 dargelegt ist;
(f) das Nukleotidmolekül die Nukleotidsequenz umfasst, die als SEQ ID NO: 70 dargelegt ist;
(g) das Nukleotidmolekül die Nukleotidsequenz umfasst, die als SEQ ID NO: 71 dargelegt ist; und/oder
(h) das Nukleotidmolekül die Nukleotidsequenz umfasst, die als SEQ ID NO: 72 dargelegt ist.

5. Nukleotidmolekül nach einem der Ansprüche 1-4, umfassend in operabler Verknüpfung von 5' bis 3':
(a) mindestens ein nicht neugeordnetes, humanes V_{H}-Gensegment,
(b) die konstruierte D_{H}-Region, wobei das konstruierte D_{H}-Gensegment ein humanes D_{H}3-3-Gensegment umfasst, unmittelbar benachbart zu der 23-mer-RSS, wobei die 23-mer-RSS unmittelbar benachbart zu dem 5'-Ende des humanen D_{H}3-3-Gensegment ist, und
(c) mindestens ein nicht neugeordnetes, humanes J_{H}-Gensegment.

6. Nukleotidmolekül nach einem der Ansprüche 1-4, umfassend in operabler Verknüpfung von 5' bis 3':
(a) mindestens ein nicht neugeordnetes, humanes V_{H}-Gensegment,
(b) die konstruierte D_{H}-Region, wobei das konstruierte D_{H}-Gensegment ein humanes D_{H}2-Gensegment umfasst, unmittelbar benachbart zu der 23-mer-RSS, wobei die 23-mer-RSS unmittelbar benachbart zu dem 3'-Ende des humanen D_{H}2-Gensegment ist, wobei optional:
(i) das konstruierte D_{H}-Gensegment ein humanes D_{H}2-2-Gensegment umfasst, unmittelbar benachbart zu 23-mer-RSS;
(ii) das konstruierte D_{H}-Gensegment ein humanes D_{H}2-8-Gensegment umfasst, unmittelbar benachbart zu 23-mer-RSS;
(iii) das konstruierte D_{H}-Gensegment ein humanes D_{H}2-15-Gensegment umfasst, unmittelbar benachbart zu 23-mer-RSS; oder
(iv) eine beliebige Kombination davon, und
(c) mindestens ein nicht neugeordnetes, humanes J_{H}-Gensegment.

7. Nukleotidmolekül nach Anspruch 5 oder Anspruch 6, wobei:
(a) das mindestens eine nicht neugeordnete, humane V_{H}-Gensegment von 5' bis 3' ein nicht neugeordnetes, humanes V_{H}1-2-Gensegment und ein nicht neigeordnetes, humanes V_{H}6-1-Gensegment umfasst, wobei das Nukleotid ferner ein oder mehrere funktionelle Nagetier-Adam6-Gene zwischen dem nicht neugeordneten, humanen V_{H}1-2-Gensegment und dem nicht neugeordneten, humanen V_{H}6-1-Gensegment umfasst, und wobei das nicht neugeordnete, humane V_{H}1-2-Gensegment, das eine oder die mehreren funktionellen Nagetier-Adam6-Gene und das nicht neugeordnete, humane V_{H}6-1-Gensegment zusammenhängend sind;
(b) das mindestens eine nicht neugeordnete, humane V_{H}-Gensegment das vollständige Repertoire an funktionellen, nicht neugeordneten, humanen V_{H}-Gensegmenten umfasst, wobei optional das vollständige Repertoire an funktionellen, nicht neugeordneten, humanen V_{H}-Gensegmenten in Keimbahnkonfiguration ist; und/oder
(c) das mindestens eine nicht neugeordnete, humane J_{H}-Gensegment ein nicht neugeordnetes, humanes J_{H}6-Gensegment umfasst, wobei optional:
(i) das mindestens eine nicht neugeordnete, humane J_{H}-Gensegment ein nicht neugeordnetes, humanes J_{H}4-Gensegment, ein nicht neugeordnetes, humanes J_{H}5-Gensegment und das nicht neugeordnete, humane J_{H}6-Gensegment umfasst; und/oder
(ii) wobei das mindestens eine nicht neugeordnete, humane J_{H}-Gensegment ein nicht neugeordnetes, humanes J_{H}1-Gensegment, ein nicht neugeordnetes, humanes J_{H}2-Gensegment, ein nicht neugeordnetes, humanes J_{H}3-Gensegment, ein nicht neugeordnetes, humanes J_{H}4-Gensegment, ein nicht neugeordnetes, humanes J_{H}5-Gensegment und ein nicht neugeordnetes, humanes J_{H}6-Gensegment umfasst, wobei ferner optional das nicht neugeordnete, humane J_{H}1-Gensegment, das nicht neugeordnete, humane J_{H}2-Gensegment, das nicht neugeordnete, humane J_{H}3-Gensegment, das nicht neugeordnete, humane J_{H}4-Gensegment, das nicht neugeordnete, humane J_{H}5-Gensegment und das nicht neugeordnete, humane J_{H}6-Gensegment in Keimbahnkonfiguration sind.

8. Nukleotidmolekül nach einem der Ansprüche 1-7, ferner umfassend:
(a) an seinem 3'-Ende, eine konstante Region der schweren Immunglobulinkette (C_{H}) oder einen Abschnitt davon;
(b) an seinem 3'-Ende, eine Nagetier-C_{H}-Region oder einen Abschnitt davon;
(c) an seinem 3'-Ende, eine Nagetier-C_{H}-Region oder einen Abschnitt davon, umfassend eine intronische Nagetier-Verstärkerregion und ein Nagetier-IgM-Gen; und/oder
(d) eine Arzneimittelauswahlkassette, die sich stromaufwärts des konstruierten D_{H}-Gensegments befindet, wobei optional die Arzneimittelauswahlkassette von einer oder mehreren ortsspezifischen Rekombinationsstellen flankiert ist.

9. Zielvektor, umfassend ein Nukleotidmolekül nach einem der Ansprüche 1-8, umfassend 5'- und 3'-Homologiearme, die konfiguriert sind, um eine homologe Rekombination mit einer Sequenz der schweren Immunglobulinkette an einem endogenen Locus der schweren Immunglobulinkette eines Nagetiers zuzulassen, wobei optional die Sequenz der schweren Immunglobulinkette eine humane oder humanisierte variable Region der schweren Immunglobulinkette umfasst.

10. Verfahren des Modifizierens einer variablen Region der schweren Immunglobulinkette, um D_{H}-D_{H}-Rekombination zu konstruieren, wobei das Verfahren Folgendes umfasst:
(a) Erhalten einer Sequenz der variablen Kette der schweren Immunglobulinkette, umfassend eine D_{H}-Region, umfassend ein oder mehrere nicht neugeordnete D_{H}-Gensegmente, wovon jedes nicht neugeordnete D_{H}-Gensegment an seiner 5'-Seite von einer ersten 12-mer-RSS und an seiner 3'-Seite von einer zweiten 12-mer-RSS flankiert ist,
wobei optional das eine oder die mehrere nicht neugeordneten D_{H}-Gensegmente mindestens ein nicht neugeordnete, humanes D_{H}-Gensegment umfassen, wobei ferner optional das mindestens eine nicht neugeordnete, humane D_{H}-Gensegment alle funktionellen, humanen D_{H}-Gensegmente umfasst, die sich zwischen einem nicht neugeordneten, humanen D_{H}1-1-Gensegment und einem nicht neugeordneten, humanen D_{H}7-27-Gensegment erstrecken und diese beinhalten, wobei optional alle funktionellen, humanen D_{H}-Gensegmente in Keimbahnkonfiguration sind, und
(b) Konstruieren der D_{H}-Region, sodass diese ferner mindestens ein konstruiertes D_{H}-Gensegment umfasst, wobei das konstruierte D_{H}-Gensegment ein D_{H}-Gensegment unmittelbar benachbart zu einer 23-mer-RSS umfasst,
wobei, in der resultierenden konstruierten D_{H}-Region, das konstruierte D_{H}-Gensegment und mindestens eines aus dem einen oder den mehreren nicht neugeordneten D_{H}-Gensegmenten operabel verknüpft sind und in der Lage sind, um in einem D_{H}-D_{H}-Rekombinationsereignis gemäß der 12/23-Regel teilzunehmen,
wobei optional die resultierende konstruierte D_{H}-Region nur humane D_{H}-Gensegmente umfasst.

11. Verfahren nach Anspruch 10, wobei die D_{H}-Region aus Anspruch 10(a) zwei oder mehr nicht neugeordnete D_{H}-Gensegmente umfasst, wovon jedes auf seiner 5'-Seite von einer ersten 12-mer-RSS und auf seiner 3'-Seite von einer zweiten 12-mer-RSS flankiert ist, und
wobei das Konstruieren Folgendes umfasst
(a) Ersetzen von mindestens einem der zwei oder mehreren nicht neugeordneten D_{H}-Gensegmente mit dem konstruierten D_{H}-Gensegment, wie in Anspruch 10(b) definiert,
(b) Ersetzen der ersten 12-mer-RSS oder der zweiten 12-mer-RSS aus mindestens einem der zwei oder mehreren nicht neugeordneten D_{H}-Gensegmente mit einer 23-mer-RSS, oder
(c) eine Kombination aus (a) und (b),
wobei optional die Sequenz der variablen Kette der schweren Immunglobulinkette ferner eine J_{H}-Region umfasst, umfassend mindestens ein nicht neugeordnetes J_{H}-Gensegment,
wobei die J_{H}-Region mit der D_{H}-Region operabel verknüpft ist, und
wobei das Ersetzen von mindestens einem der zwei oder mehreren nicht neugeordneten D_{H}-Gensegmente mit dem konstruierten D_{H}-Gensegment nach Anspruch 10(b) das Deletieren des mindestens einen nicht neugeordneten J_{H}-Gensegments umfasst,
wobei ferner optional die J_{H}-Region ein vollständiges Repertoire an humanen Keimbahn-J_{H}-Gensegmenten umfasst, umfassend ein nicht neugeordnetes, humanes J_{H}1-Gensegment, ein nicht neugeordnetes, humanes J_{H}2-Gensegment, ein nicht neugeordnetes, humanes J_{H}3-Gensegment, ein nicht neugeordnetes, humanes J_{H}4-Gensegment, ein nicht neugeordnetes, humanes J_{H}5-Gensegment und ein nicht neugeordnetes, humanes J_{H}6-Gensegment, wobei optional das nicht neugeordnete, humane J_{H}1-Gensegment, das nicht neugeordnete, humane J_{H}2-Gensegment, das nicht neugeordnete, humane J_{H}3-Gensegment, das nicht neugeordnete, humane J_{H}4-Gensegment, das nicht neugeordnete, humane J_{H}5-Gensegment und das nicht neugeordnete, humane J_{H}6-Gensegment in Keimbahnkonfiguration sind, und
wobei das Deletieren von mindestens einem Keimbahn-J_{H}-Gensegment das Deletieren des nicht neugeordneten, humanen J_{H}1-Gensegments, des nicht neugeordneten, humanen JH2-Gensegments und des nicht neugeordneten, humanen J_{H}3-Gensegments, und optional ferner das Deletieren des nicht neugeordneten, humanen J_{H}4-Gensegments und des nicht neugeordneten, humanen JH5-Gensegments umfasst.

12. Verfahren nach Anspruch 10 oder 11, wobei:
(a) das konstruierte D_{H}-Gensegment nach Anspruch 10(b) Folgendes umfasst
(i) ein humanes D_{H}3-3-Gensegment, unmittelbar benachbart zu der 23-mer-RSS, wobei optional die 23-mer-RSS unmittelbar benachbart zu dem 5'-Ende des D_{H}3-3-Gensegments ist,
(ii) ein humanes D_{H}2-2-Gensegment, unmittelbar benachbart zu der 23-mer-RSS, wobei optional die 23-mer-RSS unmittelbar benachbart zu dem 3'-Ende des D_{H}2-2-Gensegments ist,
(iii) ein humanes D_{H}2-8-Gensegment, unmittelbar benachbart zu der 23-mer-RSS, wobei optional die 23-mer-RSS unmittelbar benachbart zu dem 3'-Ende des D_{H}2-8-Gensegments ist,
(iv) ein humanes D_{H}2-15-Gensegment, unmittelbar benachbart zu der 23-mer-RSS, wobei optional die 23-mer-RSS unmittelbar benachbart zu dem 3'-Ende des D_{H}2-15-Gensegments ist, oder
(v) eine beliebige Kombination aus (i)-(iv);
(b) das Konstruieren das Ersetzen des am meisten 3' nicht neugeordneten D_{H}-Gensegments der D_{H}-Region nach Anspruch 10(a) mit dem konstruierten D_{H}-Gensegment nach Anspruch 10(b) umfasst; und/oder
(c) das Konstruieren das Ersetzen von mindestens einem des einen oder der mehreren nicht neugeordneten D_{H}-Gensegmente umfasst, das dem konstruierten D_{H}-Gensegment nach Anspruch 10(b) entspricht, mit dem konstruierten D_{H}-Gensegment nach Anspruch 10(b) umfasst.

13. Locus der schweren Immunglobulinkette, umfassend das Nukleotidmolekül nach einem der Ansprüche 1-8, den Zielvektor nach Anspruch 9 oder die variable Region der schweren Immunglobulinkette, modifiziert gemäß dem Verfahren nach einem der Ansprüche 10-12.

14. Nagetiergenom, umfassend das Nukleotidmolekül nach einem der Ansprüche 1-8, den Zielvektor nach Anspruch 9, die variable Region der schweren Immunglobulinkette, modifiziert gemäß dem Verfahren nach einem der Ansprüche 10-12, oder den Locus der schweren Immunglobulinkette nach Anspruch 13, wobei das Nagetiergenom optional ein Nagetier-Keimbahngenom ist.

15. Nagetiergenom nach Anspruch 14, wobei das Nagetiergenom eine variable Region der schweren Immunglobulinkette umfasst, die Folgendes umfasst:
(a) nicht neugeordnete V_{H}-Gensegmente,
(b) die konstruierte D_{H}-Region, wobei optional die konstruierte D_{H}-Region nur humane D_{H}-Gensegmente umfasst, und
(c) nicht neugeordnete J_{H}-Gensegmente,
wobei (a)-(c) in operabler Verknüpfung sind, sodass, bei Rekombination, die variable Region der schweren Immunglobulinkette eine neu geordnete Sequenz der variablen Region der schweren Kette umfasst, die eine variable Domäne der schweren Immunglobulinkette codiert, wobei optional die neu geordnete Sequenz der variablen Region der schweren Kette nach einem V_{H}(D_{H}-D_{H})J_{H}-Rekombinationsereignis gebildet ist,
wobei optional:
(aa) das konstruierte D_{H}-Gensegment ein D_{H}-Gensegment unmittelbar benachbart zu einer 3' 23-mer-RSS umfasst;
(bb) das konstruierte D_{H}-Gensegment ein D_{H}-Gensegment unmittelbar benachbart zu einer 5' 23-mer-RSS umfasst;
(cc) due variable Region der schweren Immunglobulinkette eine humane variable Region der schweren Immunglobulinkette ist, umfassend nur humane V_{H}-Gensegmente, humane D_{H}-Gensegmente und humane J_{H}-Gensegmente, wobei optional die humane variable Region der schweren Immunglobulinkette mit einer C_{H}-Region operabel verknüpft ist, wobei ferner optional die C_{H}-Region eine endogene C_{H}-Region ist, optional an einem endogenen Locus der schweren Immunglobulinkette;
(dd) die nicht neugeordneten V_{H}-Gensegmente das vollständige Repertoire an funktionellen, nicht neugeordneten, humanen V_{H}-Gensegmenten umfassen, wobei optional das vollständige Repertoire an funktionellen, nicht neugeordneten, humanen V_{H}-Gensegmenten in Keimbahnkonfiguration ist;
(ee) die nicht neugeordneten J_{H}-Gensegmente ein humanes J_{H}6-Gensegment umfassen; und/oder
(ff) (i) dem Nagetiergenom ein endogenes Adam6-Gen fehlt; oder
(ii) das Nagetiergenom ferner ein oder mehrere Nagetier-Adam6-Gene umfasst, wobei optional:
das eine oder die mehreren Nagetier-Adam6-Gene ein endogenes Nagetier-Adam6-Gen umfassen;
das eine oder die mehreren Nagetier-Adam6-Gene zwischen zwei humanen V_{H}-Gensegmenten eingefügt sind, wobei optional das eine oder die mehreren Nagetier-Adam6-Gene zwischen einem nicht neugeordneten, humanen V_{H}1-2-Gensegment und einem nicht neugeordneten, humanen V_{H}6-1-Gensegment eingefügt sind
das eine oder die mehreren eine oder mehreren Nagetier-Adam6-Gene anstelle von einem humanen Adam6-Pseudogen eingefügt sind; und/oder
das eine oder die mehreren eine oder mehreren Nagetier-Adam6-Gene zwischen einem nicht neugeordneten, humanen V_{H}-Gensegment und einem nicht neugeordneten, humanen D_{H}-Gensegment eingefügt sind.

16. Nagetiergenom nach Anspruch 14 oder 15, wobei:
(a) das Nagetiergenom ferner ein Gen der terminalen Desoxyribonukleotidyltransferase umfasst;
(b) das Nagetiergenom **dadurch gekennzeichnet ist, dass** es für die konstruierte D_{H}-Region homozygot ist oder das Nagetier **dadurch gekennzeichnet ist, dass** es für die konstruierte D_{H}-Region heterozygot ist; und/oder
(c) das Nagetiergenom ein Rattengenom oder ein Mausgenom ist.

17. Nagetierzelle, umfassend das Nagetiergenom nach einem der Ansprüche 14 bis 16, wobei optional die Nagetierzelle eine Rattenzelle oder eine Mauszelle ist.

18. Nagetierzelle nach Anspruch 17, wobei die Nagetierzelle eine embryonale Nagetier-Stammzelle ist.

19. Nagetier, erzeugt aus der embryonalen Nagetier-Stammzelle nach Anspruch 18, wobei das Nagetier das Nagetiergenom in seiner Keimbahn umfasst.

20. Nagetier nach Anspruch 19, wobei:
(a) das Nagetier ferner ein somatisches Genom umfasst, das eine codierende Sequenz einer neugeordneten schweren Kette V_{H}(D_{H}A-D_{H}B)J_{H} umfasst,
wobei optional die codierende Sequenz einer neugeordneten schweren Kette V_{H}(D_{H}A-D_{H}B)J_{H} mit einer Gensequenz einer konstanten IgM-Region operabel verknüpft ist und/oder wobei D_{H}A oder D_{H}B von dem konstruierten D_{H}-Gensegment oder einem Abschnitt davon abgeleitet ist und mindestens 9 Basenpaare von D_{H}A oder D_{H}B identisch mit mindestens 9 Basenpaaren des konstruierten D_{H}-Gensegments ist; und/oder
(b) mindestens 95 % aller neugeordneten codierenden VDJ-Sequenzen der schweren Kette in dem Nagetier eine CDR3-Länge von mindestens 10 Aminosäuren haben, wobei optional mindestens 70 % aller neugeordneten VDJ-Gensequenzen der schweren Kette in dem Nagetier eine CDR3-Länge von mindestens 11 Aminosäuren haben, wobei optional mindestens 15 % aller neugeordneten VDJ-Gensequenzen der schweren Kette in dem Nagetier eine CDR3-Länge von mindestens 14 Aminosäuren haben, ferner optional **dadurch gekennzeichnet, dass** das Nagetier mindestens eine mindestens eine 3-fach erhöhte Frequenz von D_{H}-D_{H}-Rekombination im Vergleich zu der Frequenz von D_{H}-D_{H}-Rekombination in einem Bezugsnagetier aufweist.

21. Verfahren des Herstellens eines Nagetiers, dessen Genom eine konstruierte D_{H}-Region umfasst, wobei das Verfahren Folgendes umfasst:
(A) Erzeugen des Nagetiers aus der embryonischen Stammzelle nach Anspruch 18, oder
(B)
(a) Modifizieren des Genoms einer embryonischen Nagetier-Stammzelle, sodass es ein DNA-Fragment umfasst, umfassend ein oder mehrere konstruierte D_{H}-Gensegmente, die jeweils ein D_{H}-Gensegment unmittelbar benachbart zu einer 23-mer-RSS umfassen, wobei optional das DNA-Fragment das Nukleotidmolekül nach einem der Ansprüche 1-8, den Zielvektor nach Anspruch 9, die variable Region der schweren Immunglobulinkette, modifiziert gemäß dem Verfahren nach einem der Ansprüche 10-12, oder die schwere Immunglobulinkette nach Anspruch 13 umfasst, und
(b) Erzeugen des Nagetiers unter Verwendung der modifizierten embryonischen Nagetier-Stammzelle aus (B)(a).

22. Verfahren nach Anspruch 21, wobei das Nagetier eine Ratte oder eine Maus ist.

23. Verfahren des Erzeugens eines Antikörpers oder Erhalten einer Nukleinsäure, die diesen codiert, wobei das Verfahren Folgendes umfasst
Immunisieren eines Nagetiers mit einem Antigen, wobei das Nagetier ein Keimbahngenom umfasst, umfassend eine konstruierte D_{H}-Region, die ein oder mehrere konstruierte D_{H}-Gensegmente umfasst, die jeweils unmittelbar benachbart zu einer 23-mer RSS sind, wobei optional das Nagetier das Nagetier nach einem beliebigen der Ansprüche 19 bis 20 ist oder nach Anspruch 21 hergestellt ist, und
Zulassen, dass das Nagetier eine Immunantwort auf das Antigen erzeugt, einschließlich eines Antikörpers, oder Nukleinsäure, die diesen codiert, der an das Antigen bindet,
wobei optional:
(a) das Verfahren eines ist, das ferner das Gewinnen des Antikörpers oder der Nukleinsäure, die diesen codiert, aus dem Nagetier oder einer Nagetierzelle umfasst, wobei die Nagetierzelle eine B-Zelle oder ein Hybridom ist;
(b) ein oder mehrere konstruierte D_{H}-Gensegmente, die jeweils unmittelbar benachbart zu einer 23-mer-RSS sind, ein D_{H}-Gensegment umfassen, das an ein 5' 23-mer-RSS operabel verknüpft ist;
(c) ein oder mehrere konstruierte D_{H}-Gensegmente, die jeweils unmittelbar benachbart zu einer 23-mer-RSS sind, ein D_{H}-Gensegment umfassen, das an ein 3' 23-mer-RSS operabel verknüpft ist; und/oder
(d) das Nagetier eine Ratte oder eine Maus ist.

## Revendications

1. Molécule nucléotidique comprenant une région de diversité de chaîne lourde d'immunoglobuline (D_{H}) modifiée par ingénierie (« région D_{H} modifiée par ingénierie ») comprenant :
(i) un segment de gène D_{H} modifié par ingénierie comprenant un segment de gène D_{H} immédiatement adjacent à une RSS 23-mère (« segment de gène D_{H} modifié par ingénierie ») et
(ii) un segment de gène D_{H} non réarrangé flanqué sur son côté 5' par une première RSS 12-mère et sur son côté 3' par une seconde RSS 12-mère (« segment de gène D_{H} non réarrangé »),
dans laquelle (i) le segment de gène D_{H} modifié par ingénierie et (ii) le segment de gène D_{H} non réarrangé sont fonctionnellement liés de telle sorte que (i) le segment de gène D_{H} modifié par ingénierie et (ii) le segment de gène D_{H} non réarrangé soient capables de se joindre dans un événement de recombinaison D_{H}-D_{H} selon la règle 12/23,
dans laquelle la molécule nucléotidique est appropriée pour obtenir des rongeurs ayant la région D_{H} modifiée par ingénierie, et
optionnellement dans laquelle la région D_{H} modifiée par ingénierie comprend seulement des segments de gène D_{H} d'humain.

2. Molécule nucléotidique selon la revendication 1, dans laquelle la molécule nucléotidique comprend en outre :
(a) au moins un segment de gène variable (V_{H}) non réarrangé de chaîne lourde d'immunoglobuline (« segment de gène V_{H} non réarrangé ») qui est en amont de, et fonctionnellement lié à, la région D_{H} modifiée par ingénierie ;
(b) au moins un segment de gène de jonction (J_{H}) non réarrangé de chaîne lourde d'immunoglobuline (« segment de gène J_{H} non réarrangé ») qui est en aval de, et fonctionnellement lié à, la région D_{H} modifiée par ingénierie ; ou
(c) une association de (a) et de (b).

3. Molécule nucléotidique selon la revendication 2, dans laquelle :
(a) l'au moins un segment de gène V_{H} non réarrangé comprend un segment de gène V_{H}6-1 non réarrangé d'humain, l'au moins un segment de gène J_{H} non réarrangé comprend un segment de gène J_{H}6 non réarrangé d'humain, ou une combinaison de ceux-ci ;
(b) l'au moins un segment de gène J_{H} non réarrangé comprend un segment de gène J_{H}4 non réarrangé d'humain, un segment de gène J_{H}5 non réarrangé d'humain, et un segment de gène J_{H}6 non réarrangé d'humain ;
(c) l'au moins un segment de gène J_{H} non réarrangé comprend un segment de gène J_{H}1 non réarrangé d'humain, un segment de gène J_{H}2 non réarrangé d'humain, un segment de gène J_{H}3 non réarrangé d'humain, un segment de gène J_{H}4 non réarrangé d'humain, un segment de gène J_{H}5 non réarrangé d'humain, et un segment de gène J_{H}6 non réarrangé d'humain, optionnellement dans laquelle le segment de gène J_{H}1 non réarrangé d'humain, le segment de gène J_{H}2 non réarrangé d'humain, le segment de gène J_{H}3 non réarrangé d'humain, le segment de gène J_{H}4 non réarrangé d'humain, le segment de gène J_{H}5 non réarrangé d'humain, et le segment de gène J_{H}6 non réarrangé d'humain sont en configuration de lignée germinale ; et/ou
(d) l'au moins un segment de gène V_{H} non réarrangé comprend le répertoire complet de segments de gène V_{H} non réarrangés fonctionnels d'humain s'étendant entre et incluant un segment de gène V_{H}3-74 non réarrangé d'humain et un segment de gène V_{H}1-6 non réarrangé d'humain (« répertoire complet de segments de gène V_{H} non réarrangés fonctionnels d'humain »), optionnellement dans laquelle le répertoire complet de segments de gène V_{H} non réarrangés fonctionnels d'humain est en configuration de lignée germinale.

4. Molécule nucléotidique selon l'une quelconque des revendications précédentes, dans laquelle :
(a) la molécule nucléotidique comprend en outre un ou plusieurs gènes Adam6 fonctionnels de rongeur, optionnellement dans laquelle les un ou plusieurs gènes Adam6 fonctionnels de rongeur sont situés entre un segment de gène V_{H}1-2 d'humain et un segment de gène V_{H}6-1 d'humain et/ou remplace un gène Adam6 d'humain ;
(b) le segment de gène D_{H} modifié par ingénierie comprend la RSS 23-mère immédiatement adjacente à l'extrémité 5' du segment de gène D_{H} ;
(c) le segment de gène D_{H} modifié par ingénierie comprend la RSS 23-mère immédiatement adjacente à l'extrémité 3' du segment de gène D_{H} ;
(d) le segment de gène D_{H} modifié par ingénierie comprend :
(i) un segment de gène D_{H}3-3 d'humain immédiatement adjacent à la RSS 23-mère, optionnellement dans laquelle la RSS 23-mère est immédiatement adjacente à l'extrémité 5' du segment de gène D_{H}3-3 ;
(ii) un segment de gène D_{H}2-2 d'humain immédiatement adjacent à la RSS 23-mère, optionnellement dans laquelle la RSS 23-mère est immédiatement adjacente à l'extrémité 3' du segment de gène D_{H}2-2 ;
(iii) un segment de gène D_{H}2-8 d'humain immédiatement adjacent à la RSS 23-mère, optionnellement dans laquelle la RSS 23-mère est immédiatement adjacente à l'extrémité 3' du segment de gène D_{H}2-8 ;
(iv) un segment de gène D_{H}2-15 d'humain immédiatement adjacent à la RSS 23-mère, optionnellement dans laquelle la RSS 23-mère est immédiatement adjacente à l'extrémité 3' du segment de gène D_{H}2-15 ; ou
(v) une quelconque association de (i)-(iv) ;
(e) la molécule nucléotidique comprend la séquence nucléotidique présentée par SEQ ID n° : 52 ou comprend la séquence nucléotidique présentée par SEQ ID n° : 61 ;
(f) la molécule nucléotidique comprend la séquence nucléotidique présentée par SEQ ID n° : 70 ;
(g) la molécule nucléotidique comprend la séquence nucléotidique présentée par SEQ ID n° : 71 ; et/ou
(h) la molécule nucléotidique comprend la séquence nucléotidique présentée par SEQ ID n° : 72.

5. Molécule nucléotidique selon l'une quelconque des revendications 1 à 4, comprenant en liaison fonctionnelle de 5' à 3' :
(a) au moins un segment de gène V_{H} non réarrangé d'humain,
(b) la région D_{H} modifiée par ingénierie, dans laquelle le segment de gène D_{H} modifié par ingénierie comprend un segment de gène D_{H}3-3 d'humain immédiatement adjacent à la RSS 23-mère, dans laquelle la RSS 23-mère est immédiatement adjacente à l'extrémité 5' du segment de gène D_{H}3-3 d'humain, et
(c) au moins un segment de gène J_{H} non réarrangé d'humain.

6. Molécule nucléotidique selon l'une quelconque des revendications 1 à 4, comprenant en liaison fonctionnelle de 5' à 3' :
(a) au moins un segment de gène V_{H} non réarrangé d'humain,
(b) la région D_{H} modifiée par ingénierie, dans laquelle le segment de gène D_{H} modifié par ingénierie comprend un segment de gène D_{H}2 d'humain immédiatement adjacent à la RSS 23-mère, dans laquelle la RSS 23-mère est immédiatement adjacente à l'extrémité 3' du segment de gène D_{H}2 d'humain, optionnellement dans laquelle :
(i) le segment de gène D_{H} modifié par ingénierie comprend un segment de gène D_{H}2-2 d'humain immédiatement adjacent à la RSS 23-mère ;
(ii) le segment de gène D_{H} modifié par ingénierie comprend un segment de gène D_{H}2-8 d'humain immédiatement adjacent à la RSS 23-mère ;
(iii) le segment de gène D_{H} modifié par ingénierie comprend un segment de gène D_{H}2-15 d'humain immédiatement adjacent à la RSS 23-mère ; ou
(iv) une quelconque association de ceux-ci, et
(c) au moins un segment de gène J_{H} non réarrangé d'humain.

7. Molécule nucléotidique selon la revendication 5 ou la revendication 6, dans laquelle :
(a) l'au moins un segment de gène V_{H} non réarrangé d'humain comprend, de 5' à 3', un segment de gène V_{H}1-2 non réarrangé d'humain et un segment de gène V_{H}6-1 non réarrangé d'humain, dans laquelle le nucléotide comprend en outre un ou plusieurs gènes Adam6 fonctionnels de rongeur entre le segment de gène V_{H}1-2 non réarrangé d'humain et le segment de gène V_{H}6-1 non réarrangé d'humain, et dans laquelle le segment de gène V_{H}1-2 non réarrangé d'humain, les un ou plusieurs gènes Adam6 fonctionnels de rongeur, et le segment de gène V_{H}6-1 non réarrangé d'humain sont contigus ;
(b) l'au moins un segment de gène V_{H} non réarrangé d'humain comprend le répertoire complet de segments de gène V_{H} non réarrangés fonctionnels d'humain, optionnellement dans laquelle le répertoire complet de segments de gène V_{H} non réarrangés fonctionnels d'humain est en configuration de lignée germinale ; et/ou
(c) l'au moins un segment de gène J_{H} non réarrangé d'humain comprend un segment de gène J_{H}6 non réarrangé d'humain, optionnellement dans laquelle :
(i) l'au moins un segment de gène J_{H} non réarrangé d'humain comprend un segment de gène J_{H}4 non réarrangé d'humain, un segment de gène J_{H}5 non réarrangé d'humain, et le segment de gène J_{H}6 non réarrangé d'humain ; et/ou
(ii) dans laquelle l'au moins un segment de gène J_{H} non réarrangé d'humain comprend un segment de gène J_{H}1 non réarrangé d'humain, un segment de gène J_{H}2 non réarrangé d'humain, un segment de gène J_{H}3 non réarrangé d'humain, un segment de gène J_{H}4 non réarrangé d'humain, un segment de gène J_{H}5 non réarrangé d'humain, et un segment de gène J_{H}6 non réarrangé d'humain, en outre optionnellement dans laquelle le segment de gène J_{H}1 non réarrangé d'humain, le segment de gène J_{H}2 non réarrangé, le segment de gène J_{H}3 non réarrangé, le segment de gène J_{H}4 non réarrangé, le segment de gène J_{H}5 non réarrangé, et le segment de gène J_{H}6 non réarrangé sont en configuration de lignée germinale.

8. Molécule nucléotidique selon l'une quelconque des revendications 1 à 7, comprenant en outre :
(a) à son extrémité 3', une région constante (C_{H}) d'immunoglobuline de chaîne lourde ou une partie de celle-ci ;
(b) à son extrémité 3', une région C_{H} de rongeur ou une partie de celle-ci ;
(c) à son extrémité 3', une région C_{H} de rongeur ou une partie de celle-ci comprenant une région d'activateur intronique de rongeur et un gène IgM de rongeur ; et/ou
(d) une cassette de sélection de médicament située en amont du segment de gène D_{H} modifié par ingénierie, optionnellement dans laquelle la cassette de sélection de médicament est flanquée par un ou plusieurs sites de recombinaison spécifique de site.

9. Vecteur de ciblage, comprenant une molécule nucléotidique de l'une quelconque des revendications 1 à 8, comprenant des bras d'homologie 5' et 3' configurés pour permettre une recombinaison homologue avec une séquence de chaîne lourde d'immunoglobuline à un locus de chaîne lourde d'immunoglobine endogène de rongeur, optionnellement dans lequel la séquence de chaîne lourde d'immunoglobuline comprend une région variable de chaîne lourde d'immunoglobuline d'humain ou humanisée.

10. Procédé de modification d'une région variable de chaîne lourde d'immunoglobuline pour créer par ingénierie une recombinaison D_{H}-D_{H}, le procédé comprenant :
(a) l'obtention d'une séquence de chaîne variable de chaîne lourde d'immunoglobuline comprenant une région D_{H} comprenant un ou plusieurs segments de gène D_{H} non réarrangés, dont chaque segment de gène D_{H} non réarrangé est flanqué sur son côté 5' par une première RSS 12-mère et sur son côté 3' par une seconde RSS 12-mère,
optionnellement dans lequel les un ou plusieurs segments de gène D_{H} non réarrangés comprennent au moins un segment de gène D_{H} non réarrangé d'humain, en outre optionnellement dans lequel l'au moins un segment de gène D_{H} non réarrangé d'humain comprend tous les segments de gène D_{H} fonctionnels d'humain s'étendant entre, et incluant, un segment de gène D_{H}1-1 non réarrangé d'humain et un segment de gène D_{H}7-27 non réarrangé d'humain, optionnellement dans lequel tous les segments de gène D_{H} fonctionnels d'humain sont en configuration de lignée germinale, et
(b) la modification par ingénierie de la région D_{H} pour comprendre en outre au moins un segment de gène D_{H} modifié par ingénierie, dans lequel le segment de gène D_{H} modifié par ingénierie comprend un segment de gène D_{H} immédiatement adjacent à une RSS 23-mère,
dans lequel, dans la région D_{H} modifiée par ingénierie résultante, le segment de gène D_{H} modifié par ingénierie et au moins un des un ou plusieurs segments de gène D_{H} non réarrangés sont fonctionnellement liés et capables de se joindre dans un événement de recombinaison D_{H}-D_{H} selon la règle 12/23,
optionnellement dans lequel la région D_{H} modifiée par ingénierie résultante comprend seulement des segments de gène D_{H} d'humain.

11. Procédé selon la revendication 10, dans lequel la région D_{H} selon la revendication 10(a) comprend deux, ou plus, segments de gène D_{H} non réarrangés, dont chacun est flanqué sur son côté 5' par une première RSS 12-mère et sur son côté 3' par une seconde RSS 12-mère, et
dans lequel la modification par ingénierie comprend
(a) le remplacement d'au moins un des deux, ou plus, segments de gène D_{H} non réarrangés avec le segment de gène D_{H} modifié par ingénierie tel que défini dans la revendication 10(b),
(b) le remplacement de la première RSS 12-mère ou de la seconde RSS 12-mère d'au moins un des deux, ou plus, segments de gène D_{H} non réarrangés avec une RSS 23-mère, ou
(c) une association de (a) et de (b),
optionnellement dans lequel la séquence de chaîne variable de chaîne lourde d'immunoglobuline comprend en outre une région J_{H} comprenant au moins un segment de gène J_{H} non réarrangé,
dans lequel la région J_{H} est fonctionnellement liée à la région D_{H}, et
dans lequel le remplacement d'au moins un des deux, ou plus, segments de gène D_{H} non réarrangés avec le segment de gène D_{H} modifié par ingénierie tel que défini dans la revendication 10(b) comprend la délétion de l'au moins un segment de gène J_{H} non réarrangé,
en outre optionnellement dans lequel la région J_{H} comprend un répertoire complet de segments de gène J_{H} de lignée germinale d'humain comprenant un segment de gène J_{H}1 non réarrangé d'humain, un segment de gène J_{H}2 non réarrangé d'humain, un segment de gène J_{H}3 non réarrangé d'humain, un segment de gène J_{H}4 non réarrangé d'humain, un segment de gène J_{H}5 non réarrangé d'humain, et un segment de gène J_{H}6 non réarrangé d'humain, optionnellement dans lequel le segment de gène J_{H}1 non réarrangé d'humain, le segment de gène J_{H}2 non réarrangé d'humain, le segment de gène J_{H}3 non réarrangé d'humain, le segment de gène J_{H}4 non réarrangé d'humain, le segment de gène J_{H}5 non réarrangé d'humain, et le segment de gène J_{H}6 non réarrangé d'humain sont en configuration de lignée germinale, et
dans lequel la délétion d'au moins un segment de gène J_{H} de lignée germinale comprend la délétion du segment de gène J_{H}1 non réarrangé d'humain, du segment de gène J_{H}2 non réarrangé d'humain, et du segment de gène J_{H}3 non réarrangé d'humain, et optionnellement en outre la délétion du segment de gène J_{H}4 non réarrangé d'humain et du segment de gène J_{H}5 non réarrangé d'humain.

12. Procédé selon la revendication 10 ou 11, dans lequel :
(a) le segment de gène D_{H} modifié par ingénierie tel que défini dans la revendication 10(b) comprend
(i) un segment de gène D_{H}3-3 d'humain immédiatement adjacent à la RSS 23-mère, optionnellement dans lequel la RSS 23-mère est immédiatement adjacente à l'extrémité 5' du segment de gène D_{H}3-3,
(ii) un segment de gène D_{H}2-2 d'humain immédiatement adjacent à la RSS 23-mère, optionnellement dans lequel la RSS 23-mère est immédiatement adjacente à l'extrémité 3' du segment de gène D_{H}2-2,
(iii) un segment de gène D_{H}2-8 d'humain immédiatement adjacent à la RSS 23-mère, optionnellement dans lequel la RSS 23-mère est immédiatement adjacente à l'extrémité 3' du segment de gène D_{H}2-8,
(iv) un segment de gène D_{H}2-15 d'humain immédiatement adjacent à la RSS 23-mère, optionnellement dans lequel la RSS 23-mère est immédiatement adjacente à l'extrémité 3' du segment de gène D_{H}2-15, ou
(v) une quelconque association de (i)-(iv) ;
(b) la modification par ingénierie comprend le remplacement du segment de gène D_{H} non réarrangé le plus 3' de la région D_{H} telle que définie dans la revendication 10(a) avec le segment de gène D_{H} modifié par ingénierie tel que défini dans la revendication 10(b) ; et/ou
(c) la modification par ingénierie comprend le remplacement d'au moins un des un ou plusieurs segments de gène D_{H} non réarrangés qui correspondent au segment de gène D_{H} modifié par ingénierie tel que défini dans la revendication 10(b) avec le segment de gène D_{H} modifié par ingénierie tel que défini dans la revendication 10(b).

13. Locus de chaîne lourde d'immunoglobine comprenant la molécule nucléotidique de l'une quelconque des revendications 1 à 8, le vecteur de ciblage de la revendication 9, ou la région variable de chaîne lourde d'immunoglobuline modifiée selon le procédé de l'une quelconque des revendications 10 à 12.

14. Génome de rongeur comprenant la molécule nucléotidique de l'une quelconque des revendications 1 à 8, le vecteur de ciblage de la revendication 9, la région variable de chaîne lourde d'immunoglobuline modifiée selon le procédé de l'une quelconque des revendications 10 à 12, ou le locus de chaîne lourde d'immunoglobine de la revendication 13, dans lequel le génome de rongeur est optionnellement un génome de lignée germinale de rongeur.

15. Génome de rongeur selon la revendication 14, dans lequel le génome de rongeur comprend une région variable de chaîne lourde d'immunoglobuline qui comprend :
(a) des segments de gène V_{H} non réarrangés,
(b) la région D_{H} modifiée par ingénierie, optionnellement dans lequel la région D_{H} modifiée par ingénierie comprend seulement des segments de gène D_{H} d'humain, et
(c) des segments de gène J_{H} non réarrangés,
dans lequel (a)-(c) sont en liaison fonctionnelle de telle sorte que, lors de la recombinaison, la région variable de chaîne lourde d'immunoglobuline comprenne une séquence de région variable de chaîne lourde réarrangée encodant un domaine variable de chaîne lourde d'immunoglobuline, optionnellement dans lequel la séquence de région variable de chaîne lourde réarrangée est formée après un événement de recombinaison V_{H}(D_{H}-D_{H})J_{H},
optionnellement dans lequel :
(aa) le segment de gène D_{H} modifié par ingénierie comprend un segment D_{H} immédiatement adjacent à une RSS 23-mère de 3' ;
(bb) le segment de gène D_{H} modifié par ingénierie comprend un segment de gène D_{H} immédiatement adjacent à une RSS 23-mère de 5' ;
(cc) la région variable de chaîne lourde d'immunoglobuline est une région variable de chaîne lourde d'immunoglobuline d'humain comprenant seulement des segments de gène V_{H} d'humain, des segments de gène D_{H} d'humain, et des segments de gène J_{H} d'humain, optionnellement dans lequel la région variable de chaîne lourde d'immunoglobuline d'humain est fonctionnellement liée à une région C_{H}, en outre optionnellement dans lequel la région C_{H} est une région C_{H} endogène, optionnellement à un locus de chaîne lourde d'immunoglobine endogène ;
(dd) les segments de gène V_{H} non réarrangés comprennent le répertoire complet de segments de gène V_{H} non réarrangés fonctionnels d'humain, optionnellement dans lequel le répertoire complet de segments de gène V_{H} non réarrangés fonctionnels d'humain est en configuration de lignée germinale ;
(ee) les segments de gène J_{H} non réarrangés comprennent un segment de gène J_{H}6 d'humain ; et/ou
(ff) (i) le génome de rongeur n'a pas de gène Adam6 endogène ; ou
(ii) le génome de rongeur comprend en outre un ou plusieurs gènes Adam6 de rongeur, optionnellement dans lequel :
les un ou plusieurs gènes Adam6 de rongeur comprend un gène Adam6 de rongeur endogène ;
les un ou plusieurs gènes Adam6 de rongeur sont insérés entre deux segments de gène V_{H} d'humain, optionnellement dans lequel les un ou plusieurs gènes Adam6 de rongeur sont insérés entre un segment de gène V_{H}1-2 non réarrangé d'humain et un segment de gène V_{H}6-1 non réarrangé d'humain,
les un ou plusieurs un ou plusieurs gènes Adam6 de rongeur sont insérés à la place d'un pseudogène Adam6 d'humain ; et/ou
les un ou plusieurs un ou plusieurs gènes Adam6 de rongeur sont insérés entre un segment de gène V_{H} non réarrangé d'humain et un segment de gène D_{H} non réarrangé d'humain.

16. Génome de rongeur selon la revendication 14 ou 15, dans lequel :
(a) le génome de rongeur comprend en outre un gène de désoxynucléotidyl-transférase terminale ;
(b) le génome de rongeur est **caractérisé en ce qu'**il est homozygote pour la région D_{H} modifiée par ingénierie ou le génome de rongeur est **caractérisé en ce qu'**il est hétérozygote pour la région D_{H} modifiée par ingénierie ; et/ou
(c) le génome de rongeur est un génome de rat ou un génome de souris.

17. Cellule de rongeur comprenant le génome de rongeur selon l'une quelconque des revendications 14 à 16, optionnellement dans laquelle la cellule de rongeur est une cellule de rat ou une cellule de souris.

18. Cellule de rongeur selon la revendication 17, dans laquelle la cellule de rongeur est une cellule souche embryonnaire de rongeur.

19. Rongeur généré à partir de la cellule souche embryonnaire de rongeur de la revendication 18, dans lequel le rongeur comprend le génome de rongeur dans sa lignée germinale.

20. Rongeur selon la revendication 19, dans lequel :
(a) le rongeur comprend en outre un génome somatique qui comprend une séquence de codage V_{H}(D_{H}A-D_{H}B)J_{H} de chaîne lourde réarrangée,
optionnellement dans lequel la séquence de codage V_{H}(D_{H}A-D_{H}B)J_{H} de chaîne lourde réarrangée est fonctionnellement liée à une séquence de gène de région constante IgM et/ou dans lequel D_{H}A ou D_{H}B est dérivé du segment de gène D_{H} modifié par ingénierie ou d'une partie de celui-ci et au moins 9 paires de base de D_{H}A ou de D_{H}B sont identiques à au moins 9 paires de base du segment de gène D_{H} modifié par ingénierie ; et/ou
(b) au moins 95 % de toutes les séquences de codage VDJ de chaîne lourde réarrangées dans le rongeur ont une longueur CDR3 d'au moins 10 acides aminés, optionnellement dans lequel au moins 70 % de toutes les séquences de gène VDJ de chaîne lourde réarrangées dans le rongeur ont une longueur CDR3 d'au moins 11 acides aminés, optionnellement dans lequel au moins 15 % de toutes les séquences de gène VDJ de chaîne lourde réarrangées dans le rongeur ont une longueur CDR3 d'au moins 14 acides aminés, en outre optionnellement **caractérisé en ce que** le rongeur présente au moins une au moins une fréquence multipliée par 3 de recombinaison D_{H}-D_{H} par rapport à la fréquence de recombinaison D_{H}-D_{H} dans un rongeur de référence.

21. Procédé de création d'un rongeur dont le génome comprend une région D_{H} modifiée par ingénierie, le procédé comprenant :
(A) la génération du rongeur à partir de la cellule souche embryonnaire de la revendication 18, ou
(B)
(a) la modification du génome d'une cellule souche embryonnaire de rongeur pour comprendre un fragment d'ADN comprenant un ou plusieurs segments de gène D_{H} modifiés par ingénierie, chacun comprenant un segment de gène D_{H} immédiatement adjacent à une RSS 23-mère, optionnellement dans lequel le fragment d'ADN comprend la molécule nucléotidique de l'une quelconque des revendications 1 à 8, le vecteur de ciblage de la revendication 9, la région variable de chaîne lourde d'immunoglobuline modifiée selon le procédé de l'une quelconque des revendications 10 à 12, ou la chaîne lourde d'immunoglobuline de la revendication 13, et
(b) la génération du rongeur en utilisant la cellule souche embryonnaire modifiée de rongeur de (B)(a).

22. Procédé selon la revendication 21, dans lequel le rongeur est un rat ou une souris.

23. Procédé de production d'un anticorps ou d'obtention d'un acide nucléique encodant ce dernier, le procédé comprenant
l'immunisation d'un rongeur avec un antigène, lequel rongeur comprend un génome de lignée germinale comprenant une région D_{H} modifiée par ingénierie qui comprend un ou plusieurs segments de gène D_{H} modifiés par ingénierie qui sont chacun immédiatement adjacents à une RSS 23-mère, optionnellement dans lequel le rongeur est le rongeur de l'une quelconque des revendications 19 et 20 ou créé selon la revendication 21, et
la permission au rongeur de produire une réponse immunitaire à l'antigène incluant un anticorps, ou acide nucléique encodant de dernier, qui se lie à l'antigène,
optionnellement dans lequel :
(a) le procédé est un comprenant en outre la récupération de l'anticorps, ou de l'acide nucléique encodant ce dernier, à partir du rongeur ou d'une cellule de rongeur, dans lequel la cellule de rongeur est une cellule B ou un hybridome ;
(b) un ou plusieurs segments de gène D_{H} modifiés par ingénierie qui sont chacun immédiatement adjacents à une RSS 23-mère comprennent un segment de gène D_{H} fonctionnellement lié à une RSS 23-mère de 5' ;
(c) un ou plusieurs segments de gène D_{H} modifiés par ingénierie qui sont chacun immédiatement adjacents à une RSS 23-mère comprennent un segment de gène D_{H} fonctionnellement lié à une RSS 23-mère de 3' ; et/ou
(d) le rongeur est un rat ou une souris.
